# EUROPEAN PATENT APPLICATION

(11) **EP 1 847 535 A1**
(43) Date of publication of application: **24.10.2007**
(21) Application number: 06712870.2
(22) Date of filing: 02.02.2006
(51) Int. Cl.: C07D 401/04, A61K 31/454, A61K 31/4545, A61K 31/496, A61K 31/5377, A61P 13/02, C07D 413/14

(54) **1-(PIPERIDIN-4-YL)-1H-INDOLE DERIVATIVE**

(30) Priority: 04.02.2005 JP 2005028413
(71) Applicant: Eisai R&D Management Co., Ltd., Tokyo 112-8088 (JP)
(72) Inventor: SUZUKI, Yuichi, c/o Tsukuba Research Lab., Tsukuba-shi, Ibaraki 300-2635 (JP); ITO, Koichi, c/o Tsukuba Research Lab., Tsukuba-shi, Ibaraki 300-2635 (JP); SASAKI, Atsushi, c/o Tsukuba Research Lab., Tsukuba-shi, Ibaraki 300-2635 (JP); UENO, Koshi, c/o Tsukuba Research Lab., Tsukuba-shi, Ibaraki 300-2635 (JP); SHINMYO, Daisuke, c/o Tsukuba Research Lab., Tsukuba-shi, Ibaraki, 300-2635 (JP); SAKAI, Miyuki, c/o Tsukuba Research Lab., Tsukuba-shi, Ibaraki, 300-2635 (JP); ISHIHARA, Hiroki, c/o Tsukuba Research Lab., Tsukuba-shi, Ibaraki 300-2635 (JP); KUBOTA, Atsuhiko, c/o Tsukuba Research Lab., Tsukuba-shi, Ibaraki, 300-2635 (JP)
(74) Representative: HOFFMANN EITLE
(86) International application number: PCT/JP2006/301727
(87) International publication number: WO 2006/082872

(57) **Abstract**

The present invention provides a compound represented by the formula (1) or a pharmacologically acceptable salt thereof, or a hydrate thereof (provided that a compound in which all of R4a, R4b, and R4c are hydrogen atoms is excluded.): 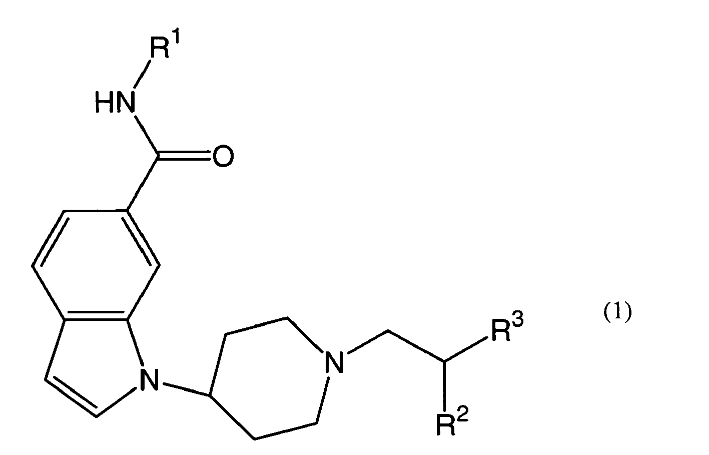 [wherein R1 represents a hydrogen atom, R2 represents a hydrogen atom, R3 represents the formula: 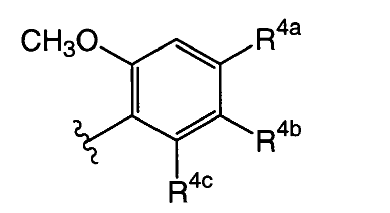 wherein R4a, R4b, and R4c are the same as or different from each other and each represents a hydrogen atom, a C₁₋₆ alkyl group or a C₁₋₆ alkoxy group, etc.]

## Description

### TECHNICAL FIELD

This invention relates to 1-(piperidin-4-yl)-1X-indole derivatives or pharmacologically acceptable salts thereof, or hydrates thereof, and a pharmaceutical composition containing the foregoing as an active ingredient, which have the beneficial effect for treatment or prevention to the dysuria.

### BACKGROUND ART

The dysuria poses a big problem with social aging in the field of care or a clinical medical treatment. Micturition is a kind of mechanisms (urine storage, voiding) in which the urine produced by filtration, re-absorption and secretion in a nephron is excreted through the ureter, the urinary bladder, and the urethra. It is known that a mechanism of micturition is reflection through parasympathetic nerve (pelvic nerve etc.), while a storage mechanism is mainly receiving regulation by the smooth muscle of a bladder neck part and the sphincter part urethra under sympathetic-nerve control. When a constant amount of urine is accumulated in the urinary bladder, an urge to urinate occurs, micturition is prompted, and control of the lower-order sacral-spinal-cord center which is a reflex arc is released, normal micturition arises by contracting a urinary-bladder wall smooth muscle (detrusor muscle), and causing relaxation of an external urethra sphincter muscle by reflection simultaneously. And urinary disturbance is a generic term for the state which generated an abnormality in these series of mechanisms of micturition, and is a severe uropathy which presents with abnormality of voiding difficulty, urinary incontinence and a urination state / urinary stream / uresiesthesia such as urinary volume / frequency of urination / enuresis.

The various subjective symptoms induced by the dysuria is called the lower urinary-tract symptoms, and are divided roughly into the urine storage symptoms including a frequent urination, a feeling of urgency of urination, urinary incontinence, etc., and the voiding symptoms containing a voiding difficulty, the anuresis, etc. It is reported that the 5-HT_{1A} receptor which is one of the serotonin (5-hydroxytryptamine=5-HT) receptors is connected with the lower urinary-tract symptoms in recent years. (For example, refer to Non-patent literature 1 : J. Pharmacol. Exp. Ther., 262: 1, 181-189 (1992), Patent document 1 : WO96/05817, Patent document 2 : WO97/31637, and Patent document 3 : USP No. 5990114). Herein, serotonin is a bioactive amine known as a neurotransmitter and the involvement of this amine to various functions, such as a smooth muscle relaxant effect, a platelet-aggregation effect, a gastroenteric functional adjustment effect, the body-temperature adjustment in a central nerve system, appetite, sleep, a pain sensation, a sexual act, insecurity, a depression, cognition, a memory, etc., is known.

As with the report concerning the role of a 5-HT_{1A} receptor in the above-mentioned lower urinary-tract symptoms, it is disclosed in Non-patent literature 1 that the 5-HT_{1A} receptor plays a role in a micturition reflex, moreover, in Patent document 1 that the compounds which interact with 5-HT_{1A} receptor are useful for the prevention of urinary incontinence. Furthermore, use of compounds which have affinity for the 5-HT_{1A} receptor in the treatment of the lower urinary-tract symptoms is disclosed in Patent documents 2 and 3.

Furthermore, it is disclosed that compounds which have 5-HT_{1A} receptor antagonism represented by the following formula are effective as the lower urinary-tract symptoms therapeutic agent (for example, Patent document 4 : WO99/06384.). 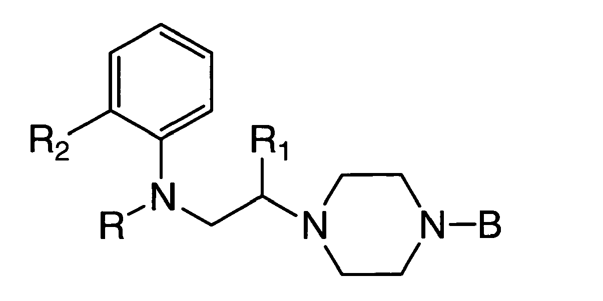 [wherein R represents a hydrogen atom, etc.; R₁ represents a hydrogen atom, etc.; R₂ represents a halogen atom, etc.; B represents a monocyclic aryl group, etc.]

In research of the micturition reflex using rats, a 5-HT_{1A} receptor agonist rises a micturition reflex (for example, refer to Non-patent literature 1), on the other hand, it is reported that another side and this receptor antagonist suppresses the rhythmic contraction and the micturition reflex measured by cystometrogram. Moreover, it is also reported that the 5-HT_{1A} receptor partial agonist suppressed the micturition reflex attenuated the 5-HT_{1A} receptor partial agonist corresponding to the grade of the action property which the medicine has (for example, refer to Non-patent literature 2 : J. Pharmacol Exp. Ther., 290 : 3, 1258-69 (1999))
From these findings, the compound which has 5-HT_{1A} receptor-binding property is anticipated as a new dysuria therapeutic agent based on a new mechanism of action (for example, refer to Non-patent literature 3 : Drugs 63 (23), 2595 -611 (2003)).

On the basis of these backgrounds, it is disclosed that the compound represented by the following formula has excellent 5-HT_{1A} receptor-binding property, and has a beneficial effect on the treatment or prevention of the dysuria (for example, refer to Patent document 5: Japanese Patent Unexamined Publication No. 2002-114684). 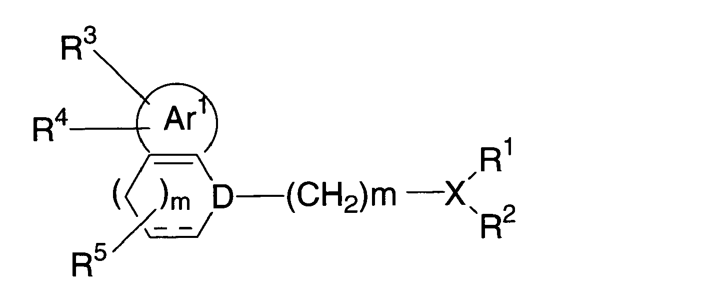 [wherein Ar¹ ring represents a benzene ring, etc., D represents a nitrogen atom, etc. , R³ and R⁴ are the same as or different from each other and each represents a hydrogen atom, etc. , R⁵ represents a hydrogen atom, etc., R¹ and R² represent a hydrogen atom, etc. or represent together the ring which contains X in combination, or m represents an integer of 0, 1 to 6.]

### DISCLOSURE OF THE INVENTION

### PROBLEM TO BE SOLVED BY THE INVENTION

However, the compound group represented by the formula disclosed in Patent document 5, experiment 1 in said document ([³H]-(+/-)-8-hydroxy-2-(dipropylamino)tetralin binding experiment), it is related with Experiment 2 (5-HT_{1A} receptor-antagonism examination), Experiment 3 (antagonism with respect to the 5-HT_{1A} receptor-agonist induced hypothermic effect in rats), and Experiment 4 (inhibitory effect of the rhythmic contraction in rats). Although each test method is disclosed in detail, not only test compounds but those concrete test results (pharmacological effect) are not disclosed at all.
Therefore, from the content disclosed in Patent document 5, it is unknown whether the compound represented by the formula actually has the excellent 5-HT1A receptor agonistic or antagonistic action and has a beneficial effect on the treatment or prevention of dysuria.
In addition, the indole derivatives which have a piperidine ring have been disclosed as the treatment or preventive agent of the lower urinary-tract symptoms (for example, refer to Patent document 6 : WO2005/108389). However, in said Patent document 6, the derivative only with which the nitrogen atom of a piperidine ring has a bicyclic group is disclosed but the derivative with which the nitrogen atom of a piperidine ring has a monocyclic group is not specifically disclosed.

Then, the present inventors take the above-mentioned situation into consideration, and the present invention aims at providing the compound which has excellent affinity for the 5-HT_{1A} receptor and representing this receptor antagonism, and can demonstrate the more excellent effect in the treatment or prevention of the lower urinary-tract symptoms, especially the urine storage symptoms.

### MEANS TO SOLVE THE PROBLEM

The present inventors eagerly examined the synthesis and the pharmacological activity of compounds which have 5-HT_{1A} receptor-binding ability. As a result, the present inventors have completed the present invention by discovering that the 1-(piperidine-4-yl)-1*H*-indole derivatives based on this invention exhibit the excellent 5-HT_{1A} receptor antagonism, and are particularly useful for the treatment or prophylactic agent for the lower urinary-tract symptoms, specifically urine storage symptoms, an urinary frequency, or an urinary incontinence.

In the first aspect of the present invention, the present invention provides:
[1] a compound represented by the following formula (1) or a pharmacologically acceptable salt thereof, or a hydrate thereof,

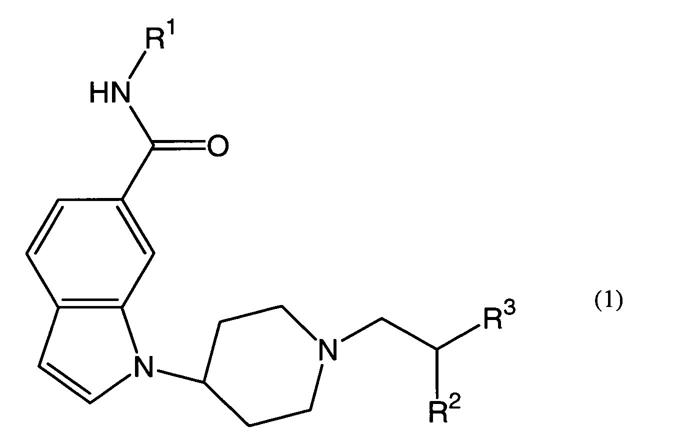

wherein R1 represents a hydrogen atom or a methyl group;
R2 represents a hydrogen atom, a halogen atom, a C₁₋₆ alkyl group, or a hydroxyl group; and
R3 represents a group selected from the group consisting of the formulas: 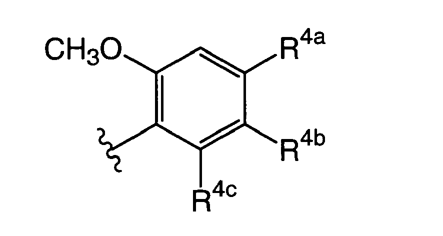 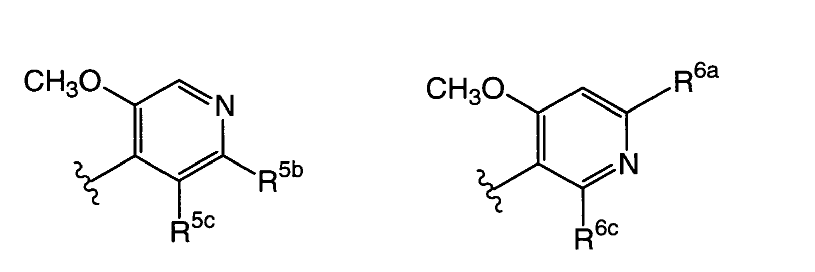 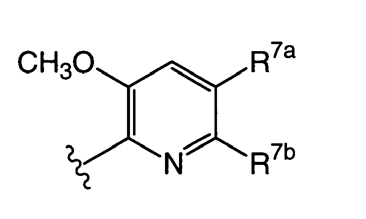 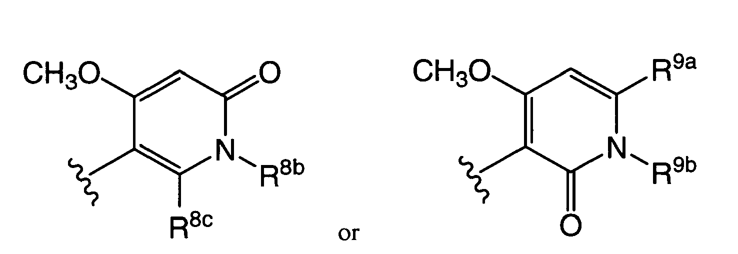
wherein R4a, R4b, R4c, R5b, R5c, R6a, R6c, R7a, R7b, R8c, and R9a are the same as or different from each other and each represents the group selected from following A1 group; R8b and R9b represent a phenyl group which may be substituted by one to three groups selected from a C₁₋₆ alkyl group or following B1 group; (provided that a compound in which all of R4a, R4b, and R4c are hydrogen atoms and a compound in which all of R6a, and R6c are hydrogen atoms are excluded.)

<A1 group>
   (1) a hydrogen atom;
   (2) a halogen atom;
   (3) a hydroxyl group;
   (4) a nitro group;
   (5) a cyano group;
   (6) a C₁₋₆ alkyl group (wherein said C₁₋₆ alkyl group may be substituted by the substituent selected from the group consisting of a hydroxyl group, a cyano group, a C₁_₆ alkoxy group, a di(C₁₋₆ alkyl)amino group, and a di (C₁₋₆ alkyl)aminocarbonyl group);
   (7) a trifluoromethyl group;
   (8) a C₁₋₆ alkoxy group (wherein said C₁₋₆ alkoxy group may be substituted by the substituent selected from the group consisting of a hydroxyl group, a C₁₋₆ alkoxy group, a di (C₁₋₆ alkyl) amino group, and a piperidyl group);
   (9) a phenyl group which may be substituted with one to three group selected from following B1 group;
   (10) a benzyloxy group which may be substituted with one to three group selected from following B1 group;
   (11) a group represented by the formula -NR²¹-R²² (wherein R²¹ represents a hydrogen atom or a C₁₋₆ alkyl group, R²² represents a hydrogen atom, a C₁₋₆ alkyl group, a C₂₋₇ alkylcarbonyl group, or a C₁₋₆ alkylsulfonyl group);
   (12) formula -CO-R²³ (wherein R²³ represents a C₁₋₆ alkyl group or a di (C₁₋₆ alkyl) amino group.); and
   (13) formula -X-R²⁴ (wherein R²⁴ represents a 5- to 6-membered heterocyclic group or a heteroaryl group which may be substituted with one to three group selected from C1 group, X represents a single bond, - (CH₂)ₘ- group (m represents 1 to 3), an oxygen atom, a carbonyl group, a -(CH₂)ₙCO- group (n represents 1 to 3), or a -N(CH₃)- group.).
<B1 group>
   a C₁₋₆ alkyl group,
   a C₁₋₆ alkoxy group, and
   a halogen atom.
<C1 group>
   a C₁_₆ alkyl group;
   a C₁_₆ alkoxy group;
   a C₂₋₇ alkyl carbonyl group;
   a halogen atom;
   a benzyloxycarbonyl group; and
   a N-methyl acetamide group.

In the above-mentioned compounds or pharmacologically acceptable salts thereof, or hydrates thereof, suitable compounds are listed below.
[2] in item [1], wherein R1 represents a hydrogen atom.

[3] in item [1] or [2], wherein R2 represents a hydrogen atom.

[4] in any one of items [1] to [3], wherein R3 is represented by the formula: 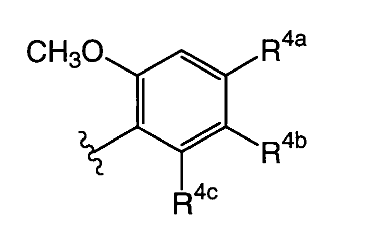
(wherein R4a, R4b, and R4c are same meaning as defined in item [1]).

[5] in any one of items [1] to [3], wherein R3 is represented by the following formula: 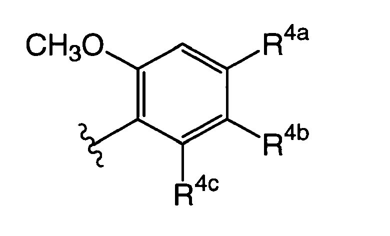
(wherein R4a, R4b, and R4c are the same as or different from each other, and each represents the group selected from the following A2 group.)

<A2 group>
   (1) a hydrogen atom;
   (2) a halogen atom;
   (3) a hydroxyl group;
   (4) a cyano group;
   (5) a C₁₋₆ alkyl group (wherein said C₁₋₆ alkyl group may be substituted by the substituent selected from the group consisting of a hydroxyl group, a cyano group, a C₁₋₆ alkoxy group, a di (C₁₋₆ alkyl)amino group, and a di(C₁₋₆ alkyl)aminocarbonyl group);
   (6) a trifluoromethyl group;
   (7) a C₁_₆ alkoxy group (wherein said C₁_₆ alkoxy group may be substituted by the substituent selected from the group consisting of a hydroxyl group, a C₁₋₆ alkoxy group, a di (C₁₋₆ alkyl) amino group, and a piperidyl group);
   (8) a phenyl group which may be substituted by one to three group selected from the following B1 group;
   (9) a group represented by the formula -NR²¹-R²² (wherein R²¹ represents a hydrogen atom or a C₁₋₆ alkyl group; R²² represents a hydrogen atom, a C₁₋₆ alkyl group, C₂₋₇ alkylcarbonyl group, or C₁₋₆ alkylsulfonyl group) ;
   (10) formula -CO-R²³ (wherein R²³ represents a C₁₋₆ alkyl group or a di(C₁₋₆ alkyl) amino group.); and
   (11) formula -X-R²⁴ (wherein R²⁴ represents a 5- to 6-membered heterocyclic group or a heteroaryl group which may be substituted by one to three group selected from a C1 group; X represents a single bond, a -(CH₂)ₘ- group (m represents 1 to 3), an oxygen atom, a carbonyl group, a - (CH₂)ₙCO- group (n represents 1 to 3) or a -N(CH₃)-group.).
<B1 group>
   a C₁₋₆ alkyl group;
   a C₁₋₆ alkoxy group; and
   a halogen atom.
<C1 group>
   a C₁₋₆ alkyl group;
   a C₁₋₆ alkoxy group;
   a C₂₋₇ alkylcarbonyl group;
   a halogen atom;
   a benzyloxycarbonyl group; and
   a N-methyl acetamide group.

[6] in any one of items [1] to [3], wherein R3 is represented by the formula: 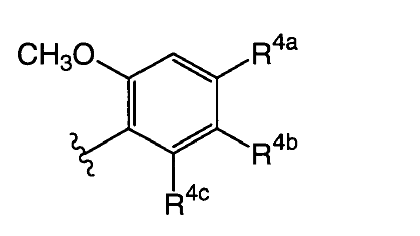
(wherein R4a, R4b, and R4c are the same as or different from each other and each represents the group selected from the following A3 group.)

<A3 group>
   (1) a hydrogen atom;
   (2) a halogen atom;
   (3) a cyano group;
   (4) a C₁₋₆ alkyl group (wherein said C₁₋₆ alkyl group may be substituted by the substituent selected from the group consisting of a hydroxyl group, a cyano group, and C₁₋₆ alkoxy group.);
   (5) a trifluoromethyl group;
   (6) a C₁₋₆ alkoxy group (wherein said C₁₋₆ alkoxy group may be substituted by the substituent selected from the group consisting of a hydroxyl group and a C₁₋₆ alkoxy group.) ;
   (7) a group represented by the formula -NR²¹-R²² (wherein R²¹ represents a hydrogen atom or a C₁₋₆ alkyl group; R²² represents a hydrogen atom, a C₁₋₆ alkyl group, a C₂₋₇ alkylcarbonyl group, or a C₁₋₆ alkylsulfonyl group.);
   (8) formula -CO-R²³ (wherein R²³ represents a C₁₋₆ alkyl group.); and
   (9) formula -X-R²⁴ (Wherein R²⁴ represents a 5- to 6-membered heterocyclic group or a heteroaryl group which may be substituted by one to three group selected from the following C1 group; X represents a single bond and a (CH₂)ₘ-group (m represents 1 to 3), an oxygen atom, a carbonyl group, a -(CH₂)ₙCO- group (n represents 1 to 3), or a -N(CH₃)-group.)
<C1 group>
   a C₁₋₆ alkyl group;
   a C₁₋₆ alkoxy group;
   a C₂₋₇ alkylcarbonyl group;
   a halogen atom;
   a benzyloxycarbonyl group; and
   a N-methyl acetamide group.

[7] in any one of items [1] to [3], wherein R3 is represented by the formula: 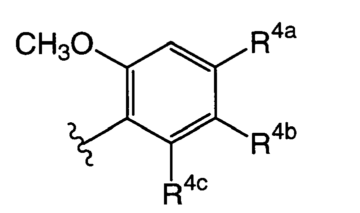
(wherein R4a, R4b, and R4c are the same as or different from each other and each represents the group selected by following A4 group.)

<A4 group>
   (1) a hydrogen atom;
   (2) an unsubstituted C₁₋₆ alkoxy group;
   (3) a C₁₋₆ alkoxy-C₁₋₆ alkyl group; and
   (4) a morpholino group.

[8] in any one of items [1] to [3], wherein R3 is represented by the formula: 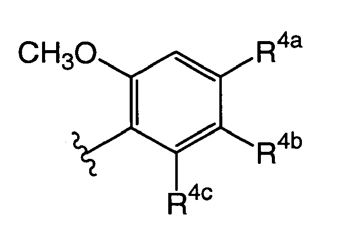
(wherein R4a, R4b, and R4c are the same as or different from each other and each represents the group selected by following A5 group.)

<A5 group>
   (1) a hydrogen atom;
   (2) a methoxy group;
   (3) a methoxymethyl group; and
   (4) a morpholino group.

[9] in any one of items [1] to [6], wherein R24 represents a group selected from the following D1 group (wherein said group may be substituted by the one to three group selected from C1 group.)

<C1 group>
   a C₁_₆ alkyl group;
   a C₁_₆ alkoxy group;
   a C₂₋₇ alkyl carbonyl group;
   a halogen atom;
   a benzyloxycarbonyl group; and
   N-methyl acetamide group.
<D1 group>
   a pyrrolidinyl group;
   a piperidyl group;
   a pyridyl group;
   a piperazinyl group;
   an oxazolyl group;
   an isoxazolyl group;
   an oxo pyrrolidinyl group;
   an oxo piperazinyl group;
   a pyridonyl group; and
   a morpholinyl group.

[10] in any one of Claims 1 to 6, wherein R24 represents a group selected from the following D2 group (wherein said group may be substituted by the one to three group selected from C2 group.)

<C2 group>
   a methyl group;
   a acetyl group; and
   a N-methyl acetamide group.
<D2 group>
   a pyrrolidinyl group;
   a piperidyl group;
   an oxo pyrrolidinyl group; and
   a morpholinyl group.

As the suitable compound of 1-(piperidinyl)indole derivatives according to the present invention, the present invention provides:
[11] a compound selected from the group consisting of the following compound groups or a pharmacologically acceptable salt thereof, or a hydrate thereof:
   1-[1-[2-(2-methoxy-4-methoxymethylphenyl)ethyl]piperidin-4-yl]-1*H*-indole-6-carboxamide;
   1-[1-[2-(2,4,6-trimethoxyphenyl)ethyl]piperidin-4-yl]-1*H*-indole-6-carboxamide;
   1-[1-[2-(2,3,6-trimethoxyphenyl)ethyl]piperidin-4-yl]-1*H*-indole-6-carboxamide;
   1-[1-[2-(4,6-dimethoxypyridin-3-yl)ethyl]piperidin-4-yl]-1*H*-i ndole-6-carboxamide;
   1-[1-[2-(2,5-dimethoxyphenyl)ethyl]piperidin-4-yl]-1*H*-indole-6-carboxamide ;
   1-[1-[2-(2,6-dimethoxyphenyl)ethyl]piperidin-4-yl]-1*H*-indole-6-carboxamide; and
   1-[1-[2-(2-methoxy-6-morpholinophenyl)ethyl]piperidin-4-yl]-1 H-indole-6-carboxamide.

In another aspect of the present invention, the present invention provides: [12] a pharmaceutical composition comprising the compound according to any one of items [1] to [11] or a pharmacologically acceptable salt thereof, or a hydrate thereof, as an active ingredient.

In further another aspect of the present invention, the present invention provides: [13] a treating or preventing agent for a lower urinary tract symptom comprising the compound according to any one of items [1] to [11] or a pharmacologically acceptable salt thereof, or a hydrate thereof, as an active ingredient.

In still another aspect of the present invention, the present invention provides: [14] a treating or preventing agent for a micturition symptom comprising the compound according to any one of items [1] to [11] or a pharmacologically acceptable salt thereof, or a hydrate thereof, as an active ingredient.

In other aspect of the present invention, the present invention provides: [15] a treating or preventing agent for a frequent urination or urinary incontinence comprising the compound according to any one of items [1] to [11] or a pharmacologically acceptable salt thereof, or a hydrate thereof, as an active ingredient.

The term "lower urinary tract symptom" used in the present specification represents a generic designation of the disease associated with dysfunction in the urinary collection mechanism and dysfunction in the mechanism of urea excretion. Also, the term "frequent urination" used in the present specification represents a symptom which falls into a symptom that the frequency of urination increases, and the term "urinary incontinence" used in the present specification means that urine is in condition to be drained involuntariness unconsciously, including involuntary micturition, incontinentia ureterica, stress incontinence, reflex incontinence, overflow incontinence, etc.. Further, the term "storage symptoms" used in the present specification refers to a sympton involving frequent urination, urinary urgency, urinary incontinence, etc..

The term "halogen atom" used in the present specification refers to a fluorine atom, a chlorine atom, a bromine atom or an iodine atom; preferred examples of halogen atom include a fluorine atom or a chlorine atom.

The term "C₁₋₆ alkyl group" used in the present specification refers to a linear chain or branched chain alkyl group containing 1 to 6 carbon atoms, for example, includes a methyl group, an ethyl group, a *n*-propyl group, an isopropyl group, a *n*-butyl group, an isobutyl group, a *tert*-butyl group, a *n*-pentyl group, an isopentyl group, a neopentyl group, a *n*-hexyl group, 1-methylpropyl group, 1,2-dimethylpropyl group, a 2-methylbutyl group, a 1,2-dimethylbutyl groups, a 1-ethyl-2-methylpropyl group, a 1,1,2-trimethylpropyl group, a 1-methylbutyl group, a 1,1-dimethylbutyl group, a 2,2-dimethylbutyl group, a 2-ethylbutyl group, a 1,3-dimethylbutyl group, a 2-methylpentyl group, 3-methylpentyl group, etc.. Preferred examples of such groups include a methyl group or an ethyl group.

The term "C₁₋₆ alkoxy group" used in the whole present specification refers to a group in which hydrogen atom of the linear chain or the branched chain of alkyl group is substituted for an oxygen atom, for example, includes a methoxy group, an ethoxy group, a *n-*propoxy group, an isopropoxy group, a sec-propoxy group, a *n-*butoxy group, an isobutoxy group, a sec-butoxy group, a *tert*-butoxy group, a *n-*pentyloxy group, an isopentyloxy group, a sec-pentyloxy group, a *tert*-pentyloxy group, a *n-*hexyloxy group, an isohexyloxy group, a 1,2-dimethyl propoxy group, a 2-methylbutoxy group, a 1,2-dimethylbutoxy group, a 1-ethyl-2-methylpropoxy group, a 1,1,2-trimethylpropoxy group, a 1,1-dimethyl butoxy group, a 2,2-dimethylbutoxy group, a 2-ethylbutoxy group, a 1,3-dimethylbutoxy group, a 2-methylpentyloxy group, a 3-methylpentyloxy group, a hexyloxy group, etc.. Preferred examples of such groups include a methoxy group or an ethoxy group.

The term "di- (C₁₋₆ alkyl) amino group" used in the present specification refers to a group in which two hydrogen atoms of the amino group is substituted for C1-6 alkyl group, for example, includes a N, N-dimethylamino group, a N-ethyl-N-methylamino group, a N, N-diethylamino group, a N-methyl-N-propylamino group, a N-ethyl-N-propyl amino group, a N, N-dipropylamino group, a N-butyl-N-methylamino group, a N-butyl-N-ethylamino group, a N-butyl-N-propylamino group, a N, N-dibutylamino group, a N-methyl-N-pentylamino group, a N-ethyl-N-pentylamino group, a N-pentyl-N-propylamino group, a N-butyl-N-pentylamino group, a N, N-dipentylamino group, a N-hexyl-N-methylamino group, a N-ethyl-N-hexylamino group, a N-hexyl-N-propylamino group, a N-butyl-N-hexylamino group, a N-hexyl-N-pentylamino group, a N, N-dihexylamino group, etc.. Preferred examples of such groups include a N, N-dimethylamino group or a N, N-diethylamino group.

The term "di- (C₁₋₆ alkyl) amino carbonyl group" used in the present specification refers to a group in which a hydrogen atom of an amino carbonyl group is substituted for C1-6 alkyl group, for example, include a N, N-dimethylaminocarbonyl group, a N-ethyl-N-methylaminocarbonyl group, a N, N-diethylaminocarbonyl group, a N-methyl-N-propylaminocarbonyl group, a N-ethyl-N-propylaminocarbonyl group, a N, N-dipropylaminocarbonyl group, a N-butyl-N-methylaminocarbonyl group, a N-butyl-N-ethylaminocarbonyl group, a N-butyl-N-propylaminocarbonyl group, a N,N-dibutylaminocarbonyl group, a N-methyl-N-pentylaminocarbonyl group, a N-ethyl-N-pentylamino carbonyl group, a N-pentyl-N-propylaminocarbonyl group, a N-butyl-N-pentylaminocarbonyl group, a N, N-dipentylamino carbonyl group, a N-hexyl-N-methylamino carbonyl group, a N-ethyl-N-hexylamino carbonyl group, a N-hexyl-N-propylaminocarbonyl group, a N-butyl-N-hexylaminocarbonyl group, a N-hexyl-N-pentylaminocarbonyl group, a N, N-dihexylaminocarbonyl group, etc.. Preferred examples of such groups include a N, N-dimethylaminocarbonyl group or a N, N-diethylaminocarbonyl group.

The term "C₂₋₇ alkyl carbonyl group" used in the whole present specification refers to a carbonyl group to which the above-mentioned "C1-6 alkyl group" is bound, for example, include acetyl, propionyl, butyryl, isobutyryl, valeryl, isovaleryl, pivaloyl, hexanoyl or a heptanoyl group, etc.. Preferred examples of such groups include acetyl or propionyl.

The term "C₁₋₆ alkyl sulfonyl group" used in the present specification refers to a linear or branched chain alkylsulfonyl group containing 1 to 6 carbon atoms, for example, include a methyl sulfonyl, an ethylsulfonyl, a propylsulfonyl, an isopropyl sulfonyl, a butylsulfonyl, an isobutylsulfonyl, a *s*-butylsulfonyl, a *tert*-butylsulfonyl, a pentylsulfonyl, an isopentylsulfonyl, a 2-methylbutylsulfonyl, a neopentylsulfonyl, a 1-ethylpropylsulfonyl, a hexylsulfonyl, a 4-methylpentylsulfonyl, a 3-methylpentylsulfonyl, a 2-methylpentylsulfonyl, 1-methylpentylsulfonyl, a 3,3-dimethylbutylsulfonyl, a 2,2-dimethylbutylsulfonyl, a 1,1-dimethylbutylsulfonyl, a 1,2-dimethylbutylsulfonyl, a 1,3-dimethylbutylsulfonyl, a 2,3-dimethylbutylsulfonyl or a 2-ethylbutylsulfonyl group, etc.. Preferred examples of such groups include a methylsulfonyl or an ethylsulfonyl group.

The term "5- to 6-membered heterocyclic group or a heteroaryl ring group" used in the present specification refers to a 5- to 6-membered heterocyclic group containing 1 to 3 atoms containing a sulfur atom, an oxygen atom and an nitrogen atom, for example, include a pyrrolidinyl group, an oxo pyrrolidinyl group, a pyrolinyl group, a pyrrolyl group, a furyl group, a thienyl group, an imidazolyl group, a pyrazolyl group, an oxazolyl group, an isoxazolyl group, a thiazolyl group, an isothiazolyl group, a pyrazolonyl group, a piperazinyl group, an oxo piperazinyl group, piperidyl group, a morpholinyl group, a pyranyl group, a pyridyl group, a pyrazinyl group, a pyrimidinyl group, a pyridazinyl group, a pyridonyl group, a triazolyl group. Preferred examples of such groups include a pyrrolidinyl group, an oxo pyrrolidinyl group, an oxazolyl group, an isoxazolyl group, a piperazinyl group, an oxo piperazinyl group, a piperidyl group, a morpholinyl group, a pyridyl group or a pyridonyl group.

The term "C₁₋₆ alkoxy -C₁₋₆ alkyl group" used in the present specification refers to a group in which a hydrogen atom of the above definition "C₁₋₆ alkyl group" is substituted for the above definition "C₁₋₆ alkoxy group", for example, include a methoxy methyl group, a methoxyethyl group, an ethoxymethyl group, an ethoxyethyl group.

The term "pharmacologically acceptable salt" used in the present specification is not limited insofar as it forms a pharmacologically acceptable salt with the compound represented by the above formula (I). Preferably, hydrogen halides (for example, hydrochloride, hydrobromide and hydroiodide), inorganic acid salts (for example, sulfate, nitrate, perchlorate, phosphate, carbonate and bicarbonate), organic carboxylic acid salts (for example, acetate, oxalate, maleate, tartarate, fumarate, citrate), organic sulfonic acid salts (for example, methanesulfonate, trifluoromethanesulfonate, ethanesulfonate, benzenesulfonate, toluenesulfonate and camphor sulfonate) amino acid salts (for example, aspartate and glutamate).

Note that in the present specification, the structural formula of the compound may represent a constant isomer for convenience, but all geometric isomers producing in the conformation of the compound according to the present invention, optical isomer based on the asymmetric carbon, stereoisomer, isomer such as the tautomer and mixture of isomer are included in the present invention, it is not limited to the formula' s description for convenience, the present invention encompasses even a mixture of isomers. Thus, the compound according to the present invention exists as an optically active substance and racemate having an asymmetric carbon atom intramolecularly, although the present invention is not limited thereto. Further, a crystal polymorph sometimes exists for the compound according to the present invention, and all crystal polymorphs thereof are included in the present invention. The crystal polymorph is sometimes single or sometimes a mixture thereof, and both are included in the present invention.

### ADVANTAGEOUS EFFECT OF THE INVENTION

1-(Piperidin-4-yl)-1*H*-indole derivatives represented by formula (1) according to the present invention or the pharmacologically acceptable salts thereof, or hydrates thereof have an excellent 5-HT_{1A} receptor antagonistic action, and are useful as a therapeutic or preventive agent for the lower urinary tract symptoms.

### BEST MODE FOR CARRYING OUT THE INVENTION

The examples and pharmacological tests will be described below for explaining the advantageous effect of the compound according to the present invention. The examples are provided for illustrative purposes only, and are not intended to limit the scope of the invention.

The compound represented by formula (1) according to the present invention can be produced by methods describe below.

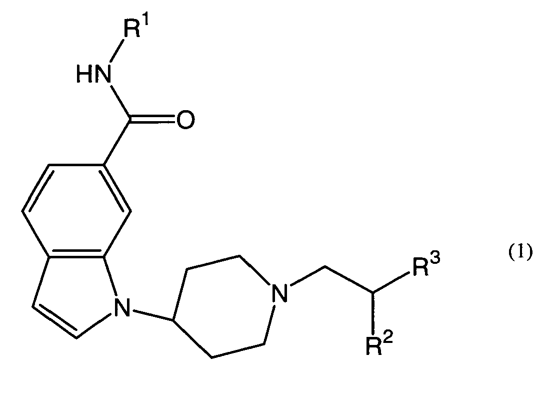

[wherein R¹, R² and R³ have the same meaning as defined in the above.]
For examples, the compound represented by the formula (1) can be produced by the following four types of production methods. Note that the term "room temperature" described below refers to a temperature approximately between 15 °C and 30 °C.

### [Production method 1]

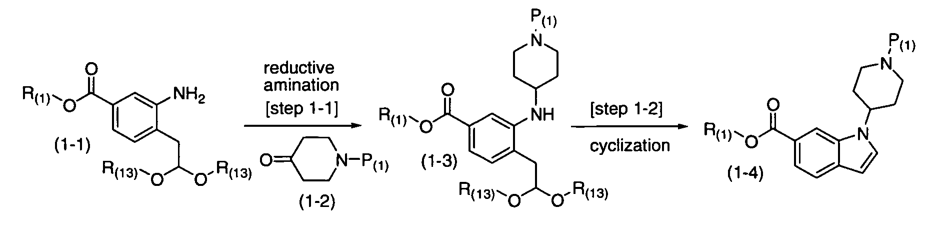

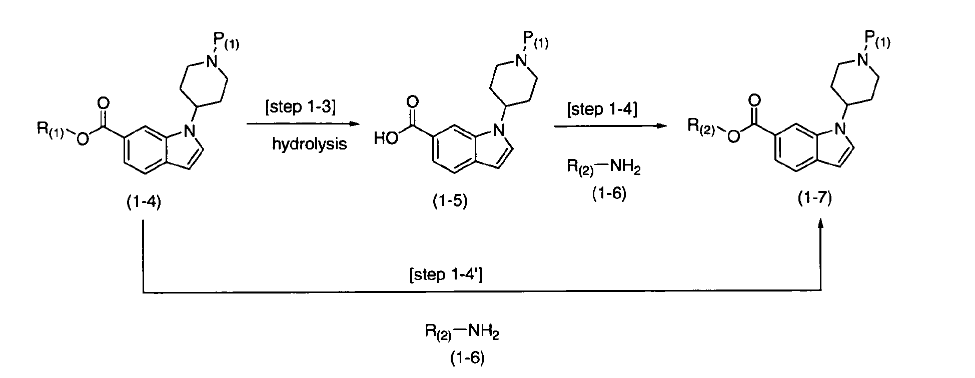

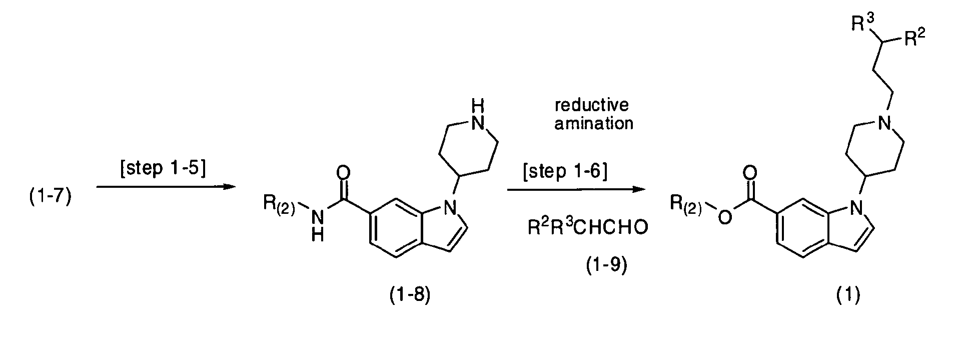

wherein R₍₁₎ represents a lower alkyl group such as a methyl group or an ethyl group, a lower aralkyl group such as a benzyl group, etc. ; R₍₂₎ represents a hydrogen atom or a methyl group; R₍₁₃₎ represents a methyl group, an ethyl group, etc., which are capable of being hydrolyzed; P₍₁₎ represents a protecting group for an amino group, which is able to be deprotected, such as a benzyloxycarbonyl group, *tert*-butyloxycarbonyl group, etc.; and R² and R³ have the same meanings as described above.

[Production method 1] is a method for producing the compound represented by formula (1) according to the present invention, which uses compound (1-1) as a raw material and conducts multi-stage steps ranging from [Step 1-1] to [Step 1-6]. Compound (1-1) can also be produced from a commercially available compound according to the methods known to persons skilled in the art. Examples of such known methods may include: Coe, J. W. ; Vetelino, M. G. ; Bradlee, M. J. ; Tetrahedron Lett. , 37 (34), 6045-6048 (1996), Arai, E. ; Tokuyama, H. ; Linsell, M. S. ; Fukuyama, T. ; Tetrahedron Lett., 39(1), 71-74 (1998), Tisher, A. N., Lanza, T. J.; Tetrahedron Lett., 27(15), 1653 (1986), Sakamoto Takao, Kondo Yoshinori, Yamanaka Hiroshi, Chem. Pharm. Bull., Vol. 34, p. 2, 362 (1986). With regard to compound (1-2) and compound (1-6), commercially available compounds may directly be used, or these compounds may also be produced from commercially available compounds according to methods known to persons skilled in the art. Compound (1-9) and compound (1-10) may be produced from commercially available compounds according to methods known to persons skilled in the art, or may also be produced by the method described in production examples in the present examples.

### [Step 1-1]

Step 1-1 is a step of obtaining compound (1-3) by reductive amination of compound (1-1) and compound (1-2). The reaction of this step can be carried out under the same conditions as those commonly used for the reductive amination of a carbonyl compound and an amine compound. The reduction reaction in this step is not particularly limited. Examples of such a reaction may include a reductive amination reaction using a reducing agent such as borane or a boron hydride complex compound, and a catalytic reduction reaction using a metal catalyst under a hydrogen atmosphere.

Examples of the reductive amination reaction using the boron hydride complex compound may include methods described in publications such as W. S. Emerson, Organic Reactions, 4, 174 (1948), C. F. Lane, Synthesis, 135 (1975), J. C. Ctowell and S. J. Pedegimas, Synthesis, 127 (1974), A. F. Abdel-Magid, K. G. Carson, B. D. Harris, C. A. Maryanoff and R. D. Shah, Journal of Organic Chemistry, 61, 3849 (1996). Examples of the boron hydride complex compound used herein may include sodium borohydride, sodium cyanoborohydride, sodium triacetoxyborohydride. When the boron hydride complex compound is used as reducing agent, a solvent is not particularly limited, as long as it does not inhibit the reaction and dissolve a starting material to a certain extent. Example of a solvent used herein may include methanol, ethanol, tetrahydrofuran, N,N-dimethylformamide, dichloromethane, 1,2-dichloroethane, etc..

When the present reaction is carried out in the coexistence of an acid, preferred results such as the improvement of yield can be obtained. Preferred examples of such acid may include: but are not limited to, mineral acids such as hydrochloric acid, organic acids such as acetic acid, and Lewis acids such as zinc chloride, a boron trifluoride diethyl ether complex, or titanium (IV) tetraisopropoxide.

Compound (1-2) is used against compound (1-1) at a ratio of 0.8 to 2.5 equivalents, preferably 1 to 1.5 equivalents. A boron hydride complex compound is used against compound (1-1) at a ratio of 1 to 3 equivalents, and preferably 1 to 1. 5 equivalents. The reaction time is not particularly limited. It is generally between 0.5 and 48 hours, preferably between 0.5 and 12 hours. The reaction temperature of this step is not particularly limited. The reaction temperature is generally between -78°C and a solvent-reflux temperature, preferably between a temperature on ice and a room temperature.

When a catalytic reduction reaction is carried out under a hydrogen atmosphere, a solvent used is not particularly limited, as long as it does not inhibit the reaction. Examples of the solvent may include methanol, ethanol, tetrahydrofuran, and 1, 4-dioxane. Examples of the metal catalyst used for the reaction may include palladium, platinum oxide, and Raney nickel.

The reaction time is not particularly limited. The reaction time is generally between 1 and 48 hours, preferably between 1 and 24 hours. The reaction conditions are not particularly limited. The reaction can be carried out at a temperature between a room temperature and a solvent-reflux temperature at a pressure between a normal pressure and a pressure of 150 atmospheres, preferably at a temperature between a room temperature and 60°C at a pressure between a normal pressure and a pressure of 5 atmospheres.

### [Step 1-2]

Step 1-2 is a step of obtaining compound (1-4) by the ring closure of compound (1-3) with an acid. The reaction can be carried out under the same reaction conditions as those described in, for example, Coe, J. W. ; Vetelino, M. G. ; Bradlee, M. J.; Tetrahedron Lett., 37(34), 6045-6048 (1996), Arai, E. ; Tokuyama, H.; Linsell M. S.; Fukuyama, T.; Tetrahedron Lett., 39(1), 71-74 (1998), Tishler, A. N., Lanza, T. J.; Tetrahedron Lett., 27(15), 1653 (1986), Sakamoto Takao, Kondo Yoshinori, Yamanaka Hiroshi, Chem. Pharm. Bull., Vol. 34, p. 2362 (1986).

A solvent used in the present reaction is not particularly limited, as long as it does not inhibit the reaction and dissolve a starting material to a certain extent. Examples of such a solvent may include: water; mixed solvent consisting of water and an organic solvent such as methanol, ethanol, tetrahydrofuran, 1,4-dioxane, benzene, or toluene; and organic solvents such as methanol, ethanol, tetrahydrofuran, 1,4-dioxane, benzene, or toluene. The present reaction can be carried out by using appropriate acid at a ratio between 1 equivalent and a large excess amount against the aforementioned compound in the solvent. Examples of the acid used herein may include acetic acid, hydrogen chloride, hydrochloric acid, hydrobromic acid, sulfuric acid, nitric acid, trifluoroacetic acid, p-toluenesulfonic acid, p-toluenesulfonic acid-pyridinium salt, and camphorsulfonic acid.

The reaction time is not particularly limited. The reaction time is generally between 1 and 24 hours, preferably between 1 and 4 hours. The reaction temperature of this step is generally between a temperature on ice and a solvent-reflux temperature. It is to be noted that [Step 1-1] and [Step 1-2] can also be carried out by one-pot reaction without isolating compound (1-3).

### [Step 1-3]

Step 1-3 is a step of obtaining compound (1-5) by alkaline hydrolysis of compound (1-4). The reaction can be carried out under the same reaction conditions as those described in, for example, Matassa, V. G. ; Brown, F. J. ; Bernstein, P. R. ; Shapion, H.S.; Maduskuie, T. P. J.; Cronk, L. A.; Vacek, E. P.; Yee, Y. K.; Snyder, D. W.; Krell, R. D.; Lerman, C. L; Maloney, J. J.; J. Med. Chem., 33 (9), 2621-2629 (1990). Specifically, for example, a base such as sodium hydroxide is added to a solution containing compound (1-4). The mixture is then stirred for several hours to 1 day. Thereafter, the resultant mixture is treated with an acid such as citric acid, to give compound (1-5).

A solvent used in the present reaction is not particularly limited, as long as it does not inhibit the reaction and dissolve a starting material to a certain extent. Examples of such a solvent may include methanol, ethanol, 2-propanol, tetrahydrofuran, and 1,4-dioxane. The base used herein is not particularly limited. Preferred examples of such a base may include sodium hydroxide, potassium hydroxide, and lithium hydroxide. The amount of a base used against compound (1-4) is between 1 equivalent and an excess amount, preferably between 1 and 20 equivalents.

The reaction time of this step is not particularly limited. The reaction time is generally between 1 and 24 hours preferably between 1 and 6 hours. The reaction temperature of this step is not particularly limited. It is generally between a room temperature and a solvent-reflux temperature.

When an ester is a benzyl ester or allyl ester, carboxylic acid can be obtained under the same conditions as those generally used for the deprotection of a protecting group for a carboxylic acid compound (which are condition described in the literature such as T. W. Green and P. G. M. Wuts, "Protective Groups in Organic Chemistry, Second Edition", John Wiley & Sons (1991), p.248-251.

### [Step 1-4]

Step 1-4 is a step of obtaining compound (1-7) by condensing compound (1-5) and compound (1-6) with a condensing agent. The condensation reaction of compound (1-5) and (1-6) with a condensing agent can be carried out under the same conditions as commonly used conditions described in the following publications. Such known methods include Rosowsky, A.; Forsch, R. A. ; Moran, R, G. ; Freisheim, J. H. ; J. Med. Chem. , 34 (1), 227-234 (1991), Brzostwska, M.; Brossi, A.; Flippen-Anderson, J. L.; Heterocycles, 32 (10), 1969-1972 (1991), Romero, D.L. ; Morge, R. A.; Biles, C.; Berrios-Pena, N.; May, P. D.; Palmer, J. R.; Johnson, P. D.; Smith, H. W.; Busso, M.; Tan, C. -K. ; Voorman, R. L. ; Reusser, F. ; Althaus, I. W. ; Downey, K. M. ; So, A. G. ; Resnick, L. ; Tarpley, W. G. , Aristoff, P. A. ; J. Med. Chem. , 37 (7), 999-1014 (1994). Compound (1-6) may be either a free form or a salt.

A solvent used in the present reaction is not particularly limited, as long as it does not inhibit the reaction. Examples of such a solvent may include tetrahydrofuran, 1,4-dioxane, ethyl acetate, methyl acetate, methylene chloride, chloroform, N,N-dimethylformamide, toluene, and xylene. Examples of the condensing agent may include CDI (N,N'-carbonyldiimidazole), Bop (1H-1,2,3-benzotriazol-1-yloxy(tri(dimethylamino))phosphonium hexafluorophosphate), WSC (1-ethyl-3-(3-dimethylaminopropyl)carbodiimide hydrochloride), DCC (N,N-dicyclohexylcarbodiimide), and diethylphosphoryl cyanide. Compound (1-6) is used against compound (1-5) at a ratio between 1 equivalent and an excess amount. In addition, an organic base such as triethylamine may also be added at a ratio between 1 equivalent and an excess amount, as necessary.

The reaction time is not particularly limited. The reaction temperature is generally between 0.5 and 48 hours, preferably between 0.5 and 24 hours. The reaction temperature of this step is not particularly limited, and varies depending on a raw material used, a solvent used, etc.. The reaction temperature is preferably between a temperature on ice and a solvent-reflux temperature.

Moreover, compound (1-7) can also be produced from compound (1-5) and compound (1-6) according to alternative methods described in (1) and (2) below.

### Alternative method (1)

Compound (1-5) is converted into a mixed acid anhydride. Then, the mixed acid anhydride is allowed to react with compound (1-6), to give compound (1-7). Such a mixed acid anhydride can be synthesized by means known to persons skilled in the art. For example, compound (1-5) is allowed to react with a chloroformic ester such as ethyl chloroformate in the presence of a base such as triethylamine. Such a chloroformic ester and a base are used at a ratio between 1 and 2 equivalents against compound (1-5). The reaction temperature is between -30°C and a room temperature, preferably between -20°C and a room temperature.

A step of condensing a mixed acid anhydride and compound (1-6) is carried out, for example, by allowing the mixed acid anhydride to react with compound (1-6) in a solvent such as methylene chloride, tetrahydrofuran, or N,N-dimethylformamide. Compound (1-6) is used at a ratio between 1 equivalent and a large excess amount against the mixed acid anhydride.

The reaction time of the above condensing step is not particularly limited. The reaction time is generally between 0.5 and 48 hours, preferably between 0.5 and 12 hours. The reaction temperature of the above condensing step is generally between -20°C and 50°C, more preferably between -20°C and a room temperature.

### Alternative method (2)

Compound (1-5) is converted into an activated ester. Then, the activated ester is allowed to react with compound (1-6), to give compound (1-7). A step of obtaining such an activated ester can be carried out by allowing compound (1-5) to react with activated ester-synthesizing reagent in a solvent such as 1,4-dioxane, tetrahydrofuran, or N,N-dimethylformamide, in the presence of a condensing agent such as DCC. N-Hydroxysuccinimide is an example of such an activated ester-synthesizing reagent. Such an activated ester-synthesizing reagent and the condensing agent are used at a ratio between 1 and 1.5 equivalents against compound (1-5). The reaction time is not particularly limited. The reaction time is generally between 0.5 and 48 hours, preferably between 0.5 and 24 hours. The reaction temperature is between -20°C and 50°C, preferably between -20°C and a room temperature.

A step of condensing an activated ester and compound (1-6) is carried out by allowing the activated ester to react with compound (1-6) in a solvent such as methylene chloride, tetrahydrofuran, or N,N-dimethylformamide. Compound (1-6) is used at a ratio between 1 equivalent and an excess amount against the activated ester. The reaction time of the above condensing step is not particularly limited. The reaction time is generally between 0.5 and 48 hours, preferably between 0.5 and 24 hours. The reaction temperature of the above condensing step is between -20°C and 50°C, preferably between -20°C and a room temperature.

### [Step 1-4']

Step 1-4' is a step of obtaining compound (1-7) by condensing compound (1-4) and compound (1-6). This condensation reaction can be carried out under the same conditions as those commonly used for the condensation reaction of an ester compound and an amine compound. Known methods may include Dodd, J. H.; Guan, J.; Schwender, C. F.; Synth. Commun., 23(7), 1003-1008 (1993), Sim, T. B.; Yoon, N. M.; Synlett (10), 827-828 (1994). An amine compound (1-6) used may be either a free form or a salt.

A solvent used in the present reaction is not particularly limited, as long as it does not inhibit the reaction. Examples of such a solvent may include methanol, ethanol, 1-propanol, 2-propanol, 1-butanol, tetrahydrofuran, 1,4-dioxane, toluene, xylene, and acetic acid. In addition, it is also possible to use an amine compound (1-6) as a solvent. Compound (1-6) is used at a ratio between 1 equivalent and an excess amount against compound (1-4).

The reaction time of this step is not particularly limited. The reaction time is generally between 1 and 48 hours, preferably between 1 and 24 hours. The reaction temperature of this step is not particularly limited, and varies depending on a raw material used, a solvent used, etc.. It is preferably between a room temperature and a solvent-reflux temperature.

Moreover, in the present reaction, acids such as p-toluenesulfonic acid or camphorsulfonic acid, Lewis acids such as trimethylaluminum, or bases such as sodium hydride may be added to the reaction, thereby obtaining good results such as the reduction of the reaction time or the improvement of yield. Furthermore, a well-closed pressure-resisting container such as an autoclave may be used to heat a reaction mixture to a high temperature between 100°C and 250°C, thereby obtaining good results such as the reduction of the reaction time.

### [Step 1-5]

Step 1-5 is a step of obtaining compound (1-8) by deprotecting a protecting group for the secondary amine of compound (1-7). The deprotection reaction can be carried out under the same conditions as those commonly used for the deprotection of a protecting group for an amino compound. Such conditions are described in publications such as W. Green and P. G. M. Wuts, "Protective Groups in Organic Chemistry, Second Edition", John Wiley & Sons (1991), p.309-405. When the amino group of compound (1-7) is protected by a benzyloxycarbonyl group, for example, the protecting group is deprotected by hydrogenolysis of compound (1-7) using palladium on carbon as a catalyst in a solvent such as alcohol, to give compound (1-8).

### [Step 1-6]

Step 1-6 is a step of obtaining a compound represented by general formula (I) by the reductive amination of compound (1-8) and compound (1-9). In this step, compound (1-8) and compound (1-9) are used as raw materials, and the method described in the aforementioned production method ([Step 1-1]) is applied, so as to synthesize the compound represented by general formula (I). In addition, a commercially available compound may directly be used as compound (1-9), or the above compound may also be produced from a commercially available compound by a method known to persons skilled in the art. Moreover, it can also be produced by the method described below in production method A, production method B or production examples in the present examples. Furthermore, the used compound (1-8) may be either a free form or a salt.

### [Production method 2]

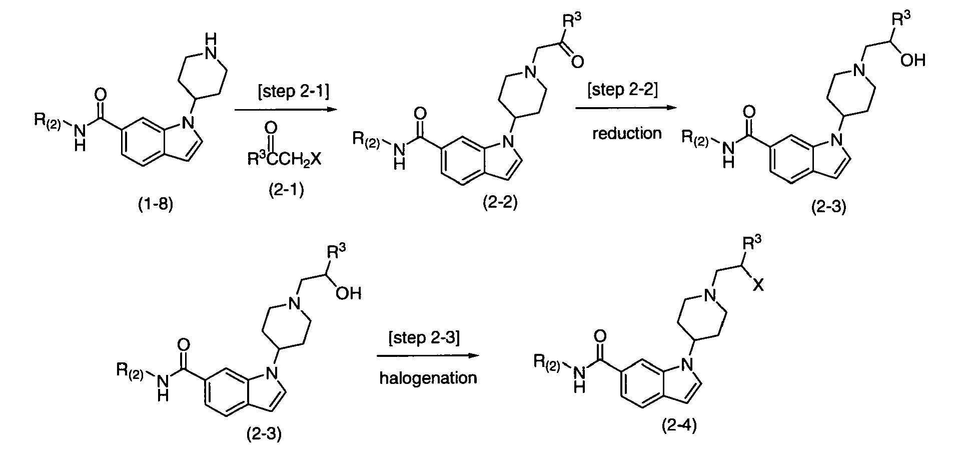

[wherein R₍₂₎ represents a hydrogen atom or a methyl group, example of X include a halogen atom such as a fluorine atom, a chlorine atom, a bromine atom or an iodine atom, R³ has the same meaning as defined above.]

Production method 2 is a method of producing the compound represented by formula (1) according to the present invention, via a process of which uses multi-stage steps ranging from [Step 2-1] to [Step 2-2], then [step 2-3] using compound (1-8) as a raw material. Note that compound (2-3) and compound (2-4) are included in the compound represented by formula (1) according to the present invention.

### [Step 2-1]

Step 2-1 is a step of obtaining the compound of the formula (1) by nucleophilic substitution reaction between compound (1-8) and compound (2-1). This step is carried out under the same conditions as those commonly used for the reaction between a secondary amine and halogen compound (for example, conditions described in Hiraki, Y.; Terada, T.; Okaji, Y.; Yamazaki, T.; Tetrahedron Lett., 31(33), 4755-4758 (1990)). Note that as compound (2-1), a commercially available compound can be used as is, compound (2-1) can be produced by a method well-known to an artisan having the ordinary skills from a commercially available compound. Further, compound (2-1) can be produced using a method as described in production examples in the present examples. Furthermore, the used compound (1-8) may be either a free form or a salt.

A solvent used in the reaction of this step is not particularly limited as long as it does not inhibit the reaction and dissolves a starting material to some extent. Examples of such solvents may include methanol, ethanol, propanol, tetrahydrofuran, benzene, toluene, xylene, acetonitrile, dichloromethane, chloroform, N,N-dimethylformamide, dimethyl sulfoxide.

Compound (2-1) is used against compound (1-8) at a ratio of 1 to 10 equivalents, preferably 1 to 5 equivalents. The reaction time of this step is not limited in particular. The reaction time is generally being between 1 and 72 hours, preferably being between 1 and 48 hours. The reaction temperature of this step is generally between room temperature and solvent-reflux temperature, preferably between room temperature and -100 °C.

Further, good results such as yield improvement can be obtained by adding a base. The base used is not limited in particular as far as reaction is not inhibited. Preferred examples of such base may include sodium carbonate, potassium carbonate, sodium hydroxide, potassium hydroxide, diazabicyclo undecene, sodium hydride, potassium hydride, sodium methoxide, potassium methoxide, potassium *tert*-butoxide, triethylamine, diisopropylethylamine, etc..

### [Step 2-2]

Step 2-2 is a step of obtaining compound (2-3) by reducing a carbonyl group of compound (2-2) with a reducing agent. The reduction reaction of this step is not limited in particular, but the reduction is carried out under the same conditions as those of commonly used for the reduction of a carbonyl group into alcohol (for example, H. C. Brown, S. Krishnamurhy, Tetrahedron, 35, 567 (1979), H. C. Brown, S. Krishnamurhy, AldrichchimicaActa, 12 (1), 3 (1979), etc.).

Examples of the reducing agent used in this step may include metal hydrides, more specifically, it is possible to use sodium borohydride, lithium aluminum hydride, diisobutylaluminum hydride. Solvents used in this step are not limited in particulay, as long as it does not inhibit the reaction and dissolve a starting material to a certain extent. Examples of the solvent may include dichloromethane, tetrahydrofuran, diethyl ether, etc..

The reaction temperature of this step is generally between -100 °C and a room temperature, preferably between -78 °C and a room temperature. The reaction time of this step is not limited in particular, but the reaction time is generally between 0.5 and 48 hours, preferably between 0.5 and 24 hours.

### [Step 2-3]

Step 2-3 is a step of obtaining a halogen compound (2-4) by reacting between compound (2-3) with a halogenating agent. More specifically, as will be described in Examples 36 below, this step is a step in which a hydroxyl group is replaced with fluorine. Examples of such methods may include (i) a method in which a hydroxyl group is first modified with a functional group with a good leaving group such as methanesulfonate, then fluorine ion is reacted; (ii) a fluorinating method in which a hydroxyl group is converted into a reactive intermediate which has a good leaving ability in the reaction system by reacting a fluorinating agent. For the method (ii), it is preferable to use dimethylamino sulfur-trifluoride as the fluorinating agent. Examples of this method may include a method described in, for example, M. Hudliky, Org. React., 35, 513 (1988), etc.. Examples of other fluorinating agents may include hydrogen fluoride, sulfur tetrafluoride, (2-chloro-1,1,2-trifluoroethyl)diamine, 1,1,2,3,3,3-hexafluoro-1-diethylaminopropane, 2,2-difluoro-1,3-dimethylimidazolyzine, [bis(2-methoxyethyl)amino]sulfur trifluroide, difluoro triphenylphosphorane, trifluorodiphenylphospholane, etc..

A solvent used in this step is not limited in particular, as long as it does not inhibit the reaction, examples of such solvent may include methanol, 1, 4-dioxane, tetrahydrofuran, dichloromethane, 1,2-dichloroethane, chloroform, etc.. The halogenating agent is used against compound (2-3) at a ratio of 1 equivalent to an excess amount, preferably 1 equivalent to 2 equivalents.

The reaction time of this step is not limited in particular, but generally between 1 and 48 hours, preferably between 1 and 24 hours. The reaction temperature of this step is not limited in particular, but generally between -100 °C and ice-cooling temperature, preferably between -78 °C and ice-cooling temperature.

### [Production method 3]

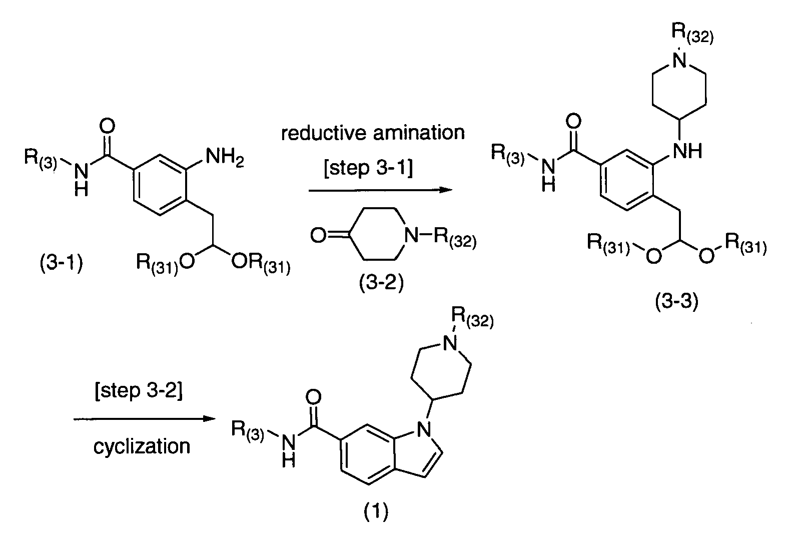

[wherein R₍₃₎ represents a hydrogen atom or a methyl group; R₍₃₁₎ represents a methyl group, an ethyl group, etc., which are capable of being hydrolyzed; R₍₃₂₎ represents CH₂CH₂R²R³; R² and R³ have the same meaning as defined above.]

Production method 3 is a method of obtaining the compound represented by formula (1) according to the present invention, which uses compound (3-1) as a raw material and performs [Step 3-1] and [Step 3-2]. Compounds (3-1) can be produced from a commercially available compound according to a method well-known to persons skilled in the art. Also, compound (3-2) can be produced from a commercially available compound according to a method well-known to person skilled in the art.

### [Step 3-1]

Step 3-1 is a step of producing compound (303) by using compound (3-1) and a compound (3-2) as raw materials and applying the method described in the above [Step 1-1].

### [Step 3-2]

Step 3-2 is a step of producing the compound represented by formula (1) by using compound (3-3) as a raw material and applying the method described in the above Step 1-2.

### [Production method 4]

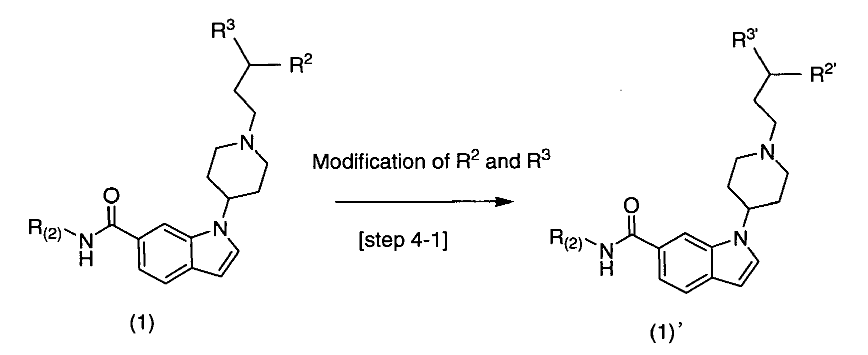

[wherein R(₂), R² and R³ have the same meaning as defined above; R²' and R³' represent R² and R³, respectively, which are appropriately modified.]

Production method 4 is a method for producing the compounds represented by formula (1)' from the compound represented by formula (1) as a raw material. Here, the compound represented by forumala (1)' is included in the compound represented by formula (1). Note that the compound represented by the formula (1) can be synthesized by the production method 1 described above.

### [Step 4-1]

Step 4-1 is a step of obtaining the compound represented by formula (1)' by modification of R2 and R3 in the compound represented by formula (1). The modification of R² and R³ can carried out by performing various reactions well-known to persons skilled in the art, or by the combined use of various reactions. The known reaction which can be used for modification of R² and R³, for examples, conversion (reduction) of carbonyl group into alcohol, conversion of a hydroxyl group into an ether group by use of halogenated alkyl group, conversion into a hydroxyl group from an alkoxy group, conversion (reduction) into amino group of a nitro group, conversion into an amide group from an amino group, etc.. In addition, the compound represented by formula (1)' can also be produced by methods described in the present examples.

Next, when the compound represented by formula (1) according to the present invention is synthesized, compound (1-9) used in [Production method 1] as a raw material will be described in detail. This compounds-(1-9) can be produced by [Production method A] or [Production method B]. The final compounds produced by these methods A and B may sometimes be indicated by different formulas to explain each step. However, all these compounds correspond to compound (1-9).

### [Production method A](synthesis of compound (1-9))

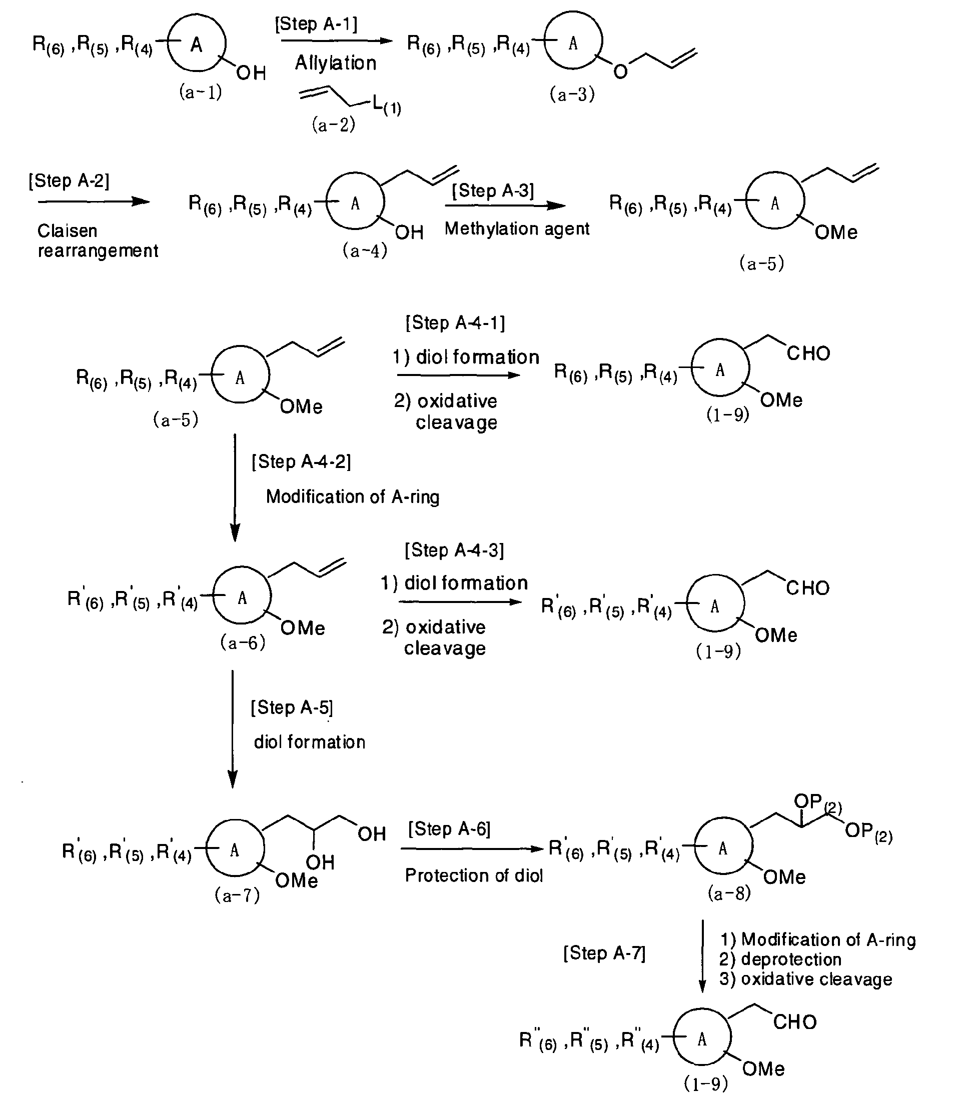

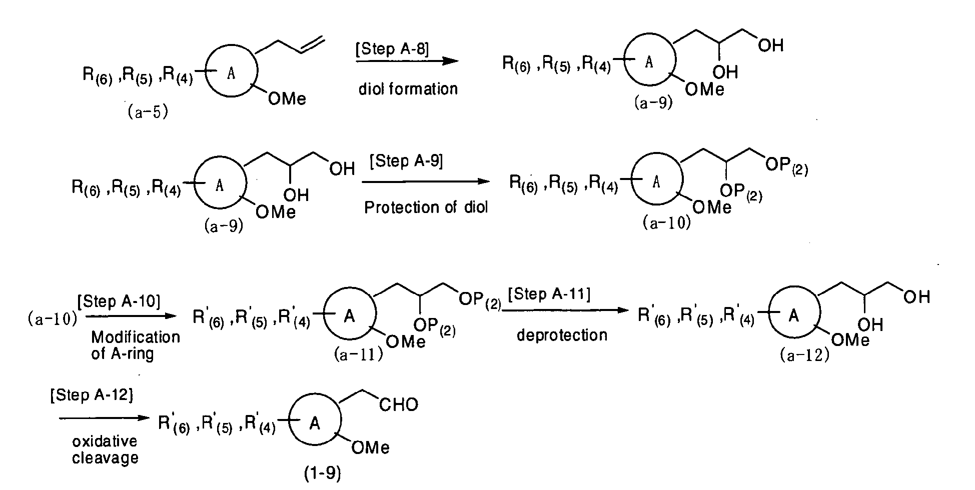

[wherein A ring represents a benzene ring, R₍₄₎ and R₍₅₎ and R₍₆₎ are the same as or different from each other and each represents the group selected from the following A1 group,
<A1 group>
   (1) a hydrogen atom, (2) a halogen atom, (3) a hydroxyl group, (4) a nitro group, (5) a cyano group, (6) a C₁₋₆ alkyl group (wherein said C₁₋₆ alkyl group may be substituted by a substituent selected from the group consisting of a hydroxyl group, a cyano group, a C₁₋₆ alkoxy group, a di (C₁₋₆ alkyl) amino group, and a di (C₁₋₆ alkyl) aminocarbonyl group),(7) a trifluoromethyl group, (8) a C₁₋₆ alkoxy group (wherein said C₁_₆ alkoxy group may be substituted by a substituent selected from the group consisting of a hydroxyl group, a C₁₋₆ alkoxy group, a di (C₁₋₆ alkyl) amino group, and a piperidyl group),
   (9) a phenyl group which may be substituted with one to three group selected from following B1 group, (10) a benzyloxy group which may be substituted with one to three groups selected from following B1 group, (11) a formula -NR²¹-R²² (wherein R²¹ represents a hydrogen atom or a C₁₋₆ alkyl group, R²² represents a hydrogen atom, a C₁₋₆ alkyl group, a C₂₋₇ alkylcarbonyl group, or a C₁₋₆ alkylsulfonyl group), (12) a formula -CO-R²³ (wherein R²³ represents a C₁₋₆ alkyl group or a di (C₁₋₆ alkyl) amino group, (13) a formula -X-R²⁴ (wherein R²⁴ represents a 5- or 6-membered heterocyclic group or heteroaryl group which may be substituted with the one to three groups selected from C1 group, and X represents a single bond and a -(CH₂)ₘ-group (m represents 1 to 3), an oxygen atom, a carbonyl group, a -(CH₂)ₙCO-group (n represents 1 to 3), or a -N (CH₃) - group, R₍₄₎', R₍₅₎', R₍₆₎', R₍₄₎", R₍₅₎" and R₍₆₎" represent a group in which R₍₄₎, R₍₅₎, and R₍₆₎ are suitably modified, L₍₁₎ represents a leaving group, for example, a halogen atom(a chlorine atom, a bromine atom, iodine atom) or sulfonyloxy hydroxy groups, such as or a methanesulfonyloxy group, p-toluenesulfonyloxy group and trifluoromethanesulfonyloxy group.
<B1 group>
   a C₁₋₆ alkyl group;
   a C₁₋₆ alkoxy group; ; and
   a halogen atom.
<C1 group>
   a C₁₋₆ alkyl group;
   a C₁₋₆ alkoxy group;
   a C₂₋₇ alkyl carbonyl group;
   a halogen atom;
   a benzyloxycarbonyl group; and
   a N-methyl acetamide group.

A commercially available compound can directly be used as compound (a-1), or the above compound can also be produced from a commercially available compound by a method known to persons skilled in the art. Moreover, it can be also produced by production examples in the present examples. A commercially available compound can directly be used as compound (a-2), or the above compound can also be produced from a commercially available compound by a method known to persons skilled in the art. Moreover, it can be also produced by production examples in the present examples.

### [Step A-1]

Step A-1 is a step of obtaining compound (a-3) by allylation reaction of compound (a-1) and compound (a-2) as a raw material. This reaction can be carried out under the same conditions as those used in the allylation reaction of allyl halide with a phenol derivative (including a heterocyclic ring) (which are conditions described in, for example, Nichols D.E.; Snyder, S.E. ; Oberlender, R.; Johnson,M,P.; Huang,X.; J. Med. Chem., 34 (1), 276-281 (1991), Sato,H.: Dan,T.; Onuma,E.; Tanaka,H.; Aoki, B. ; Koga,H.; Chem. Pharm. Bull., 39 (7), 1760-1772 (1991).

Specifically, a base is allowed to react with a solution containing compound (a-1) to obtain phenoxide, and the phenoxide compound is then allowed to react with compound (a-2), so as to obtain compound (a-3). This reaction can be carried out by allowing an appropriate base to react with the above compound at a ratio between 1 equivalent and an excess amount, in an organic solvent such as acetone, 2-butanone, acetonitrile, N,N-dimethylformamide, dimethyl sulfoxide, benzene or toluene, or a mixed solvent thereof. Examples of such a base used herein may include sodium carbonate, potassium carbonate, sodium hydroxide, potassium hydroxide, diazabicycloundecene, sodium hydride, potassium hydride, sodium methoxide, potassium methoxide, potassium *tert*-butoxide, etc..

Compound (a-2) is used against compound (a-1) at a ratio between 1 and 3 equivalents, preferably between 1 and 1.7 equivalents. The reaction time of this step is not particularly limited. The reaction time is generally between 1 and 48 hours, preferably between 1 and 24 hours. The reaction temperature of this step is generally between a temperature on ice and a solvent-reflux temperature. There may be cases where preferred results such as the improvement of yield or the reduction of the reaction time can be obtained by the coexistence of an ammonium salt such as tetra-*n-*butylammonium chloride, tetra-*n-*butylammonium bromide, or tetra-*n-*butylammonium iodide.

### [Step A-2]

Step A-2 is a step of obtaining compound (a-4) by subjecting compound (a-3) to Claisen rearrangement reaction. This reaction can be carried out under the same conditions as those described in, for example, Nichols, D. E.; Snyder, S. E.; Oberlender, R.; Johnson, M. P. ; Huang, X. ; J. Med. Chem., 34 (1), 276-281 (1991), Sato, H. ; Dan, T. ; Onuma, E. ; Tanaka, H. ; Aoki, B. ; Koga, H. ; Chem. Pharm. Bull., 39 (7), 1760-1772 (1991).

Specifically, a solution containing compound (a-3) is heated, so as to obtain compound (a-4). This reaction of this step can be carried out in the absence of solvent, or in an organic solvent such as N,N-dimethylaniline, N,N-diethylaniline, N-methylpyrrolidone, or dichlorobenzene. The reaction temperature of this step is generally between 100°C and a solvent-reflux temperature, preferably between 160°C and 210°C. Also, this reaction is preferably carried out in a nitrogen or argon atmosphere. There may be cases where preferred results such as the reduction of the reaction time or the improvement of yield can be obtained by performing this reaction using a microwave reactor.

Note that there may be cases where a regioisomer is synthesized in this reaction (Claisen rearrangement), although it depends on the type of a raw material. When an allyloxy group is defined at position 1, compounds formed by transferring an allyl group to any one of position 2, 4, or 6, are also included in the scope of present invention.

### [Step A-3]

Step A-3 is a step of obtaining compound (a-6) by the methylation reaction of compound (a-4) with compound (a-5). This reaction can be carried out under the same conditions as those used in the alkylation (methylation) reaction of a phenol derivative (including a heterocyclic ring) with a methyl halide or dimethyl sulfate (which are conditions described in, for example, Chilin, A.; Rodighiero, P.; Pastorini, G.; Guitto, A.; J. Org. Chem., 56 (3), 980-983 (1991), Dike, S. Y.; Merchant, J. R.; Sapre, N. Y.; Tetrahedron, 47 (26), 4775-4786 (1991)).

Specifically, a base is allowed to react with a solution containing compound (a-4) to obtain phenoxide, and the phenoxide compound is then allowed to react with compound (a-5), so as to obtain compound (a-6). This reaction can be carried out by allowing an appropriate base to react with the above compound at a ratio between 1 equivalent and an excess amount against compound (a-4), in an organic solvent such as acetone, 2-butanone, acetonitrile, N,N-dimethylformamide, dimethyl sulfoxide, benzene or toluene, or a mixed solvent thereof. Examples of such a base used herein may include sodium carbonate, potassium carbonate, sodium hydroxide, potassium hydroxide, diazabicycloundecene, sodium hydride, potassium hydride, sodium methoxide, potassium methoxide, potassium *tert*-butoxide, etc..

Examples of such a methylating reagent may include methyl iodide, methyl bromide, methyl chloride, and dimethyl sulfate. Compound (a-5) is used against compound (a-4) at a ratio between 1 and 5 equivalents, preferably between 1 and 3 equivalents. The reaction time of this step is not particularly limited. The reaction time is generally between 0.5 and 48 hours, preferably between 0.5 and 24 hours. The reaction temperature of this step is generally between a temperature on ice and a solvent-reflux temperature.

There may be cases where preferred results such as the improvement of yield or the reduction of the reaction time can be obtained by the coexistence of an ammonium salt such as tetra-*n-*butylammonium chloride, tetra-*n-*butylammonium bromide, tetra-*n-*butylammonium iodide, etc..

### [Step A-4-1]

Step A-4-1 is a step of obtaining compound (1-9) by the oxidative cleavage of olefin in the allyl part of compound (a-5). The reaction can be carried out under the same conditions as those commonly used in an oxidative cleavage reaction of obtaining aldehyde from olefin. An oxidative cleavage reaction used in the present reaction is not particularly limited. An oxidative cleavage reaction involving ozone oxidation, the use of osmium tetroxide (wherein an oxidizing agent may be used in combination), the use of K₂OsO₄ (wherein an oxidizing agent is used in combination), the use of chromic acid, or electrode oxidation, may be an example of such an oxidative cleavage reaction.

An oxidizing agent is used against compound (a-5) at a ratio between a catalytic amount (0.01 equivalent) and an excess amount. An oxidizing agent that is used in combination is used against the above oxidizing agent at a ratio between 1 equivalent and an excess amount.

Examples of such an oxidative cleavage reaction involving ozone oxidation may include methods described in, for example, Jagadeesh, S. G. ; Krupadanam, G. L. D. ; Srimannarayana, G.; Synth. Commun., 31 (10), 1547-1557 (2001), Cannon, J. G.; Roufos, I.; J. Heterocycl. Chem., 27 (7), 2093-2095 (1990). In an oxidative cleavage reaction involving the ozone oxidation of olefin, specifically, for example, oxygen-flow containing several percentage of ozone (prepared with an ozone generator) is applied to a solution containing compound (a-5), and then, the generated ozonide (hydroperoxide, when methanol is used as a solvent) is treated with a reducing agent without being isolated, so as to obtain compound (1-9).

A solvent used in the present reaction is not particularly limited, as long as it does not inhibit the reaction and dissolves a starting material to a certain extent. Preferred examples of the solvents may include methylene chloride, ethyl acetate, and methanol. The reaction temperature of this step is generally between -100°C and a room temperature, more preferably between -78°C and a room temperature. The reaction time of this step is not particularly limited. The reaction time is generally between 0.5 and 48 hours, preferably between 0.5 and 24 hours.

In addition, the method described in Lai, G. ; Anderson, W. K.; Tetrahedron Lett., 34 (43), 6849-6852 (1993) is an example of an oxidative cleavage reaction using osmium tetroxide (wherein an oxidizing agent may be used in combination), K₂OsO₄ (wherein an oxidizing agent is used in combination), AD-mix-α(β), etc.. The oxidative cleavage reaction of olefin using osmium tetroxide, etc. can be carried out under the same conditions as commonly used reaction conditions (for example, conditions described in the aforementioned publications). An oxidizing agent used in combination is not particularly limited. Examples of such an oxidizing agent may include sodium periodate, etc..

A solvent used herein may be a mixed solvent consisting of water and an organic solvent such as ether, tetrahydrofuran, 1,4-dioxane, or acetone. The reaction temperature is generally between a temperature on ice and a room temperature. An oxidative cleavage reaction using osmium tetroxide can also be carried out by two-step reaction, wherein olefin is oxidized with osmium tetroxide (that may be used together with an oxidizing agent) into 1,2-diol, and then aldehyde is obtained from the 1,2-diol using an oxidizing agent such as lead tetraacetate or sodium periodate. Such two-step reaction can be carried out under the same conditions as commonly used reaction conditions (for example, conditions described in Masquelin, T.; Hengartner, U.; Streith, J.; Synthesis, 7, 780-786 (1995), Banfield, S. C.; England, D. B. ; Kerr, M. A. ; Org. lett., 3 (21), 3325-3327 (2001)). Examples of an oxidizing agent used when olefin is converted into 1,2-diol may include N-methylmorpholine N-oxide, and K₃Fe(CN)₆. A solvent used herein is a mixed solvent consisting of water and an organic solvent such as acetonitrile, acetone, *tert*-butanol, or tetrahydrofuran. The reaction temperature is generally between a temperature on ice and a room temperature. The reaction time is not particularly limited. It is generally between 0.2 and 48 hours, preferably between 0.2 and 24 hours.

In addition, examples of such an oxidizing agent used when 1,2-diol is converted into aldehyde may include lead tetraacetate, sodium periodate, etc.. Examples of the solvent used herein may include organic solvents such as benzene, toluene, methylene chloride, ether, tetrahydrofuran, 1,4-dioxane, or acetone, and mixed solvents consisting of water and these organic solvents. The reaction temperature is generally between a temperature on ice and a room temperature. The reaction time is not particularly limited. The reaction time is generally between 5 minutes and 48 hours, preferably between 5 minutes and 24 hours.

### [Step A-4-2]

Step A-4-2 is a step of obtaining compound (a-6) by modifying (converting) ring A of compound (a-5) as appropriate. Modification (conversion) of ring A of compound (a-5) of the present invention can be carried out by performing various reactions known to persons skilled in the art, or by the combined use of various reactions. The above compound can also be produced by the method described in production examples in the present examples. The term "modification (conversion) of ring A" includes the modification (conversion) of a substituent (R₍₄₎, R₍₅₎, or R₍₆₎).

Specific examples of various reactions known to persons skilled in the art may include: an oxidation of converting alcohol into a carbonyl compound such as aldehyde or ketone; an oxidation of converting an aldehyde compound into carboxylic acid; a reduction of converting ester, carboxylic acid, or nitrile into aldehyde or alcohol; a nitration reaction of an aromatic ring; a halogenation of an aromatic ring; a reduction from a nitro group into an amino group; a reduction of a carbon-carbon double bond or a triple bond due to hydrogenation in the presence of a transition metal catalyst; an esterification of carboxylic acid; hydrolysis of an ester into carboxylic acid; synthesis of an aldehyde compound by hydrolysis of an enol ether compound; a conversion of hydrolyzing nitrile into an amide compound or carboxylic acid; a reduction of an amide compound into an amino compound; a hydroboration; an oximation of a carbonyl compound such as aldehyde or ketone; a nitrilation of an oxime group; an N-alkylation using a reductive amination; a method of synthesizing amides using an acylation of an amino group; a sulfonamidation of an amino group; an amidation by the condensation of a carboxylic acid compound and an amino compound; an amidation reaction by the condensation of an ester compound and an amino compound; an amidation by the condensation of acid chloride and an amino compound; a condensation between an amino group and a hydroxyl group, which uses N,N'-carbonyldiimidazole, phosgene, or triphosgene; a condensation between amide and a hydroxyl group, which uses N,N'-carbonyldiimidazole, phosgene, or triphosgene; a reaction of converting a hydroxyl group into fluorine using a DAST (dimethylaminosulfur trifluoride) reagent, etc.; an *O*-alkylation of alcohol or phenols; an N-alkylation of an amide group; an N-alkylation of an urethane compound; an alkylation of a carbonyl group into α-position by a reaction with alkyl halide following the treatment of a carbonyl compound with a base such as LDA (lithium diisopropyl amide) ; a demethylation reaction from an anisole derivative into a phenol derivative; a reaction of converting a hydroxyl group into a leaving group, such as mesylation or bromination of a hydroxyl group; a nucleophilic substitution reaction between a compound having a leaving group such as a bromo group and an amine compound; a nucleophilic substitution reaction between a compound having a leaving group such as a bromo group and sodium cyanide; a nucleophilic reaction of a carbonyl group with a Grignard reagent or alkyl or phenyl lithium; Wittig reaction; Horner-Emmons reaction; Mitsunobu reaction; Beckmann rearrangement; synthesis of benzoxazole by Beckmann rearrangement; Curtius rearrangement; Baeyer-Villiger reaction; Dieckmann condensation; a coupling reaction using a transition metal (for example, Suzuki coupling reaction, Ulmann-type coupling reaction, Sonogashira reaction), the coupling reaction of S. L. Buchwald et al. between an amino compound and halogenated aryl compounds, Stille coupling reaction, etc.); a reaction of synthesizing isoxazole by a 1,3-dipole addition; a reaction of synthesizing oxazole using an aldehyde compound and a TOSMIC reagent (tosylmethyl isocyanide); metallation due to halogen-metal exchange; a formylation or amidation due to the reaction between a metallated (lithiated) compound (lithiation, etc.) and a formylating agent such as N,N-dimethylformamide or an amidating agent such as dimethylcarbamoyl chloride; a reaction of converting a pyridine compound into a quaternary compound using methyl iodide or benzyl bromide; a reduction reaction of a quaternary pyridine compound into piperidine due to hydrogenation in the presence of a transition metal catalyst; a method of synthesizing a ketone compound due to the decarboxylation of a 1,3-ketoester compound; and protection and deprotection of various functional groups described in the publication, T. W. Green and P. G. M. Wuts, "Protective groups in Organic Chemistry, Second Edition", John Wiley & Sons (1991). However, examples are not limited to these reactions.

### [Step A-4-3]

Step A-4-3 is a step of producing compound (1-9) by using compound (a-6) as a raw material and applying the method described in said Step A-4-1.

### [Step A-5]

Step A-5 is a step of producing compound (a-7) by using compound (a-6) as a raw material and applying the method described in said Step A-4-1.

### [Step A-6]

Step A-6 is a step of obtaining compound (a-8) by protecting 1,2-diol of compound (a-7). The reaction can be carried out under the same conditions as those commonly used for the protection of 1,2-diol (for example, conditions described in publications such as T. W. Green and P. G. M. Wuts, "Protective groups in Organic Chemistry, Second Edition", John Wiley & Sons (1991), pp. 118-142).

Specifically, a protecting group (acetonide) can be introduced into 1,2-diol under conditions in which 2,2-dimethoxypropane, pyridinium p-toluenesulfonate at a catalytic amount, etc., are allowed to react with the above compound in an acetone solvent.

### [Step A-7]

Step A-7 is a step of obtaining compound (1-9) by using compound (a-8) as a raw material and applying the method described above Step A-4-2. By this method, ring A is modified (converted) as appropriate, the protecting group of 1,2-diol is deprotected, and oxidative cleavage is conducted, thereby obtaining compound (1-9).

The reaction can be carried out under the same conditions as those commonly used in the deprotection of 1,2-diol (for example, conditions described in publications such as T. W. Green and P. G. M. Wuts, "Protective groups in Organic Chemistry, Second Edition", John Wiley & Sons (1991), pp. 118-142). For example, 1,2-diol can be obtained by deprotecting the protecting group (acetonide) of 1,2-diol under conditions in which a 4N hydrogen chloride-ethyl acetate solution is allowed to act on the compound in an ethyl acetate solvent. Oxidative cleavage can be carried out using the method described in the said Step A-4-1.

### [Step A-8]

Step A-8 is a step of producing compound (a-9) by using compound (a-5) as a raw material and applying the method described in the said Step A-4-1.

### [Step A-9]

Step A-9 is a step of producing compound (a-10) by using compound (a-9) as a raw material and applying the method described in the said Step A-6.

### [Step A-10]

Step A-10 is a step of producing compound (a-11) by using compound (a-10) as a raw material and applying the method described in the said Step A-4-2.

### [Step A-11]

Step A-11 is a step of producing compound (a-12) by using compound (a-11) as a raw material and applying the method described in the said Step A-7.

### [Step A-12]

Step A-12 is a step of producing compound (1-9) by using compound (a-12) as a raw material and applying the method described in the said Step A-4-1.

[Production method B](synthesis method of compound (1-9))

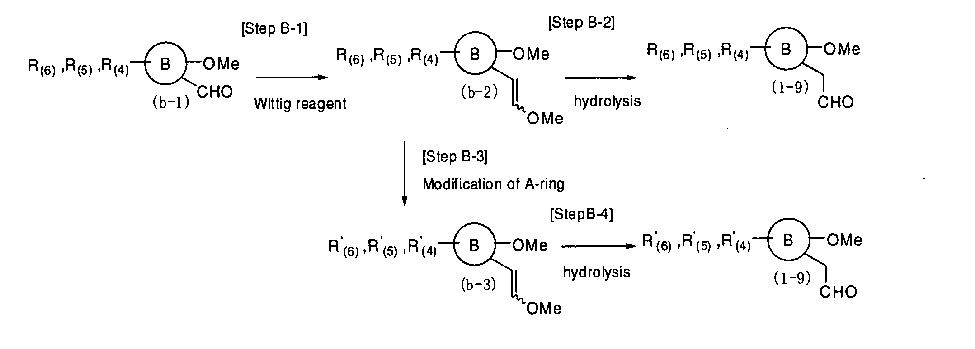

[wherein B ring represents a benzene ring, a pyridine ring, or a pyridone ring, R₍₄₎, R₍₅₎, R₍₆₎ R'₍₄₎, R'₍₅₎, and R'₍₆₎ have the same meaning described above.]

A commercially available compound may directly be used as compound (b-1), or the above compound may also be produced from a commercially available compound by a method known to persons skilled in the art. Moreover, it can also be produced using production examples in the present examples, etc..

### [Step B-1]

Step B-1 is a step of obtaining compound (b-2), which has one more carbon atom by Wittig reaction of compound (b-1). The reaction can be carried out under same conditions as those commonly used for aldehyde and a Wittig reagent (Wittig reaction) (methoxymethyltriphenylphosphonium chloride) (for example, conditions described in Gibson, S. E.; Guillo, N.; Middleton, R. J,; Thuilliez, A.; Tozer, M. J.; J. Chem. Soc., Perkin Trans. I, 4, 447-455 (1997)).

Specifically, for example, a Wittig reagent (methoxymethyltriphenylphosphonium chloride) is allowed to react with a base, and it is then allowed to react with compound (b-1), so as to obtain compound (b-2).

The reaction of this step can be carried out by allowing a base to act on a Wittig reagent at a ratio between 0.8 and 1 equivalent with respect to the reagent in an organic solvent such as ether, tetrahydrofuran, 1,4-dioxane, 1,2-dimethoxyethane, benzene, or toluene. Examples of such a base used herein may include sodium hydride, potassium hydride, sodium methoxide, potassium methoxide, potassium *tert*-butoxide, *n-*butyllithium, LDA (lithium diisopropylamide), etc..

The reaction time of this step is not particularly limited. The reaction time is generally between 5 minutes and 24 hours, preferably between 5 minutes and 12 hours. The reaction temperature of this step is generally between -78°C and a room temperature, more preferably between a temperature on ice and a room temperature.

### [Step B-2]

Step B-2 step of obtaining compound (1-9) by allowing compound (b-2) to react with acid. The reaction can be carried out under the same conditions as those described in, for example, Gibson, S. E.; Guillo, N.; Middleton, R. J.; Thuilliez, A.; Tozer, M. J.; J. Chem. Soc., Perkin Trans. I, 4, 447-455 (1997).

Specifically, for example, compound (b-2) is dissolved in 5N hydrochloric acid, etc. followed by heating, so as to obtain compound (1-9). The reaction can be carried out by allowing acid to react with the above compound at a ratio between 1 equivalent and an excess amount to the compound, in a mixed solvent consisting of water and an organic solvent such as methanol, ethanol, tetrahydrofuran, or 1,4-dioxane, or in an organic solvent such as methanol, ethanol, tetrahydrofuran, 1,4-dioxane, ethyl acetate, methylene chloride, or acetonitrile. Preferred examples of acid used herein may include hydrogen chloride, hydrochloric acid, hydrobromic acid, sulfuric acid, nitric acid, trifluoroacetic acid, and formic acid. In addition, it is also possible to convert the compound into aldehyde with trimethylsilyl iodide (which may be generated from trimethylsilyl chloride and sodium iodide in the reaction system).

The reaction time of this step is not particularly limited. The reaction time is generally between 0.5 and 24 hours, preferably between 0.5 and 12 hours. The reaction temperature of this step is generally between a temperature on ice and a solvent-reflux temperature.

### [Step B-3]

Step B-3 is a step of synthesizing compound (b-3) by appropriately modifying ring A, using compound (b-2) as a raw material and applying the above production method ([Step A-4-2]).

### [Step B-4]

Step B-4 is a step of producing compound (b-3) by appropriately modifying ring A, using compound (b-2) as a raw material and applying the above production method ([Step B-2]).

The compound represented by formula (1) according to the present invention or pharmacologically acceptable salts thereof, or hydrates thereof have excellent 5-HT_{1A} receptor antagonistic action and is useful as the therapeutic or preventive agent for lower urinary tract symptoms.

In the case of using the compounds represented by formula (1) according to the present invention or pharmacologically acceptable salts thereof, or hydrates thereof as an agent for treating or preventing the above-mentioned symptoms, said compounds can be formulated by common methods with itself or mixed with appropriate amount of pharmacologically acceptable additive, diluent, etc..

Preferred dosage forms in the present invention include a tablet, a powder, a parvule, a granule, a coated tablet, a capsule, a syrup, a troche, an inhalant, a suppository, an injection, an ointment, an eye drop, an eye ointment, a nasal drop, an ear drop, a poultice, and a lotion. For formulation, commonly used additives may be used. Examples of such an additive may include an excipient, a binder, a lubricant, a coloring agent, flavor, as well as, a stabilizer, an emulsifier, an adsorption enhancer, a surfactant, a pH regulator, an antiseptic, and antioxidant, as necessary. The above described agent can be formulated by mixing ingredients that are commonly used as raw materials for pharmaceutical formulations according to common methods. Examples of such ingredients may include; (1) animal or vegetable oils such as soybean oil, tallow, or synthetic glyceride; (2) hydrocarbons such as liquid paraffin, squalane, or solid paraffin; (3) ester oils such as octyldodecyl myristate or isopropyl myristate; (4) higher alcohols such as cetostearyl alcohol or behenyl alcohol; (5) silicone resins; (6) silicone oils; (7) surfactants such as polyoxyethylene fatty acid ester, sorbitan fatty acid ester, glycerin fatty acid ester, polyoxyethylene sorbitan fatty acid ester, polyoxyethylene hydrogenated castor oil, or a polyoxyethylene-polyoxypropylene block copolymer; (8) water soluble polymers such as hydroxyethyl cellulose, polyacrylic acid, a carboxyvinyl polymer, polyethylene glycol, polyvinylpyrrolidone, or methyl cellulose; (9) lower alcohols such as ethanol or isopropanol; (10) polyvalent alcohols such as glycerin, propylene glycol, dipropylene glycol, or sorbitol; (11) sugars such as glucose or sucrose; (12) inorganic powders such as silicic acid anhydride, magnesium aluminum silicate, or aluminum silicate; and (13) purified water.

Among above additives, the following are used respectively as (1) an excipient such as lactose, corn starch, saccharose, glucose, mannitol, sorbit, crystalline cellulose, and silicon dioxide, etc.; (2) a bindersuch as polyvinyl alcohol, polyvinyl ether, methylcellulose, ethylcellulose, gum Arabic, Tragacanth, gelatin, shellac, hydroxypropylmethylcellulose, hydroxypropylcellulose, polyvinylpyrrolidone, a polypropylene glycol-polyoxyethylene block polymer, and meglumine, etc.; (3) a disintegrant such as starch, agar, gelatin powder, crystalline cellulose, calcium carbonate, sodium bicarbonate, calcium citrate, dextrin, pectin, and carboxymethylcellulose calcium, etc.; (4) a lubricant such as magnesium stearate, talc, polyethylene glycol, silica, and hydrogenated vegetable oil, etc.; (5) all kind of coloring agent which is allowed for addition to pharmaceuticals; (6) flavor such as cocoa powder, menthol, aromatic powder, peppermint oil etc.; (7) a stabilizer such as p-hydroxybenzoate esters like methylparaben and propylparaben, alcohols like chlorobutanol, benzyl alcohol, and phenethyl alcohol, phenols like phenol and cresol, sorbic acid, etc.; (8) antioxidant such as an ascorbic acid and α-tocopherol which is allowed for addition to pharmaceuticals.

The dosage of the pharmaceutical composition according to the present invention varies depending on the degree of symptoms, age, sex, body weight, dosage form, the type of salts, sensitivity difference to medical agent, specific type of symptoms, etc.. In general, the pharmaceutical composition is orally administered once or divided over several administrations per day, at a dosage approximately between 30 µg and 10 g, preferably between 100 µg and 5 g, more preferably between 100 µg and 100 mg to adults. When the pharmaceutical composition is administered by injection, it is administered once or divided over several administrations per day, at a dosage approximately between 30 µg and 1 g, preferably between 100 µg and 500 mg, more preferably between 100 µg and 30 mg to adults.

### EXAMPLE

By illustrating Production examples, examples, test and pharmaceutical production examples as follows, the present invention will be explained in detail, although the scope of the present invention is not limited to these examples.

### Production example 1

### Synthesis of 1-(piperidin-4-yl)-1H-indole-6-carboxamide

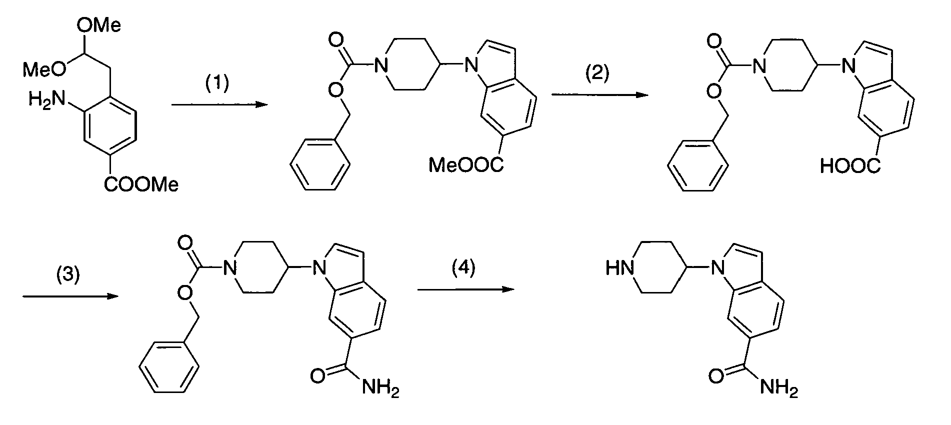

### (1) Synthesis of methyl

### 1-(1-benzyloxycarbonylpiperidin-4-yl)-1H-indole-6-carboxylate

44.3 g of methyl 3-amino-4-(2,2-dimethoxyethyl)benzoate synthesized according to the publication (Tetrahedron Letters, Vol. 37, No. 34, pp. 6045-6048) and 64.9 g of benzyl 4-oxo-1-piperidinecarboxylate were dissolved in 485 ml of acetic acid, followed by stirring at room temperature. Approximately 20 minutes later, 58.9 g of sodium triacetoxyborohydride was added to the reaction solution. Then, the reaction solution was further stirred for 2 hours. Thereafter, 485 ml of water was added to the reaction solution, and the resulting mixture was heated to a temperature between 100°C and 115°C. Approximately 3 hours later, the reaction solution was cooled, and then concentrated under a reduced pressure. Thereafter, water and ethyl acetate were added, so as to separate the organic layer. The resulting organic layer was washed with a saturated sodium bicarbonate aqueous solution and brine, and dried over anhydrous magnesium sulfate. After removing the drying agent by filtration, the organic layer was concentrated under a reduced pressure, and the residue was then purified by NH silica gel column chromatography (hexane / ethyl acetate). The resulting solid was suspended in a mixed solvent consisting of hexane and *tert*-butyl methyl ether, followed by filtration to give 64.6 g of the title compound.
¹H-NMR (CDCl₃)
δ: 1.80-2.05(m,2H), 2.05-2.23(m,2H), 2.92-3.15(m,2H), 3.96(s,3H), 4.30-4.60(m,3H), 5.18(s,2H), 6.58(dd, *J*=0.4, 2.8Hz,1H), 7.30-7.45(m,6H), 7.64(dd, J=0.4, 8.4Hz,1H), 7.80(dd, J=1.6,8.4Hz, 1H), 8.14(s, 1H).

### (2) Synthesis of

### 1-(1-benzyloxycarbonylpiperidin-4-yl)-1H-indole-6-carboxylic acid

90.0 g of methyl 1-(1-benzyloxycarbonylpiperidin-4-yl)-1*H*-indole-6-carboxylate was dissolved in a mixed solution consisting of 760 ml of methanol and 200 ml of tetrahydrofuran. Thereafter, 92 ml of a 5 N sodium hydroxide aqueous solution was added to the reaction solution, and the mixture was then heated to a temperature between 60°C and 70°C. After completion of the reaction, the reaction solution was cooled, and 65.0 g of ammonium chloride was added, followed by concentration under a reduced pressure. A 5% KHSO₄ aqueous solution was added to the residue, so as to adjust pH to be 5 to 6, followed by extraction with ethyl acetate. The organic layer was washed with water and brine, and then dried over anhydrous magnesium sulfate. After removing the drying agent by filtration, the organic layer was concentrated under a reduced pressure. Thereafter, the residue was solidified, and then collected from a mixed solvent consisting of hexane and *tert*-butyl methyl ether by filtration to give 75.6 g of the title compound.
¹H-NMR(CDCl3)
δ: 1.80-2.04(m,2H), 2.06-2.21(m,2H), 2.94-3.16(m,2H), 4.30-4.58(m,3H), 5.19(s,2H), 6.60(dd, *J*=0.8,3.6Hz,1H), 7.30-7.44(m,6H), 7.68(dd, *J*=0.8,8.4Hz,1H), 7.88(dd, *J*=1.6,8.4Hz,1H), 8.22(s,1H).

### (3) Synthesis of Benzyl 4-(6-carbamoyl-1H-indol-1-yl) piperidine-1-carboxylate

75.0 g of 1-(1-benzyloxycarbonylpiperidin-4-yl)-1H-indole-6-carboxylic acid was dissolved in 620 ml of tetrahydrofuran. Thereafter, 38.6 g of 1,1'-carbonylbis-1*H*-imidazole was added. The reaction solution was stirred at room temperature for 1.5 hours, and 134 ml of 28% ammonium water was then added. After completion of the reaction, the reaction solution was concentrated under a reduced pressure, followed by extraction with ethyl acetate. The resulting organic layer was washed with brine and a saturated ammonium chloride aqueous solution. Tetrahydrofuran was added to the separated organic layer, and a partially solidified title compound was dissolved therein, followed by drying over anhydrous magnesium sulfate. After removing the drying agent by filtration, the organic layer was concentrated under a reduced pressure. The solidified title compound was collected by filtration. The thus collected title compound was suspended in tetrahydrofuran, and the mixture was heated, followed by filtration. The thus collected title compound was then suspended in a mixed solvent consisting of tetrahydrofuran and methanol, and the resulting mixture was heated, followed by filtration. The generated filtrates were gathered and concentrated, and thus, the title compound was obtained in the same above manner. The total amount of the title compounds was 64.8 g.
¹H-NMR(CDCl3)
δ: 1.93(brs,2H), 2.04-2.18(m,2H), 3.02(brs,2H), 4.26-4.60(m,3H), 5.18(s,2H), 6.58(dd, J=0.8,3.2Hz,1H), 7.28-7.44(m,7H), 7.65(dd, *J*=0.4,8.4Hz, 1H), 8.10(s,1H)

### (4) Synthesis of 1-(piperidin-4-yl)-1H-indole-6-carboxamide

43 g of benzyl 4-(6-carbamoyl-1*H*-indol-1-yl)piperidine-1-carboxylate was suspended in a mixed solution consisting of 400 ml of methanol and 600 ml of tetrahydrofuran. Thereafter, 3.3 g of 10% palladium carbon was added. This suspension was substituted by hydrogen, which was then stirred at room temperature. After completion of the reaction, 10% palladium carbon was filtered off from the reaction solution, and the reaction solution was then concentrated under a reduced pressure. Tetrahydrofuran was added to the residue, and the resulting mixture was concentrated again under a reduced pressure. Tetrahydrofuran was added to the generated residue, followed by stirring, so as to solidify the title compound. Thereafter, tetrahydrofuran and ether were added followed by cooling on ice. The solidified title compound was collected by filtration. The generated filtrate was concentrated, and thus, the title compound was obtained in the same above manner. The total amount of the title compounds was 25.2 g.
¹H-NMR(CDCl3)
δ: 1.88-2.02(m,2H), 2.06-2.16(m,2H), 2.80-2.92(m,2H), 3.22-3.32(m,2H), 4.46(tt, J=4.0,12.0Hz,1H), 6.58(dd, *J*=0.8,3.2Hz, 1H), 7.36-7.44(m,2H), 7.65(d, *J*=8.4Hz, 1H), 8.11(s, 1H).

### Production example 2

### Synthesis of the N-methyl 1-(piperidin-4-yl) -1H-indole-6-carboxamide

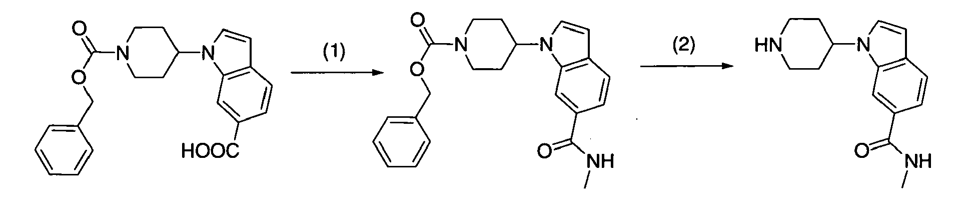

### (1) Synthesis of N-methyl

### 1-(1-benzyloxycarbonylpiperidin-4-yl)-1H-indole-6-carboxamide

2.00 g of 1-(1-benzyloxycarbonylpiperidin-4-yl)-1H-indole-6-carboxylic acid was dissolved in 20 ml of tetrahydrofuran, and 1.03 g of 1,1'-carbonylbis-1H-imidazole was then added. The resulting mixture was stirred at room temperature for 1.5 hours, and 4.11 ml of a 40% methylamine aqueous solution was added. After completion of the reaction, the reaction solution was extracted with ethyl acetate. The organic layer was washed with a saturated sodium bicarbonate aqueous solution, a saturated ammonium chloride aqueous solution, and brine. Thereafter, the organic layer was dried over anhydrous magnesium sulfate. After removing the drying agent by filtration, the organic layer was concentrated under a reduced pressure, and the residue was then purified by NH silica gel column chromatography (ethyl acetate) and silica gel column chromatography (hexane /ethyl acetate) to give 1.77 g of the title compound.
¹H-NMR(CDCl3)
δ: 1.80-2.00(m,2H), 2.03-2.17(m,2H), 2.90-3.10(m,2H), 3.06(d, J=4.8Hz,3H), 4.30-4.58(m,3H), 5.16(s,2H), 6.21(brs,1H), 6.55(dd, *J*=0.8,3.2Hz,1H), 7.27(d, *J*=3.6Hz,1H), 7.28-7.40(m,6H), 7.61(dd, *J*=0.8,8.0Hz,1H), 8.03(s,1H).

### (2) Synthesis of N-methyl

### 1-(piperidin-4-yl)-1H-indole-6-carboxamide

1.77 g of N-methyl1-(1-benzyloxycarbonylpiperidin-4-yl)-1H-indole-6-carboxamide was dissolved in 30 ml of methanol. Then, 200 mg of 10% palladium carbon was added to the resulting solution. The reaction atmosphere was replaced with hydrogen, which was then stirred at room temperature. After completion of the reaction, 10% palladium carbon was filtered off from the reaction solution, and the reaction solution was then concentrated under a reduced pressure. The residue was purified by NH silica gel column chromatography (ethyl acetate / methanol) , followed by solidification from a mixed solution consisting of ethyl acetate, *tert*-butyl methyl ether, and methanol to give 973 mg of the title compound.
¹H-NMR(CDCl3)
δ: 1.86-1.99(m, 2H), 2.06-2.14(m, 2H), 2.84(dt, *J*=2.4, 12.4Hz, 2H), 3.06(d, *J*=4.8Hz,3H), 3.23-3.30(m,2H), 4.44(tt, *J*=4.0, 12.0Hz, 1H), 6.24(brs,1H), 6.54(dd, *J*=0.8, 3.2Hz, 1H), 7.32-7.36(m,2H), 7.61(dd, *J*=0.4, 8.4Hz, 1H), 8.04(s,1H).

### Production example 3

### Synthesis of 3-amino-4-(2,2-dimethoxyethyl)benzamide

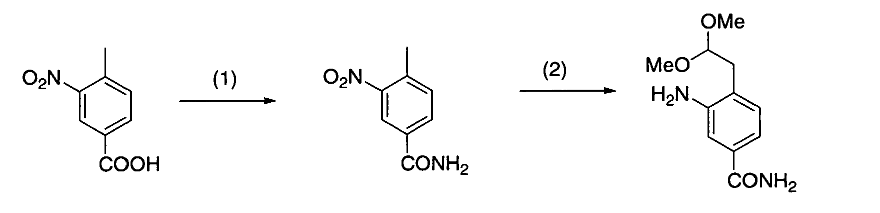

### (1) Synthesis of 3-nitro-4-methylbenzamide

20.0 g of 3-nitro-4-methylbenzoic acid was dissolved in 400 ml of tetrahydrofuran. Thereafter, 21.5 g of 1,1'-carbonyldiimidazole and 0.1 ml of dimethylformamide were added. The resulting mixture was stirred for 45 minutes. Thereafter, 20 ml of 28% ammonia water was added, followed by stirring at room temperature for 24 hours. After completion of the reaction, the reaction solution was concentrated under a reduced pressure, and the residue was separated into 600 ml of ethyl acetate and 200 ml of water. The organic layer was separated, and then washed with 200 ml of 2 N hydrochloric acid, 100 ml of water, 100 ml of a saturated sodium bicarbonate aqueous solution, and 100 ml of brine. It was then dried over anhydrous magnesium sulfate. After removing the drying agent by filtration, the filtrate was concentrated under a reduced pressure to give 19.5 of the title compound.
¹H-NMR(CDCl3) δ: 2.67(s,3H), 7.47(d, *J*=7.6Hz, 1H), 7.98(dd, *J*=7.6,2.0Hz,1H), 8.40(d, *J*=2.0Hz,1H).

### (2) Synthesis of 3-amino-4-(2,2-dimethoxyethyl)benzamide

19.5 g of 3-nitro-4-methylbenzamide and 30 g of dimethylformamide dimethyl acetal were dissolved in 200 ml of dimethylformamide. The resulting mixture was stirred at 140°C for 20 hours. The mixture was then concentrated under a reduced pressure. Thereafter, 360 ml of methanol and 25 g of chlorotrimethylsilane were added to the residue. The resulting solution was heated to reflux for 16 hours. The reaction solution was cooled and then concentrated under a reduced pressure. Water and ethyl acetate were added, so as to divide the organic layer. The organic layer was separated, and then washed with a saturated sodium bicarbonate aqueous solution and brine. It was then dried over anhydrous magnesium sulfate. The mixture was filtered through 100 g of a silica gel layer, and then washed with ethyl acetate. Thereafter, the filtrate was concentrated under a reduced pressure. 0.9 g of 10% palladium carbon was added to 150 ml of a methanol solution containing the resulting crude product, and the mixture was intensively stirred in a hydrogen atmosphere. After completion of the reaction, the catalyst was removed by filtration, and the filtrate was concentrated under a reduced pressure. The resulting residue was purified by NH silica gel column chromatography (hexane / ethyl acetate) to give 11.5 g of the title compound.
¹H-NMR(CDCl3)
δ: 2.90(d, *J*=5.2Hz,2H), 3.38(s,6H), 4.16-4.24(br,1H), 4.99(t, *J*=5.2Hz,3H), 5.52-5.67(br,1H), 5.93-6.10(br,1H) 7.07(dd, *J*=1.6,7.6Hz,1H), 7.10(d, *J*=7.6Hz,1H), 7.18(d, *J*=1.6Hz,1H).

### Production example 4

### (5) Synthesis of 4-(2-bromo-6-methoxybenzyl)-2,2-dimethyl-[1,3]dioxolane

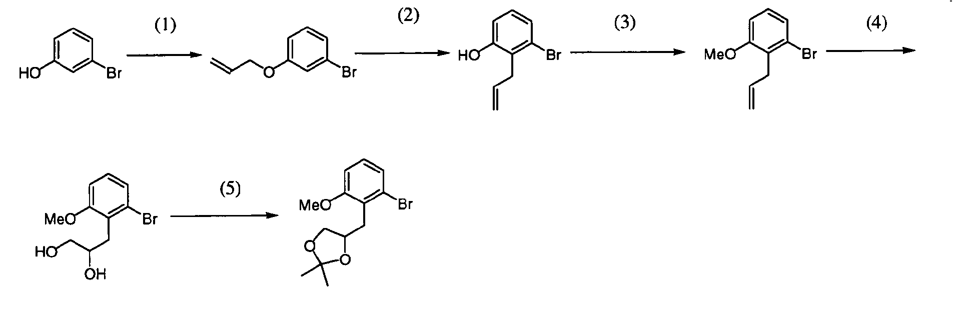

### (1) Synthesis of 1-allyloxy-3-bromobenzene

25.2g of 3-bromophenol was dissolved in 100ml of N,N-dimethylformamide, 20.3g of potassium carbonate and 19ml of allyl bromide were added at room temperature. This reaction liquid was stirred at room temperature for 5 hours. Water and ethyl acetate were added to the reaction liquid, and the organic layer was separated. The resulting organic layer was washed with brine and then dried over anhydrous sodium sulfate. After removing the drying agent by filtration, the organic layer was concentrated under a reduced pressure, and the residue was purified by NH silica gel column chromatography (hexane-ethyl acetate) to give 28.8g of the title compound.
¹H-NMR(CDCl3)
δ (ppm): 4.50-4.52(m,2H), 5.29(dd, *J*=10.0,1.6Hz, 1H), 5.40(dd, *J*=17.2,1.6Hz, 1H), 5.95-6.08(m,1H), 6.82-6.85(m,1H), 7.05-7.08(m,3H), 7.12(dd, *J*=8.4,8.0Hz, 1H).

### (2) Synthesis of 2-allyl-3-bromophenol

28.8g of 1-allyloxy-3-bromobenzene was dissolved in 60ml of diethylaniline under nitrogen atmosphere and the mixture was heated to reflux for 4 hours. Ethyl acetate was added after standing to cool the reaction liquid and washed with 5N HCl three times. The resulting organic layer washed additionally with water three times was washed with brine and then dried over anhydrous sodium sulfate. After removing the drying agent by filtration, the organic layer was concentrated under a reduced pressure, and the residue was purified by silica gel column chromatography (hexane-ethyl acetate) to give 20.2g of the title compound. 10.6g of 2-allyl-5-bromophenol which was regioisomer was also obtained.
¹H-NMR(CDCl3)
δ (ppm): 3.60-3.64(m,2H), 5.08-5.15(m,2H), 5.26 (s,1H), 5.90-6.02 (m,1H), 6.75(dd, J=8.4,0.8Hz, 1H), 6.96(dd, *J*=8.4,8.0Hz, 1H), 7.15(dd, *J*=8.0,0.8Hz, 1H).

### 2-allyl-5-bromophenol

¹H-NMR(CDCl3)
δ (ppm) : 3.35(d, *J*=6.4Hz, 2H), 5.10-5.20 (m,3H), 5.92-6.02 (m,1H), 6.93-7.03(m,3H).

### (3) Synthesis of 1-allyl-4-bromo-2-methoxybenzene

12.2g of 2-allyl-3-bromophenol was dissolved in 50ml of N,N-dimethylformamide, 8g of potassium carbonate and 7.2ml of methyl iodide were added at room temperature. This reaction liquid was stirred overnight at room temperature. Water and ethyl acetate were added to the reaction liquid, and the organic layer was separated. The resulting organic layer was washed with brine and then dried over anhydrous sodium sulfate. After removing the drying agent by filtration, the organic layer was concentrated under a reduced pressure, and the residue was purified by silica gel column chromatography (hexane-ethyl acetate) to give 8.4g of the title compound.
¹H-NMR(CDCl3)
δ (ppm) : 3.57 (td, *J*= 6*.*0, 1.6Hz, 2H), 3.81 (s,3H), 4.97-5.05 (m, 2H), 5.85-5.95(m, 1H), 6.79 (d, J=8.4Hz, 1H), 7.03(dd, *J=* 8.4, 8.0Hz, 1H), 7.15(dd, J=8.0,1.2Hz, 1H).

### (4) Synthesis of 3-(2-bromo-6-methoxyphenyl)propane-1,2-diol

21g of AD-mix-β was suspended to 80ml of *tert*-butanol and 80ml of water, 4.7g of 1-allyl-4-bromo-2-methoxybenzene dissolved in 15ml of *tert*-butanol was added at room temperature. This reaction liquid was stirred overnight at room temperature. 24.8g of sodium sulfite was added to the reaction liquid, which was stirred at room temperature for 1 hour. Then water and ethyl acetate were added to reaction liquid, and the organic layer was separated. The resulting organic layer was washed with brine and then dried over anhydrous sodium sulfate. After removing the drying agent by filtration, the organic layer was concentrated under a reduced pressure to give 8.4g of the title compound.
¹H-NMR(CDCl3)
δ (ppm): 2.14-2.20(m,1H), 2.39(d, *J*=5.6Hz, 1H), 3.03(dd, *J*=13.6,6.4Hz, 1H), 3.10(dd, *J*=13.6,7.2Hz, 1H), 3.48-3.58(m,1H), 3.62-3.69(m,1H), 3.85(s, 3H), 3.96-4.05(m,1H), 6.83(d, *J*=7.6Hz, 1H), 7.07(dd, *J*=8.0,7.6Hz, 1H), 7.19(d, *J*=8.0Hz, 1H).

### (5) Synthesis of 4-(2-bromo-6-methoxybenzyl)-2,2-dimethyl -[1,3]dioxolane

8.4g of 3-(2-bromo-6-methoxyphenyl)propane-1,2-diol was dissolved in 200ml of acetone, 7.7ml of 2,2-dimethoxypropane and 402mg of p-toluenesulfonic acid monohydrate were added at room temperature. This reaction liquid was stirred at room temperature under nitrogen atmosphere overnight. The solvent was concentrated under a reduced pressure, water and ethyl acetate were added, and the organic layer was separated. The resulting organic layer was washed with brine and then dried over anhydrous sodium sulfate. After removing the drying agent by filtration, the organic layer was concentrated under a reduced pressure, and the residue was purified by silica gel column chromatography (hexane-ethyl acetate) to give 6.6g of the title compound.
¹H-NMR(CDCl3)
δ (ppm): 1.35(s,3H), 1.48(s,3H), 3.10(dd, *J*=13,8.4Hz, 1H), 3.21(dd, *J*=13,5.2Hz, 1H), 3.80-3.84(m,1H), 3.82(s,3H), 3.89(dd, *J*=8.0,5.6Hz, 1H), 4.37-4.44(m,1H), 6.79(dd, *J*=8.0,1.2Hz, 1H), 7.06(dd, *J*=8.4,8.0Hz, 1H), 7.15(dd, *J*=8.4,1.2Hz, 1H).

### Production example 5

### Synthesis of the N-(2-allyl-3-methoxyphenyl)acetamide

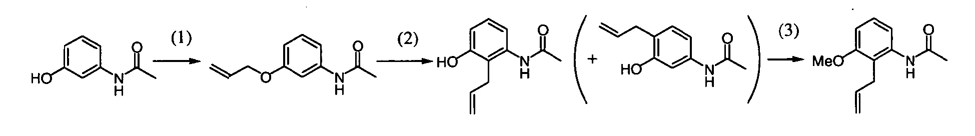

### (1) Synthesis of the N-(3-allyloxyphenyl)acetamide

14.2g of N-(3-hydroxyphenyl)acetamide was dissolved in 70ml of N,N-dimethylformamide, 13g of potassium carbonate and 12.2ml of allyl bromide were added at room temperature. This reaction liquid was stirred overnight at room temperature. Water and ethyl acetate were added to reaction liquid, and the organic layer was separated. The resulting organic layer was washed with brine and then dried over anhydrous sodium sulfate. After removing the drying agent by filtration, the organic layer was concentrated under a reduced pressure, and the residue was purified by NH silica gel column chromatography (hexane-ethyl acetate) to give 20.6g of the title compound.
¹H-NMR(CDCl3)
δ (ppm): 2.16(s,3H), 4.52(td, *J*=5.2,1.6Hz, 2H), 5.27 (dd, *J*=10.4,1.6Hz, 1H), 5.40(dd, *J*=21.2,1.6Hz, 1H), 6.03(ddd, *J*=21.2,10.4Hz,5.2Hz, 1H), 6.67 (d, *J*=8.0Hz, 1H), 6.95 (d, *J*=8.4Hz, 1H), 7.18(dd, *J*=8.4,8.0Hz, 1H), 7.42(br,1H).

### (2) Synthesis of the N-(2-allyl-3-hydroxyphenyl)acetamide

20.6g of N-(3-allyloxyphenyl)acetamide was dissolved in 50ml of dimethylaniline under nitrogen atmosphere, which was heated to reflux for 5 hours. Ethyl acetate was added after standing to cool the reaction liquid and washed with 5N HCl three times. The resulting organic layer washed additionally with water three times was washed with brine and then dried over anhydrous sodium sulfate. After removing the drying agent by filtration, the organic layer was concentrated under a reduced pressure, and the residue was purified by silica gel column chromatography (hexane-ethyl acetate) to give 12.3g of a mixture of the title compound and the corresponding regioisomer, N-(4-allyl-3-methoxyphenyl)acetamide. Compound was used in the next reaction without further purification.

### (3) Synthesis of the N-(2-allyl-3-methoxyphenyl)acetamide

7. 7g of a mixture of N- (2-allyl-3-hydroxyphenyl) acetamide and the N-(4-allyl-3-hydroxyphenyl)acetamide was dissolved in 50ml of N,N-dimethylformamide, 5.8g of potassium carbonate and 5.1ml of methyl iodide were added at room temperature. This reaction liquid was stirred overnight at room temperature. Water and ethyl acetate were added to reaction liquid, and the organic layer was separated. The resulting organic layer was washed with brine and then dried over anhydrous sodium sulfate. After removing the drying agent by filtration, the organic layer was concentrated under a reduced pressure, and the residue was purified by silica gel column chromatography (hexane-ethyl acetate) to give 1.6g of the title compound. 4.2g of the corresponding regioisomer, N-(4-allyl-3-methoxyphenyl) acetamide was also obtained.
¹H-NMR(CDCl3)
δ (ppm): 2.15(s,3H), 3.45(d, J=6.0Hz, 2H), 3.81(s,3H), 5.02(d, *J*=17.2Hz, 1H), 5.11(d, *J*=10.0Hz, 1H), 5.88-6.00(m,1H), 6.71(d, J=8.4Hz, 1H), 7.21(dd, *J*=8.4,8.0Hz, 1H), 7.50(d, *J*=8.0Hz, 1H).
<regioisomer = N-(4-allyl-3-methoxyphenyl)acetamide>
¹H-NMR(CDCl3)
δ (ppm): 2.16(s,3H), 3.32(d, *J*=7.4Hz, 2H), 3.81(s,3H), 4.98-5.05(m,2H), 5.89-6.00 (m,1H), 6.76(dd, *J*=8.0,2.4Hz, 1H), 7.03(d, *J*=8.0Hz, 1H), 7.18 (br,1H), 7.36(d, *J*=2.4Hz, 1H).

### Production example 6

### Synthesis of 4-(3-bromo-6-methoxybenzyl)-2,2-dimethyl-[1,3]dioxolane

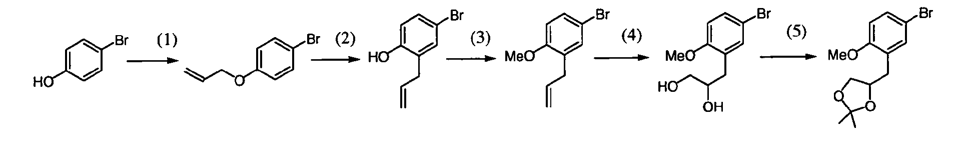

### (1) Synthesis of 1-allyloxy-4-bromobenzene

19.9g of 4-bromophenol was dissolved in 100ml of N,N-dimethylformamide, 19.2g of potassium carbonate and 15ml of allyl bromide were added at room temperature. This reaction liquid was stirred overnight at room temperature. Water and ethyl acetate were added to reaction liquid, and the organic layer was separated. The resulting organic layer was washed with brine and then dried over anhydrous sodium sulfate. After removing the drying agent by filtration, the organic layer was concentrated under a reduced pressure, and the residue was purified by silica gel column chromatography (hexane-ethyl acetate) to give 26.8g of the title compound.
¹H-NMR(CDCl3)
δ (ppm) : 4.48-4.54 (m, 2H), 5.29 (d, *J*=10.0Hz, 1H), 5.40 (d, J=17.2Hz, 1H), 5. 96-6.08 (m, 1H), 6.78 (d, *J*=8.4Hz, 2H), 7.36 (d, J=8.4Hz, 2H).

### (2) The synthesis of 2-allyl-4-bromophenol

26.8g of 1-allyloxy-4-bromobenzene was dissolved in 60ml of dimethylaniline under nitrogen atmosphere, which was heated to reflux for 10 hours. Ethyl acetate was added after standing to cool with reaction liquid and washed with 5N HCl twice. The resulting organic layer washed additionally with water twice was washed with brine and then dried over anhydrous sodium sulfate. After removing the drying agent by filtration, the organic layer was concentrated under a reduced pressure, and the residue was purified by silica gel column chromatography (hexane-ethyl acetate) to give 19.2g of the title compound.
¹H-NMR(CDCl3)
δ (ppm) : 3.36(d, *J*=6.4Hz, 2H), 5.12-5.19 (m, 3H), 5.93-6.05 (m,1H), 6.69(dd, *J*=7.6,1.2Hz, 1H), 7.18-7.23(m,2H).

### (3) Synthesis of 2-allyl-4-bromo-1-methoxybenzene

19.2g of 2-allyl-4-bromophenol was dissolved in 50ml of N,N-dimethylformamide, 13g of potassium carbonate and 9ml of methyl iodide were added at room temperature. This reaction liquid was stirred overnight at room temperature. Water and ethyl acetate were added to reaction liquid, and the organic layer was separated. The resulting organic layer was washed with brine and then dried over anhydrous sodium sulfate. After removing the drying agent by filtration, the organic layer was concentrated under a reduced pressure, and the residue was purified by silica gel column chromatography (hexane-ethyl acetate) to give 14.8g of the title compound.
¹H-NMR(CDCl3)
δ (ppm) : 3.33 (dd, *J*=5.2,1.2Hz, 2H), 3.80 (s,3H), 5.02-5.08 (m, 2H), 5.78-5.98 (m,1H), 6.71(d, *J*=7.2Hz, 1H), 7.22-7.29(m,2H).

### (4) Synthesis of 3-(3-bromo-6-methoxyphenyl)propane-1,2-diol

51.6g of AD-mix-β was suspended to 160ml of *tert*-butanol and 160ml of water, 11g of 2-allyl-4-bromo-1-methoxybenzene dissolved in 40ml of *tert*-butanol was added at room temperature. This reaction liquid was stirred overnight at room temperature. 53g of sodium sulfite was added to reaction liquid, which was stirred at room temperature for 1 hour. Water and ethyl acetate were added to reaction liquid, and then the organic layer was separated. The resulting organic layer was washed with brine and then dried over anhydrous sodium sulfate. After removing the drying agent by filtration, the organic layer was concentrated under a reduced pressure to afford 21.5g of the title compound. This compound was used in the next reaction without further purification.

### (5) Synthesis of 4-(3-bromo-6-methoxybenzyl)-2,2-dimethyl-[1,3]dioxolane

21.5g of 3-(3-bromo-6-methoxyphenyl)propane-1,2-diol was dissolved in 300ml of acetone, 30ml of 2,2-dimethoxypropane and 852mg of p-toluenesulfonic acid monohydrate were added at room temperature. This reaction liquid was stirred at room temperature under nitrogen atmosphere overnight. The solvent was concentrated under a reduced pressure, water and ethyl acetate were added, and the organic layer was separated. The resulting organic layer was washed with brine and then dried over anhydrous sodium sulfate. After removing the drying agent by filtration, the organic layer was concentrated under a reduced pressure, and the residue was purified by silica gel column chromatography (hexane-ethyl acetate) to give 13.9g of the title compound.
¹H-NMR (CDCl3)
δ (ppm) : 1.35(s,3H), 1.42(s,3H), 2.78(dd, *J*=14.0, 6.8Hz,1H), 2.91(dd, *J*=14.0,6.0Hz, 1H), 3.63(dd, *J*=8.0,6.8Hz, 1H), 3.79(s,3H), 3.95(dd, *J*=8.0, 6.0Hz,1H), 4.33(tt, *J*=6.8,6.0Hz, 1H), 6.70(d, *J*=9.2Hz, 1H), 7.27-7.31(m,2H).

### Production example 7

### Synthesis of 4-benzyloxycarbonylmethylaminopiperidine

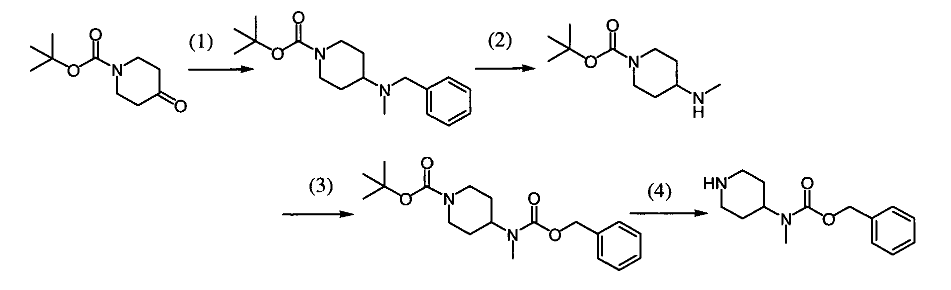

### (1) Synthesis of 4-benzylmethylamino-1-tert-butoxycarbonyl piperidine

10.1g of 1-*tert*-butoxycarbonylpiperidin-4-one and 9.8ml of benzylmethylamine were dissolved into 100ml of dichloromethane, 5.8ml of acetic acid and 16g of sodium triacetoxyborohydride were added to the reaction liquid, the reaction liquid was stirred overnight at room temperature. Aqueous sodium hydroxide, dichloromethane were added to the reaction liquid, and the organic layer was separated. The resulting organic layer was washed with brine and then dried over anhydrous sodium sulfate. After removal of the drying agent by filtration, the organic layer was concentrated under a reduced pressure. The residue was purified by NH silica gel column chromatography (hexane-ethyl acetate) to give 10.5g of the title compound.
¹H-NMR(CDCl3)
δ (ppm): 1.45(s,9H), 1.45-1.60(m,2H), 1.75-1.83(m,2H), 2.18(s,3H), 2.52-2.62(m,1H), 2.62-2.74(m,2H), 3.56(s,2H), 4.08-4.21(m,2H), 7.20-7.32(m,5H).

### (2) Synthesis of 4-methylamino-1-tert-butoxycarbonyl piperidine

10.5g of 4-benzylmethylamino-1-*tert-*butoxycarbonyl piperidine was dissolved in 100ml of methanol, and catalytic amount of palladium hydroxide-carbon was added. The mixture was stirred under hydrogen atmosphere at room temperature overnight. Hydrogen in the reaction system was replaced with nitrogen, catalyst was removed by filtration through filter paper. The solvent was concentrated under a reduced pressure to afford 6.97g of the title compound.
¹H-NMR(CDCl3)
δ (ppm): 1.17-1.28(m,2H), 1.44(s,9H), 1.80-1.88(m,2H), 2.42(s,3H), 2.45-2.54(m,1H), 2.72-2.84(m,2H), 3.94-4.10(m,2H).

### (3) Synthesis of 4-benzyloxycarbonylmethylamino -1-tert-butoxycarbonylpiperidine

6.97g of 4-methylamino-1-*tert*-butoxycarbonylpiperidine and 9ml of triethylamine were dissolved into 100ml of dichloromethane, 33ml of benzyl chloroformate was added in an ice-cooling. An ice bath was removed, and the reaction liquid was stirred overnight at room temperature. Water and chloroform were added to the reaction liquid, and the organic layer was separated. The resulting organic layer was washed with brine and then dried over anhydrous sodium sulfate. After removing the drying agent by filtration, the organic layer was concentrated under a reduced pressure, the residue was purified by silica gel column chromatography (hexane-ethyl acetate) to give 10.8g of the title compound.
¹H-NMR (CDCl3) 5 (ppm): 1.46(s,9H), 1.55-1.67(m,4H), 2.65-2.83(m,5H), 4.08-4.24(m,3H), 5.10(s,2H), 7.26-7.36(m,5H).

### (4) Synthesis of 4-benzyloxycarbonylmethylaminopiperidine

4.43g of 4-benzyloxycarbonylmethylamino-1-*tert*-butoxycarbonyl piperidine was dissolved in 30ml of methanol, 10ml of 4N HCl ethyl acetate solution was added at room temperature, which was stirred overnight at room temperature. A saturated sodium carbonate aqueous solution and ethyl acetate was added to this reaction liquid, the organic layer was separated. The resulting organic layer was washed with brine and then dried over anhydrous sodium sulfate. After removing the drying agent by filtration, the organic layer was concentrated under a reduced pressure, the residue was purified by NH silica gel column chromatography (ethyl acetate / methanol) to give 2.76g of the title compound.
¹H-NMR(CDCl3)
δ (ppm): 1.60-1.70(m,4H), 2.60-2.76(m,2H), 2.82(s,3H), 3.08-3.16(m,2H), 4.03-4.20(m,1H), 5.15(s,2H), 7.28-7.40(m,5H).

### Example 1

### Synthesis of 1-[1-[2-(5-acetyl-2-methoxyphenyl)-ethyl] piperidin-4-yl]-1H-indole-6-carboxamide

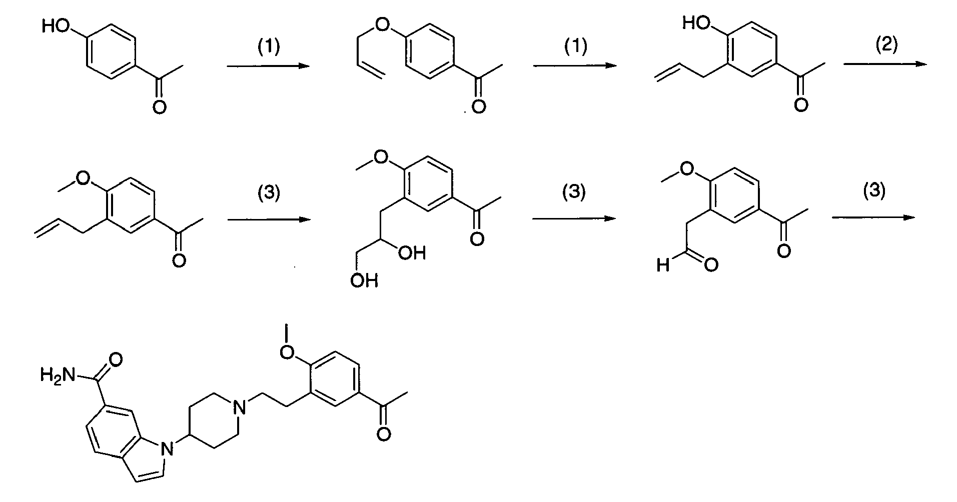

### (1) Synthesis of 3-allyl-4-hydroxyacetophenone

8.81g of 4-hydroxyacetophenone was dissolved in 90ml of N,N-dimethylformamide, 13.4g of potassium carbonate and 9.39g of allyl bromide were added, and the reaction liquid was stirred overnight. The reaction liquid was diluted in ethyl acetate, and washed with saturated ammonium aqueous solution and brine. The organic layer was concentrated under a reduced pressure after drying over magnesium sulfate, and purified by silica gel column chromatography (hexane-ethyl acetate) to give 11.3g of 4-allyloxyacetophenone.
Under nitrogen atmosphere, 11.3g of 4-allyloxyacetophenone was dissolved in 20ml of N,N-dimethylaniline, and heated to reflux for 12 hours. The reaction liquid was diluted in ethyl acetate after standing to cool at room temperature, and washed with 5N HCl, water, saturated sodium bicarbonate aqueous solution and brine. The organic layer was concentrated under a reduced pressure after drying over magnesium sulfate, and purified by silica gel column chromatography (hexane-ethyl acetate), then solidified by hexane-diethyl ether, to give 8.68g of the title compound.
¹H-NMR (CDCl3)
δ: 2.55(s,3H), 3.44-3.48(m,2H), 5.16-5.23(m,2H), 5.54 (s,1H), 5.96-6.07 (m,1H), 6.85 (d, *J*=8.8Hz,1H), 7.76-7.81 (m,2H).

### (2) Synthesis of 3-allyl-4-methoxyacetophenone

8.68g of 3-allyl-4-hydroxyacetophenone was dissolved in 150ml of N,N-dimethylformamide, 10.2g of potassium carbonate and 10. 5g of iodomethane were added and stirred overnight. Reaction liquid was diluted in ethyl acetate and washed with saturated ammonium aqueous solution and brine. The organic layer was concentrated under a reduced pressure after drying over magnesium sulfate, and the residue was purified by silica gel column chromatography (hexane-ethyl acetate) to give 8.94g of the title compound.
¹H-NMR(CDCl3)
δ: 2.56 (s, 3H), 3.41(d, *J*=6.8Hz,2H), 3.90 (s,3H), 5.03-5.10(m,2H), 5.93-6.04(m,1H), 6.88(d, *J*=8.4Hz,1H), 7.78(d, *J*=2.4Hz,1H), 7.85(dd, *J*=2.4,8.4Hz,1H).

### (3) Synthesis of 1-[1-[2-(5-acetyl-2-methoxyphenyl)ethyl] piperidin-4-yl]-1H-indole-6-carboxamide

212mg of 3-allyl-4-methoxyacetophenone was dissolved in 14ml of *tert*-butanol-water (1:1), 1.56g of AD-mix-β was added, and the mixture was stirred overnight at room temperature. In an ice-cooling, 1. 67g of sodium sulfite was added, and the mixture was stirred for 1 hour at room temperature. The reaction liquid was diluted in ethyl acetate and washed with brine. The organic layer was concentrated under a reduced pressure after drying over magnesium sulfate, and 224mg of 3-(5-acetyl-2-methoxyphenyl)propane-1,2-diol was obtained. This compound was used in the next reaction without further purification.
224mg of 3-(5-acetyl-2-methoxyphenyl)propane-1,2-diol was dissolved in 3ml of tetrahydrofuran and 7ml of methanol, aqueous 7ml solution of 0.43g of sodium metaperiodate was added in an ice-cooling and the mixture was stirred for 30 minutes at room temperature. The reaction liquid was diluted in ethyl acetate and washed with water and brine. The organic layer was concentrated under a reduced pressure after drying over magnesium sulfate to afford 177mg of (5-acetyl-2-methoxyphenyl)acetaldehyde. This compound was used in the next reaction without further purification. 190mg of 1-(piperidin-4-yl)-1*H*-indole-6-carboxamide synthesized in production example 1 and 177mg of (5-acetyl-2-methoxyphenyl)acetaldehyde were dissolved in 10ml of methylene chloride, 0.09ml of acetic acid was added, and stirred for 20 minutes at room temperature. 0.25g of sodium triacetoxyborohydride was added afterwards and stirred for 3 hours at room temperature. Saturated sodium bicarbonate aqueous solution was added to reaction liquid and the reaction liquid was extracted with chloroform. The extract was concentrated under reduced pressure after drying over magnesium sulfate, and purified by silica gel column chromatography (methanol-ethyl acetate) to give 117mg of the title compound.
¹H-NMR(DMSO-d6)
δ: 1.93-2.08(m,4H), 2.23-2.32(m,2H), 2.52-2.61(m,2H), 2.53(s,3H), 2.79-2.85(m,2H), 3.09-3.15(m,2H), 3.89(s,3H), 4.37-4.47 (m,1H), 6.50(d, *J*=3.2Hz,1H), 7.07(d, *J*=8.8Hz,1H), 7.21(brs,1H), 7.54-7.60 (m,2H), 7.67 (d, *J*=3.2Hz,1H), 7.82(d, *J*=2.4Hz,1H), 7.87(dd, *J*=2.4,8.8Hz,1H), 7.91 (brs,1H), 8.13 (s,1H).

### Example 2

### Synthesis of 1-[1-[2-(2-methoxy-5-trifluoromethylphenyl)-ethyl]piperidin-4-yl]-1H-indole-6-carboxamide

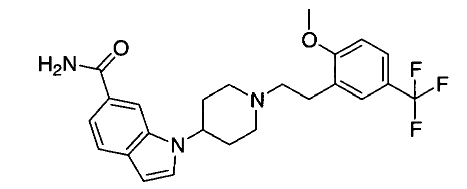

Example 1 was followed from 4-hydroxybenzotrifluoride, the title compound was obtained.
¹H-NMR(DMSO-d6)
δ: 1.93-2.07(m,4H), 2.22-2.33(m,2H), 2.52-2.61(m,2H), 2.80-2.88(m,2H), 3.07-3.15(m,2H), 3.89(s,3H), 4.37-4.47(m,1H), 6.48-6.52(m,1H), 7.15(d, *J*=8.8Hz,1H), 7.21(brs,1H), 7.52-7.61(m,4H), 7.64-7.68(m,1H), 7.92(brs,1H), 8.13(s,1H).

### Example 3

### Synthesis of 1-[1-[2-(3-acetyl-2,6-dimethoxyphenyl)ethyl] piperidin-4-yl]-1H-indole-6-carboxamide

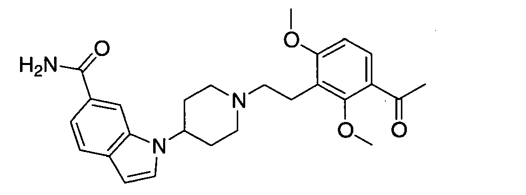

The title compound was obtained by synthesizing from 2-hydroxy-4-methoxyacetophenone in accordance with the methods in example 1.
¹H-NMR(DMSO-d6)
δ: 1.93-2.10-(m,4H), 2.25-2.34(m,2H), 2.45-2.53(m,2H), 2.54(s,3H), 2.78-2.85(m,2H), 3.09-3.17(m,2H), 3.73(s,3H), 3.87(s,3H), 4.37-4.47(m,1H), 6.51(d, *J*=3.2Hz,1H), 6.89(d, *J*=8.8Hz, 1H), 7.21 (brs, 1H), 7.53-7. 62 (m, 3H), 7.68 (d, *J*=3.2Hz, 1H), 7.91(brs,1H), 8,13(s,1H).

### Example 4

### Synthesis of 1-[1-[2-(2-benzyloxy-6-methoxyphenyl)ethyl] piperidin-4-yl]-1H-indole-6-carboxamide

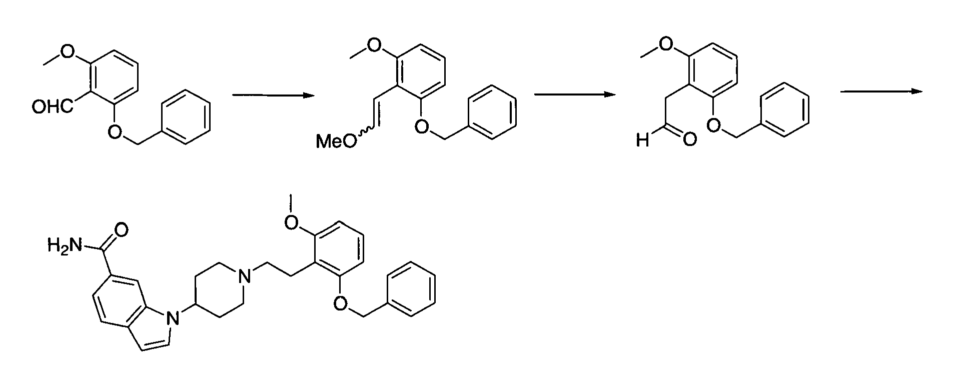

Under nitrogen atmosphere, 1.18g of potassium *tert*-butoxide was added to a suspension of 4.38g of (methoxymethyl)triphenylphosphonium chloride in 40ml of tetrahydrofuran in an ice-cooling, and stirred for 5 minutes. 1.50g of 2-methoxy-6-(phenylmethoxy)benzaldehyde (CAS No: 61227-36-9) was added in an ice-cooling, stirred for 10 minutes, and stirred for another 30 minutes at room temperature. The reaction liquid was diluted in ethyl acetate and washed with water and brine. The organic layer was concentrated under a reduced pressure after drying over magnesium sulfate, and purified by NH silica gel column chromatography (hexane-ethyl acetate), to give 1.75g of 1-benzyloxy-3-methoxy-2-(2-methoxyvinyl)benzene including a little triphenylphosphine. This compound was used in the next reaction without further purification.
800mg of 1-benzyloxy-3-methoxy-2-(2-methoxyvinyl)benzene was dissolved in 16ml of 2N HC1-tetrahydrofuran (1:1), the mixture was stirred for 2 hours at 70°C. The reaction liquid was diluted in ethyl acetate after standing to cool to room temperature, and washed with water and brine. The organic layer was concentrated under a reduced pressure after drying over magnesium sulfate to afford (2-benzyloxy-6-methoxyphenyl)acetaldehyde. This compound was used in the next reaction without further purification.
490mg of 1-(piperidin-4-yl)-1*H*-indole-6-carboxamide synthesized in production example 1 and 790mg of (2-benzyloxy-6-methoxyphenyl)acetaldehyde were dissolved in 20ml of tetrahydrofuran, 0.20ml of acetic acid and 640mg of sodium triacetoxyborohydride were added, and stirred overnight. Saturated sodium bicarbonate aqueous solution was added to the reaction liquid, and the reaction liquid was extracted with chloroform. The extract was concentrated under a reduced pressure after drying over magnesium sulfate and purified by silica gel column chromatography (ethyl acetate-methanol) to give 861mg of the title compound.
¹H-NMR(DMSO-d6)
δ: 1.87-2.07(m,4H), 2.17-2.27(m,2H), 2.42-2.49(m,2H), 2.79-2.87(m,2H), 3.03-3.11(m,2H), 3.79(s,3H), 4.36-4.44(m,1H), 5.12(s,2H), 6.50(d, *J*=3.2Hz, 1H), 6.64(d, *J*=8.4Hz, 1H), 6.72(d, J=8.4Hz,1H), 7.14(dd, J=8.4,8.4Hz,1H), 7.21(brs,1H), 7.29-7.35(m, 1H), 7.40(dd, *J*=7.2,7.2Hz,2H), 7.48(d, *J*=7.2Hz,2H), 7.53-7.61(m,2H), 7.66(d, *J*=3.2Hz, 1H), 7.91(brs,1H) 8.12(s,1H).

### Example 5

### Synthesis of 1-[1-[2-(2,6-dimethoxyphenyl)ethyl] piperidin-4-yl]-1H-indole-6-carboxamide

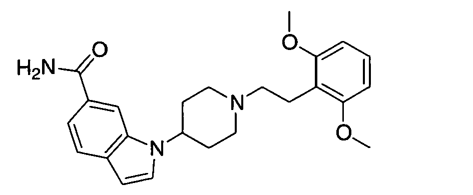

The title compound was obtained by synthesizing from 2,6-dimethoxybenzaldehyde in accordance with the methods in example 4.
¹H-NMR (DMSO-d6)
δ: 1.92-2.08 (m, 4H), 2.20-2.28 (m,2H), 2.37-2.44 (m,2H), 2.75-2.82 (m, 2H), 3.07-3.14 (m, 2H), 3.78 (s,6H), 4.37-4.47 (m,1H), 6.50(d, *J*=3.2Hz, 1H), 6.63 (d, *J*=8.4Hz, 2H), 7.15(dd, *J*=8.4,8.4Hz,1H), 7.21(brs,1H), 7.53-7.60 (m, 2H), 7.67 (d, *J*=3.2Hz,1H), 7.91 (brs, 1H), 8.12 (s,1H).

### Example 6

### Synthesis of 1-[1-[2-(2,4-dimethoxyphenyl)ethyl] piperidin-4-yl]-1H-indole-6-carboxamide

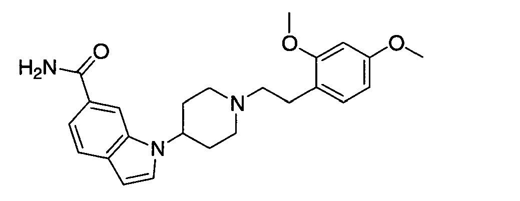

The title compound was obtained by synthesizing from 2,4-dimethoxybenzaldehyde in accordance with the methods in example 4.
¹H-NMR(DMSO-d6)
5: 1.92-2.08(m,4H), 2.20-2.28(m,2H), 2.47-2.53(m,2H), 2.65-2.72(m,2H), 3.06-3.13(m,2H), 3.74(s,3H), 3.78(s,3H), 4.37-4.47(m,1H), 6.45 (dd, *J*=2.8,8.4Hz,1H), 6.50(d, *J*=3.2Hz,1H), 6.52(d, *J*=2.8Hz,1H), 7.07(d, J=8.4Hz,1H), 7.21(brs,1H), 7.53-7.60(m,2H), 7.67(d, *J*=3.2Hz, 1H), 7.91 (brs,1H), 8.12 (s,1H).

### Example 7

### Synthesis of 1-[1-[2-(2-fluoro-6-methoxyphenyl)ethyl] piperidin-4-yl]-1H-indole-6-carboxamide

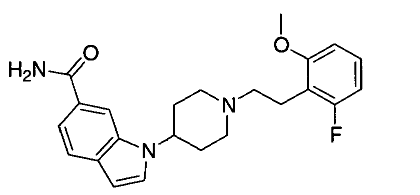

The title compound was obtained by synthesizing from 2-fluoro-6-methoxybenzaldehyde (CAS NO: 146137-74-8) in accordance with the methods in example 4.
¹H-NMR (DMSO-d6)
δ: 1.92-2.07(m,4H), 2.23-2.31(m,2H), 2.46-2.53(m,2H), 2.77-2.83(m,2H), 3.07-3.14(m,2H), 3.83(s,3H), 4.37-4.46(m,1H), 6.50(d, *J*=3.2Hz, 1H), 6.78(dd, *J*=9.6,9.6Hz, 1H), 6.84(d, *J*=8.0Hz, 1H), 7.18-7.26(m,2H), 7.54-7.61(m,2H), 7.67(d, *J*=3.2Hz, 1H), 7.91(brs,lH), 8.12(s,1H)

### Example 8

### Synthesis of 1-[1-[2-(2,4,6-trimethoxyphenyl)ethyl] piperidin-4-yl]-1H-indole-6-carboxamide

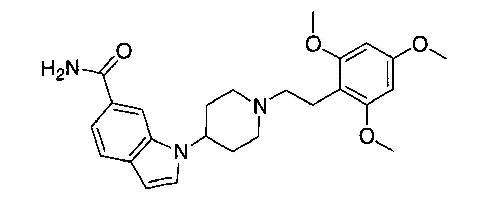

The title compound was obtained by synthesizing from 2,4,6-trimethoxybenzaldehyde in accordance with the methods in example 4.
¹H-NMR(DMSO-d6)
δ: 1.93-2.09(m,4H), 2.18-2.27(m,2H), 2.33-2.40(m,2H), 2.66-2.72(m,2H), 3.06-3.14(m,2H), 3.77(s,3H), 3.77(s,6H), 4.36-4.46(m,1H), 6.22(s,2H), 6.51 (d, *J*=3.2Hz,1H), 7.22(brs,1H), 7.54-7.61(m,2H), 7.68(d, *J*=3.2Hz,1H), 7.92(brs,1H), 8.13(s,1H).

### Example 9

### Synthesis of 1-[1-[2-(2,4,5-trimethoxyphenyl)ethyl] piperidin-4-yl]-1H-indole-6-carboxamide

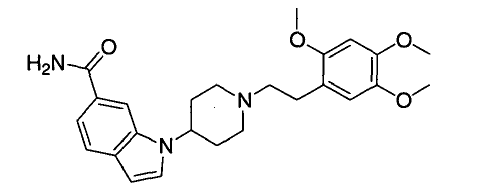

The title compound was obtained by synthesizing from 2,4,5-trimethoxybenzaldehyde in accordance with the methods in example 4.
¹H-NMR(DMSO-d6)
δ: 1.92-2.08(m,4H), 2.20-2.30(m,2H), 2.45-2.54(m,2H), 2.65-2.72(m,2H), 3.07-3.13(m,2H), 3.70(s,3H), 3.77(s,3H), 3.77(s,3H), 4.37-4.47(m,1H), 6.50(d, J=2.8Hz,1H), 6.66(s,1H), 6.82(s,1H), 7.21(brs,1H), 7.53-7.61(m,2H), 7.67(d, *J*=2.8Hz,1H), 7.91(brs,1H), 8.12(s,1H).

### Example 10

### Synthesis of 1-[1-[2-(2,3,6-trimethoxyphenyl)ethyl] piperidin-4-yl]-1H-indole-6-carboxamide

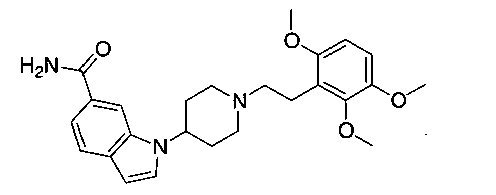

The title compound was obtained by synthesizing from 2,3,6-trimethoxybenzaldehyde (CAS NO: 5556-86-5) in accordance with the methods in example 4.
¹H-NMR(DMSO-d6)
δ: 1.93-2.10(m,4H), 2.23-2.31(m,2H), 2.42-2.48(m,2H), 2.74-2.81(m,2H), 3.08-3.14(m,2H), 3.74(s,3H), 3.75(s,3H), 3.75(s,3H), 4.37-4.47(m,1H), 6.50(d, *J*=3.2Hz,1H), 6.66(d, *J*=9.2Hz,1H), 6.84(d, *J*=9.2Hz,1H), 7.21(brs,1H), 7.54-7.61(m,2H), 7.67(d, *J*=3.2Hz,1H), 7.91(brs,1H), 8.13(s,1H).

### Example 11

### Synthesis of 1- [1- [2- (3-methoxypyridine-2-yl) ethyl] piperidin-4-yl]-1H-indole-6-carboxamide

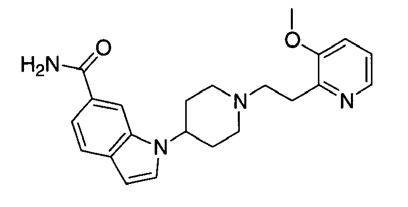

The title compound was obtained by synthesizing from 3-methoxypyridine-2-carbaldehyde (CAS NO: 1849-53-2) in accordance with the methods in example 4.
¹H-NMR(DMSO-d6) 5: 1.92-2.07(m,4H), 2.23-2.32(m,2H), 2.68-2.74(m,2H), 2.92-2.98(m,2H), 3.08-3.14(m,2H), 3.83(s,3H), 4.37-4.47(m,1H), 6.50(d, *J*=3.2Hz,1H), 7.17-7.24(m,1H), 7.22(dd, *J*=4.4,8.0Hz,1H), 7.36 (dd, *J*=1.2,8.0Hz,1H), 7.53-7.60(m,2H), 7.67(d, *J*=3.2Hz,1H), 7.91(brs,1H), 8.06(dd, *J*=1.2,4.4Hz,1H), 8.13(s,1H)

### Example 12

### Synthesis of 1-[1-[2-(5-acetylamino-4-hydroxy-2-methoxyphenyl)ethyl] piperidin-4-yl]-1H-indole-6-carboxamide

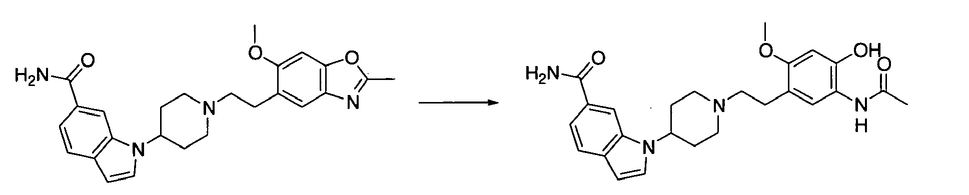

52mg of 1-[1-[2-(6-methoxy-2-methylbenzoxazole-5-yl)ethyl]piperidin-4-yl]-1*H*-indole-6-carboxamide was dissolved in 50ml of 1000ml aqueous solution of sodium chloride (2.0g) and conc. HCl (7.0ml), and the mixture was shaken for 6 hours at about 40°C. The reaction liquid was neutralized by adding saturated sodium bicarbonate aqueous solution and then extracted with chloroform. The extract was concentrated under a reduced pressure after drying over magnesium sulfate and crystallized form ethyl acetate to give 44mg of the title compound.
¹H-NMR(DMSO-d6)
δ: 1.91-2.10(m,4H), 2.06(s,3H), 2.17-2.31(m,2H), 2.40-2.55(m,2H), 2.59-2.70(m,2H), 3.04-3.15(m,2H), 3.73(s,3H), 4.37-4.48(m,1H), 6.48(s,1H), 6.50(d, *J*=3.2Hz, 1H), 7.15-7.26(m,2H), 7.52-7.61(m,2H), 7.67(d, *J*=3.2Hz, 1H), 7.91(brs,1H), 8.13(s,1H), 9.40(s,1H), 9.58(s,1H).

### Example 13

### Synthesis of 1-[1-[2-(5-acetyl-2,4-dimethoxyphenyl)ethyl] piperidin-4-yl]-1H-indole-6-carboxamide

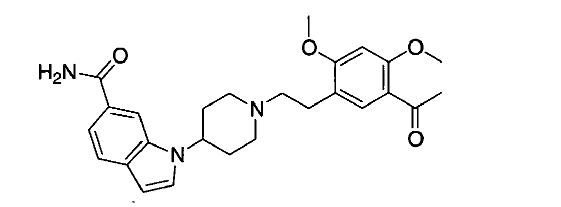

The title compound was obtained by synthesizing from 2-hydroxy-4-methoxy-5-prenylphenyl methyl ketone (Synth. Commun., 20 (5), 649 (1990)) in accordance with the methods in example 1-(2) to (3).
¹H-NMR(DMSO-d6)
δ: 1.92-2.07(m,4H), 2.20-2.30(m,2H), 2.46-2.53(m,2H), 2.50(s,3H), 2.67-2.75(m,2H), 3.06-3.14(m,2H), 3.92(s,3H), 3.94(s,3H), 4.37-4.47(m,1H), 6.50(d, *J*=3.2Hz,1H), 6.70(s,1H), 7.21(brs,1H), 7.53(s, 1H); 7.52-7.61(m,2H), 7.66(d, *J*=3.2Hz,1H), 7.91(brs,1H), 8.13(s,1H).

### Example 14

### Synthesis of 1-[1-[2-[5-(1-hydroxyethyl)-2-methoxyphenyl] ethyl]piperidin-4-yl]-1H-indole-6-carboxamide

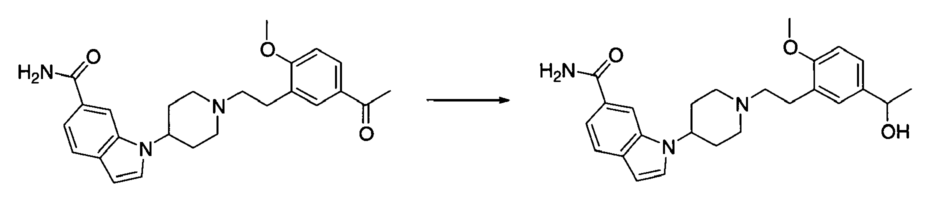

36mg of 1-[1-[2-(5-acetyl-2-methoxyphenyl)ethyl] piperidin-4-yl]-1H-indole-6-carboxamide synthesized in example 1 was dissolved in 3ml of methanol, 7mg of sodium borohydride was added in an ice-cooling, and stirred for 3 hours at room temperature. The reaction liquid was concentrated under a reduced pressure, brine was added to the residue and was extracted with chloroform. The extract was concentrated under a reduced pressure after drying over magnesium sulfate, and purified by NH silica gel column chromatography (ethyl acetate-methanol) to give 28mg of the title compound.
¹H-NMR(DMSO-d6)
δ: 1.30(d, *J*=6.8Hz,3H), 1.90-2.10(m,4H), 2.21-2.32(m,2H), 2.48-2.58(m,2H), 2.72-2.80(m,2H), 3.07-3.16(m,2H), 3.78(s,3H), 4.37-4.48(m,1H), 4.60-4.70(m,1H), 4.99(d, *J*=4.4Hz,1H), 6.51(d, J=2.8Hz,1H), 6.86-6.92(m,1H), 7.12-7.16(m,2H), 7.21(brs,1H), 7.53-7.61 (m,2H), 7.67 (d, *J*=2.8Hz,1H), 7. 92 (brs,1H), 8.13 (s,1H).

### Example 15

### Synthesis of 1-[1-[2-[5-(1-hydroxy-1-methylethyl)-2-methoxyphenyl]ethyl]piperidin-4-yl]-1H-indole-6-carboxamide

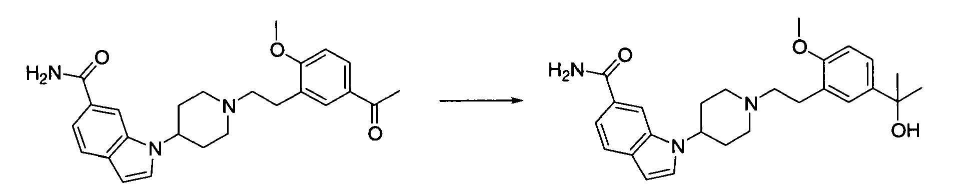

Under nitrogen atmosphere, 62mg of 1-[1-[2-(5-acetyl-2-methoxyphenyl)ethyl]piperidin-4-yl]-1*H-*indole-6-carboxamide synthesized in example 1 was dissolved in 5ml of tetrahydrofuran, 0.36ml of methyllithium (1.04M diethyl ether solution) was added at -78°C, and was stirred for 30 minutes. 0.36ml of methyllithium was added at -78°C, and the mixture was stirred for 15 minutes. After quenching with saturated ammonium aqueous solution, the mixture was slowly raised to a room temperature. Saturated ammonium aqueous solution was added to the reaction liquid, and extracted with chloroform. The extract was concentrated under a reduced pressure after drying over magnesium sulfate, and purified by NH silica gel column chromatography (methanol-ethyl acetate) to give 42mg of the title compound.
¹H-NMR(DMSO-d6)
δ: 1.40(s,6H), 1.92-2.00(m,4H), 2.22-2.31(m,2H), 2.50-2.57(m,2H), 2.73-2.80(m,2H), 3.07-3.15(m,2H), 3.77(s,3H), 4.37-4.47(m,1H), 4.86(s,1H), 6.51(d, *J*=3.2Hz,1H), 6.86(d, J=8.4Hz,1H), 7.18-7.28(m,3H), 7.54-7.60(m,2H), 7.67(d, *J*=3.2Hz,1H), 7.91 (brs, 1H), 8.13(s,1H).

### Example 16

### Synthesis of 1-[1-[2-(2-hydroxy-6-methoxyphenyl)ethyl] piperidin-4-yl]-1H-indole-6-carboxamide

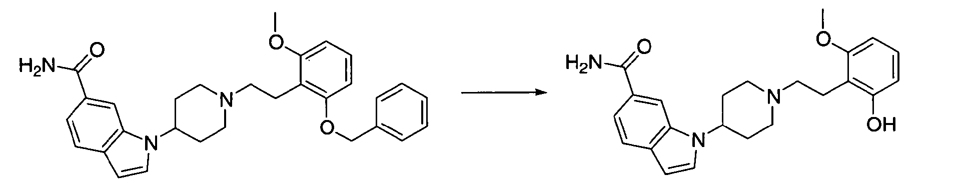

827mg of 1-[1-[2-(2-benzyloxy-6-methoxyphenyl)ethyl] piperidin-4-yl]-1*H*-indole-6-carboxamide synthesized in example 4 was dissolved in 20ml of methanol, 150mg of 10% palladium carbon was added, and stirred for 4 hours under hydrogen atmosphere. Filtering off palladium carbon and concentrating under a reduced pressure afforded 680mg of the title compound.
¹H-NMR(DMSO-d6)
δ: 1.97-2.07(m,4H), 2.25-2.34(m,2H), 2.44-2.53(m,2H), 2.74-2.81(m,2H), 3.11-3.18(m,2H), 3.74(s,3H), 4.39-4.50(m,1H), 6.43(d, J=8.4Hz,1H), 6.44(d, *J*=8.4Hz,1H), 6.51(d, J=3.2Hz,1H), 6.96(dd, *J*=8.4, 8.4Hz,1H), 7.22(brs,1H), 7.53-7.61(m,2H), 7.67(d, *J*=3.2Hz,1H), 7.91(brs,1H), 8.13(s,1H) 10.08(brs,1H).

### Example 17

### Synthesis of 1-[1-[2-[2-methoxy-6-(2-methoxyethoxy)phenyl] ethyl]piperidin-4-yl]-1H-indole-6-carboxamide

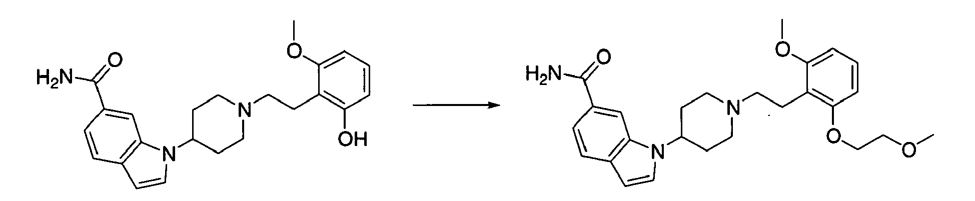

70mg of 1-[1-[2-(2-hydroxy-6-methoxyphenyl)ethyl] piperidin-4-yl]-1H-indole-6-carboxamide synthesized in example 16 was dissolved in 2ml of N,N-dimethylformamide, 49mg of potassium carbonate and 37mg of (2-bromoethyl) methyl ether were added, and stirred for 1.5 hours at 70°C. 49mg of potassium carbonate and 37mg of (2-bromoethyl) methyl ether were further added, and the mixture was stirred for 2 hours at 70°C. Brine was added to the mixture after standing to cool to room temperature, and extracted with chloroform. The extract was concentrated under a reduced pressure after drying over magnesium sulfate, and purified by NH silica gel column chromatography (ethyl acetate-methanol) to give 54mg of the title compound.
¹H-NMR(DMSO-d6)
δ: 1.92-2.10(m,4H), 2.22-2.31(m,2H), 2.40-2.50(m,2H), 2.76-2.83(m,2H), 3.08-3.15(m,2H), 3.33(s,3H), 3.65-3.70(m,2H), 3.78(s,3H), 4.06-4.11(m,2H), 4.36-4.47(m,1H), 6.50(d, *J*=3.2Hz,1H), 6.92(d, *J*=8.4Hz,1H), 6.92(d, *J*=8.4Hz, 1H), 7.13(dd, *J*=8.4,8.4Hz,1H), 7.21(brs,1H), 7.54-7.60(m,2H), 7.68(d, *J*=3.2Hz,1H), 7.91(brs,1H), 8.13(s,1H).

### Example 18

### Synthesis of 1-[1-[2-(2-ethoxy-6-methoxyphenyl)ethyl] piperidin-4-yl]-1H-indole-6-carboxamide

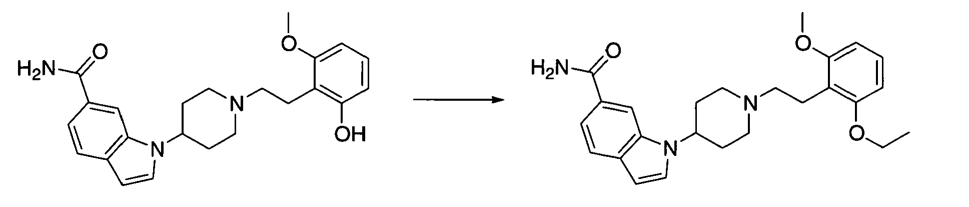

The title compound was obtained by synthesizing from 1-[1-[2-(2-hydroxy-6-methoxyphenyl)ethyl] piperidin-4-yl]-1*H*-indole-6-carboxamide (prepared in example 16) in accordance with the methods in example 17.
¹H-NMR(DMSO-d6)
δ: 1.34(t, *J*=7.2Hz,3H), 1.92-2.20(m,4H), 2.20-2.30(m,2H), 2.38-2.47(m,2H), 2.75-2.82(m,2H), 3.08-3.16(m,2H), 3.77(s,3H), 4.02(q, J=7.2Hz,2H) 4.37-4.47(m,1H), 6.50(d, J=3.2Hz,1H), 6.60(d, *J*=8.4Hz,1H) 6.60(d, J=8.4Hz,1H) 7.12(dd, *J*=8.4,8.4Hz,1H), 7.21(brs,1H), 7.53-7.61(m,2H), 7.68(d, *J*=3.2Hz,1H), 7.91(brs,1H), 8.13(s,1H)

### Example 19

### Synthesis of 1-[1-[2-(2-isobutoxy-6-methoxyphenyl)ethyl] piperidin-4-yl]-1H-indole-6-carboxamide

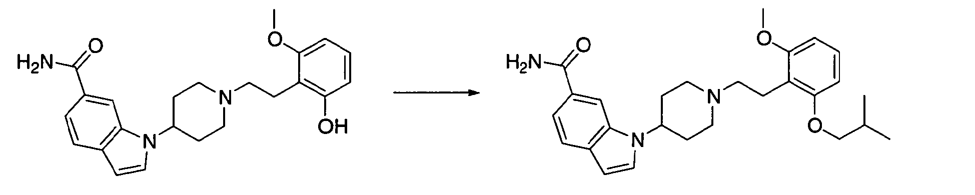

The title compound was obtained by synthesizing from 1- [1- [2- (2-hydroxy-6-methoxyphenyl) ethyl] piperidin-4-yl]-1*H*-indole-6-carboxamide (prepared in example 16) in accordance with the methods in example 17.
¹H-NMR(DMSO-d6)
δ : 1.03(d, *J*=6.8Hz,6H), 1.92-2.00(m,5H) 2.21-2.32(m,2H), 2.40-2.47(m,2H), 2.77-2.85(m,2H), 3.07-3.15(m,2H), 3.74(d, *J*=6.8Hz,1H), 3.78(s,3H), 4.37-4.47 (m,1H), 6.50(d, J=3.2Hz,1H), 6.59(d, J=8.4Hz,1H), 6.61(d, J=8.4Hz,1H), 7.12(dd, J=8.4,8.4Hz,1H), 7.21(brs,1H), 7.53-7.60(m,2H), 7.68(d, J=3.2Hz,1H), 7.91(brs,1H), 8.13(s,1H).

### Example 20

### Synthesis of 1-[1-[2-[2-(2-dimethylaminoethoxy)-6-methoxyphenyl]ethyl]piperidin-4-yl]-1H-indole-6-carboxamide

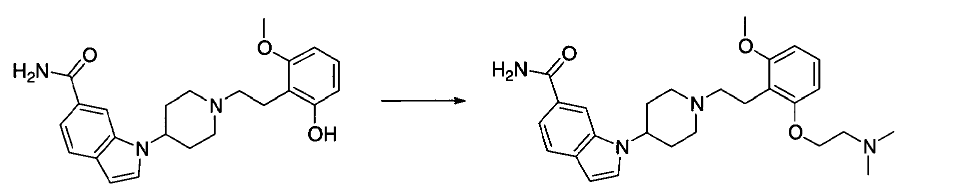

The title compound was obtained by synthesizing from 1-[1-[2-(2-hydroxy-6-methoxyphenyl)ethyl] piperidin-4-yl]-1H-indole-6-carboxamide (prepared in example 16) in accordance with the methods in example 17.
¹H-NMR(DMSO-d6)
δ: 1.91-2.10(m,4H), 2.20-2.30(m,2H), 2.24(s,6H), 2.20-2.30(m, 2H), 2.39-2.48(m, 2H), 2.65(t, *J*=6.0Hz,2H), 2.75-2.82(m,2H), 3.06-3.14(m,2H), 3.78(s,3H), 4.04(t, *J*=6.0Hz,2H), 4.37-4.47(m,1H), 6.50(d, *J*=2.8Hz,1H), 6.61(d, J=8.0Hz,1H 6.62(d, *J*=8.0Hz,1H), 7.12(dd, *J*=8.0,8.0Hz,1H), 7.21(brs,1H), 7.53-7.61(m,2H), 7.68(d, *J*=2.8Hz, 1H), 7. 91 (brs, 1H), 8.13(s, 1H).

### Example 21

### Synthesis of 1-[1-[2-[2-methoxy-6-[(2-piperidin-1-yl)ethoxy] phenyl]ethyl]piperidin-4-yl]-1H-indole-6-carboxamide

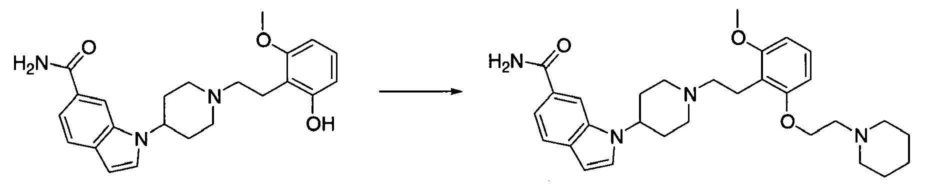

The title compound was obtained by synthesizing from 1- [1- [2- (2-hydroxy-6-methoxyphenyl) ethyl] piperidin-4-yl]-1*H*-indole-6-carboxamide (prepared in example 16) in accordance with the methods in example 17.
¹H-NMR(DMSO-d6)
δ : 1.34-1.42(m,2H), 1.46-1.54(m,4H), 1.92-2.10(m,4H), 2.23-2.32(m,2H), 2.38-2.55(m,6H), 2.65-2.72(m,2H), 2.75-2.83(m,2H), 3.07-3.15(m,2H), 3.77(s,3H), 4.05(t, *J*=5.6Hz, 2H), 4.37-4.47(m,1H), 6.50(d, *J*=3.2Hz, 1H), 6.61(d, *J*=8.4Hz, 1H), 6.62(d, *J*=8.4Hz, 1H), 7.12(dd, J=8.4,8.4Hz,1H), 7.21(brs,1H), 7.54-7.61(m,2H), 7.68(d, J=3.2Hz,1H), 7.91(brs, 1H), 8.13(s, 1H).

### Example 22

### Synthesis of 1-[1-[2-[2-(2-hydroxyethoxy)-6-methoxyphenyl] ethyl]piperidin-4-yl]-1H-indole-6-carboxamide

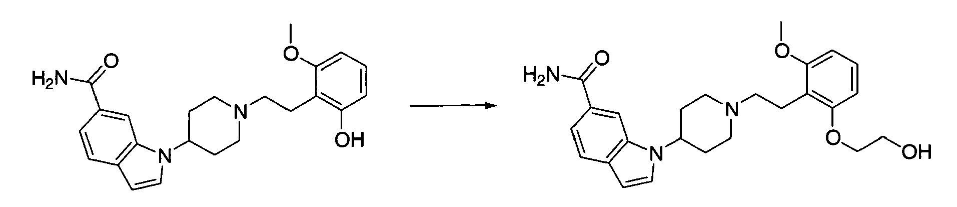

The title compound was obtained by synthesizing from 1-[1-[2-(2-hydroxy-6-methoxyphenyl)ethyl] piperidin-4-yl]-1*H*-indole-6-carboxamide (prepared in example 16) in accordance with the methods in example 17.
¹H-NMR (DMSO-d6)
δ: 1.92-2.10(m,4H), 2.20-2.30(m,2H), 2.40-2.48(m,2H), 2.77-2.85(m,2H), 3.08-3.16(m,2H), 3.70-3.77(m,2H), 3.78(s,3H), 3.98(t, *J*=4.8Hz,2H), 4.37-4.46(m,1H), 4.80(t, *J*=5.6Hz,1H), 6.50(d, *J*=2.8Hz,1H), 6.61(d, *J*=8.4Hz,1H), 6.61(d, *J*=8.4Hz,1H), 7.12 (dd, *J*=8.4,8.4Hz,1H), 7.21(brs,1H), 7.53-7.61(m,2H), 7.67 (d, *J*=2.8Hz,1H), 7.92(brs,1H), 8.13(s,1H)

### Example 23

### Synthesis of 1-[1-[2-(5-acetylamino-2-methoxyphenyl)ethyl] piperidin-4-yl]-1H-indole-6-carboxamide

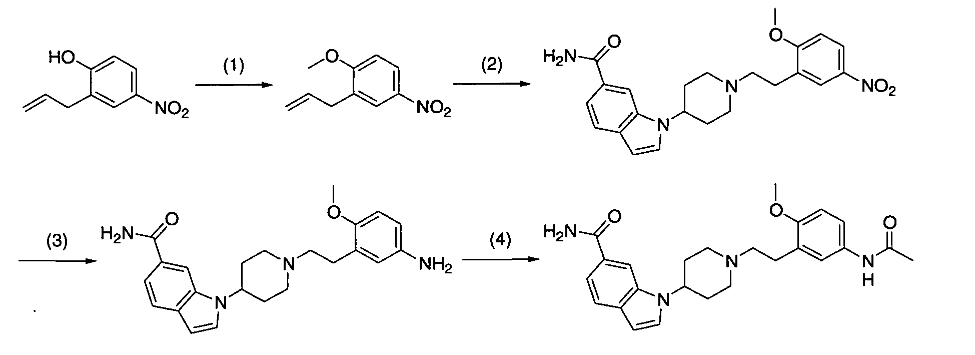

### (1) Synthesis of 1-allyl-2-methoxy-5-nitrobenzene

31.6g of 4-nitro-2-(2-propenyl) phenol (CAS NO: 19182-96-8) was dissolved in 200ml of N,N-dimethylformamide, 36.6g of potassium carbonate and 30.0g of iodomethane were added, which was stirred for 1.5 hours. The reaction liquid was diluted in ethyl acetate and washed with water and brine. The organic layer was concentrated under a reduced pressure after drying over magnesium sulfate, and purified by NH silica gel column chromatography (ethyl acetate) to give 32.7g of the title compound.
¹H-NMR(CDCl3)
5: 3.42(d, J=6.8Hz,2H), 3.94(s,3H), 5.07-5.17(m,2H), 5.91-6.03(m,1H), 6.90(d, J=9.2Hz,1H), 8.06(d, J=2.4Hz,1H), 8.15(dd, J=2.4,9.2Hz,1H).

### (2) Synthesis of 1-[1-[2-(2-methoxy-5-nitrophenyl)ethyl] piperidin-4-yl]-1H-indole-6-carboxamide

Example 1-(3) was followed from 1-allyl-2-methoxy -5-nitrobenzene, the title compound was obtained.
¹H-NMR(DMSO-d6)
δ : 1.94-2.03(m,4H), 2.25-2.33(m,2H), 2.57-2.63(m,2H), 2.83-2.90(m,2H), 3.08-3.15(m,2H), 3.96(s,3H), 4.38-4.47(m,1H), 6.50(d, *J*=3.2Hz,1H), 7.17-7.24(m,2H), 7.53-7.60(m,2H), 7.66(d, *J*=3.2Hz,1H), 7.91(brs,1H), 8.11-8.18(m,3H).

### (3) Synthesis of 1-[1-[2-(5-amino-2-methoxyphenyl)ethyl] piperidin-4-yl]-1H-indole-6-carboxamide

178mg of 1-[1-[2-(2-methoxy-5-nitrophenyl)ethyl] piperidin-4-yl]-1*H*-indole-6-carboxamide was dissolved in 5ml of methanol, 30mg of 10% palladium carbon was added, and the mixture was stirred for 4 hours under hydrogen atmosphere. Filtering off palladium carbon and concentrating under a reduced pressure afforded are 166mg of the title compound.
¹H-NMR (DMSO-d6)
5: 1.92-2.09(m,4H), 2.21-2.30(m,2H), 2.47-2.56(m,2H), 2.60-2.67(m,2H), 3.06-3.13(m,2H), 3.66(s,3H), 4.37-4.47(m,1H), 4.50-4.56(m,2H), 6.39(d, *J*=2.8Hz,1H), 6.50(d, *J*=3.2Hz,1H), 6.66(d, *J*=8.8Hz,1H), 7.21(brs,1H), 7.53-7.61(m,2H), 7.68(d, *J*=3.2Hz,1H), 7.91(brs,1H), 8.13(s,1H).

### (4) Synthesis of 1-[1-[2-(5-acetylamino-2-methoxyphenyl)ethyl] piperidin-4-yl]-1H-indole-6-carboxamide

40mg of 1-[1-[2-(5-amino-2-methoxyphenyl)ethyl] piperidin-4-yl]-1H-indole-6-carboxamide was dissolved in 2ml of acetic anhydride-pyridine (1:1), which was stirred overnight at room temperature. The reaction liquid was concentrated under a reduced pressure, 1N aqueous sodium hydroxide was added to the residue, and extracted with chloroform. The extract was concentrated under a reduced pressure after drying over magnesium sulfate, and purified by NH silica gel column chromatography (ethyl acetate-methanol) to give 40mg of the title compound.
¹H-NMR(DMSO-d6)
δ: 1.92-2.09(m,4H), 2.00(s,3H), 2.22-2.31(m,2H), 2.50-2.57(m,2H), 2.69-2.76(m,2H), 3.07-3.14(m,2H), 3.76(s,3H), 4.37-4.47(m,1H), 6.50(d, *J*=3.2Hz,1H) 6.88(d, *J*=8.8Hz,1H), 7.21(brs,1H), 7.36(d, *J*=2.4Hz,1H), 7.39(dd, J=2.4,8.8Hz,1H), 7.54-7.61(m,2H), 7.67(d, *J*=3.2Hz, 1H),7.91(brs, 1H), 8.13(s,1H), 9.72(s, 1H).

### Example 24

### Synthesis of 1-[1-[2-(5-dimethylamino-2-methoxyphenyl)ethyl] piperidin-4-yl]-1H-indole-6-carboxamide

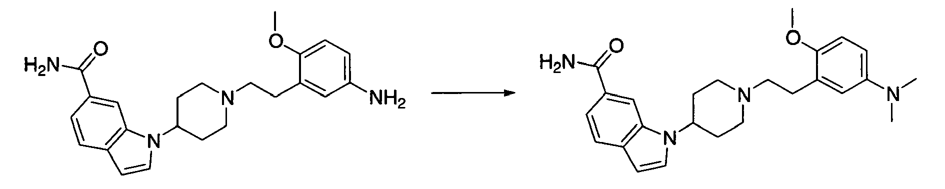

59mg of 1-[1-[2-(5-amino-2-methoxyphenyl)ethyl] piperidin-4-yl]-1*H*-indole-6-carboxamide synthesized in example 23- (3) was dissolved in 5ml of acetonitrile, 1ml of 37% formalin, 47mg of sodium cyanoborohydride and 1ml of acetic acid were added, which was stirred overnight. Saturated sodium bicarbonate aqueous solution was added to reaction liquid, and extracted with chloroform. The extract was concentrated under a reduced pressure after drying over magnesium sulfate, and purified by NH silica gel column chromatography (ethyl acetate) to give 35mg of the title compound.
¹H-NMR(DMSO-d6) δ: 1.93-2.09(m,4H), 2.22-2.31(m,2H), 2.50-2.57(m,2H), 2.69-2.76(m,2H), 2.80(s,6H), 3.08-3.15(m,2H), 3.71(s,3H), 4.37-4.47(m,1H), 6.50(d, *J*=3.2Hz, 1H), 6.57(dd, *J*=3.2,8.8Hz,1H), 6.67(d, *J*=3.2Hz,1H), 6.82(d, *J*=8.8Hz,1H), 7.21(brs,1H), 7.54-7.60(m,2H), 7.67(d, *J*=3.2Hz,1H), 7.91(brs,1H), 8.13(s,1H).

### Example 25

### Synthesis of 1-[1-[2-[2-methoxy-5-(2-oxopyrrolidin-1-yl) phenyl]ethyl]piperidin-4-yl]-1H-indole-6-carboxamide

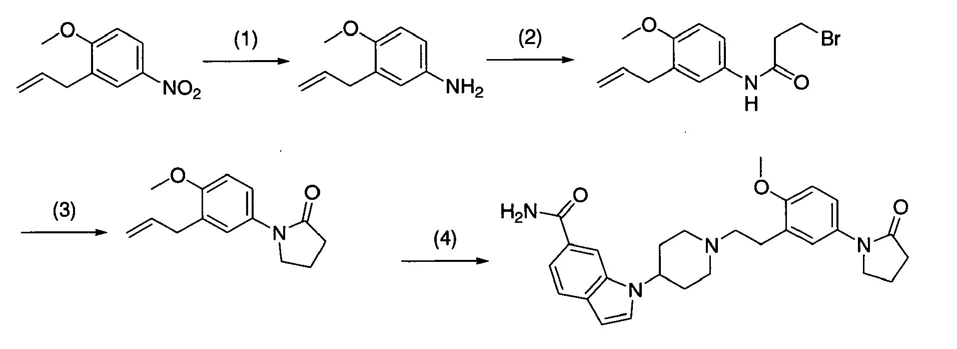

### (1) Synthesis of 1-allyl-5-amino-2-methoxybenzene

15.0g of 1-allyl-2-methoxy-5-nitrobenzene, 33.4g of ammonium chloride and 17.5g of iron suspended in 270ml of ethanol and 55ml of water, and was heated to reflux for 1 hour. The insoluble material was removed by filtration after standing to cool to room temperature, and the filtrate was concentrated under a reduced pressure. The residue was diluted in ethyl acetate and washed with saturated sodium bicarbonate aqueous solution and brine. The organic layer was concentrated under a reduced pressure after drying over magnesium sulfate, and purified by silica gel column chromatography (hexane-ethyl acetate) to give 11.1g of the title compound.
¹H-NMR(CDCl3)
δ: 3.32(d, J=6.4Hz,2H), 3.39(brs,2H), 3.76(s,3H), 5.00-5.10(m,2H), 5.91-6.03(m,1H), 6.51-6.57(m,2H), 6.67-6.73(m,1H).

### (2) Synthesis of N-(3-allyl-4-methoxyphenyl)-4-bromobutanamide

500mg of 1-allyl-5-amino-2-methoxybenzene was dissolved in tetrahydrofuran 10ml, 1.06ml of triethylamine and 0.41ml of 4-bromobutyryl chloride was added in an ice-cooling, and stirred for 1 hour at room temperature. The reaction liquid was diluted in ethyl acetate and washed with water and brine. The organic layer was concentrated under a reduced pressure after drying over magnesium sulfate, and purified by NH silica gel column chromatography (hexane-ethyl acetate) to give 924mg of the title compound.
¹H-NMR(CDC13)
δ : 2.27(tt, *J*=5.2,6.0Hz,2H), 2.53(t, *J*=6.0Hz,2H), 3.35(d, J=6.0Hz,2H), 3.53(t, J=5.2Hz,2H), 3.80(s,3H), 5.00-5.09(m,2H), 5.90-6.01(m,1H), 6.79(d, *J*=8.8Hz,1H), 7.03(brs,1H), 7.17(d, J=2.4Hz,1H), 7.38(dd, *J*=2.4,8.8Hz,1H).

### (3) Synthesis of 1-(3-allyl-4-methoxyphenyl)pyrrolidin-2-one

170mg of N-(3-allyl-4-methoxyphenyl)-4-bromobutanamide was dissolved in 3ml of N,N-dimethylformamide, 22mg of 60% sodium hydride was added in an ice-cooling, and the mixture was stirred overnight at room temperature. The reaction liquid was diluted in ethyl acetate and washed with saturated ammonium aqueous solution and brine. The organic layer was concentrated under a reduced pressure after drying over magnesium sulfate, and purified by silica gel column chromatography (hexane-ethyl acetate) to give 55mg of the title compound.
¹H-NMR(CDCl3)
δ: 2.14 (tt, *J*=7.2,8.0Hz,2H), 2.58 (t,*J*=8.0Hz,2H), 3.37 (d, J=6.4Hz,2H), 3.81(t, *J*=7.2Hz,2H), 3.81(s,3H), 5.00-5.08(m,2H), 5.91-6.03(m,1H), 6.83(d, *J*=8.8Hz,1H), 7.28(d, *J*=2.8Hz,1H), 7.40(dd, *J*=2.8,8.8Hz,1H).

### (4) Synthesis of 1-[1-[2-[2-methoxy-5-(2-oxopyrrolidin-1-yl) phenyl]ethyl] piperidin-4-yl]-1H-indole-6-carboxamide

The title compound was obtained by synthesizing from 1-(3-allyl-4-methoxyphenyl) pyrrolidin-2-one in accordance with the methods in example 1-(3).
¹H-NMR (DMSO-d6)
δ: 1.92-2.10(m,6H), 2.22-2.31(m,2H), 2.40-2.58(m,4H), 2.72-2.80(m,2H), 3.07-3.15(m,2H), 3.79(s,3H), 3.75-3.82(m,2H), 4.37-4.47(m,1H), 6.50(d, J=2.8Hz,1H), 6.95(d, J=8.8Hz,1H), 7.2.1(brs,1H), 7.42(dd, *J*=2.4,8.8Hz,1H), 7.48(d, *J*=2.4Hz,1H), 7.52-7.60(m,2H), 7.67(d, *J*=2.8Hz,1H), 7.91(brs,1H), 8.13(s,1H).

### Example 26

### Synthesis of 1-[1-[2-[5-(methylpropionylamino)-2-methoxyphenyl]ethyl]piperidin-4-yl]-1H-indole-6-carboxamide

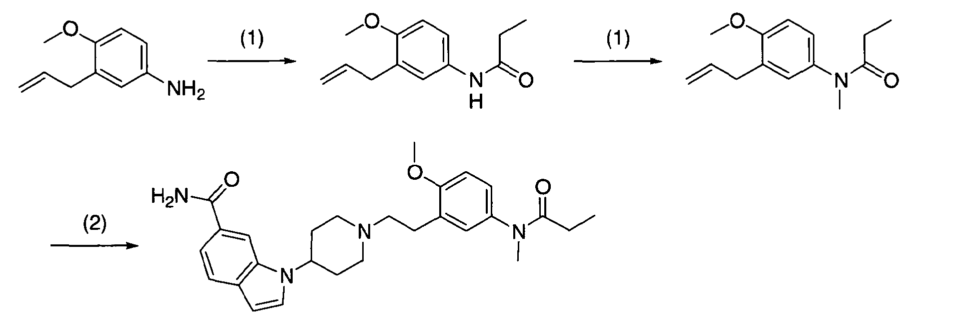

### (1) Synthesis of N-(3-allyl-4-methoxyphenyl)-N-methyl propionamide

500mg of 1-allyl-5-amino-2-methoxybenzene was dissolved in 10ml of tetrahydrofuran, 1.27ml of triethylamine and 0.32ml of propionyl chloride were added in an ice-cooling, which was stirred for 1 hour at room temperature. The reaction liquid was diluted in ethyl acetate and washed with water and brine. The organic layer was concentrated under a reduced pressure after drying over magnesium sulfate, and purified by silica gel column chromatography (hexane-ethyl acetate) to give 662mg of N-(3-allyl-4-methoxyphenyl)propionamide.
254mg of N-(3-allyl-4-methoxyphenyl)propionamide was dissolved in 5ml of N,N-dimethylformamide, 56mg of 60% sodium hydride was added in an ice-cooling, which was stirred for 30 minutes at room temperature. 0.25g of iodomethane was then added and the mixture was stirred overnight. The reaction liquid was diluted in ethyl acetate and washed with saturated ammonium aqueous solution and brine. The organic layer was concentrated under a reduced pressure after drying over magnesium sulfate, and purified by NH silica gel column chromatography (hexane-ethyl acetate) to give 263mg of the title compound.
¹H-NMR(CDCl3)
δ: 1.04(t, *J*=7.2Hz,3H), 2.06(q, *J*=7.2Hz,2H), 3.22(s,3H), 3.37(d, J=6.8Hz,2H), 3.85(s,3H), 5.03-5.12(m,2H), 5.91-6.03(m,1H), 6.84(d, *J*=8.4Hz,1H), 6.94 (d, *J*=2.8Hz,1H), 6.99(dd, *J*=2.8,8.4Hz,1H).

### (2) Synthesis of 1-[1-[2-[5-(N-methylpropionylamino)-2-methoxyphenyl]ethyl]piperidin-4-yl]-1H-indole-6-carboxamide

The title compound was obtained by synthesizing from N-(3-allyl-4-methoxyphenyl)-N-methylpropionamide in accordance with the methods in example 1-(3).
¹H-NMR (DMSO-d6)
δ: 0.91(t, J=7.6Hz,3H), 1.91-2.07(m,6H), 2.20-2.31(m,2H), 2.52-2.62 (m,2H), 2.72-2.81 (m,2H), 3.04-3.16(m,2H), 3.11(s,3H), 3.83(s,3H), 4.37-4.47(m,1H), 6.50(d, *J*=3.2Hz,1H), 7.00(d, *J*=8.0Hz,1H), 7.13(d, *J*=8.0Hz,1H), 7.14 (s,1H), 7.21(brs,1H), 7.53-7.61(m,2H), 7.64(d, *J*=3.2Hz,1H), 7.91(brs,1H) 8.12(s,1H).

### Example 27

### Synthesis of 1-[1-[2-[5-(acetylmethylamino)-2-methoxyphenyl] ethyl]piperidin-4-yl]-1H-indole-6-carboxamide

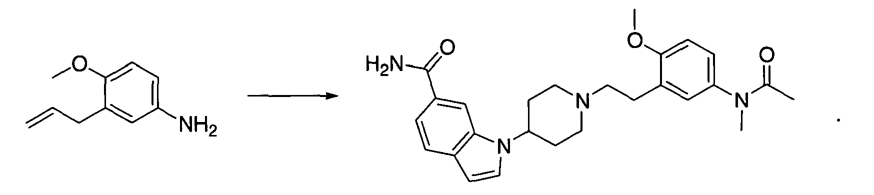

The title compound was obtained by synthesizing from 1-allyl-5-amino-2-methoxybenzene in accordance with the methods in example 26.
¹H-NMR(DMSO-d6)
δ : 1.75(s,3H), 1.92-2.07(m,4H), 2.20-2.31(m,2H), 2.51-2.61(m,2H), 2.73-2.81(m,2H), 3.05-3.15(m,2H), 3.11(s,3H), 3.82(s,3H), 4.35-4.47(m,1H), 6.50(d, *J*=3.2Hz,1H), 7.00(d, *J*=8.0Hz,1H 7.10-7.28(m,2H), 7.21(brs,1H), 7.52-7.60(m,2H), 7.64(d, *J*=3.2Hz, 1H), 7.91(brs, 1H), 8.12(s,1H).

### Example 28

### Synthesis of 1-[1-[2-[5-(methanesulfonylmethylamino)-2-methoxyphenyl]ethyl]piperidin-4-yl]-1H-indole-6-carboxamide

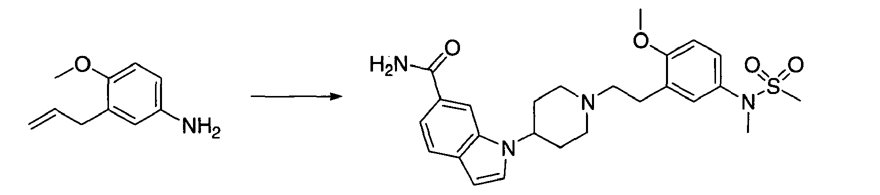

The title compound was obtained by synthesizing from 1-allyl-5-amino-2-methoxybenzene in accordance with the methods in example 26.
¹H-NMR(DMSO-d6)
δ: 1.92-2.07(m,4H), 2.21-2.32(m,2H), 2.51-2.60(m,2H), 2.73-2.81(m,2H), 2.92(s,3H), 3.07-3.15(m,2H), 3.19(s,3H), 3.82(s,3H), 4.37-4.47(m,1H), 6.50(d, J=3.6Hz,1H), 6.98(d, *J*=8.4Hz,1H), 7.17-7.28(m,3H), 7.53-7.61(m,2H), 7.66(d, *J*=3.6Hz,1H), 7.91 (brs,1H), 8.12 (s,1H).

### Example 29

### Synthesis of 1-[1-[2-[5-(acetylethylamino)-2-methoxyphenyl] ethyl]piperidin-4-yl]-1H-indole-6-carboxamide

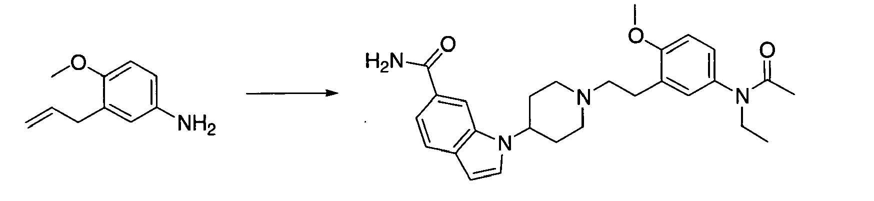

The title compound was obtained by synthesizing from 1-allyl-5-amino-2-methoxybenzene in accordance with the methods in example 26.
¹H-NMR (DMSO-d6)
δ: 1.00(t, *J*=7.0Hz,3H), 1.71(s,3H), 1.90-2.04(m,4H), 2.20-2.31(m,2H), 2.52-2.62(m,2H), 2.73-2.81(m,2H), 3.05-3.14(m,2H), 3.59(q, *J*=6.9Hz,2H), 3.83(s,3H), 4.37-4.47(m,1H), 6.50(d, *J*=3.2Hz,1H), 7.01(d, *J*=8.4Hz,1H), 7.06-7.13(m,2H), 7.21(brs,1H), 7.52-7.62-(m,2H), 7.63(d, *J*=3.2Hz,1H), 7.91(brs,1H), 8.12(s,1H)

### Example 30

### Synthesis of 1-[1-[2-[2-methoxy-5-(4-methylpiperazin-1-carbonyl)phenethyl]piperidin-4-yl]-1H-indole-6-carboxamide

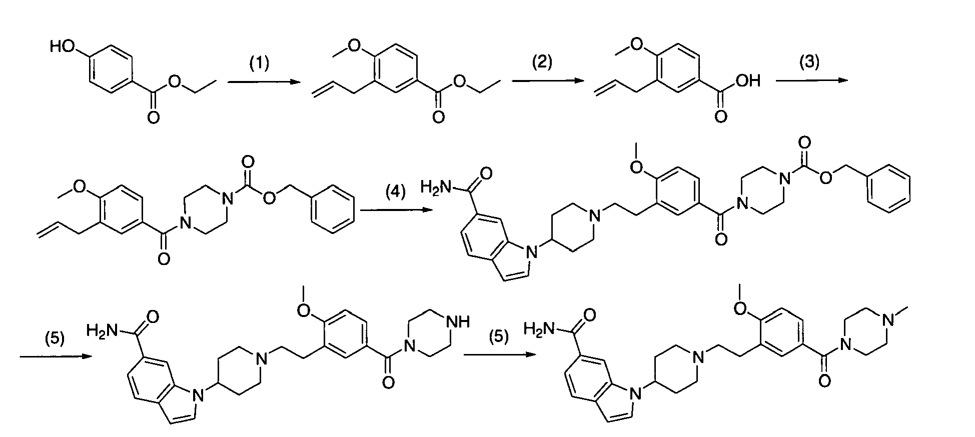

### (1) Synthesis of ethyl 3-allyl-4-methoxybenzoate

The title compound was obtained by synthesizing from ethyl 4-hydroxybenzoate in accordance with the methods in example 1- (1) to (2).
¹H-NMR(CDCl3)
δ:1.38(t, *J*=7.2Hz,3H), 3.38(d, *J*=6.8Hz,2H), 3.87(s,3H), 4.34(q, J=7.2Hz,2H), 5.01-5.09(m,2H), 5.92-6.04 (m,1H), 6.85(d, *J*=8.8Hz,1H), 7.82(d, *J*=2.4Hz,1H), 7.91(dd, *J*=2.4,8.8Hz,1H).

### (2) Synthesis of 3-allyl-4-methoxybenzoic acid

5.20g of ethyl 3-allyl-4-methoxybenzoate was dissolved in 40ml of ethanol, 7.1ml of 5N aqueous sodium hydroxide was added in an ice-cooling. 7.1ml of 5N aqueous sodium hydroxide was added after stirring for 2 hours at room temperature, 7.1ml of 5N aqueous sodium hydroxide was added after stirring another 2 hours, and then stirred overnight. 20ml of 5N HCl was added in an ice-cooling and then concentrated under a reduced pressure. 2N HCl was added to the residue, which was extracted with methylene chloride. The extract was concentrated under a reduced pressure after drying over magnesium sulfate to give 4.72g of the title compound.
¹H-NMR(CDCl3)
δ: 3.40(d, J=6.8Hz,2H), 3.91(s,3H), 5.04-5.12(m,2H), 5.93-6.05(m,1H), 6.88(d, *J*=8.0Hz,1H), 7.88(d, *J*=2.0Hz,1H), 7.98(dd, *J*=2.0,8.0Hz,1H).

### (3) Synthesis of benzyl 4-(3-allyl-4-methoxybenzoyl) piperazine-1-carbonxylate

Under nitrogen atmosphere, 500mg of 3-allyl-4-methoxybenzoic acid was dissolved in 10ml of methylene chloride, 0.34ml of oxalyl chloride and 0.05ml of N,N-dimethylformamide were added in an ice-cooling, which was stirred for 30 minutes at room temperature. After the reaction liquid was concentrated under a reduced pressure, the residue was dissolved in 15ml of tetrahydrofuran, 2.0ml of triethylamine and 2.86g of benzyl 1-piperazine carboxylate were added in an ice-cooling, and then was stirred for 2 hours at room temperature. The reaction liquid was diluted in ethyl acetate and washed with water, 2N HCl, water, saturated sodium bicarbonate aqueous solution and brine. The organic layer was concentrated under a reduced pressure after drying over magnesium sulfate, and purified by silica gel column chromatography (hexane-ethyl acetate) to give 984mg of the title compound.
¹H-NMR(CDCl3)
δ: 3.38(d, *J*=6.8Hz,2H), 3.43-3.74(m,8H), 3.86(s,3H), 5.03-5.09(m,2H), 5.15(s,2H), 5.90-6.02(m,1H), 6.85(d, *J*=8.4Hz,1H), 7.22(d, *J*=1.6Hz,1H), 7.28(dd, *J*=1.6,8.4Hz,1H), 7.30-7.42(m,5H).

### (4) Synthesis of benzyl

### 4-[3-[2-[4-(6-carbamoylindol-1-yl)]-4-piperidin-1-yl] ethylmethoxybenzoyl]piperazine-1-carbonxylate

The title compound was obtained by synthesizing from benzyl 4-(3-allyl-4-methoxybenzoyl)piperazine-1-carbonxylate in accordance with the methods in example 1-(3).
¹H-NMR(DMSO-d6)
δ: 1.92-2.07(m,4H), 2.20-2.31(m,2H), 2.52-2.60(m,2H), 2.75-2.83(m,2H), 3.07-3.15(m,2H), 3.38-3.60(m,8H), 3.84(s,3H), 4.37-4.47(m,1H), 5.10(s,2H), 6.49(d, *J*=3.2Hz,1H), 7.02(d, *J*=8.4Hz,1H), 7.21(brs,1H), 7.27-7.40(m,7H), 7.54-7.60(m,2H), 7.66(d, *J*=3.2Hz,1H), 7.91(brs,1H), 8.13(s,1H)

### (5) Synthesis of 1-[1-[2-[2-methoxy-5-(4-methylpiperazin-1-carbonyl)phenyl]ethyl]piperidin-4-yl]-1H-indole-6-carboxamide

285mg of benzyl 4-[3-[2-[4-(6-carbamoylindol-1-yl)]-4-piperidin-1-yl]ethylmethoxybenzoyl]piperazine-1-carbonxylate was dissolved in 8ml of methanol, 55mg of 10% palladium carbon was added, which was stirred for 6 hours under hydrogen atmosphere. Palladium carbon was removed by filtration, and the mixture was concentrated under a reduced pressure to yield 222mg of 1-[1-[2-[2-methoxy-5-(piperazine-1-carbonyl)phenyl]ethyl] piperidin-4-yl]-1H-indole-6-carboxamide. This compound was used in the next reaction without further purification.
138mg of 1-[1-[2-[2-methoxy-5-(piperazine-1-carbonyl)phenyl]ethyl]piperidin-4-yl]-1*H*-indole-6-carboxamide was dissolved in 5ml of acetonitrile, 1ml of 37% formalin, 89mg of sodium cyanoborohydride and 0.2ml of acetic acid were added, and was stirred for 1 hour at room temperature. Saturated sodium bicarbonate aqueous solution was added to reaction liquid and extracted with chloroform. The extract was concentrated under a reduced pressure after drying over magnesium sulfate, purified by NH silica gel column chromatography (ethyl acetate-methanol), and further purified by high performance liquid chromatography (ODS-AM; acetonitrile-water) to give 34mg of the title compound.
¹H-NMR(DMSO-d6)
δ: 1.92-2.06(m,4H), 2.18(s,3H), 2.22-2.37(m,6H), 2.52-2.60(m,2H), 2.76-2.82(m,2H), 3.07-3.14(m,2H), 3.40-3.57(m,4H), 3.84(s,3H), 4.37-4.47 (m,1H),6.50 (d, *J*=3.2Hz,1H), 6.99-7.03(m,1H), 7.18-7.28(m,3H), 7.54-7.60(m,2H), 7.66(d, *J*=3.2Hz,1H), 7.91(brs,1H), 8.12(s,1H).

### Example 31

### Synthesis of 1-[1-[2-[5-(4-acetylpiperazine-1-carbonyl)-2-methoxyphenyl]ethyl]piperidin-4-yl]-1H-indole-6-carboxamide

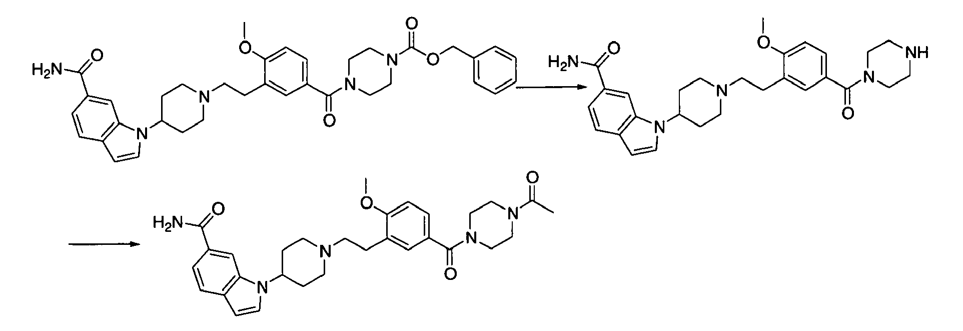

285mg of benzyl 4-[3-[2-[4-(6-carbamoylindol-1-yl)]-4-piperidin-1-yl]ethylmethoxybenzoyl]piperazine-1-carboxylate was dissolved in 8ml of methanol, 55mg of 10% palladium carbon was added, which was stirred for 6 hours under hydrogen atmosphere. Palladium carbon was removed by filtration, and the mixture was concentrated under a reduced pressure to give 222mg of 1-[1-[2-[2-methoxy-5-(piperazine-1-carbonyl)phenyl]ethyl] piperidin-4-yl]-1*H*-indole-6-carboxamide. This compound was used in the next reaction without further purification.
84mg of 1-[1-[2-[2-methoxy-5-(piperazine-1-carbonyl)phenyl]ethyl]piperidin-4-yl]-1*H*-indole-6-carboxamide was dissolved in 4ml of acetic anhydride-pyridine (1:1), and then was stirred for 1 hour at room temperature. The reaction liquid was concentrated under a reduced pressure, saturated sodium bicarbonate aqueous solution was added to the residue, and extracted with chloroform. The extract was concentrated under a reduced pressure after drying over magnesium sulfate, and purified by high performance liquid chromatography (ODS-AM; acetonitrile-water) to give 62mg of the title compound.
¹H-NMR(DMSO-d6)
δ : 1.93-2.06(m,4H), 2.01(s,3H), 2.22-2.31(m,2H), 2.53-2.61(m,2H), 2.75-2.83(m,2H), 3.07-3.15(m,2H), 3.41-3.58(m,8H), 3.85(s,3H), 4.37-4.48(m,1H), 6.50(d, *J*=3.2Hz,1H), 7.02 (d, *J*=8.0Hz,1H), 7.21(brs,1H), 7.26-7.32(m,2H), 7.53-7.60(m,2H), 7.66(d, *J*=3.2Hz,1H), 7.91(brs,1H), 8.12(s,1H).

### Example 32

### Synthesis of 1-[1-[2-[2-methoxy-5-(morpholine-4-carbonyl) phenyl]ethyl]piperidin-4-yl]-1H-indole-6-carboxamide

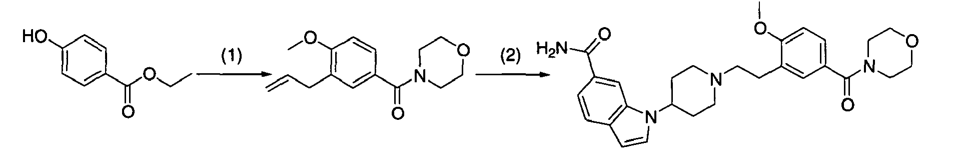

### (1) Synthesis of (3-allyl-4-methoxyphenyl)morpholin-4-yl methanone

The title compound was obtained by synthesizing from ethyl 4-hydroxybenzoate in accordance with the methods in example 30-(1), (2) and (3).
¹H-NMR(CDCl3)
δ: 3.38(d, *J*=6.4Hz,2H), 3.53-3.80(m,8H), 3.85(s,3H), 5.02-5.09(m,1H), 5.90-6.02(m,1H), 6.84(d, *J*=8.8Hz,1H), 7.22(d, *J*=2.4Hz,1H), 7.28(dd, *J*=2.4,8.8Hz,1H).

### (2) Synthesis of 1-[1-[2-[2-methoxy-5-(morpholine-4-carbonyl) phenyl]ethyl]piperidin-4-yl]-1H-indole-6-carboxamide

The title compound was obtained by synthesizing from (3-allyl-4-methoxyphenyl)morpholin-4-yl methanone in accordance with the methods in example 1-(3).
¹H-NMR(DMSO-d6)
δ: 1.92-2.07(m,4H), 2.22-2.31(m,2H), 2.52-2.60(m,2H), 2.75-2.82(m,2H), 3.08-3.14(m,2H), 3.44-3.64(m,8H), 3.84(s,3H), 4.37-4.47(m,1H), 6.50(d, *J*=3.2Hz, 1H), 7.01(d, *J*=8.0Hz,1H), 7.21(brs,1H), 7.26-7.31(m,2H), 7.53-7.60(m,2H), 7.66(d, *J*=3.2Hz,1H), 7.91(brs,1H), 8.12(s,1H).

### Example 33

### Synthesis of 1-[1-[2-(five-diethylcarbamoyl-2-methoxyphenyl) ethyl]piperidin-4-yl]-1H-indole-6-carboxamide

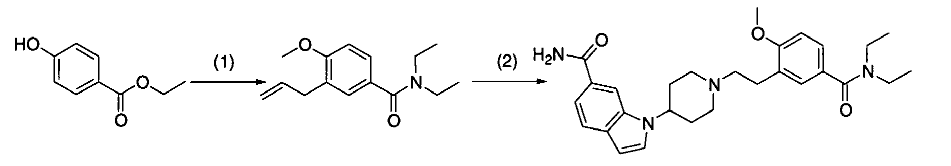

### (1) Synthesis of 3-allyl-N,N-diethyl-4-methoxybenzamide

The title compound was obtained by synthesizing from ethyl 4-hydroxybenzoate in accordance with the methods in example 30-(1), (2) and (3).
¹H-NMR(CDCl3)
δ: 1.05-1.77(m,6H), 3.20-3.60(m,4H), 3.38(d, *J*=6.0Hz,2H), 3.84(s,3H), 5.01-5.08(m,2H), 5.90-6.02 (m,1H), 6.82(d, *J*=8.0Hz,1H), 7.17(d, *J*=2.4Hz,1H), 7.24(dd, *J*=2.4,8.0Hz,1H).

### (2) Synthesis of 1-[1-[2-(5-diethylcarbamoyl-2-methoxyphenyl) ethyl]piperidin-4-yl]-1H-indole-6-carboxamide

The title compound was obtained by synthesizing from 3-allyl-N, N-diethyl-4-methoxybenzamide in accordance with the methods in example 1-(3).
¹H-NMR(DMSO-d6)
δ: 1.05-1.17(m,6H), 1.92-2.07(m,4H), 2.22-2.31(m,2H), 2.51-2.60(m,2H), 2.75-2.82(m,2H), 3.06-3.15(m,2H), 3.22-3.40(m,8H), 3.84(s,3H), 4.37-4.47(m,1H), 6.50(d, *J*=3.2Hz, 1H), 6.99(d, *J*=8.8Hz, 1H), 7.16-7.25(m,3H), 7.53-7.61 (m, 2H), 7.65 (d, *J*=3.2Hz, 1H), 7.91(brs, 1H), 8.12(s, 1H).

### Example 34

### Synthesis of 1-[1-[2-[2-methoxy-5-(pyrrolidine-1-carbonyl) phenyl]ethyl]piperidin-4-yl]-1H-indole-6-carboxamide

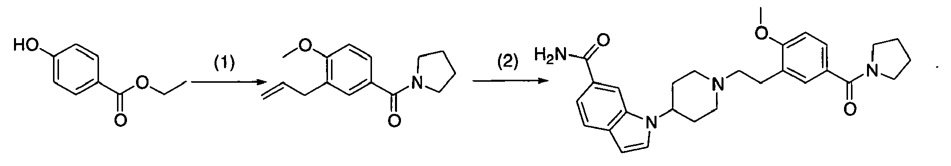

### (1) Synthesis of (3-allyl-4-methoxyphenyl)pyrrolidin-1-yl methanone

The title compound was obtained by synthesizing from ethyl 4-hydroxybenzoate in accordance with the methods in example 30- (1), (2) and (3).
¹H-NMR(CDCl3)
δ: 1.81-2.00(m,4H), 3.38(d, *J*=6.0Hz,2H), 3.42-3.52(m,2H), 3.57-3.67 (m,2H), 3.85 (s, 3H), 5.01-5.08 (m, 2H), 5.91-6.02 (m, 1H), 6.83(d, *J*=7.2Hz,1H), 7.33-7.37(m,1H), 7.38-7.43(m,1H).

### (2) Synthesis of 1- [1-[2-[2-methoxy-5-(pyrrolidine-1-carbonyl) phenyl]ethyl] piperidin-4-yl]-1H-indole-6-carboxamide

The title compound was obtained by synthesizing from (3-allyl-4-methoxyphenyl)pyrrolidin-1-yl methanone in accordance with the methods in example 1-(3).
¹H-NMR (DMSO-d6)
δ: 1.75-1.91(m,4H), 1.92-2.07(m,4H), 2.22-2.31(m,2H), 2.52-2.60(m,2H), 2.75-2.83(m,2H), 3.07-3.15(m,2H), 3.41-3.49(m,4H), 3.85(s,3H), 4.37-4.47(m,1H), 6.50(d, *J*=3.2Hz,1H), 6.99 (d, *J*=8.8Hz,1H), 7.21 (brs, 1H) , 7.38-7.44(m,2H), 7.54-7.61 (m, 2H), 7.66 (d, *J*=3.2Hz, 1H), 7.91 (brs, 1H), 8.13 (s,1H).

### Example 35

### Synthesis of 1-[1-[2-(2,5-dimethoxyphenyl)-2-hydroxyethyl] piperidin-4-yl]-1H-indole-6-carboxamide

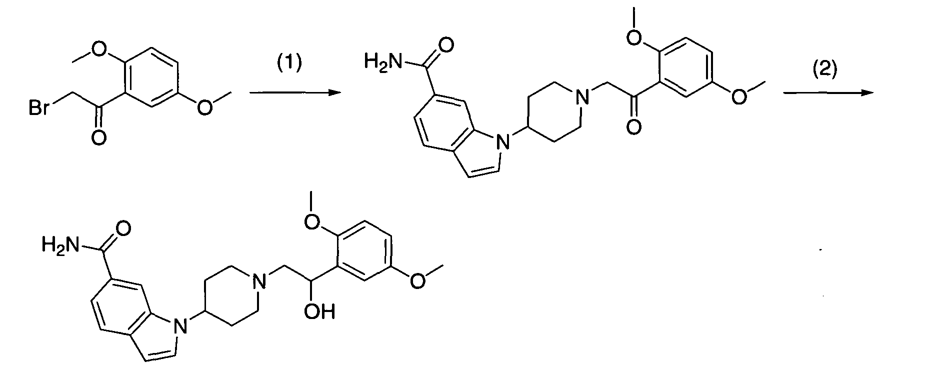

### (1) Synthesis of 1- [1- [2- (2, 5-dimethoxyphenyl) -2-oxoethyl] piperidin-4-yl]-1H-indole-6-carboxamide

200mg of 1-(piperidin-4-yl)-1*H*-indole-6-carboxamide synthesized in Production example 1 was dissolved in 10ml of methylene chloride, 0.19g of triethylamine and 0.38g of 2-bromo-2',5'-dimethoxyacetophenone was added an in ice-cooling, which was stirred for 3 hours at room temperature. The reaction liquid was diluted in chloroform, and washed with water and brine. The organic layer was concentrated under a reduced pressure after drying over magnesium sulfate, and purified by silica gel column chromatography (ethyl acetate-methanol) to give 344mg of the title compound.
¹H-NMR (DMSO-d6)
δ: 1.90-2.07(m,4H), 2.47-2.55(m,2H), 3.00-3.07(m,2H), 3.74(s,3H), 3.84(s,2H), 3.85(s,3H), 4.37-4.47(m,1H), 6.50(d, *J*=3.2Hz,1H), 7.09-7.15(m,3H), 7.21(brs,1H), 7.53-7.61(m,2H), 7.68(d, *J*=3.2Hz,1H), 7.92(brs,1H), 8.12(s,1H).

### (2) Synthesis of 1-[1-[2-(2,5-dimethoxyphenyl)-2-hydroxyethyl] piperidin-4-yl]-1H-indole-6-carboxamide

159mg of 1-[1-[2-(2,5-dimethoxyphenyl)-2-oxoethyl] piperidin-4-yl]-1*H*-indole-6-carboxamide was dissolved in 5ml of methanol and 2ml of methylene chloride, 23mg of sodium borohydride was added in an ice-cooling, which was stirred for 2 hours at room temperature. The reaction liquid was concentrated under a reduced pressure, water was added to the residue, and extracted with chloroform. The extract was concentrated under a reduced pressure after drying over magnesium sulfate, and purified by NH silica gel column chromatography (ethyl acetate-methanol) to give 152mg of the title compound.
¹H-NMR (DMSO-d6)
δ: 1.90-2.11(m,4H), 2.32-2.52(m,4H), 3.07-3.20(m,2H), 3.71(s,3H), 3.75(s,3H), 4.35-4.46(m,1H), 4.93(d, *J*=4.4Hz,1H), 5.04-5.10(m,1H) 6.50(d, *J*=3.2Hz,1H), 6.76(dd, *J*=3.2,8.8Hz,1H), 6.88(d, *J*=8.8Hz,1H), 7.03(d, *J*=3.2Hz,1H), 7.21(brs,1H), 7.53-7.61(m,2H), 7.66 (d, *J*=3.2Hz,1H), 7.91(brs,1H), 8.12(s,1H).

### Example 36

### Synthesis of 1-[1-[2-(2,5-dimethoxyphenyl)-2-fluoroethyl] piperidin-4-yl]-1H-indole-6-carboxamide

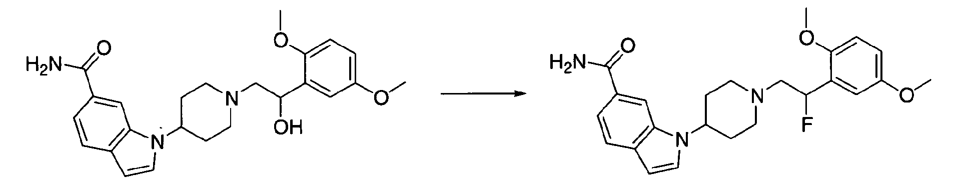

Under nitrogen atmosphere, 38mg of diethylaminosulfur trifluoride was dissolved in 3ml of methylene chloride, 2ml of methylene chloride solution of 66mg of 1-[1-[2-(2,5-dimethoxyphenyl)-2-hydroxyethyl]piperidin-4-yl]-1H-indole-6-carboxamide synthesized in example 35 was added at -78°C, which was stirred for 1 hour. After stirring in an ice-cooling for 30 minutes, water was added. A saturated sodium bicarbonate aqueous solution was then added, and extracted with chloroform. The extract was concentrated under a reduced pressure after drying over magnesium sulfate, which was purified by silica gel column chromatography (ethyl acetate-methanol) to give 16mg of the title compound.
¹H-NMR (DMSO-d6)
δ: 1.92-2.00(m,4H), 2.37-2.50(m,2H), 2.57-2.73(m,1H), 2.83-2.96(m,1H), 3.06-3.21(m,2H), 3.73(s,3H), 3.78(s,3H), 4.38-4.48(m,1H), 5.88-6.05(m,1H), 6.51(d, *J*=3.2Hz,1H), 6.88-6.94(m,2H), 6.99(d, *J*=8.8Hz, 1H), 7.21(brs, 1H), 7.54-7.61(m,2H), 7.69(d, *J*=3.2Hz,1H), 7.91(brs,1H), 8.13(s,1H).

### Example 37

### Synthesis of 1-[1-[2-(2,5-dimethoxyphenyl)propyl]piperidin-4-yl]-1H-indole-6-carboxamide

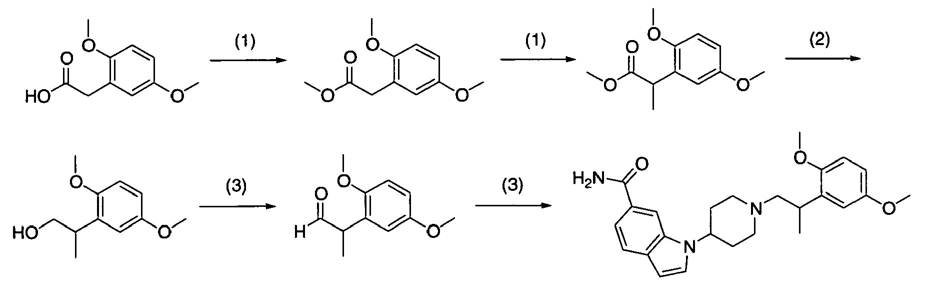

### (1) Synthesis of methyl 2-(2,5-dimethoxyphenyl)propionate

19.6g of 2,5-dimethoxyphenylacetic acid was dissolved in 100ml of methanol, 0.3ml sulfuric acid was added, and heated to reflux overnight. The mixture was concentrated under a reduced pressure after standing to cool to room temperature, the residue was diluted to diethyl ether and washed with water, saturated sodium bicarbonate aqueous solution and brine. The organic layer was purified by silica gel column chromatography (diethyl ether) after drying over magnesium sulfate to give 20.7g of methyl 2,5-dimethoxyphenylacetate.
5.00g of methyl 2,5-dimethoxyphenylacetate was dissolved in 100ml of tetrahydrofuran, 1.05g of 60% sodium hydride was added in an ice-cooling, which stirred for 15 minutes at room temperature. In the ice-cooling, 4.39g of iodomethane was added, and was stirred overnight. The reaction liquid was diluted to diethyl ether and washed with water and brine. The organic layer was concentrated under a reduced pressure after drying over magnesium sulfate, and purified by silica gel column chromatography (hexane-ethyl acetate) to give 4.12g of the title compound.
¹H-NMR(CDCl₃)
δ: 1.44(d, *J*=6.4Hz,3H), 3.66(s,3H), 3.77(s,3H), 3.78(s,3H), 4.03(q, *J*=6.4Hz, 1H), 6.75(dd, *J*=3.2, 8.8Hz, 1H), 6.80(d, *J*=3.2Hz,1H), 6.81(d, *J*=8.8Hz,1H).

### (2) Synthesis of 2-(2,5-dimethoxyphenyl)propan-1-ol

2.00g of methyl 2-(2,5-dimethoxyphenyl)propionate was dissolved in 40ml of tetrahydrofuran, 0.85g of 80% lithium aluminum hydride was added in an ice-cooling, which was stirred for 2 hours at room temperature. In the ice-cooling, water, and then 2N HCl was added and extracted with ethyl acetate. The extract was washed with 2N HCl, saturated sodium bicarbonate aqueous solution and brine. The organic layer was concentrated under a reduced pressure after drying over magnesium sulfate to yield 1.70g of the title compound.
¹H-NMR(CDCl3)
δ : 1.25(d, *J*=6.8Hz,3H), 3.36-3.46(m,1H), 3.67-3.75(m,2H), 3.77(s,3H), 3.79(s,3H), 6.72(dd, *J*=2.8,8.8Hz,1H), 6.79(d, J=2.8Hz,1H), 6.81(d, *J*=8.8Hz,1H).

### (3) Synthesis of 1- [1- [2- (2, 5-dimethoxyphenyl) -propyl] piperidin-4-y1]-1H-indole-6-carboxamide

Under nitrogen atmosphere, 126mg of 2-(2,5-dimethoxyphenyl)propan-1-ol was dissolved in 2ml of triethylamine and 4ml of dimethyl sulfoxide, 0.82g of sulfur trioxide-pyridine was added, and stirred for 30 minutes at room temperature. The reaction liquid was diluted with diethyl ether and washed with water, saturated ammonium aqueous solution, saturated sodium bicarbonate aqueous solution, water and brine. The organic layer was concentrated under a reduced pressure after drying over magnesium sulfate, and purified by silica gel column chromatography (hexane-ethyl acetate) to yield 81mg of 2-(2,5-dimethoxyphenyl)propionaldehyde with slightly sulfurous odor. This compound was used in the next reaction without further purification.
70mg of 1-(piperidin-4-yl)-1*H*-indole-6-carboxamide, which was synthesized in Production example 1, and 80mg of 2- (2,5-dimethoxyphenyl)propionaldehyde were dissolved in 3ml of tetrahydrofuran, 0.05ml of acetic acid and 91mg of sodium triacetoxyborohydride was added, which was stirred for 7 hours at room temperature. Saturated sodium bicarbonate aqueous solution was added to reaction liquid, and extracted with chloroform. The extract was concentrated under a reduced pressure after drying over magnesium sulfate, and purified by silica gel column chromatography (ethyl acetate-methanol) to give 99mg of the title compound.
¹H-NMR(DMSO-d6)
δ: 1.18(d, *J*=6.8Hz,3H), 1.90-2.03(m,4H), 2.08-2.18(m,1H), 2.20-2.30 (m,1H), 2.41-2.50(m,2H), 2.97-3.05(m,1H), 3.08-3.15 (m,1H), 3.30-3.40(m,1H), 3.70(s,3H), 3.74(s,3H), 4.33-4.43(m,1H), 6.49(d, *J*=3.2Hz,1H), 6.73(dd, *J*=2.8,9.2Hz,1H), 6.78(d, *J*=2.8Hz,1H), 6.88(d, *J*=9.2Hz,1H), 7.20 (brs,1H), 7.53-7.60(m,2H), 7.67(d, *J*=3.2Hz,1H), 7.90 (brs,1H), 8.11 (s,1H).

### Example 38

### Synthesis of 1-[1-[2-(5-dimethylcarbamoyl-2-methoxyphenyl) ethyl]piperidin-4-yl]-1H-indole-6-carboxamide

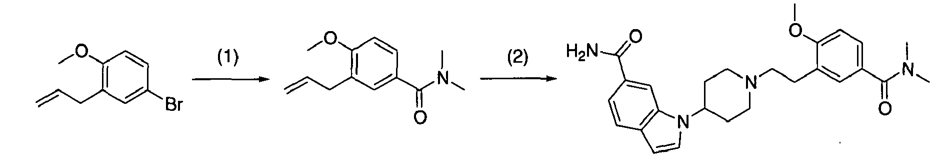

### (1) Synthesis of 3-allyl-4-methoxy-N,N-dimethylbenzamide

600mg of 3-allyl-l-bromo-4-methoxybenzene (CAS No: 114303-65-0) was dissolved in 15ml of tetrahydrofuran under nitrogen atmosphere, 1.83ml of *n-*butyllithium (1.59M hexane solution) was added at -78°C, which was stirred for 15 minutes. 0.29ml of N,N-dimethylformamide was added at -78°C and the mixture was slowly raised to 0°C. After quenching with saturated ammonium aqueous solution, the reaction liquid was diluted in ethyl acetate and washed with saturated ammonium aqueous solution and brine. The organic layer was concentrated under a reduced pressure after drying over magnesium sulfate, and purified by silica gel column chromatography (hexane-ethyl acetate) to give 370mg of the title compound.
¹H-NMR(CDCl3)
δ: 3.03(brs,6H), 3.37(d, J=6.4Hz,2H), 3.83(s,3H), 5.01-5.08(m,2H), 5.90-6.02(m,1H), 6.83(d, *J*=8.4Hz,1H), 7.23(d, *J*=2.4Hz,1H), 7.29(dd, *J*=2.4,8.4Hz,1H).

### (2) Synthesis of 1-[1-[2-(5-dimethylcarbamoyl-2-methoxyphenyl) ethyl]piperidin-4-yl]-1H-indole-6-carboxamide

The title compound was obtained by synthesizing from 3-allyl-4-methoxy-N,N-dimethylbenzamide in accordance with the methods in example 1-(3).
¹H-NMR(DMSO-d6)
δ: 1.92-2.06(m,4H), 2.21-2.31(m,2H), 2.52-2.60(m,2H), 2.75-2.82(m,2H), 2.96(s,6H), 3.06-3.14(m,2H), 3.84(s,3H), 4.36-4.47(m,1H), 6.50(d, *J*=3.2Hz,1H), 7.00(d, *J*=8.8Hz,1H), 7.21(brs,1H), 7.25-7.30(m,2H), 7.53-7.61(m,2H), 7.66(d, *J*=3.2Hz,1H), 7.91(brs,1H), 8.12(s,1H).

### Example 39

### Synthesis of 1-[1-[2-(5-dimethylcarbamoyl-2-methoxyphenyl) ethyl]piperidin-4-yl]-N-methyl-1H-indole-6-carboxamide

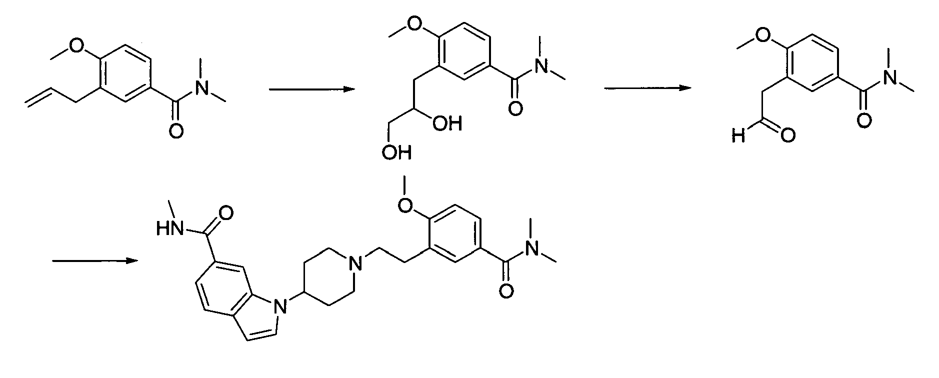

104mg of 3-allyl-4-methoxy-N,N-dimethylbenzamide was dissolved in 10ml of *tert*-butanol-water (1:1), 0.66g of AD-mix-β was added, which was stirred overnight at room temperature. 0.71g of sodium sulfite was added in an ice-cooling, which was stirred for 1 hour at room temperature. Brine was added to reaction liquid, and extracted with methylene chloride. The extract was concentrated under a reduced pressure after drying over magnesium sulfate to yield 150mg of 3-(2,3-dihydroxypropyl)-4-methoxy-N,N-dimethylbenzamide. This compound was used in the next reaction without further purification.
150mg of 3-(2,3-dihydroxypropyl)-4-methoxy-N,N-dimethylbenzamide was dissolved in 2ml of tetrahydrofuran and 5ml of methanol, aqueous 5ml solution of 0.20g of sodium metaperiodate was added in an ice-cooling, which was stirred for 30 minutes at room temperature. The reaction liquid was diluted in ethyl acetate and washed with water and brine. The organic layer was concentrated under a reduced pressure after drying over magnesium sulfate to yield 95mg of 4-methoxy-N,N-dimethyl-3-(2-oxoethyl)benzamide. This compound was used in the next reaction without further purification.
70mg of 1-(piperidin-4-yl)-N-methyl-1*H*-indole-6-carboxamide and 95mg of 4-methoxy-N,N-dimethyl-3-(2-oxoethyl)benzamide were dissolved in 6ml of methylene chloride, 0.03ml of acetic acid and 86mg of sodium triacetoxyborohydride were added, which was stirred for 3 hours at room temperature. Saturated sodium bicarbonate aqueous solution was added to reaction liquid and extracted with chloroform. The extract was concentrated under a reduced pressure after drying over magnesium sulfate, and purified by silica gel column chromatography (methanol-ethyl acetate) to give 120mg of the title compound.
¹H-NMR (DMSO-d6)
δ: 1.93-2.07(m,4H), 2.22-2.31(m,2H), 2.53-2.60(m,2H), 2.76-2.83(m,2H), 2.82(d, *J*=4.4Hz,3H), 2.96(s,6H), 3.07-3.15(m,2H), 3.84(s,3H), 4.36-4.45(m,1H), 6.50(d, *J*=3.2Hz, 1H), 7.00(d, *J*=9.2Hz, 1H), 7.25-7.30(m,2H), 7.51-7.58(m,2H), 7.65(d, *J*=3.2Hz, 1H), 8.05(s,1H), 8.30-8.37(m, 1H).

### Example 40

### Synthesis of 1-[1-[2-[5-(acetyl methylamino)-2-methoxyphenyl] ethyl]piperidin-4-yl]-N-methyl-1H-indole-6-carboxamide

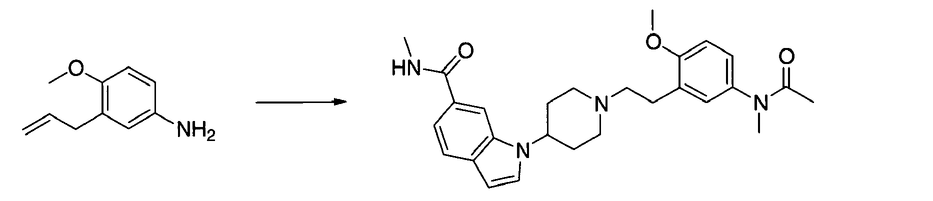

The title compound was obtained by synthesizing from 1-allyl-5-amino-2-methoxybenzene in accordance with the methods in example 1-(1) and example 39.
¹H-NMR (DMSO-d6)
δ: 1.76(s,3H), 1.92-2.06(m,4H), 2.21-2.31(m,2H), 2.52-2.61(m,2H), 2.73-2.81(m,2H), 2.82(d, *J*=4.4Hz,3H), 3.05-3.15(m,2H), 3.11(s,3H), 3.83(s,3H), 4.35-4.46(m,1H), 6.50(d, *J*=2.8Hz,1H), 7.00(d, *J*=8.0Hz,1H), 7.11-7.18(m,2H), 7.50-7.59 (m,2H), 7.64(d, *J*=2.8Hz, 1H), 8.05(s, 1H), 8.30-8.37 (m, 1H).

### Example 41

### Synthesis of 1-[1-[2-(2-methoxy-5-methoxymethylphenyl)ethyl] piperidin-4-yl]-1H-indole-6-carboxamide

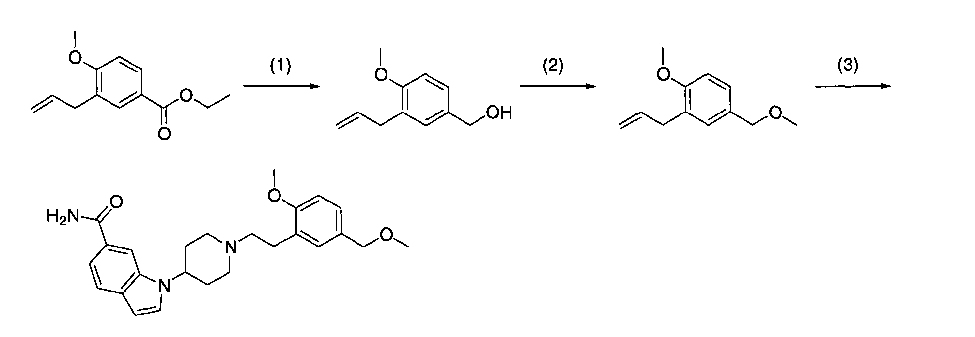

### (1) Synthesis of (3-allyl-4-methoxyphenyl)methanol

1.95g of ethyl 3-allyl-4-methoxybenzoate was dissolved in 50ml of tetrahydrofuran, 0.84g of lithium aluminum hydride was added in an ice-cooling, which was stirred for 30 minutes in an ice-cooling and for another 30 minutes at room temperature. 0.84ml of water, 0.84ml of 5N aqueous sodium hydroxide and 2.52ml of water were added to reaction liquid in an ice-cooling, which was stirred at room temperature. The reaction liquid was filtered through Celite, the filtrate was concentrated under a reduced pressure and purified by silica gel column chromatography (hexane-ethyl acetate) to give 1.50g of the title compound.
¹H-NMR(CDCl3)
δ: 3.38 (d, *J*=6.4Hz, 2H), 3.83 (s, 3H), 4.60 (s, 2H) , 5.02-5.09 (m,2H), 5.93-6.05 (m,1H), 6.84(d, *J*=8.0Hz,1H) 7.15(d, *J*=2.0Hz,1H) 7.20(dd *J*=2.0,8.0Hz,1H).

### (2) Synthesis of 2-allyl-1-methoxy-4-(methoxymethyl)benzene

0.93g of (3-allyl-4-methoxyphenyl)methanol was dissolved in 20ml of tetrahydrofuran, 0.30g of 60% sodium hydride was added in an ice-cooling, which was stirred at room temperature for 15 minutes. 2.22g of iodomethane was added in an ice-cooling, which was stirred for 3 hours at room temperature. The reaction liquid was diluted in ethyl acetate and washed with saturated ammonium aqueous solution and brine. The organic layer was concentrated under a reduced pressure after drying over magnesium sulfate, and purified by NH silica gel column chromatography (hexane-ethyl acetate) to give 879mg of the title compound.
¹H-NMR(CDCl3)
δ: 3.36(s,3H), 3.38(d, J=6.4Hz,2H), 3.83(s,3H), 4.37(s,2H), 5.02-5.09(m,2H), 5.93-6.05(m,1H) 6.83(d, J=8.0Hz,1H), 7.12(d, J=2.4Hz,1H), 7.16(dd, J=2.4,8.0Hz,1H).

### (3) Synthesis of 1- [1- [2- (2-methoxy-5-methoxymethylphenyl)ethyl]piperidin-4-yl]-1H-indole-6-carboxamide

The title compound was obtained by synthesizing from 2-allyl-1-methoxy-4-(methoxymethyl)benzene in accordance with the methods in example 1-(3).
¹H-NMR(DMSO-d6)
5: 1.92-2.09(m,4H), 2.22-2.31(m,2H), 2.50-2.58(m,2H), 2.73-2.80(m,2H), 3.07-3.15(m,2H), 3.25(s,3H), 3.80(s,3H), 4.31(s,2H), 4.37-4.47(m,1H), 6.51(d, J=3.2Hz,1H), 6.90-6.96(m,1H), 7.11-7.16(m,2H), 7.21(brs,1H), 7.53-7.61 (m, 2H), 7.67 (d, *J=*3.2Hz, 1H), 7.91(brs, 1H), 8.13(s, 1H).

### Example 42

### Synthesis of 1-[1-[2-(5-hydroxymethyl-2-methoxyphenyl)ethyl] piperidin-4-yl]-1H-indole-6-carboxamide

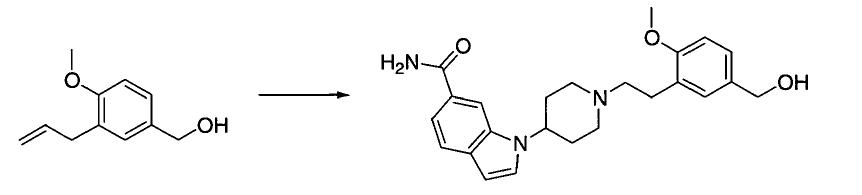

The title compound was obtained by synthesizing from (3-allyl-4-methoxyphenyl)methanol in accordance with the methods in example 1-(3).
¹H-NMR(DMSO-d6)
δ: 1.91-2.10(m,4H), 2.22-2.31(m,2H), 2.50-2.57(m,2H), 2.73-2.80(m,2H), 3.07-3.15(m,2H), 3.78(s,3H), 4.40(d, J=5.6Hz,2H), 4.37-4.47(m,1H), 5.01(t, *J*=5.6Hz,1H), 6.50(d, *J*=3.2Hz,1H), 6.88-6.92(m,1H), 7.09-7.14(m,2H), 7.21(brs,1H), 7.53-7.61(m,2H), 7.67(d, *J*=3.2Hz, 1H), 7.91 (brs, 1H), 8.13 (s,1H).

### Example 43

### Synthesis of 1-[1-[2-(2,5-dimethoxyphenyl)-ethyl] piperidin-4-yl]-1H-indole-6-carboxamide

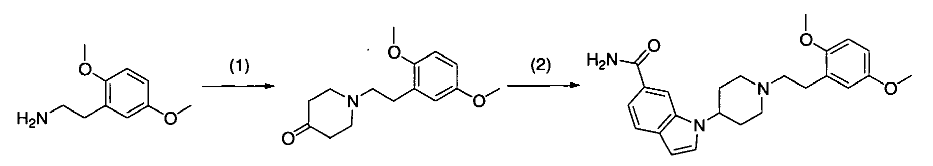

### (1) Synthesis of 1-[2-(2,5-dimethoxyphenyl)ethy1] piperidin-4-one

The title compound was obtained by synthesizing from 2,5-dimethoxyphenylamine in accordance with the methods disclosed in J. Org. Chem., 1991,56,2701.
¹H-NMR(CDCl3)
δ : 2.45-2.52(m,4H), 2.66-2.73(m,2H), 2.79-2.88(m,6H), 3.77(s,3H), 3.79(s,3H), 6.69-6.81(m,3H).

### (2) Synthesis of 1-[1-[2-(2,5-dimethoxyphenyl)ethyl] piperidin-4-yl]-1H-indole-6-carboxamide

50mg of 3-amino-4-(2,2-dimethoxyethyl)benzamide, which was synthesized in Production example 3, and 117mg of 1-[2-(2,5-dimethoxyphenyl)ethyl]piperidin-4-one were dissolved in 3ml of acetic acid, 0.32g of sodium sulfate was added, which was stirred for 30 minutes at room temperature. 0.14g of sodium triacetoxyborohydride was added and stirred for 1 hour, 3ml of water was added and stirred for 2 hours at 100°C. Vacuum concentration was made after standing to cool at room temperature, saturated sodium bicarbonate aqueous solution was added to the residue and extracted with methylene chloride. The extract was concentrated under a reduced pressure after drying over magnesium sulfate, and purified by silica gel column chromatography (ethyl acetate-methanol) to give 90mg of the title compound.
¹H-NMR(DMSO-d6)
δ: 1.93-2.08(m,4H), 2.22-2.31(m,2H), 2.51-2.57(m,2H), 2.71-2.77(m,2H), 3.07-3.14(m,2H), 3.70(s,3H), 3.74(s,3H), 4.37-4.47(m,1H), 6.50(d, *J=* 3.2Hz,1H), 6.73(dd, *J=*3.2,8.8Hz,1H) 6.80(d, *J*=3.2Hz,1H), 6.87(d, *J*=8.8Hz,1H), 7.21(brs,1H), 7.54-7.60(m,2H), 7.67(d, *J*=3.2Hz, 1H),7.91(brs,1H), 8.13(s,1H).

### Example 44

### Synthesis of 1-[1-(2-methoxy-6-oxazol-5-ylphenethyl)piperidin-4-yl]-1H-indole-6-carboxamide

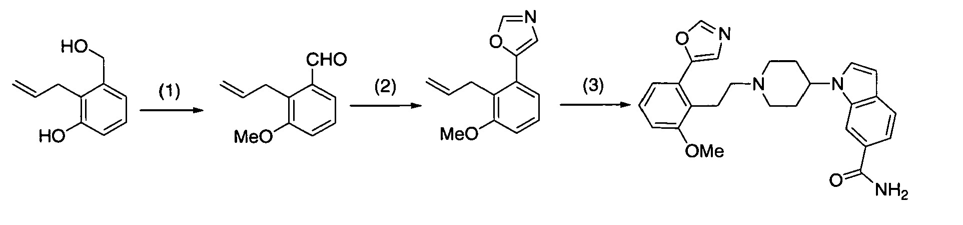

### (1) Synthesis of 2-allyl-3-methoxybenzaldehyde

1.00g of 2-allyl-3-methoxybenzyl alcohol which was prepared from 2-allyl-3-hydroxybenzylalcohol *(*Tetrahedron 56 (2000) 13, 1873-1882) according to example 1- (2) was dissolved in 30ml of dichloromethane, 3.09g of Dess-Martin reagent was added. After completion of reaction, a saturated sodium thiosulfate solution was added and extracted with chloroform. The organic layer was washed with saturated sodium bicarbonate aqueous solution, dried over anhydrous magnesium sulfate. After removal of drying agent by filtration, concentration was carried out under a reduced pressure, and the residue was purified by silica gel column chromatography (hexane-ethyl acetate) to give 920mg of the title compound.
¹H-NMR(CDCl₃)
5: 3.82-3.86(m,2H), 3.87(s,3H), 4.88-5.03(m,2H), 5.95-6.06(m,1H), 7.11(dd, *J*=1*.*2,8.0Hz,1H), 7.34 (t, *J*=8.0Hz,1H), 7.47(dd, *J*=1.2,8.0Hz,1H), 10.26(s,1H).

### (2) Synthesis of 5-(2-allyl-3-methoxyphenyl)oxazole

300mg of 2-allyl-3-methoxybenzaldehyde was dissolved in 10ml of methanol, 498mg of tosylmethyl isocyanide, 470mg of potassium carbonate were added and the mixture was heated to reflux. After completion of reaction, ethyl acetate and brine were added, and the organic layer was partitioned. The organic layer was dried over anhydrous magnesium sulfate, concentration was carried out under a reduced pressure after removal of drying agent by filtration and the residue was purified by silica gel column chromatography (hexane-ethyl acetate) to give 273mg of the title compound.
¹H-NMR(CDCl3)
δ : 3.52-3.56(m,2H), 3.86(s,3H), 4.84-4.92(m,1H), 4.98-5.03(m,1H), 5.93-6.04(m,1H), 6.92(dd, *J*=1.2,8.0Hz, 1H), 7.20(dd, *J*=1.2,8.0Hz,1H), 7.24 (s,1H), 7.28 (t, *J*=8.0Hz,1H), 7.93 (s,1H).

### (3) Synthesis of 1-[1-(2-methoxy-6-oxazol-5-ylphenethyl)-piperidin-4-yl]-1H-indole-6-carboxamide

The title compound was obtained from 5-(2-allyl-3-methoxyphenyl)oxazole in accordance with the methods in example 1-(3).
¹H-NMR(CDCl3)
δ: 2.04-2.20(m,4H), 2.22-2.36(m,2H), 2.58-2.67 (m,2H), 2.98-3.06(m,2H), 3.14-3.24(m,2H), 3.89 (s,3H), 4.33-4.44 (m,1H), 6.57 (dd, *J*=0.8,3.2Hz,1H), 6.92(dd, *J*=0.8,8.4Hz,1H), 7.16(dd, *J*=1.2,8.0Hz,1H), 7.20-7.32(m,2H), 7.38-7.43(m,2H), 7.63(dd, *J*=0.8,8.4Hz,1H), 7.98 (s,1H), 8.09(s,1H).

### Example 45

### Synthesis of 1-[1-(2-methoxy-5-oxazol-5-ylphenethyl)-piperidin-4-yl]-1H-indole-6-carboxamide

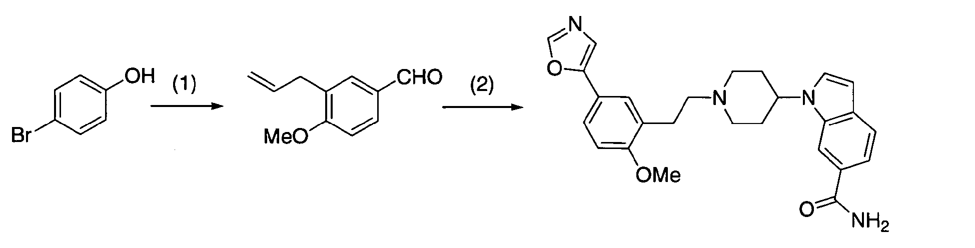

### (1) Synthesis of 3-allyl-4-methoxybenzaldehyde

2-allyl-4-bromoanisole, which was synthesized from 4-bromophenol according to example 1-(1) and (2), was dissolved in 50ml of tetrahydrofuran, which was cooled to -70°C, 4.78ml of *n-*butyllithium solution (2.60 mol/l hexane solution) was added. After stirring for 15 minutes, 4.10ml of N,N-dimethylformamide was added and then raised to a room temperature. After completion of reaction, saturated ammonium aqueous solution and ethyl acetate were added, and the organic layer was partitioned. The organic layer was washed with brine, then dried over anhydrous magnesium sulfate. After removal of drying agent by filtration, concentration was carried out under a reduced pressure, and the residue was purified by silica gel column chromatography (hexane-ethyl acetate) to give 1.76g of the title compound.
¹H-NMR(CDCl3)
5: 3.39-3.43(m,2H), 3.92(s,3H), 5.04-5.11(m,2H), 5.92-6.03(m,1H), 6.95(d, J=8.4Hz,1H), 7.68(d, *J*=2.4Hz,1H), 7.74(dd, *J*=2.4,8.4Hz,1H), 9.85(s,1H).

### (2) Synthesis of 1-[1-(2-methoxy-5-oxazol-5-ylphenethyl) piperidin-4-yl]-1H-indole-6-carboxamide

The title compound was obtained in accordance with the methods in example 44-(2) and (3).
¹H-NMR(CDCl3)
5: 2.08-2.20(m,4H), 2.26-2.36(m,2H), 2.64-2.72(m,2H), 2.86-2.94(m,2H), 3.18-3.26(m,2H), 3.88(s,3H), 4.34-4.45(m,1H) 6.57(dd, *J*=0.8,3.2Hz,1H), 6.90(d, *J*=8.4Hz,1H), 7.23(s,1H), 7.37-7.42(m,2H), 7.45-7.52(m,2H), 7.63(dd, *J*=0.8,8.4Hz,1H), 7.87(s,1H), 8.10(s,1H).

### Example 46

### Synthesis of 1-[1-[2-(3-methoxy-6-methoxymethylpyridin-2-yl) ethyl] piperidin-4-yl]-1H-indole-6-carboxamide

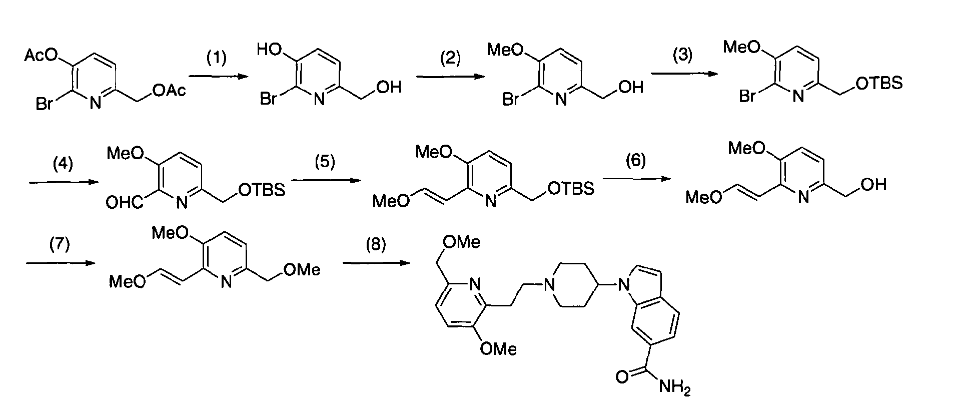

### (1) Synthesis of 2-bromo-6-hydroxymethylpyridin-3-ol

24.4g of 6-acetoxymethyl-2-bromopyridin-3-yl acetate *(*Aust. J. Chem. 1983, 36, p2565) was dissolved in 150ml of methanol, 7.13g of lithium hydroxide monohydrate was added. After completion of reaction, pH was adjusted 5 to 6 with 5N HCl, then concentrated under a reduced pressure and the obtained residue was purified by silica gel column chromatography (hexane/ethyl acetate to ethyl acetate / methanol) to give 15.1g of the title compound.
¹H-NMR(DMSO-d6)
δ: 4.40(s,2H), 5.34(brs,1H), 7.27(s,2H).

### (2) Synthesis of 2-bromo-6-hydroxymethyl-3-methoxypyridine

15g of 2-bromo-6-hydroxymethylpyridin-3-ol was dissolved in 300ml of acetone, 15.2g of potassium carbonate and 5.72ml of methyl iodide were added, which was heated to reflux. After completion of reaction, solid was filtered, and the filtrate was concentrated under a reduced pressure. The residue was purified by silica gel column chromatography (hexane/ethyl acetate to ethyl acetate / methanol) to give 13.9g of the title compound.
¹H-NMR(CDCl3)
δ: 2.73(t, *J*=6.0Hz,1H) 3.92(s,3H), 4.69(d, *J*=6.0Hz,2H), 7.15(d, *J*=8.4Hz,1H), 7.24(d, *J*=8.4Hz, 1H).

### (3) Synthesis of 2-bromo-6-(tert-butyldimethylsilyloxymethyl)-3-methoxypyridine

3.00g of 2-bromo-6-hydroxymethyl-3-methoxypyridine was dissolved in 50ml of N,N-dimethylformamide, 2.82g of imidazole, 2.40g of *tert*-butyldimethylsilyl chloride was successively added, which was stirred at room temperature. After completion of reaction, saturated ammonium aqueous solution and ethyl acetate were added, the organic layer was partitioned, the resulting organic layer was washed with brine, and then dried over anhydrous magnesium sulfate. After removal of drying agent by filtration, concentration was carried out under a reduced pressure, and the residue was purified by silica gel column chromatography (hexane-ethyl acetate) to give 4.26g of the title compound.
¹H-NMR(CDCl3)
δ: 0.11(s,6H), 0.95(s,9H), 3.91(s,3H), 4.76(s,2H), 7.16(d, *J*=8.0Hz, 1H), 7.40(d, *J*=8.0Hz, 1H).

### (4) Synthesis of 6-(tert-butyldimethylsilyloxymethyl)-3-methoxypyridine-2-carbaldehyde

The title compound was obtained from 2-bromo-6-(*tert*-butyldimethylsilyloxymethyl)-3-methoxypyridine in accordance with the methods in example 45- (1).
¹H-NMR(CDCl3)
δ: 0.12(s,6H), 0.95(s,9H), 3.96(s,3H), 4.85(s,2H), 7.44(d, *J*=8.8Hz,1H), 7.71(d, *J*=8.8Hz,1H), 10.3(s,1H).

### (5) Synthesis of 6-(tert-butyldimethylsilyloxymethyl)-3-methoxy-2-(2-methoxyvinyl)pyridine

2.21g of (methoxymethyl)triphenylphosphonium chloride was suspended in 25ml of tetrahydrofuran, and cooled on an ice. 652mg of potassium *tert*-butoxide was added thereto, which was stirred for 10 minutes, 908mg of 6-(*tert*-butyldimethylsilyloxymethyl)-3-methoxypyridine-2-carbaldehyde was then added to reaction liquid using 5ml of tetrahydrofuran. After completion of reaction, saturated ammonium aqueous solution and ethyl acetate were added and the organic layer was partitioned. The resulting organic layer was washed with brine and then dried over anhydrous magnesium sulfate. Concentration was carried out under a reduced pressure after removal of drying agent by filtration, and the residue was purified by silica gel column chromatography (hexane-ethyl acetate) to give 248mg of the title compound.
¹H-NMR(CDCl3)
δ: 0.11 (s, 6H), 0.95 (s, 9H), 3.80(s, 3H), 3.82(s, 3H), 4.81(s,2H), 5.66(d, *J*=7.6Hz, 1H), 6.26(d, *J*=7.6Hz, 1H), 7.11(d, *J*=8.8Hz, 1H), 7.25(d, *J*=8.8Hz, 1H).

### (6) Synthesis of [5-methoxy-6-(2-methoxyvinyl)pyridin-2-yl] methanol

248mg of 6-(*tert*-butyldimethylsilyloxymethyl)-3-methoxy-2-(2-methoxyvinyl)pyridine was dissolved in 5ml of tetrahydrofuran, 0.9ml of tetrabutylammonium fluoride (1 mol/L tetrahydrofuran solution) was added. After completion of reaction, the reaction liquid was concentrated under a reduced pressure and the residue was purified by NH silica gel column chromatography (ethyl acetate) to give 139mg of the title compound.
¹H-NMR(CDCl3)
δ: 3.83(s,3H), 3.84(s,3H), 4.66(s,2H), 5.71(d, *J*=7.6Hz, 1H), 6.33(d, *J*=7.6Hz, 1H), 6.93(d, *J*=8.4Hz, 1H), 7.10(d, *J*=8.4Hz, 1H).

### (7) Synthesis of 3-methoxy-6-methoxymethyl-2-(2-methoxyvinyl) pyridine

139mg of [5-methoxy-6-(2-methoxyvinyl)pyridin-2-yl]methanol was dissolved in 5ml of tetrahydrofuran at room temperature, 33mg 60% sodium hydride, 55µl of methyl iodide were added sequentially. After completion of reaction, saturated ammonium aqueous solution, ethyl acetate was added, the organic layer was partitioned, and the resulting organic layer was dried over anhydrous magnesium sulfate. Concentration was carried out under a reduced pressure after removal of drying agent by filtration and the residue was purified by NH silica gel column chromatography (ethyl acetate) to give 118mg of the title compound.
¹H-NMR(CDCl3)
δ: 3.46(s,3H), 3.81(s,3H), 3.82(s,3H), 4.56(s,2H), 5.66(d, *J*=7.6Hz,1H), 6.28(d, *J*=7.6Hz,1H), 7.10(d, *J*=8.4Hz,1H), 7.17(d, *J=* 8.4Hz,1H).

### (8) Synthesis of 1-[1-[2-(3-methoxy-6-methoxymethyl pyridin-2-yl)ethyl]piperidin-4-yl]-1H-indole-6-carboxamide

118mg of 3-methoxy-6-methoxymethyl-2-(2-methoxyvinyl) pyridine was dissolved in 1ml of tetrahydrofuran and 1ml of 5N HCl, which was heated to reflux. 0.5ml of 5N HCl was again added in between times. After completion of reaction, the reaction was diluted in chloroform and then adjusted to pH 7 with saturated sodium bicarbonate aqueous solution. The resulting organic layer dried over anhydrous magnesium sulfate. After removal of drying agent by filtration, concentration under a reduced pressure afforded 106mg of residue. 106mg of this residue and 73mg of [1-(piperidin-4-yl)-1*H*-indol-6-yl]carboxamide were dissolved in 5ml of dichloromethane, 34µl of acetic acid was added. About 15 minutes later, 95mg of sodium triacetoxyborohydride was added, which was stirred at room temperature. After completion of reaction, 5N aqueous sodium hydroxide, water and chloroform were added, the organic layer was partitioned, and the resulting organic layer was dried over anhydrous magnesium sulfate. After removal of drying agent by filtration, concentration was carried out under a reduced pressure, and the residue was purified by NH silica gel column chromatography (ethyl acetate / methanol) to give 103mg of the title compound.
¹H-NMR(CDCl3)
δ: 2.00-2.20(m,4H), 2.25-2.37(m,2H), 2.77-2.85(m,2H), 3.02-3.12(m,2H), 3.16-3.25(m,2H), 3.46(s,3H), 3.85(s,3H), 4.30-4.43(m,1H), 4.51(s,2H), 6.55(d, *J*=2.4Hz,1H), 7.12(d, *J*=8.4Hz,1H), 7.22(d, *J*=8.4Hz,1H), 7.36-7.44(m,2H), 7.63(d, *J*=8.0Hz,1H), 8.09(s,1H).

### Example 47

### Synthesis of 1-(1-[2-[3-methoxy-6-(3-methylisoxazol-5-yl) pyridine-2-yl)ethyl] piperidin-4-yl)-1H-indole-6-carboxamide

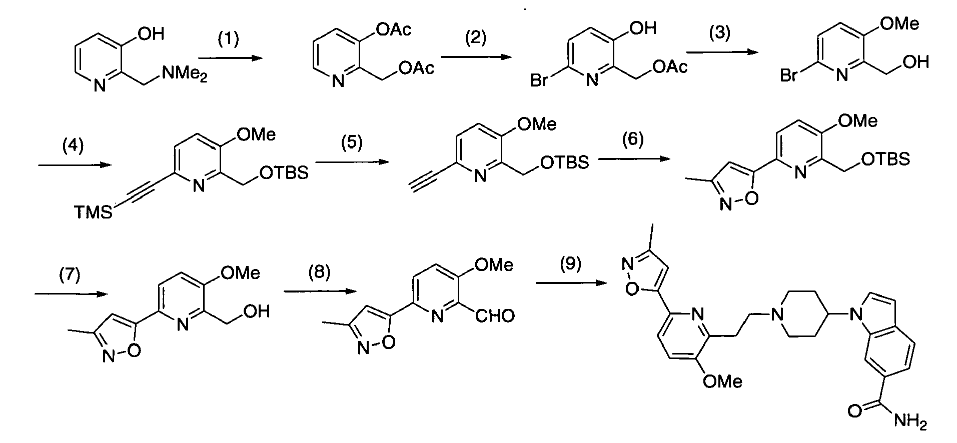

### (1) Synthesis of 2-acetoxymethylpyridin-3-yl acetate

10.2g of 2-(dimethylaminomethyl)-3-hydroxypyridine was dissolved in 45ml of acetic anhydride, which was heated to 160°C. After completion of reaction, the reaction liquid was concentrated under a reduced pressure. Methanol was added to the residue, and again concentrated, saturated sodium bicarbonate aqueous solution and ethyl acetate were added, and the organic layer was partitioned, and the resulting organic layer was dried over anhydrous magnesium sulfate. After removal of drying agent by filtration, concentration was carried out under a reduced pressure, and the residue was purified by NH silica gel column chromatography (hexane-ethyl acetate) to give 12.8g of the title compound.
¹H-NMR (CDCl3)
δ: 2.11(s,3H), 2.35 (s, 3H), 5.22 (s,2H), 7.32 (dd, *J*=4.8,8.0Hz,1H), 7.48(dd, *J*=1.6,8.0Hz,1H), 8.49(dd, *J*=1.6,4.8Hz,1H).

### (2) Synthesis of 6-bromo-2-acetoxymethyl-3-pyridinol

16.8g of 2-acetoxymethyl-3-pyridinol synthesized from 2-acetoxymethyl-pyridin-3-yl acetate according to Tetrahedron Letters, 30-(1989), p207-210 was dissolved in 300ml of dichloromethane and 400ml of tetrahydrofuran, and cooled to -30°C. 19.8g of N-bromosuccinimide was added in about 15 minutes maintaining below -20°C. 120ml of tetrahydrofuran was added on the way. Temperature was slowly raised to 10°C afterwards, about 3 hours later, the reaction was cooled to -15°C again, 1.25g of N-bromosuccinimide was added, and 1.5g of N-bromosuccinimide was added again about 3.5 hours later, after having raised temperature to -3°C, sodium thiosulfate aqueous solution was added, and the reaction liquid was concentrated under a reduced pressure. Ethyl acetate was added to the residue, the organic layer was partitioned, and the resulting organic layer was dried over anhydrous magnesium sulfate. After removal of drying agent by filtration, concentration was carried out under a reduced pressure, and the residue was purified by silica gel column chromatography (hexane-ethyl acetate) to give 15.6g of the title compound.
¹H-NMR(CDCl3)
5: 2.16(s,3H), 5.17(s,2H), 7.17(d, J=8.8Hz,1H), 7.37(d, *J*=8.8Hz,1H), 8.42(s,1H).

### (3) Synthesis of 6-bromo-2-hydroxymethyl-3-methoxypyridine

20.7g of 6-bromo-2-acetoxymethyl-3-pyridinol was dissolved in 66ml of methanol and 132ml of tetrahydrofuran, 4.43g of lithium hydroxide was added, which was stirred at room temperature. After completion of reaction, 5N HCl was added under cooling, pH was adjusted to 6 to 7, concentration was carried out under a reduced pressure. The resulting residue was dissolved in 400ml of acetone. 34.8g of potassium carbonate and 15.7ml of methyl iodide were added thereto, which was heated to reflux. About 1.5 hours later, 17.4g of potassium carbonate was added. After completion of reaction, the reaction was filtered through Celite, the filtrate was concentrated. Ethyl acetate, brine and saturated sodium bicarbonate aqueous solution were added to the residue, the organic layer was partitioned, and the resulting organic layer was dried over anhydrous magnesium sulfate. After removal of drying agent by filtration, concentration was carried out under a reduced pressure. After the residual solid was suspended in methanol-2-propanol-ethyl acetate, filtration afforded 15.4g of the title compound.
¹H-NMR(CDCl3)
δ: 3.61(t, *J*=5.2Hz, 1H), 3.85(s, 3H), 4.71(d, *J*=5.2Hz, 2H), 7.03(d, *J*=8.4Hz, 1H) 7.35(d, *J*=8.4Hz, 1H).

### (4) Synthesis of 2-(tert-butyldimethylsilyloxy methyl)-3-methoxy -6-trimethylsilylethynylpyridine

6.00g of 6-bromo-2-(*tert*-butyldimethylsilyloxymethyl)-3-methoxypyridine prepared from 6-bromo-2-hydroxymethyl-3-methoxypyridine according to example 46- (3) was dissolved in 15ml of triethylamine and 10ml of N, N-dimethylformamide, 152mg of bis(triphenylphosphine) palladium(II) chloride, 76mg of copper and 6.4ml of trimethylsilylacetylene were added sequentially, which was heated to 45-55°C. After completion of reaction, the reaction was filtered through Celite, ethyl acetate, brine and ammonia aqueous solution were added to the filtrate, the organic layer was partitioned, and the resulting organic layer was dried over anhydrous magnesium sulfate. After removal of drying agent by filtration, concentration was carried out under a reduced pressure, and the residue was purified by silica gel column chromatography (hexane-ethyl acetate) to give 5.86g of the title compound.
¹H-NMR(CDCl3)
δ: 0.10(s,6H), 0.25(s,9H), 0.90(s,9H), 3.84(s,3H), 4.80(s,2H), 7.06(d, J=8.4Hz,1H), 7.37(d, *J*=8.4Hz,1H).

### (5) Synthesis of 2-(tert-butyldimethylsilyloxymethyl)-6-acetenyl-3-methoxypyridine

5.86g of 2-(*tert*-butyldimethylsilyloxymethyl)-3-methoxy-6-trimethylsilylethynylpyridine was dissolved in 50ml of methanol and 10ml of tetrahydrofuran, 2.32g of potassium carbonate was added, which was stirred at room temperature. After completion of reaction, the reaction was filtered through Celite, the filtrate was concentrated under a reduced pressure, and the residue was purified by silica gel column chromatography (hexane-ethyl acetate) to give 4.7g of the title compound.
¹H-NMR(CDCl3)
δ: 0.10(s,6H), 0.90 (s, 9H), 3.03 (s,1H), 3.86 (s, 3H), 4.81 (s, 2H), 7.08 (d, *J*=8.4Hz, 1H), 7.40(d, *J*=8.4Hz, 1H).

### (6) Synthesis of 2-(tert-butyldimethylsilyloxymethyl)-3-methoxy-6-(3-methylisoxazol-5-yl)pyridine

4.7g of 2-(*tert*-butyldimethylsilyloxy methyl)-6-acetenyl-3-methoxypyridine, 1.33ml of nitroethane and 8.07g of 1,4-phenylenediisocyanate were suspended in 160ml of toluene, which was heated to 55°C. 0.5ml of triethylamine was added five minutes later, and temperature was raised to 85°C. About 15 hours later, 0.2ml of nitroethane and 0.1ml of triethylamine were added. The reaction liquid was stood to cool about 1.5 hours later, 8ml of water was added, which was stirred for 1 hour. The reaction liquid was filtered through Celite, the filtrate was concentrated under a reduced pressure, and the residue was purified by silica gel column chromatography (hexane-ethyl acetate) to give 2.47g of the title compound.
¹H-NMR(CDCl3)
δ: 0.13(s,6H), 0.93(s,9H), 2.36(s,3H), 3.89(s,3H), 4.88(s,2H), 6.63(s,1H), 7.20(d, *J*=8.4Hz,1H), 7.77(d, *J*=8.4Hz,1H).

### (7) Synthesis of 2-hydroxymethyl-3-methoxy-6-(3-methylisoxazol-5-yl)pyridine

4.7g of 2-(*tert*-butyldimethylsilyloxymethyl)-3-methoxy-6-(3-methylisoxazol-5-yl)pyridine was dissolved in 50ml of tetrahydrofuran, 8ml of tetrabutylammonium fluoride (1 mol/L tetrahydrofuran solution) was added. After completion of reaction, the reaction liquid was concentrated under a reduced pressure, chloroform, brine and saturated ammonium aqueous solution were added to the residue, and the organic layer was partitioned, and the resulting organic layer was dried over anhydrous magnesium sulfate. After removal of drying agent by filtration, concentration was carried out under a reduced pressure. The residue was purified by silica gel column chromatography (ethyl acetate) to give 1.62g of the title compound.
¹H-NMR(CDCl3)
δ: 2.37(s,3H), 3.91(s,3H), 4.16(t, *J*=4.8Hz,1H), 4.77(d, J=4.8Hz,2H), 6.65(s,1H), 7.22(d, *J*=8.4Hz,1H), 7.80(d, *J*=8.4Hz,1H).

### (8) Synthesis of 3-methoxy-6-(3-methylisoxazol-5-yl) pyridine-2-carbaldehyde

1.47g of 2-hydroxymethyl-3-methoxy-6-(3-methylisoxazol-5-yl)pyridine was dissolved in 70ml of chloroform, 7.5g of manganese dioxide was added, which was stirred at 60 °C from a room temperature. Manganese dioxide was added 12.5g in total on the way. After completion of reaction, the filtrate was concentrated to yield 306mg of the title compound.
¹H-NMR(CDCl3)
δ: 2.38 (s,3H), 4.03(s,3H), 6.78(s,1H), 7.51(d, *J*=8.8Hz,1H), 8.05 (d, *J*=8.8Hz,1H), 10.3(s,1H).

### (9) Synthesis of 1-(1-[2-[3-methoxy-6-(3-methylisoxazol-5-yl) pyridin-2-yl)ethyl]piperidin-4-yl)-1H-indole-6-carboxamide

The title compound was obtained by synthesizing from 3-methoxy-6-(3-methylisoxazol-5-yl) pyridine-2-carbaldehyde in accordance with the methods in example 46-(5) to (8).
¹H-NMR(CDCl₃)
δ: 2.07-2.16(m,4H), 2.28-2.39(m,2H), 2.36(s,3H), 2.84-2.93(m,2H), 3.07-3.15(m,2H), 3.19-3.28(m,2H), 3.90(s,3H), 4.34-4.46(m,1H), 6.56(d, *J*=2.8Hz, 1H), 6.63(s,1H), 7.17(d, *J*=8.4Hz, 1H), 7.37-7.43(m,2H), 7.63(d, *J*=8.4Hz, 1H), 7.71(d, *J*=8.8Hz, 1H), 8.10(s,1H).

### Example 48

### Synthesis of 1-(1-[2-[3-methoxy-6-(3-pyridyl) pyridin-2-yl) ethyl]piperidin-4-yl)-1H-indole-6-carboxamide

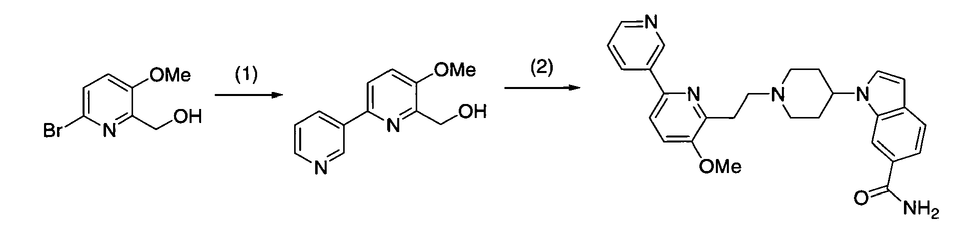

### (1) Synthesis of the 2-hydroxymethyl-3-methoxy-6-(3-pyridyl) pyridine

According to Tetrahedron Letters, 42-(2001), p2093-296, the title compound was synthesized from a 3-pyridine boronic acid and above-described 6-bromo-2-hydroxymethyl-3-methoxypyridine.
¹H-NMR(CDCl3)
δ: 3.91(s,3H), 4.39(t, *J*=4.8Hz,1H), 4 80 (d, *J*=4.8Hz,2H), 7.24(d, *J=* 8.4Hz,1H), 7.38(ddd, *J*=0.8,4.8,8.0Hz,1H), 7.67(d, *J=* 8.4Hz,1H) 8.25-8.30(m,1H), 8.60(dd, *J*=1.6,4.8Hz,1H), 9.15(dd, *J*=0.8,2.4Hz,1H).

### (2) Synthesis of 1- (1- [2- [3-methoxy-6- (3-pyridyl) pyridin-2-yl) ethyl]piperidin-4-yl)-1H-indole-6-carboxamide

The title compound was synthesized from 2-hydroxymethyl-3-methoxy-6-(3-pyridyl) pyridine as a starting material in accordance with the methods in example 47-(8) and (9).
¹H-NMR(DMSO-d6)
δ: 1.92-2.08(m,4H), 2.26-2.35(m,2H), 2.78-2.86(m,2H), 3.00-3.08(m,2H), 3.11-3.18(m,2H), 3.89(s,3H), 4.36-4.47 (m,1H), 6.48(d, *J*=2.8Hz,1H), 7.20 (brs,1H), 7.42-7.60(m, 4H), 7.65(d, *J*=3.2Hz,1H), 7.82-7.94 (m,1H), 7.89(d, *J*=8.4Hz,1H) 8.12 (s,1H), 8.36(dt, *J*=2.0,8.0Hz,1H), 8.54(dd, *J*=1.6,4.8Hz,1H).

### Example 49

### Synthesis of 1-(1-[2-[3-methoxy-6-(4-pyridyl) pyridin-2-yl) ethyl]piperidin-4-yl)-1H-indole-6-carboxamide

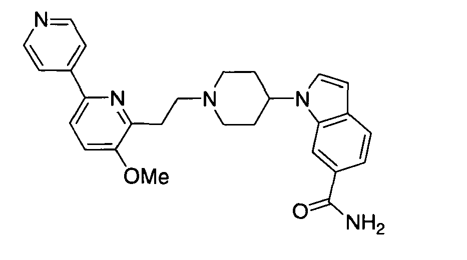

The title compound was synthesized in accordance with the methods in example 48.
¹H-NMR(CDCl3)
δ: 2.06-2.20(m,4H), 2.30-2.42(m,2H), 2.92-3.00(m,2H), 3.12-3.20(m,2H), 3.22-3.31(m,2H), 3.92(s,3H), 4.34-4.46(m,1H), 6.57(dd, *J*=0.8,3.6Hz,1H), 7.21(d, *J*=8.8Hz,1H), 7.38-7.43(m,2H), 7.64(d, *J*=8.8Hz,1H), 7.67(d, *J*=8.4Hz,1H), 7.89(dd, *J*=1.6,4.4Hz,2H), 8.12(brs,1H), 8.68(dd, *J*=1.6,4.4Hz,2H).

### Example 50

### Synthesis of 1-[1-[2-(3-chloro-5-methoxypyridine-4-yl)ethyl] piperidin-4-yl]-1H-indole-6-carboxamide

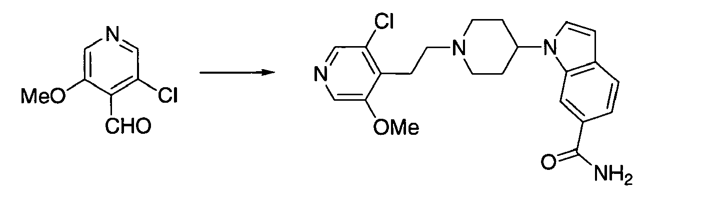

The title compound was synthesized from 3-chloro-5-methoxypyridine-4-carbaldehyde in accordance with the methods in example 46-(5) to (8).
¹H-NMR(CDCl3)
δ: 2.04-2.18(m,4H), 2.26-2.40(m,2H), 2.56-2.65(m,2H), 3.00-3.08(m,2H), 3.16-3.25(m,2H), 3.95(s,3H), 4.32-4.46(m,1H), 6.57(d, *J*=3.2Hz,1H), 7.36-7.42(m,2H), 7.63(d, *J*=8.0Hz,1H), 8.09(brs,1H), 8.11(s,1H) 8.23(s,1H).

### Example 51

### Synthesis of 1-[1-[2-(3-methoxypyridine-4-yl)ethyl] piperidin-4-yl]-1H-indole-6-carboxamide

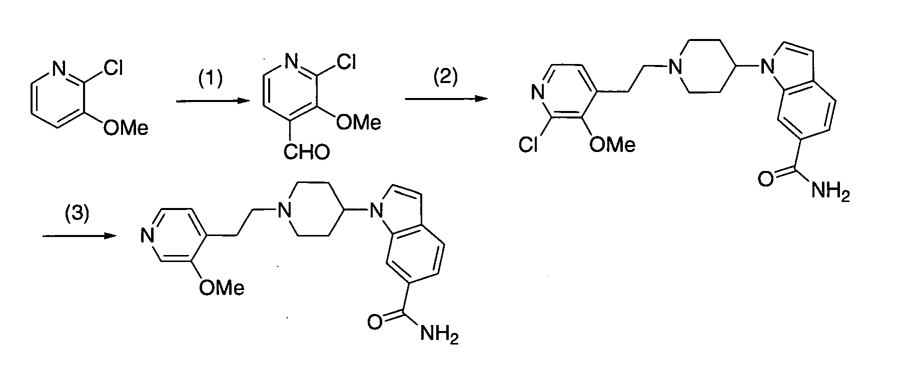

### (1) Synthesis of 2-chlor-3-methoxypyridine-4-carbaldehyde

The title compound was synthesized from 2-chlor-3-methoxypyridine in accordance with the methods disclosed in Tetrahedron 58-(2002) 309-314.
¹H-NMR(CDCl3)
5: 4.08(s,3H), 7.60(d, *J*=4.8Hz, 1H), 8.34(dd, *J*=0.8,4.8Hz, 1H), 10.4(d, *J*=0.8Hz, 1H).

### (2) Synthesis of 1-[1-[2-(2-chlor-3-methoxypyridin-4-yl)ethyl]-piperidin-4-yl]-1H-indole-6-carboxamide

The title compound was obtained from 2-chlor-3-methoxypyridine-4-carbaldehyde in accordance with the methods in example 46-(5) to (8).
¹H-NMR(CDCl3)
5: 2.00-2.18(m,4H), 2.27-2.37(m,2H), 2.66-2.74(m,2H), 2.88-2.96(m,2H), 3.12-3.22(m,2H), 3.92(s,3H), 4.34-4.45(m,1H), 6.58(d, *J*=3.2Hz,1H), 7.14(d, *J*=4.8Hz,1H), 7.36-7.44(m,2H), 7.64(d, *J*=7.6Hz,1H), 8.09(d, *J*=4.8Hz,1H), 8.11(brs,1H

### (3) Synthesis of 1-[1-[2-(3-methoxypyridine-4-yl)ethyl] piperidin-4-yl]-1H-indole-6-carboxamide

20mg of 1-[1-[2-(2-chlor-3-methoxypyridin-4-yl)ethyl] piperidin-4-yl]-1H-indole-6-carboxamide was dissolved in 5ml of methanol and 3ml of tetrahydrofuran, 6mg of 10% palladium-carbon was added, which was stirred under hydrogen atmosphere. After completion of reaction, filtration was carried out, the filtrate was concentrated under a reduced pressure, and the residue was purified by NH silica gel column chromatography (ethyl acetate / methanol) to give 12mg of the title compound.
¹H-NMR(CDCl3)
δ : 2.02-2.16(m,4H), 2.25-2.35(m,2H), 2.61-2.70(m,2H), 2.82-2.90(m,2H), 3.12-3.23(m,2H), 3.94(s,3H), 4.30-4.46(m,1H), 6.57(d, *J*=2.4Hz,1H), 7.11(d, *J*=4.8Hz,1H), 7.36-7.42(m,2H), 7.63(d, *J*=8.4Hz,1H), 8.10(brs,1H), 8.18(d, *J*=4.8Hz,1H), 8.21(s,1H).

### Example 52

### Synthesis of 1-[1-[2-(5-methoxy-2-methoxymethylpyridin-4-yl)ethyl] piperidin-4-yl]-1H-indole-6-carboxamide

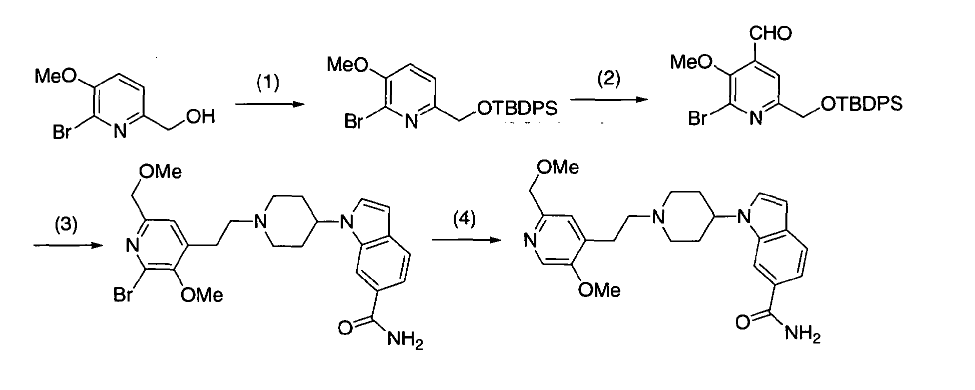

### (1) Synthesis of 2-bromo-6-(tert-butyldiphenylsilyloxymethyl)-3-methoxypyridine

4.10g of 2-bromo-6-hydroxymethyl-3-methoxypyridine was dissolved in 60ml of N,N-dimethylformamide, 3.84g of imidazole and 5.13ml of *tert*-butyldiphenylsilyl chloride were added sequentially, which was stirred at room temperature. After completion of reaction, brine and *tert*-butyl methyl ether were added, the organic layer was partitioned, and the resulting organic layer was dried over anhydrous magnesium sulfate. After removal of drying agent by filtration, concentration was carried out under a reduced pressure, and the residue was purified by silica gel column chromatography (hexane-ethyl acetate) to give 7.15g of the title compound.
¹H-NMR(CDCl3)
δ: 1.12(s,9H), 3.91(s,3H), 4.80(d, *J*=0.8Hz,2H), 7.19(d, *J*=8.0Hz,1H), 7.32-7.44(m,6H), 7.56(dt, *J*=0.8,8.0Hz,1H), 7.63-7.67(m,4H).

### (2) Synthesis of 2-bromo-6-(tert-butyl diphenylsilyloxymethyl)-3- methoxypyridine-4-carbaldehyde

The title compound was synthesized from 2-bromo-6-(*tert-*butyldiphenylsilyloxymethyl)-3-methoxypyridine in accordance with the methods in Tetrahedron 58 (2002) 309-314.
1H-NMR(CDCl3)
δ: 1.13(s,9H), 4.02(s,3H), 4.81(d, *J*=0.8Hz,2H), 7.32-7.44(m,6H), 7.62-7.68(m,4H), 7.91(t, *J*=0.8Hz,1H).

### (3) Synthesis of 1-[1-[2-(2-bromo-3-methoxy-6-methoxymethyl pyridin-4-yl)ethyl]piperidin-4-yl]-1H-indole-6-carboxamide

The title compound was obtained from 2-bromo-6-(*tert*-butyldiphenylsilyloxymethyl)-3-methoxypyridine-4-carbaldehyde in accordance with the methods in example 46- (5), (6), (7) and (8).
¹H-NMR(CDCl3)
δ: 2.00-2.16(m,4H), 2.26-2.37(m,2H), 2.67-2.75(m,2H), 2.88-2.96(m,2H), 3.12-3.22(m,2H), 3.48(s,3H), 3.89(s,3H), 4.32-4.44(m,1H), 4.51(s,2H), 6.57 (d, *J*=3.6Hz,1H) 7.27(s,1H), 7.36-7.42(m,2H), 7.63(d, *J*=8.0Hz,1H), 8.09(brs,1H).

### (4) Synthesis of 1-[1-[2-(5-methoxy-2-methoxymethylpyridin-4-yl)ethyl]piperidin-4-yl]-1H-indole-6-carboxamide

The title compound was obtained from 1- [1- [2- (2-bromo-3-methoxy-6-methoxymethylpyridin-4-yl)ethyl]piperidin-4-yl]-1H-indole-6-carboxamide in accordance with the methods in example 51.
¹H-NMR(CDCl3)
δ: 2.02-2.16(m,4H) 2.20-2.36(m,2H), 2.60-2.70(m,2H), 2.80-2.90(m,2H), 3.12-3.22(m,2H), 3.46(s,3H), 3.93(s,3H), 4.32-4.44 (m,1H), 4.51 (s,2H), 6.57 (dd, *J*=0.4,3.6Hz,1H), 7.22 (s,1H), 7.36-7.42(m,2H), 7.63(dd, *J*=0.4,8.4Hz,1H), 8.10 (s,1H), 8.14 (s,1H).

### Example 53

### Synthesis of 1-(1-[2-[2-methoxy-4-(methoxymethyl)phenyl) ethyl]piperidin-4-yl)-1H-indole-6-carboxamide

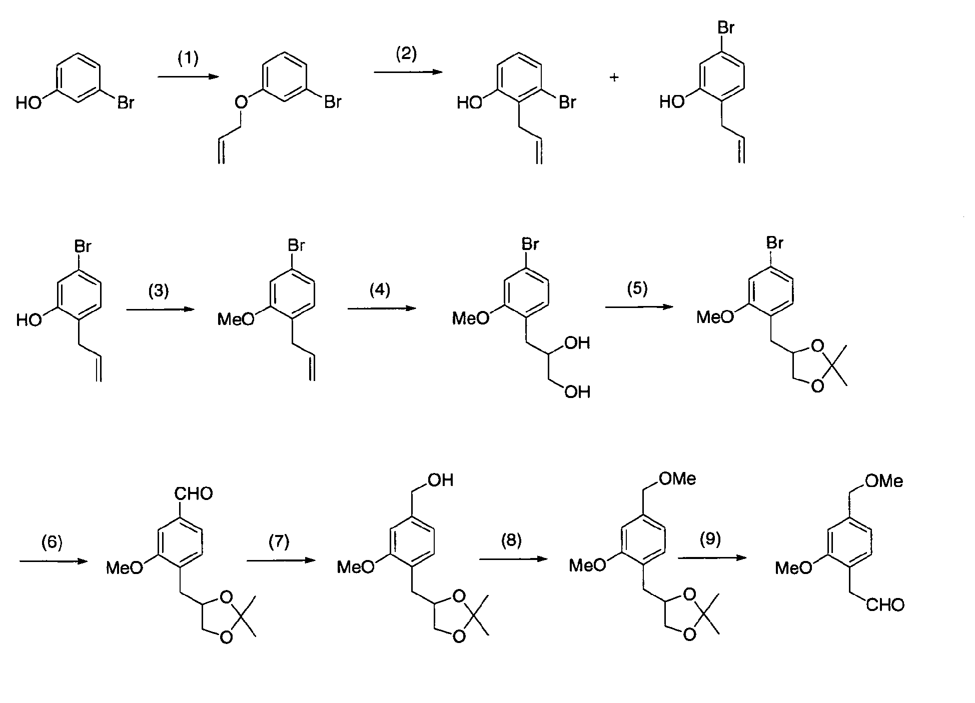

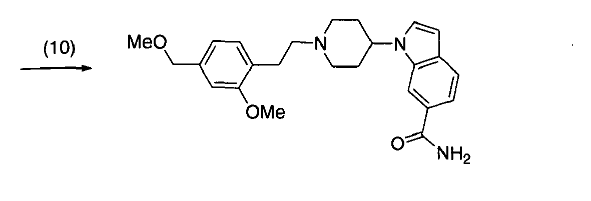

### (1) Synthesis of 1-allyloxy-3-bromobenzene

25.02g of 3-bromophenol was dissolved in 100ml of N,N-dimethylformamide, 19.98g of potassium carbonate and 18.8ml of allyl bromide were added. This reaction liquid was stirred for 19.5 hours at room temperature. Water and ethyl acetate were added to reaction liquid, and the organic layer was partitioned. The resulting organic layer was washed with water and brine, and then dried over anhydrous magnesium sulfate. After removing the drying agent by filtration, the organic layer was concentrated under a reduced pressure, the residue was purified by silica gel column chromatography (hexane-ethyl acetate) to give 31.23g of the title compound.
¹H-NMR(CDCl3)
δ : 4.50-4.52(m,2H), 5.27-5.31(m,1H), 5.37-5.43(m,1H), 5.97-6.07(m,1H), 6.82-6.85(m,1H), 7.06-7.14(m,3H).

### (2) Synthesis of 2-allyl-5-bromophenol and 2-allyl-3-bromophenol

31.23g of 1-allyloxy-3-bromobenzene was dissolved in 60ml of N,N-diethylaniline, the reaction liquid was heated to reflux overnight under nitrogen atmosphere. The reaction solution was cooled to a room temperature. Ethyl acetate was added to reaction liquid, the resulting obtained organic layer was washed with 5N HCl, water and brine, and then dried over anhydrous magnesium sulfate. After removing the drying agent by filtration, the organic layer was concentrated under a reduced pressure, and the residue was purified by NH silica gel column chromatography (hexane-ethyl acetate) to give 9.90g of 2-allyl-5-bromophenol and 16.25g of 2-allyl-3-bromophenol.
2-allyl-5-bromophenol:
¹H-NMR(CDCl3)
δ: 3.61-3.64(m,2H), 5.08-5.14(m,3H), 5.91-6.01(m,1H), 6.75(dd, *J*=0.8,8.0Hz, 1H), 6.96(dd, *J*=8.0,8.0Hz,1H), 7.15(dd, *J*=0.8,8.0Hz,1H).
2-allyl-3-bromophenol:
¹_{H}-_{NM}R(CDCl3)
δ: 3.34-3.36(m,2H), 5.11-5.17(m,3H), 5.92-6.02(m,1H), 6.94-7.02(m,3H).

### (3) Synthesis of 1-allyl-4-bromo-2-methoxybenzene

9.81g of 2-allyl-3-bromophenol was dissolved in 30ml of N,N-dimethylformamide, 5.36g of potassium carbonate and 4.82ml of methyl iodide were added. This reaction liquid was stirred for 2 days at room temperature under nitrogen atmosphere. Water and ethyl acetate were added to the reaction liquid, and the organic layer was partitioned. After the resulting organic layer was washed with sodium thiosulfate aqueous solution, water and brine, and dried over anhydrous magnesium sulfate. After removing the drying agent by filtration, the organic layer was concentrated under a reduced pressure, the residue was purified by silica gel column chromatography (hexane-ethyl acetate) to give 7.35g of the title compound.
¹H-NMR(CDCl3)
δ: 3.31-3.32(m,2H), 3.82(s,3H), 5.01-5.05(m,2H), 5.89-5.99(m,1H), 6.97-7.04(m,3H).

### (4) Synthesis of 3-(4-bromo-2-methoxyphenyl)propane-1,2-diol

To a mixture of 32.39g of AD-mix-β, 120ml of *tert*-butanoyl and 12ml of water was added a solution, which 20ml of *tert*-butanol was added to 7.35g of 1-allyl-4-bromo-2-methoxybenzene and dissolved, and the reaction liquid was stirred for 2 days at room temperature. After confirming the disappearance of starting materials, 38.88g of sodium sulfite was added to reaction liquid and the reaction liquid was stirred for 1 hour. Water and ethyl acetate were added to reaction liquid, and the organic layer was separated. The resulting organic layer was washed with brine and then dried over anhydrous magnesium sulfate. After removal of drying agent by filtration, concentration was carried out under a reduced pressure. The residue was purified by silica gel column chromatography (hexane-ethyl acetate) to give 8.25g of the title compound.
¹H-NMR(CDCl3)
δ: 2.04(t, J=6.2Hz,1H), 2.26(d, *J*=4.8Hz,1H), 2.74(dd, *J*=7.4,13.6Hz,1H) 2.81(dd, *J*=5.6,13.6Hz,1H), 3.44-3.50(m,1H), 3.60-3.65(m,1H), 3.83(s,3H), 3.88-3.95(m,1H), 6.99(d, *J*=1.4Hz,1H), 7.02(d, *J*=8.0Hz,1H),7.05(dd, *J*=1.4,8.0Hz,1H).

### (5) Synthesis of 4-(4-bromo-2-methoxybenzyl)-2,2-dimethyl-1,3-dioxolane

To a solution of 8.25g of 3-(4-bromo-2-methoxyphenyl) propane-1,2-diol, 11.66ml of 2,2-dimethoxypropane in 200ml of acetone was added 0.60g of p-toluenesulfonic acid monohydrate, calcium chloride tube was fitted and stirred overnight at room temperature. The solvent was distilled off under a reduced pressure. Water and ethyl acetate were added to the residue, and the organic layer was separated. The resulting organic layer was washed with 10% sodium carbonate aqueous solution and brine, and then dried over anhydrous magnesium sulfate. After removal of drying agent by filtration, the organic layer was concentrated under a reduced pressure. The residue was purified by silica gel column chromatography (hexane-ethyl acetate) to give 8.88g of the title compound.
¹H-NMR(CDCl3)
δ: 1.34(s,3H), 1.42(s,3H), 2.77(dd, *J*=7.0,13.6Hz,1H), 2.91(dd, *J*=6.6,13.6Hz,1H), 3.62(dd, *J*=6.6,8.0Hz,1H), 3.80(s,3H), 3.93(dd, *J*=6.0,8.0Hz,1H), 4.28-4.35(m,1H), 6.95-6.96(br,1H), 7.00-7.05(m,2H).

### (6) Synthesis of 4-[(2,2- dimethyl-1,3-dioxolan-4-yl) methyl] -3-methoxybenzaldehyde

1.77g of 4-(4-bromo-2-methoxybenzyl)-2,2-dimethyl-1,3-dioxolane was dissolved in 30ml of anhydrous tetrahydrofuran was cooled in a dry ice-acetone bath under nitrogen atmosphere. 2.68ml of *n-*butyllithium-hexane solution (2.64M) was added dropwise thereto. At this moment, internal temperature was sustained below -71 °C. The mixture was stirred for another 20 minutes. 2.28ml of N,N-dimethylformamide was added thereto. Subsequently temperature was slowly raised to a room temperature. Saturated ammonium aqueous solution and ethyl acetate was added to reaction liquid, and the organic layer was partitioned. The resulting organic layer was washed with saturation brine solution and then dried over anhydrous magnesium sulfate. After removing the drying agent by filtration, the organic layer was concentrated under a reduced pressure, the residue was purified by silica gel column chromatography (hexane-ethyl acetate) to give 0.88g of the title compound.
¹H-NMR(CDCl3)
δ: 1.35(s,3H), 1.43(s,3H), 2.92(dd, *J*=6.8,13.6Hz,1H), 3.01(dd, *J*=6.4,13.6Hz,1H), 3.66(dd, *J*=6.4,8.4Hz,1H), 3.90(s,3H), 3.98(dd, *J*=6.0,8.4Hz,1H), 4.36-4.43(m,H), 7.37-7.42(m,3H), 9.95(s,1H).

### (7) Synthesis of [4-[-(2,2-dimethyl-1,3-dioxolan-2-yl)methyl]-3-methoxyphenyl]methanol

0.88g of 4-[(2,2-dimethyl-1,3-dioxolan-4-yl)methyl]-3-methoxybenzaldehyde was dissolved in 5ml of methanol and was stirred in an ice-cooling. Until starting material was not observed by TLC, sodium borohydride was added in small portions thereto. The solvent was distilled off under a reduced pressure. 10% sodium carbonate aqueous solution and ethyl acetate was added to the residue, and the organic layer was partitioned. The resulting organic layer was washed with brine solution and then dried over anhydrous magnesium sulfate. After removing the drying agent by filtration, the organic layer was concentrated under a reduced pressure to give 0.89g of the title compound.
¹H-NMR(CDCl3)
δ : 1.34(s,3H), 1.43(s,3H), 1.64(brt,1H), 2.80(dd, *J*=7.2,13.2Hz,1H), 2.99(dd, *J*=5.4,13.2Hz,1H), 3.65(dd, *J*=6.8,8.0Hz,1H), 3.82(s,3H), 3.92(dd, *J*=6.0,8.0Hz,1H), 4.32-4.38(m,1H), 4.65(br,2H), 6.85-6.88(m,2H), 7.14(d, *J*=7.6Hz,1H).

### (8) Synthesis of 4-[2-methoxy-4-(methoxymethyl)benzyl]-2,2-dimethyl-1,3-dioxolane

0.11g of sodium hydride (60%), which was washed with hexane, suspended in 1ml of anhydrous tetrahydrofuran under nitrogen atmosphere, and was stirred in an ice-cooling. The solution which dissolved 0.56g of [4-[-(2,2-dimethyl-1,3-dioxolane-2-yl) methyl]-3-methoxyphenyl]methanol in 5ml of anhydrous tetrahydrofuran was added thereto, followed by stirring for 40 minutes at room temperature. Ice cooling was carried out again. 414µl of methyl iodide was added, which was stirred for 50 minutes at room temperature. Saturated ammonium aqueous solution and ethyl acetate was added in an ice cooling, and the organic layer was partitioned. The resulting organic layer was washed with saturation brine solution, and then dried over anhydrous magnesium sulfate. After removing the drying agent by filtration, the organic layer was concentrated under a reduced pressure, and the residue was purified by silica gel column chromatography (hexane-ethyl acetate) to give 0.3g of the title compound.
¹H-NMR(CDCl3)
5: 1.35(s,3H), 1.43(s,3H), 2.79(dd, *J*=7.4,13.4Hz,1H), 3.00(dd, *J*=5.4,13.4Hz,1H), 3.39(s,3H), 3.65(dd, *J*=6.4,8.0Hz,1H), 3.82(s,3H), 3.91(dd, *J*=6.2,8.0Hz,1H), 4.31-4.37(m,1H), 4.42(s,2H), 6.81-6.84(m,2H), 7.12(d, *J*=7.6Hz,1H).

### (9) Synthesis of [2-methoxy-4-(methoxymethyl)phenyl] acetaldehyde

145mg of 4-[2-methoxy-4-(methoxymethyl)benzyl]-2,2-dimethyl-1,3-dioxolane was dissolved in 1ml of methanol. A 4N hydrochloric acid-ethyl acetate solution was added thereto, which was stirred for 1 hour at room temperature. The solvent was distilled off under a reduced pressure. The residue was dissolved in 3ml of tetrahydrofuran and 1ml of water, saturated sodium bicarbonate aqueous solution was then added, and pH was adjusted to about 8. Next, 234mg of sodium metaperiodate was added, which was stirred vigorously. After confirming disappearance of the starting material by TLC, water and ethyl acetate were added to reaction liquid, and the organic layer was partitioned. The resulting organic layer was washed with brine solution and then dried over anhydrous magnesium sulfate. After removing the drying agent by filtration, the organic layer was concentrated under a reduced pressure to yield 111mg of crude aldehyde. This crude aldehyde was used in the next reaction without further purification.

### (10) Synthesis of 1-[1-[2-[2-methoxy-4-(methoxymethylphenyl)ethyl] piperidin-4-yl]-1H-indole-6-carboxamide

To 104mg of 1-(piperidin-4-yl)-1*H*-indole-6-carboxamide, which was synthesized in production example 1, was sequentially added 111mg of crude [2-methoxy-4-(methoxymethyl)phenyl] acetaldehyde dissolved in 5ml of dichloromethane and 48.9µl of acetic acid, the reaction liquid was stirred for 30 minutes. 137mg of sodium triacetoxyborohydride was added to the reaction liquid, and the reaction liquid was stirred for 1 hour at room temperature. 10% sodium carbonate aqueous solution and chloroform was added to reaction liquid, and the organic layer was partitioned. The resulting organic layer was washed with brine solution and then dried over anhydrous magnesium sulfate. After removing the drying agent by filtration, the organic layer was concentrated under a reduced pressure, and the residue was purified by NH silica gel column chromatography (ethyl acetate / methanol) to give 80mg of the title compound.
¹H-NMR(CDCl3)
δ: 2.09-2.15(m,4H), 2.26-2.32(m,2H), 2.62-2.66(m,2H), 2.83-2.88(m,2H), 3.19-3.22(m,2H), 3.40(s,3H), 3.85(s,3H), 3.35-4.43(m,1H), 4.43(s.2H), 5.57(br,1H), 6.14(br,1H), 6.56(d, *J*=3.2Hz,1H), 6.83-6.86(m,2H), 7.13(d, *J*=7.6Hz,1H), 7.39-7.41(m,2H), 7.63(d, *J*=8.4Hz,1H), 8.09(s,1H).

### Example 54

### Synthesis of (1-[[1-2-(methoxy-6-phenyl)phenethyl)piperidin-4-yl]-(1H)-indol-6-yl)carboxamide

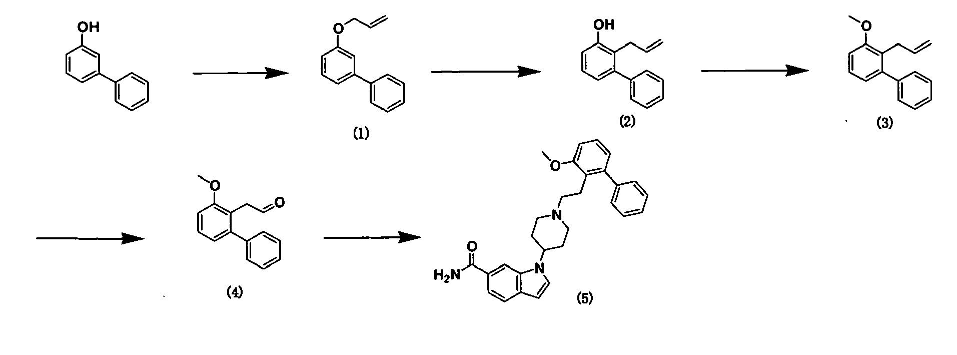

### (1) Synthesis of 3-(2-propenyloxy)biphenyl

A mixture of 1.0g of 3-hydroxybiphenyl (CAS No.580-51-8), 1.3g of allyl bromide and 2.0g of potassium carbonate in 8ml of N, N-dimethylformamide was stirred for 3 hours at room temperature. Water was added to the mixture, and the mixture was extracted with ethyl acetate. Ethyl acetate layer was washed with water and brine and dried over magnesium sulfate. The mixture was filtered and the filtrate was concentrated to yield 1.21g of the title compound as a light brown oil.
¹H-NMR(CDCl3)
δ: 4.56-4.62(m,2H), 5.27-5.33(m,1H), 5.40-5.47 (m,1H) 6.02-6.14 (m,1H), 6.87-7.60(m,9H).

### (2) Synthesis of 3-hydroxy-(2-propenyl)biphenyl

1.15g of 3-(2-propenyloxy)biphenyl in 4ml of N,N-dimethylaniline was heated at 230 °C for 40 minutes by using a microwave synthesizer. A mixture was diluted in 150ml of ethyl acetate, washed with 25ml of 5N hydrochloric acid, 50ml of water 4 times, 50ml of brine and then dried over magnesium sulfate. A mixture was filtered, and the filtrate was concentrated, to yield 1.10g of residue. 200mg of the resulting residue was separated by HPLC to give 80mg of the title compound as a colorless oil.
¹H-NMR(CDCl3)
δ: 3.32-3.37(m,2H), 5.06-5.20(m,3H), 5.94-6,08 (m,1H), 6.85-6.90(m,2H), 7.19(t, *J*=7.6Hz,1H), 7.28-7.41(m,5H)

### (3) Synthesis of 3-methoxy-(2-propenyl)biphenyl

78mg of 3-hydroxy-(2-propenyl)biphenyl, 80mg of iodomethane and 200mg of potassium carbonate in 3ml of N,N-dimethylformamide was stirred overnight at room temperature. 50ml of water was added to a mixture, and the mixture was extracted with 100ml of ethyl acetate. The ethyl acetate layer was washed with 50ml of water three times and 50ml of brine and then dried over magnesium sulfate. The mixture was filtered and the filtrate was concentrated to give 82mg of the title compound as a colorless oil.
¹H-NMR(CDCl3)
δ: 3.30(dt, *J*=2.0,6.0Hz,2H), 3.86(s,3H), 4.79(dq, *J*=2.0,17.2Hz,1H), 4.92(dq, *J*=2.0,10.4Hz,1H), 5.87-5.98(m,1H), 6.86(dd, *J*=1.2,7.6Hz,1H) 6.88(dd, *J*=1.2,8.0Hz,1H), 7.20-7.39(m,6H).

### (4) Synthesis of 2-methoxy-6-phenyl-phenylacetaldehyde

80mg 3-methoxy-(2-propenyl)biphenyl was dissolved in 6ml of tetrahydrofuran-3ml of water, 0.1ml of 3.3% osmium tetroxide aqueous solution and 300mg of sodium perchlorate were added, and the mixture was stirred for 3 hours at room temperature. 15ml of 5% aqueous sodium hydrogen sulfate was added to a mixture and the mixture was extracted with 60ml of ethyl acetate. The organic layer was washed with 30ml of water and 30ml of brine and then dried over magnesium sulfate. The mixture was filtered and the filtrate was concentrated under a reduced pressure to give of 70mg of the title compound as a brown oil.
¹H-NMR(CDCl3)
δ: 3.62(d, *J*=1.2Hz,2H), 3.86(s,3H), 6.91-6.96(m,2H), 7.22-7.25(m,1H), 7.30-7.43(m,5H), 9.66(t, *J*=1.2Hz,1H).

### (5) Synthesis of (1-[[1-2-(methoxy-6-phenyl)phenethyl) piperidine-4-yl]-(1H)-indol-6-yl)carboxamide

A mixture of 68mg of 2-methoxy-6-phenyl-phenylacetaldehyde, 70mg of [1- (piperidin-4-yl) -1*H*-indol-6-yl]carboxamide and 100mg of acetic acid in 6ml of methylene chloride was stirred for 15 minutes at room temperature, then 200mg of sodium triacetoxyborohydride was added, which was stirred for 12 hours. 10% aqueous potassium carbonate was added to a mixture, and the mixture was extracted with ethyl acetate. The ethyl acetate layer was washed with brine, dried over magnesium sulfate, then filtered, and concentrated under a reduced pressure. The residue was purified by silica gel flash column chromatography and 10% methanol-methylene chloride fractions afforded 59mg of the title compound as a colorless solid.
¹H-NMR(CDCl3)
5: 1.95-2.16(m,6H), 2.48-2.58(m,2H), 2.78-2.87(m,1H), 2.91-2.30(m,1H), 3.89(s,3H), 4.25-4.36(m,1H), 6.55(d, *J*=2.4Hz,1H), 6.86(d, *J*=7.2Hz,1H), 6.90(d, *J*=8.0Hz,1H), 7.21-7.46(m,10H), 7.62(d, *J*=8.0Hz,1H), 8.07(brs,1H).

### Example 55

### Synthesis of ([1-[2-(1-methyl-1-hydroxyethyl-6-methoxy) phenethyl)piperidin-4-yl)-(1H)-indol-6-yl]carboxamide

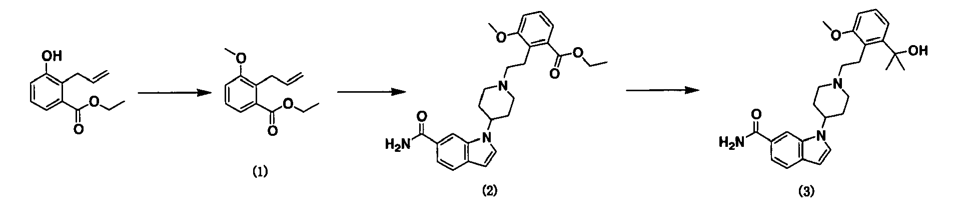

### (1) Synthesis of ethyl 3-methoxy-2-(2-propenyl)benzoate

300mg of ethyl 3-hydroxy-2-(2-propenyl)benzoate (CAS No. 53596-59-1), 150mg of iodomethane, 400mg of potassium carbonate in 8ml of N,N-dimethylformamide was stirred for 4 hours at room temperature. The mixture was partitioned between ethyl acetate and water and the ethyl acetate layer was separated. The ethyl acetate layer was washed with brine and then dried over magnesium sulfate. The mixture was filtered, the filtrate was concentrated, and the resulting residue was purified by silica gel column chromatography to give 280mg of the title compound as a colorless oil from ethyl acetate-hexane (1:9) fractions.
¹H-NMR (CDCl3)
δ: 1.38(t, *J*=7.2Hz, 3H), 3.71-3.78 (m,2H),3.84(s,3H), 4.34(q, J=7.2Hz,2H), 4.93-5.00(m,2H), 5.92-6.04(m,1H), 7.01(dd, *J*=1.2,8.0Hz,1H), 7.23(t, *J*=8.0Hz,1H) 7.39(dd, *J*=1.2,8.0Hz,1H).

### (2) Synthesis of ([1-[2-(1-ethoxycarbonyl-6-methoxy)phenethyl) piperidin-4-yl]-(1H)-indol-6-yl)carboxamide

270mg of ethyl 3-methoxy-2-(2-propenyl) benzoate was dissolved in 8ml of tetrahydrofuran 4ml of water, 0.2ml of 3.3% osmium tetroxide aqueous solution and 600mg of sodium perchlorate were added, and the mixture was stirred for 2 hours at room temperature. 5% aqueous sodium hydrogen sulfate was added to a mixture and the mixture was extracted with ethyl acetate. The organic layer was washed with water and brine and then dried over magnesium sulfate. The mixture was filtered, the filtrate was concentrated under a reduced pressure, and 210mg of the corresponding crude aldehyde was obtained through a silica gel short column.
After a mixture of 210mg of the obtained aldehyde, 200mg of [1-(piperidin-4-yl)-1*H*-indole-6-yl)carboxamide and 200mg of acetic acid in 12ml of methylene chloride was stirred for 15 minutes at room temperature, 400mg of sodium triacetoxyborohydride was added and stirred for 12 hours. 10% potassium carbonate water was added to a mixture, and was extracted with ethyl acetate. The ethyl acetate layer was washed with brine, dried over magnesium sulfate, then filtered, and concentrated under a reduced pressure. The residue was purified by silica gel flash column chromatography to give 260mg of the title compound.
¹H-NMR(CDCl3)
δ : 1.41(t, *J*=7.2Hz,3H), 2.07-2.22(m,4H), 2.30-2.44(m,2H), 2.62-2.73(m,2H), 3.15-3.30(m,4H), 3.87(s,3H), 4.37(q, *J*=7.2Hz, 2H), 6.57 (d, *J*=3.2Hz,1H) 7.01(d, *J*=8.0Hz,1H), 7.20-7.28(m,2H), 7.38-7.46(m,3H), 7.64(d, *J*=8.0Hz,1H), 8.11(brs,1H).

### (3) Synthesis of ([1-[1-(2-(1-methyl-1-hydroxyethyl-6- methoxy) phenethyl)piperidin-4-yl)-(1H)-indol-6-yl]carboxamide

260mg of ([1-[1-2-(1-ethoxycarbonyl-6-methoxy)phenethyl) piperidin-4-yl]-(1*H*)-indol-6-yl)carboxamide was dissolved in 8ml of tetrahydrofuran, and cooled to -70 °C under nitrogen atmosphere. 1.73ml of 1.04M methyl lithium ether solution was added to this solution. The mixture was stirred for 30 minutes at -70 °C and then slowly warmed to a room temperature. Ammonium chloride aqueous solution was added to the mixture, which was extracted with ethyl acetate. The ethyl acetate layer was washed with brine and dried over magnesium sulfate. The mixture was filtered, concentrated under a reduced pressure, and the residue was purified by silica gel flash column chromatography. 26mg of the title compound eluted by 10% methanol-methylene chloride was obtained as colorless solid.
¹H-NMR(DMSO-d6)
δ: ?1.53(m,6H), 1.92-2.09(m,2H), 2.29-2.38(m,2H), 2.54-2.61(m,2H), 3.04-3.11(m,2H), 3.13-3.19(m,2H), 3.78(s,3H), 4.36-4.46(m,1H), 6.49(d, *J*=3.2Hz,1H), 6.86(d, *J*=8.4Hz,1H), 7.00(dd, *J*=1.2,8.0Hz,1H), 7.09(dd, *J*=8.0,8.4Hz,1H), 7.20(brs,1H), 7.54(d, *J*=8.4Hz,1H), 7.56(dd, *J*=1.2,8.4Hz,1H) 7.65(d, *J*=3.2Hz,1H) 7.90(brs,1H) 8.12(s,1H)

### Example 56

### Synthesis of 1-[1-[2-(2-methoxy-6-morpholinophenyl)ethyl] piperidin-4-yl]-1H-indole-6-carboxamide

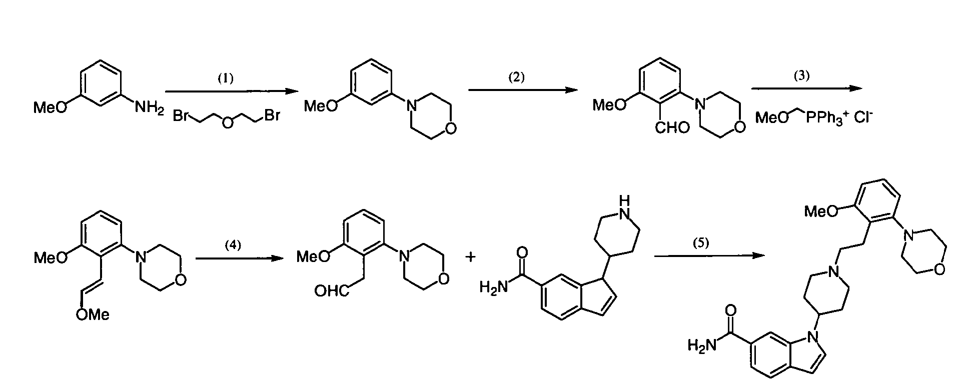

### (1) Synthesis of 1-methoxy-3-morpholinobenzene

2ml of 3-methoxyaniline was dissolved in 30ml of toluene, 7.8ml of N,N-diisopropylethylamine and 2.7ml of bis(2-bromoethyl) ether were added at room temperature. This reaction liquid was heated to reflux overnight. After the insoluble precipitation was removed by filtration, water and ethyl acetate were added to reaction liquid, and the organic layer was separated. The resulting organic layer was washed with brine and then dried over anhydrous sodium sulfate. After removing the drying agent by filtration, the organic layer was concentrated under a reduced pressure, and the residue was purified by silica gel column chromatography (hexane-ethyl acetate) to give 4.11g of the title compound.
¹H-NMR(CDCl3)
δ : 3.14-3.17(m,4H), 3.80(s,3H), 3.84-3.87(m,4H), 6.43-6.46(m,2H), 6.54(dd, 1H, *J*=1.6,7.5Hz), 7.19(dd,1H, *J*=7.5,8.4Hz).

### (2) Synthesis of 2-formyl-1-methoxy-3-morpholinobenzene

502mg of 1-methoxy-3-morpholinobenzene was dissolved in 5ml of anhydrous diethyl ether, under nitrogen atmosphere, 1.8ml of *n-*butyllithium (1.6M hexane solution) was added dropwise at room temperature. After dropping, this reaction liquid was heated to reflux for 6 hours. After reaction liquid was cooled, 438µl of N,N-dimethylformamide was dissolved in 2ml of anhydrous diethyl ether, which was added at room temperature. This reaction liquid was stirred for another 2 hours at room temperature. Water and diethyl ether were added to reaction liquid, and the organic layer was separated. The resulting organic layer was washed with brine and then dried over anhydrous sodium sulfate. After removal of drying agent by filtration, the organic layer was concentrated under a reduced pressure. The residue was purified by silica gel column chromatography (hexane-ethyl acetate) to give 653mg of the title compound (and, 643mg of starting material recovery) was obtained.
¹H-NMR(CDCl3)
δ: 3.07-3.09(m,4H), 3.89-3.91(m,4H), 3.91(s,3H), 6.63(d,1H, *J*=8.4Hz), 6.66(d,1H, *J*=8.2Hz), 7.45(dd,1H, *J*=8.2,8.4Hz), 10.38(s,1H).

### (3) Synthesis of 1-methoxy-2-(2-methoxyvinyl)-3-morpholino benzene

Under nitrogen atmosphere, 2.28g of (methoxymethyl) triphenylphosphonium chloride suspended in 10ml of tetrahydrofuran, in an ice-cooling, 241mg of potassium *tert*-butoxide was added, and the reaction liquid was stirred for 15 minutes. 214mg of 2-formyl-1-methoxy-3-morpholinobenzene was dissolved in 5ml of tetrahydrofuran, which was then added to reaction liquid in an ice-cooling. Then, reaction liquid was raised to a room temperature, and stirred for another 2 hours. Water and diethyl ether were added to the reaction liquid, and the organic layer was separated. The resulting organic layer was washed with brine and then dried over anhydrous sodium sulfate. After removal of drying agent by filtration, the organic layer was concentrated under a reduced pressure. The residue was purified by silica gel column chromatography (hexane-ethyl acetate) to give (i) 174mg and (ii) 58mg of geometric isomers of the title compound, respectively.
A <geometric isomer (i) = trans form>)
¹H-NMR(CDCl3)
5: 2.94-2.97(m,4H), 3.70(s,3H), 3.70-3.86(m,4H), 3.85(s,3H), 6.09(d,1H, *J*=13Hz), 6.66-6.69(m,2H), 7.09(dd,1H, *J*=8.1,8.2Hz), 7.57(d,1H, *J*=13Hz).
A <geometric isomer (ii) = cis form>)
¹H-NMR(CDCl3)
δ: 2.99(t,4H, J=4.6Hz), 3.67(s,3H), 3.80(t,4H, *J*=4.6Hz), 3.85 (s,3H), 5.30 (d,1H, *J*=6.8Hz), 6.16(d,1H, *J*=6.8Hz), 6.64(d, 1H, *J*=8.1Hz), 6.66(d,1H, *J*=8.2Hz), 7.18(dd,1H, *J*=8.1Hz, 8.2Hz).

### (4) Synthesis of (2-methoxy-6-morpholinophenyl)acetaldehyde

174mg of 1-methoxy-2-(2-methoxyvinyl)-3-morpholinobenzene was dissolved in 4ml of 2N HCl-tetrahydrofur (1:1), the reaction liquid was stirred for 1.5 hours at 70 °C. After the reaction liquid was stood to cool to a room temperature, saturated aqueous potassium carbonate solution and diethyl ether were added and the organic layer was separated. The resulting organic layer was washed with brine and then dried over anhydrous sodium sulfate. After removing the drying agent by filtration, the organic layer was concentrated under a reduced pressure to give 130mg of the title compound.
¹H-NMR(CDCl3)
δ: 2.86(t,4H, *J*=4.6Hz), 3.71(d, 2H, *J*=2.2Hz), 3.79(t, 4H, *J*=4.6Hz), 3.82(s, 3H), 6.76(d, 1H, *J*=8.2Hz), 6.84(d, 1H, *J*=8.1Hz), 7.29(dd,1H, *J*=8.1,8.2Hz), 9.58(d,1H, *J*=2.2Hz).

### (5) Synthesis of 1- [1-[2-(2-methoxy-6-morpholinophenyl)ethyl] piperidin-4-yl]-1H-indole-6-carboxamide

134mg of 1-(piperidin-4-yl)-1H-indole-6-carboxamide (synthesized in production example 1) and 130mg of (2-methoxy-6-morpholinophenyl)acetaldehyde was dissolved in 5ml of dichloromethane, 63µl of acetic acid and 183mg of sodium triacetoxyborohydride were added to a reaction liquid, the reaction liquid was stirred for 3 hours at room temperature. Aqueous sodium hydroxide and dichloromethane were added to the reaction liquid, and the organic layer was separated. The resulting organic layer was washed with brine and then dried over anhydrous sodium sulfate. After removal of drying agent by filtration, the organic layer was concentrated under a reduced pressure. The residue was purified by NH silica gel column chromatography (ethyl acetate) to give 108mg of the title compound.
¹H-NMR(CDCl3)
δ: 2.11-2.18(m, 4H), 2.29-2.36(m,2H), 2.60-2.64(m,2H), 2.89-2.91(m,4H), 2.98-3.02(m,2H), 3.26-3.29(m,2H), 3.83(s,3H), 3.85-3.87(m,4H), 4.37-4.42(m, 1H), 6.56(d,1H, J=2.8Hz), 6.70(d,1H, *J*=7.2Hz), 6.81(d,1H, *J*=7.2Hz), 7.16-7.20(m,1H), 7.39-7.42(m,2H), 7.63(d,1H, *J*=8.4Hz), 8.10(s, 1H).

### Example 57

### Synthesis of 1-[1-[2-[4-(dimethylcarbamoyl)-2-methoxyphenyl] ethyl]piperidin-4-yl]-1H-indole-6-carboxamide

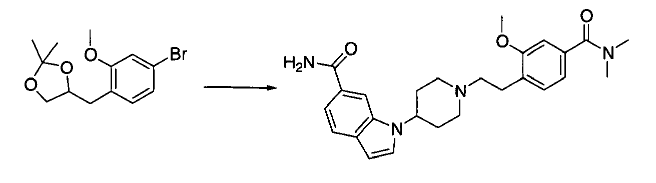

The title compound was obtained by synthesizing from 4-(4-bromo-2-methoxybenzyl)-2,2-dimethyl-[1,3]dioxolane in accordance with the methods in example 38- (1), example 53- (9) and (10).
¹H-NMR (DMSO-d6)
δ: 1.92-2.08(m,4H), 2.22-2.31(m,2H), 2.53-2.61(m,2H), 2.75-2.83(m,2H), 2.87-3.02(m,6H), 3.07-3.15(m,2H), 3.82(s,3H), 4.37-4.47(m,1H), 6.50(d, J=3.2Hz,1H), 6.90(dd, J=1.6,7.2Hz,1H), 6.96(d, *J*=1.6Hz,1H), 7.21(brs,1H), 7.24(d, *J*=7.2Hz,1H), 7.53-7.61(m,2H), 7.68(d, *J*=3.2Hz,1H), 7.91(brs,1H), 8.13(s,1H).

### Example 58

### Synthesis of 1-[1-[2-[2-methoxy-5-(1-methylpiperidin-4-yl) phenyl]ethyl]piperidin-4-yl]-N-methyl-1H-indole-6-carboxamide

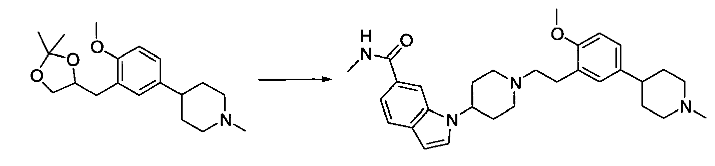

188mg of 4-[3-[(2,2-dimethyl-[1,3]dioxolan-4-yl)methyl]-4-methoxyphenyl]-1-methylpiperidine was dissolved in 2ml of methanol, 2ml of 4N hydrochloric acid-ethyl acetate solution was added, which was stirred at room temperature for 30 minutes. The reaction liquid was concentrated under a reduced pressure to afford 250mg of 3-[2-methoxy-5-(1-methylpiperidin-4-yl)phenyl]propane-1,2-diol hydrochloride. This compound was used in the next reaction without further purification.
250mg of 3-[2-methoxy-5-(1-methylpiperidin-4-yl)phenyl] propane-1,2-diol hydrochloride was dissolved in 3ml of methanol, saturated sodium bicarbonate aqueous solution was added in an ice-cooling to be pH 7, a solution of 0.43g of sodium metaperiodate in 3ml of water was added, which was stirred for 30 minutes at room temperature. Brine was added to reaction liquid, which was extracted with methylene chloride. The extract was concentrated under a reduced pressure after drying over magnesium sulfate to yield 127mg of [2-methoxy-5-(1-methylpiperidin-4-yl)phenyl]acetaldehyde. This compound was used in the next reaction without further purification.
80mg of N-methyl 1-(piperidin-4-yl)-1*H*-indole-6-carboxamide, which was synthesized in production example 2, and 127mg of [2-methoxy-5-(1-methylpiperidin-4-yl)phenyl] acetaldehyde was dissolved in 8ml of methylene chloride, and 0.05ml of acetic acid and 0.10g of sodium triacetoxyborohydride were added, which was stirred for 3 hours. Saturated sodium bicarbonate aqueous solution was added to reaction liquid, which was extracted with methylene chloride. The extract was concentrated under a reduced pressure after drying over magnesium sulfate, and purified by silica gel column chromatography (ethyl acetate-methanol), followed by high performance liquid chromatography (ODS-AM) acetonitrile-water) to give 57mg of the title compound.
¹H-NMR (CDCl3)
δ : 1.90-2.00(m,2H), 2.04-2.44(m,8H), 2.47-2.77(m,7H), 2.85-2.94(m,2H), 3.06(d, *J*=5.4Hz,3H), 3.24-3.48(m,4H), 3.82 (s, 3H), 4.35-4.46 (m, 1H), 6.36 (brs, 1H), 6.52-6.56 (m, 1H), 6.80(d, J=6.2Hz,1H), 7.02-7.08(m,2H), 7.34-7.41(m,2H), 7.59-7.63(m,1H), 8.06(s,1H).

### Example 59

### Synthesis of 1-[1-[2-[4-(acetyl methylamino)-2-methoxyphenyl] ethyl]piperidin-4-yl]-1H-indole-6-carboxamide

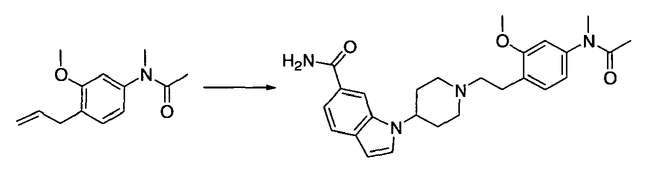

The title compound was obtained by synthesizing from N-(4-allyl-3-methoxyphenyl)-N-methyl acetamide in accordance with the methods in example 1-(3).
¹H-NMR(DMSO-d6)
δ: 1.79(s,3H), 1.92-2.10(m,4H), 2.22-2.32(m,2H), 2.50-2.61(m,2H), 2.72-2.81(m,2H), 3.06-3.20-(m,2H), 3.14(s,3H), 3.82(s,3H), 4.38-4.48(m,1H), 6.51(d, J=3.2Hz,1H), 6.83(d, *J*=8.0Hz,1H), 6.95(s,1H), 7.17-7.28(m,2H), 7.51-7.62(m,2H), 7.67(d, *J*=3.2Hz,1H), 7.91(brs,1H), 8.13(s,1H).

### Example 60

### Synthesis of 1-[1-[2-[2-methoxy-5-(2-oxo-2H-pyridin-1-yl) phenyl]ethyl]piperidin-4-yl]-1H-indole-6-carboxamide

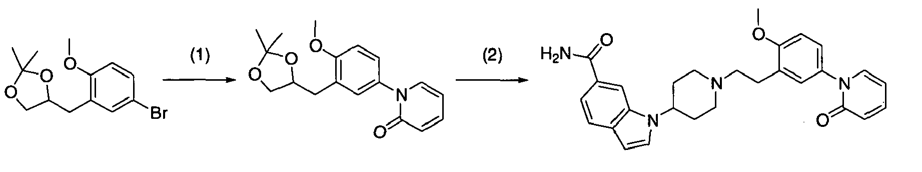

### (1) Synthesis of 1-[3-[-(2,2-dimethyl-[1,3]dioxolan-4-yl) methyl]-4-methoxyphenyl]-1H-pyridin-2-one

1.00g of 4-(5-bromo-2-methoxybenzyl)-2,2-dimethyl-[1, 3] dioxolane was dissolved in 20ml of N,N-dimethylformamide, 1.58g of 2-hydroxypyridine, 0.32g of copper iodide and 1.38g of potassium carbonate were added, which was heated at 150 °C for 6 hours under nitrogen atmosphere. The reaction liquid was diluted in ethyl acetate after standing to cool at room temperature and washed with saturated sodium bicarbonate aqueous solution and brine. The organic layer is concentrated under a reduced pressure after drying over magnesium sulfate, and purified by NH silica gel column chromatography (ethyl acetate-hexane) to give 474mg of the title compound.
¹H-NMR(CDCl3)
δ: 1.33(s,3H), 1.42(s,3H), 2.83-2.90(m,1H), 2.94-3.02(m,1H), 3.63-3.69(m,1H), 3.85(s,3H), 3.95-4.01(m,1H), 4.33-4.41(m,1H), 6.18-6.24(m,1H) 6.64(d, *J*=8.4Hz,1H), 6.91(d, *J*=8.0Hz,1H), 7.16-7.25(m,2H), 7.28-7.40(m,2H).

### (2) Synthesis of 1-[1-[2-[2-methoxy-5-(2-oxo-2H-pyridin-1-yl) phenyl]ethyl]piperidin-4-yl]-1H-indole-6-carboxamide

The title compound was obtained by synthesizing from 1-[3-[-(2,2-dimethyl-[1,3]dioxolan-4-yl)methyl]-4-methoxyphenyl]-1*H*-pyridin-2-one in accordance with the methods in example 53-(9) and (10).
¹H-NMR(DMSO-d6)
5: 1.91-2.10(m,4H), 2.23-2.33(m,2H), 2.54-2.63(m,2H), 2.76-2.84(m,2H), 3.07-3.15(m,2H), 3.86(s,3H), 4.37-4.48 (m,1H), 6.29(ddd, *J*=1.2,6.4,6.4Hz,1H), 6.46(d, *J*=8.8Hz,1H), 6.50(d, *J*=3.2Hz,1H), 7.04-7.10(m,1H), 7.16-7.27(m,3H), 7.46-7.53(m,1H), 7.53-7.64(m,3H), 7.67(d, *J*=3.2Hz,1H), 7.91(brs,1H), 8.13(s,1H).

### Example 61

### Synthesis of {1-[2-(5-cyano-2-methoxyphenyl)ethyl] piperidin-4-yl}-1H-indole-6-carboxamide

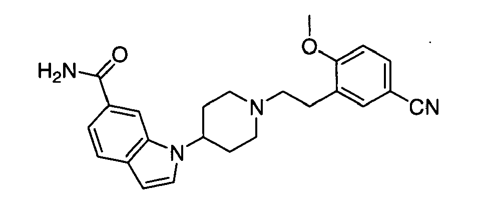

The title compound was obtained by synthesizing from 3-allyl-4-methoxy-cyanobenzene in accordance with the methods in example 1-(3).
¹H-NMR(CDCl3)
δ: 2.06-2.15(m,4H), 2.25-2.34(m,2H), 2.58-2.66(m,2H), 2.84-2.89(m,2H), 3.14-3.22(m,2H), 3.90(s,3H), 4.35-4.43(m,1H), 6.57(d, *J*=3.2Hz,1H), 6.89(d, *J*=8.4Hz,1H), 7.38-7.40(m,2H), 7.46(d, *J*=2.0Hz,1H), 7.53 (dd, *J*=2.0,8.4Hz,1H), 7.63 (d, *J*=8.0Hz,1H), 8.10(s,1H).

### Example 62

### Synthesis of {1-[2-(6-cyano-2-methoxyphenyl)ethyl] piperidin-4-yl}-1H-indole-6-carboxamide

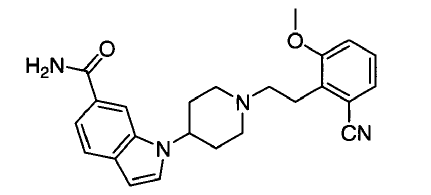

The title compound was obtained by synthesizing from 2-allyl-3-methoxy-cyanobenzene in accordance with the methods in example 1- (3).
¹H-NMR(CDCl3)
δ: 2.06-2.16(m,4H), 2.32-2.41(m,2H), 2.53-2.68(m,2H), 3.06-3.13(m,2H), 3.17-3.26(m,2H), 3.88(s,3H), 4.33-4.42(m,1H), 6.56(d, *J*=3.2Hz,1H), 7.06(d, *J*=8.4Hz,1H), 7.21-7.29(m,2H), 7.38-7.42(m,2H), 7.63(d, *J*=8.0Hz,1H), 8.08(s,1H).

### Example 63

### Synthesis of {1-[2-(6-hydroxymethyl-2-methoxyphenyl)ethyl] piperidin-4-yl}-1H-indole-6-carboxamide

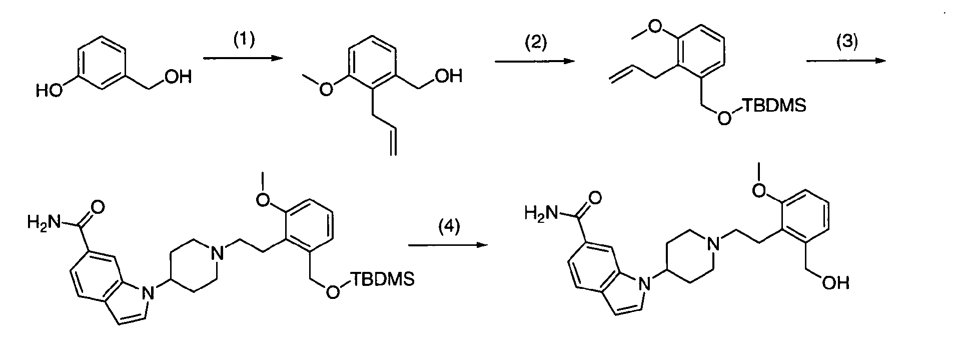

### (1) Synthesis of 2- allyl-3-methoxybenzyl alcohol

The title compound was obtained by synthesizing from 3-hydroxybenzyl alcohol in accordance with example 1- (1) and (2).
¹H-NMR(CDCl3)
δ: 3.49-3.51(m,2H), 3.82(s,3H), 4.67(s,2H), 4.89-4.99(m,2H), 5.94-6.03(m,1H), 6.85(d, *J*=8.4Hz,1H), 7.01(d, *J*=7.6Hz,1H), 7.19-7.23 (m,1H).

### (2) Synthesis of 2-allyl-6-(tert-butyldimethylsilyloxy)anisole

To a solution of 1.94g of 2-allyl-3-methoxybenzyl alcohol, 2.13g of *tert*-butyldimethylsilyl chloride in 10ml of N,N-dimethylformamide was added 1.85g of imidazole, which was stirred overnight. Water was added to reaction liquid, which was extracted with diethyl ether. The organic layer was washed with brine and dried over magnesium sulfate. Magnesium sulfate was removed by filtration and the filtrate was concentrated under a reduced pressure. Purification by silica gel column chromatography (hexane-ethyl acetate) afforded 3.05g of the title compound.
¹H-NMR(CDCl3)
δ: 0.09(s,6H), 0.94(s,9H), 3.39-3.41(m,2H), 3.81(s,3H), 4.72(s,2H), 4.86-7.96(m,2H), 5.85-5.94 (m,1H),6.79-6.81 (m,1H), 7.07-7.09(m,1H), 7.17-7.21(m 1H).

### (3) Synthesis of {1-[2-(6-(tert-butyldimethylsilyloxy)methyl-2-methoxyphenyl)ethyl]piperidin-4-yl}-1H-indole-6-carboxamide

The title compound was obtained by synthesizing from 2-allyl-6-(*tert*-butyldimethylsilyloxy)anisole in accordance with the methods in example 1-(3).
¹H-NMR(CDCl3)
δ: 0.14(s,6H), 0.94(s,9H), 2.18-2.21(m,2H), 2.88-3.02(m,2H), 3.15-3.34(m,4H), 3.43-3.56(m,2H), 3.76-3.87(m,2H), 3.87(3H,s), 4.44-4.54(m, 1H), 4.77(s,2H), 5.62(brs,1H), 6.52(d, *J*=2.8Hz,1H), 6.84(d, *J*=7.6Hz,1H), 6.98(d, *J*=7.2Hz,1H), 7.22-7.29(m,2H), 7.65(d, *J*=8.4Hz,1H), 7.74-7.77(m,1H), 8.52(s,1H), 12.82(brs,1H).

### (4) Synthesis of {1-[2-(6-hydroxymethyl-2-methoxyphenyl)ethyl] piperidin-4-yl}-1H-indole-6-carboxamide

0.25g of {1-[2-(6-(*tert*-butyldimethylsilyloxy)methyl-2-methoxyphenyl)ethyl]piperidin-4-yl}-1*H*-indole-6-carboxamide was dissolved in 5ml of methanol, *p*-toluenesulfonic acid monohydrate 0.10g was added, which was stirred for 30 minutes at room temperature. Ethyl acetate and 10% sodium carbonate aqueous solution were added followed by stirring. The organic layer was separated, washed sequentially with a mixed solvent of brine and water (1:1), and brine, and dried over magnesium sulfate. Magnesium sulfate was removed by filtration and the filtrate was concentrated under a reduced pressure. The residue was purified by NH silica gel column chromatography (ethyl acetate-methanol) to give 0.13g of the title compound.
¹H-NMR(CDCl₃)
δ: 1.89-1.95(m,2H), 2.24-2.32(m,2H), 2.37-2.47(m,2H), 2.67-2.69(m,2H), 2.89-2.96(m,4H), 3.85(s,3H), 4.20-4.28(m,1H), 4.57(s,2H), 5.52(brs,1H), 6.47-6.48(m,1H), 6.84-7.10(m,3H), 7.18-7.28(m,2H), 7.58-7.63(m,2H), 8.19 (s,1H).

### Example 64

### Synthesis of 1-{1-[2-(6-(pyridin-2-yloxy)-2-methoxyphenyl) ethyl]piperidin-4-yl}-1H-indole-6-carboxamide

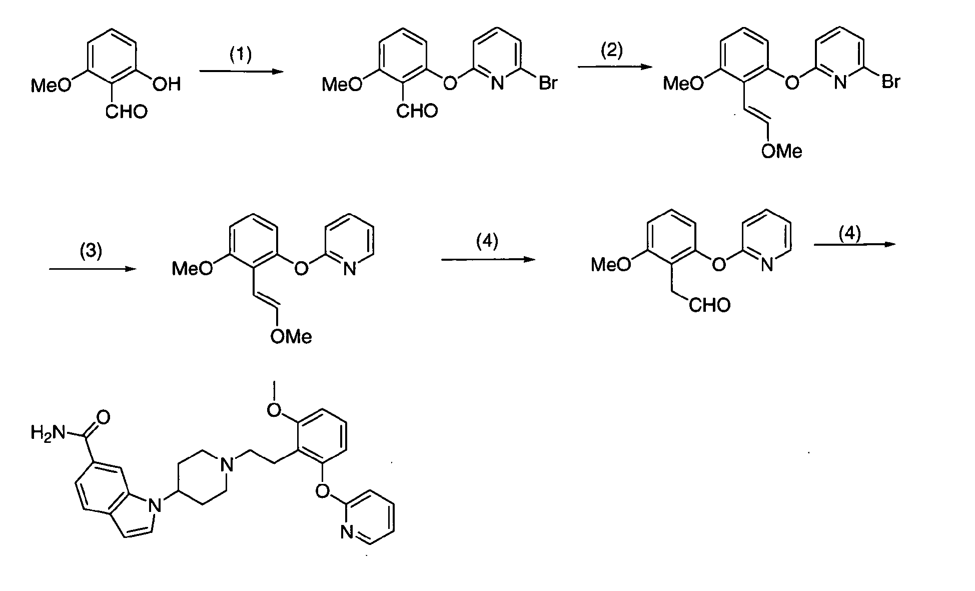

### (1) Synthesis of 2-methoxy-6-(6-bromopyridin-2-yloxy) benzaldehyde

3.06g of 2-methoxy-6-hydroxybenzaldehyde and 5.24g of 2,6-dibromopyridine were dissolved in 10ml of N-methylpyrrolidone. To this mixture was added 2.78g of potassium carbonate, under nitrogen atmosphere, the mixture was stirred for 105 minutes at 120 °C and for another 75 minutes at 120 °C. Ethyl acetate and water were added to the reaction mixture, which was stirred, the organic layer was separated. The resulting organic layer was sequentially washed with water (three times) and brine, and dried over magnesium sulfate. Magnesium sulfate was removed by filtration and the filtrate was concentrated under a reduced pressure. *tert*-Butyl methyl ether was added to the residue, collected by filtration, and 2.25g of the title compound was obtained.
¹H-NMR(CDCl3)
δ: 3.95(s,3H), 6.73-6.75(m,1H), 6.87(dd, *J*=0.6Hz,8.6Hz,1H), 6.90(dd, *J*=0.6Hz,8.2Hz,1H), 7.16(dd, *J*=0.6Hz,7.4Hz,1H), 7.50-7.55(m,1H), 10.37(s,1H).

### (2) Synthesis of 2- bromo-6-[3-methoxy-2-((E)-2-methoxyvinyl) phenoxy] pyridine

2.31g of (methoxymethyl)triphenylphosphonium chloride was suspended in 25ml of tetrahydrofuran, which was stirred in an ice cooling under nitrogen atmosphere. 0.74g of potassium *tert*-butoxide was added thereto, stirred for 10 minutes. 1.01g of 2-methoxy-6-(6-bromopyridin-2-yloxy)benzaldehyde was dissolved in 10ml of tetrahydrofuran, which was added thereto, and stirred for 10 minutes. The mixture was stirred for another 15 minutes at room temperature. To reaction liquid was added saturated ammonium aqueous solution and water, and extracted with ethyl acetate, and washed with brine, and then dried over magnesium sulfate. Magnesium sulfate was removed by filtration and the filtrate was concentrated under a reduced pressure. Purification by silica gel column chromatography (hexane-ethyl acetate) afforded 690mg of the title compound and 170mg of the (Z)-isomer of double bond.
(E)-isomer
¹H-NMR(CDCl₃)
δ: 3.58(s,3H), 3.89(s,3H), 5.83(d, *J*=12.8Hz,1H), 6.57(dd , *J*=0.6Hz,8.2Hz,1H), 6.68(dd, *J*=1.0Hz,8.2Hz,1H), 6.76(dd, *J*=1.0Hz,8.2Hz,1H), 7.07-7.11(m,1H) 7.15(dd, *J*=0.8Hz,7.6Hz,1H), 7.41-7.45(m,2H).
(Z)-isomer
¹H-NMR(CDCl3)
5: 3.45(s,3H), 3.86(s,3H), 5.12(d, *J*=6.8Hz,1H), 6.06(d, *J*=6.8Hz,1H), 6.63-6.65(m,1H), 6.73-6.77(m,2H), 7.10-7.12(m,1H), 7.19-7.23(m,1H) 7.40-7.44(m,1H).

### (3) Synthesis of 2-[3-methoxy-2-((E)-2-methoxyvinyl)phenoxy] pyridine

0.23g of 2-bromo-6-[3-methoxy-2-((E)-2-methoxyvinyl) phenoxy] pyridine was suspended in 7ml of tetrahydrofuran, under nitrogen atmosphere, which was stirred in a dry ice-acetone bath. 0.64ml of N-butyllithium (1.6M hexane solution) was added dropwise thereto, which was stirred for 30 minutes under the same condition. Next, 0.14ml of methanol was added, and stirred for 10 minutes and then stirred for another 15 minutes at room temperature. To reaction liquid was added saturated ammonium aqueous solution and water, and extracted with ethyl acetate. The organic layer was washed with brine and dried over magnesium sulfate. Magnesium sulfate was removed by filtration and the filtrate was concentrated under a reduced pressure. A mixture of this resulting residue and the resulting residue equally prepared from 0.46g of 2-bromo-6-[3-methoxy-2-((E)-2-methoxyvinyl)phenoxylpyridine was purified by silica gel column chromatography (hexane-ethyl acetate) to give 386mg of the title compound.
¹H-NMR(CDCl3)
δ : 3.56(s,3H), 3.88(s,3H), 5.85(d, *J*=12.8Hz,1H), 6.69(dd, *J*=1.0Hz,8.2Hz,1H), 6.75(dd, J=0.8Hz,8.4Hz,1H), 6.79-6.81(m,1H), 6.93-6.96(m,1H), 7.08-7.12(m,1H), 7.43(d, J=12.8Hz,1H), 7.61-7.65(m,1H), 8.17-8.19(m,1H).

### (4) Synthesis of 1-{1-[2-(6-(pyridin-2-yloxy)-2-methoxyphenyl) ethyl]piperidin-4-yl}-1H-indole-6-carboxamide

0.39g of 2-bromo-6-[3-methoxy-2-((E)-2-methoxyvinyl) phenoxy] pyridine was dissolved in 3ml of tetrahydrofuran, 2N HCl 2ml was added, which was stirred for 80 minutes at 70 °C. The reaction mixture was cooled by an ice, 10% sodium carbonate aqueous solution was added, and extracted with ethyl acetate. The organic layer was washed with brine and dried over magnesium sulfate. Magnesium sulfate was removed by filtration and the filtrate was concentrated under a reduced pressure to give 336mg of the title compound. This was used in the next reaction without further purification.
266mg of 1-(piperidin-4-yl)-1*H*-indole-6-carboxamide, which was synthesized in Production example 1, and 336mg of [2-methoxy-6-(pyridin-2-yloxy)phenyl]acetaldehyde were dissolved in 15ml of methylene chloride, 125µl of acetic acid was added, which was stirred for 5 minutes at room temperature. 0.35g of sodium triacetoxyborohydride was added afterwards, which was stirred for 7 hours at room temperature. The reaction liquid was diluted in chloroform, 5N aqueous sodium hydroxide was added, and extracted with chloroform. The organic layer was washed with brine and dried over magnesium sulfate. Magnesium sulfate was removed by filtration and the filtrate was concentrated under a reduced pressure to give 538mg of the title compound.
¹H-NMR(CDCl3)
5: 2.07-2.12(m,2H), 2.70-3.70(m,8H), 3.90(s,3H), 4.35-4.44(m,1H), 5.64(s,1H), 6.51-6.52(m, 1H), 6.67-6.70(m,1H), 6.76-6.78(m,1H), 6.97-7.05(m,2H), 7.25-7.29(m,3H), 7.51-7.75(m,3H), 8.18-8.19(m,1H), 8.39(brs,1H).

### Example 65

### Synthesis of one {1-[2-(2-cyanomethyl-6-methoxyphenyl)ethyl] piperidin-4-yl}-1H-indole-6-carboxamide

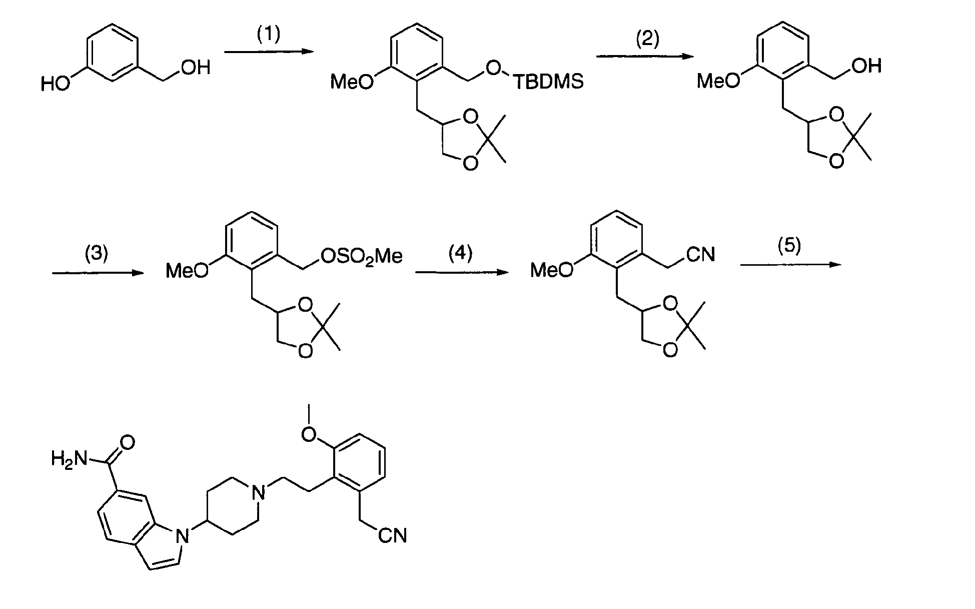

### (1) Synthesis of tert-butyl-[2-(2,2-dimethyl-[1,3] dioxolan-4-ylmethyl)-3-methoxybenzyloxy]dimethylsilane

The title compound was obtained by synthesizing from 3-hydroxybenzyl alcohol in accordance with the methods in example 53-(1) to (5).
¹H-NMR(CDCl3)
δ: 0.10 (s,3H), 0.11 (s,3H), 0.94 (s,9H), 1.31 (s,3H), 1.42 (s,3H), 2.87(dd, *J*=2.4Hz,13.6Hz,1H), 2.97(dd, *J*=2.4Hz,13.6Hz,1H), 3.66(dd, *J*=3.2Hz,8.0Hz,1H), 3.80-(s,3H), 3.94(dd, *J*=2.2Hz,8.2Hz,1H), 4.27-4.34(m,1H), 6.76-6.78 (m,1H), 7.08-7.10(m,1H), 7.18-7.22 (m,1H).

### (2) Synthesis of [2-(2,2-dimethyl-[1,3]dioxolan-4-ylmethyl)-3-methoxyphenyl]methanol

2.22g of *tert*-butyl-[2-(2,2-dimethyl-[1,3]dioxolan-4-ylmethyl)-3-methoxybenzyloxy]dimethylsilane was dissolved in 30ml of tetrahydrofuran, which was stirred at room temperature. 7.26ml of tetrabutylammonium fluoride (1.0M THF solution) was added thereto, which was stirred for 30 minutes at room temperature. Water was added to reaction liquid, which was extracted with ethyl acetate. The organic layer was washed with brine and dried over magnesium sulfate. Magnesium sulfate was removed by filtration, the filtrate was concentrated under a reduced pressure. Purification by NH silica gel column chromatography (hexane-ethyl acetate) afforded 1.50g of the title compound.
¹H-NMR(CDCl3)
δ: 1.30 (s, 3H), 1.40(s,3H), 2.81 (dd, *J*=8.4Hz, 14.0Hz, 1H), 3.18 (dd, *J*=2.6Hz,14.2Hz,1H), 3.63(dd, *J*=8.2Hz,8.2Hz,1H), 3.80-3.84(m,1H), 3.82(s,3H), 4.18(dd, *J*=5.8Hz,8.2Hz,1H), 4.31-4.37(m,1H), 4.42-4.48(m,1H), 7.74-4.77(m,1H), 6.84-6.86(m,1H), 6.98-7.00(m,1H), 7.20-7.24(m,1H).

### (3) Synthesis of 2-(2,2-dimethyl-[1,3]dioxolan-4-ylmethyl)-3-methoxybenzyloxybenzyl methanesulfonate

0.30g of [2-(2,2-dimethyl-[1,3]dioxolan-4-ylmethyl)-3-methoxyphenyl]methanol, 826µl of triethylamine was dissolved in 10ml of tetrahydrofuran, which was stirred in an ice-cooling. 92µl of methanesulfonyl chloride was added thereto, which was stirred for 20 minutes. 92µl of methanesulfonyl chloride was added again, and stirred for 20 minutes followed by stirring at room temperature for 40 minutes. A reaction mixture was cooled in an ice, saturated sodium bicarbonate aqueous solution was added, and extracted with ethyl acetate. The organic layer was washed with brine and dried over magnesium sulfate. Magnesium sulfate was removed by filtration, the filtrate was concentrated under a reduced pressure to give 450mg of the title compound. This was used in the next reaction without further purification.
¹H-NMR(CDCl3)
δ: 1.30(s,3H), 1.37(s,3H), 2.91(s,3H), 2.94(dd, *J*=3.4Hz,13.6Hz,1H), 3.10(dd, *J*=4.6Hz,13.6Hz,1H), 3.62(dd, *J*=7.4Hz,8.2Hz,1H), 3.83(s,3H), 4.04(dd, *J*=6.0Hz,8.4Hz,1H), 4.27-4.33(m,1H), 5.35(d, J=11.6Hz,1H), 5.49(d, *J*=11.6Hz,1H), 6.90-6.92(m,1H), 7.03-7.05(m,1H), 7.23-7.27(m,1H).

### (4) Synthesis of [2-(2,2-dimethyl-[1,3]dioxolan-4-ylmethyl)-3-methoxyphenyl]acetonitrile

450mg of 2-(2,2-dimethyl-[1,3]dioxolan-4-ylmethyl)-3-methoxybenzyloxybenzyl methanesulfonate was dissolved in 8ml of dimethyl sulfoxide. 0.58g of sodium cyanide was added thereto, which was stirred overnight. Water was added to reaction liquid, which was extracted with ethyl acetate-*n-*hexane. The organic layer was washed with water (three times) and brine sequentially and dried over magnesium sulfate. Magnesium sulfate was removed by filtration and the filtrate was concentrated under a reduced pressure. Purification by NH silica gel column chromatography (hexane-ethyl acetate) afforded 270mg of the title compound.
¹H-NMR(CDCl3)
δ: 1.30 (s, 3H), 1.33 (s, 3H), 2.79 (dd, *J*=3.2Hz,14.0Hz,1H), 3.06(dd, *J*=4.0Hz,14.0Hz,1H), 3.57(dd, *J*=7.8Hz,7.8Hz,1H), 3.82 (s, 3H), 3.90(d, *J*=18.2Hz,1H 3.97(d, *J*=18.2Hz,1H), 4.05(dd, *J*=6.0Hz,8.0Hz,1H), 4.25-4.32 (m,1H), 6.84-6.86 (m,1H), 7.04-7.06(m,1H), 7.21-7.25(m,1H).

### (5) Synthesis of 1-{1-[2-(2-cyanomethyl-6-methoxyphenyl)ethyl] piperidin-4-yl}-1H-indole-6-carboxamide

The title compound was obtained by synthesizing from [2-(2,2-dimethyl-[1,3]dioxolan-4-ylmethyl)-3-methoxyphenyl] acetonitrile in accordance with the methods in example 53 - (9) to (10).
¹H-NMR(CDCl3)
δ: 2.16-2.25(m,2H), 2.80-3.88(m,7H), 3.87(s,3H), 4.44-4.55(m,1H), 5.61(brs,1H), 6.55(d, *J*=2.8Hz,1H), 6.89(d, *J*=8.0Hz,1H), 6.98(d, *J*=7.6Hz,1H), 7.25-7.30(m,2H), 7.64-7.76(m,3H), 8.40(brs,1H).

### Example 66

### Synthesis of 1-{1-{2-[2-(1-hydroxyethyl)-6-methoxyphenyl] ethyl}piperidin-4-yl}-1H-indole-6-carboxamide

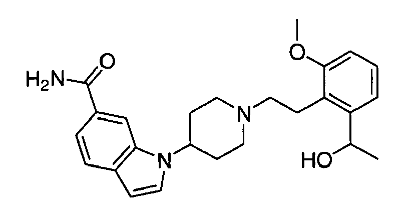

The title compound was obtained by synthesizing from 3-(1-hydroxymethyl)phenol in accordance with the methods in example 63.
¹H-NMR(CDCl3)
δ: 1.61(d, *J*=6.4Hz,3H), 1.82-1.88(m,2H), 1.98-2.06(m,1H), 2.18-2.25(m,3H), 2.44-2.73(m,4H), 2.83-2.91(m,1H), 3.04-3.09(m,1H), 3.24-3.30(m,1H), 3.85(s,3H), 4.20-4.28(m,1H), 5.08-5.13(m,1H), 5.49(brs,1H), 6.48(d, *J*=3.2Hz,1H), 6.78(brs,1H), 6.83(d, *J*=8.0Hz,1H), 7.09(d, *J*=7.2Hz,1H), 7.22-7.29(m,2H), 7.57-7.63(m,2H), 8.09(s,1H).

### Example 67

### Synthesis of 1-{1-[2-(2-acetyl-6-methoxyphenyl)ethyl] piperidin-4-yl}-1H-indole-6-carboxamide

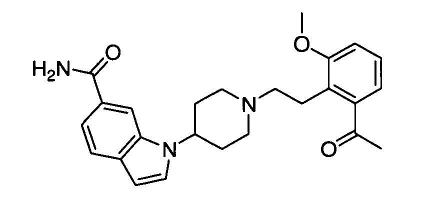

260mg of 1-{1-[2-(1-hydroxyethyl)-6-methoxyphenyl)ethyl] piperidin-4-yl}-1*H*-indole-6-carboxamide was dissolved in 4ml of methylene chloride and was stirred in an ice-cooling. 390mg of Dess-Martin reagent was added, which was stirred for 5 minutes. Sodium thiosulfate aqueous solution, a saturated sodium bicarbonate water solution and chloroform were added, which was stirred. The insoluble precipitate was removed by filtration and the organic layer was separated. The organic layer was dried over magnesium sulfate. Magnesium sulfate was removed by filtration and the filtrate was concentrated under a reduced pressure. Re-precipitation from chloroform-ethyl acetate-*tert*-butyl methyl ether gave 35mg of the title compound.
¹H-NMR(CDCl3)
δ: 2.08-2.45(m,6H), 2.59(s,3H), 2.63-2.72(m,2H), 3.05-3.09 (m,2H), 3.22-3.32 (m,2H), 3.87 (s,3H), 4.34-4.44 (m,1H), 4.81-6.50(brs,2H), 6.56(d, *J*=3.2Hz,1H), 6.98-7.00 (m,1H), 7.20-7.28(m,2H), 7.39-7.44(m,2H), 7.63(d, *J*=8.0Hz,1H), 8.09 (s,1H).

### Example 68

### Synthesis of 1-{1-{2-[2-methoxy-6-(morpholinomethyl)phenyl] ethyl}piperidin-4-yl}-1H-indole-6-carboxamide

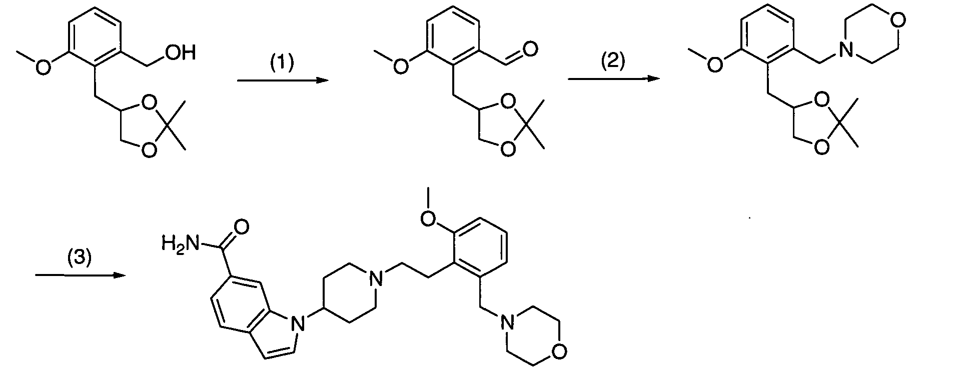

### (1) Synthesis of 2-(2,2-dimethyl-[1,3]dioxolan-4-ylmethyl)-3-methoxybenzaldehyde

8.65g of [2-(2,2-dimethyl-[1,3]dioxolan-4-ylmethyl)-3-methoxyphenyl] methanol prepared in example 65- (2) was dissolved in acetone 400ml. Activated manganese dioxide 29.8g was added, which was stirred for 4 days at room temperature. Manganese dioxide was removed by filtration and the filtrate was concentrated under a reduced pressure. Purification by silica gel column chromatography (hexane-ethyl acetate) gave 7.09g of the title compound.
¹H-NMR(CDCl3)
δ: 1.30(s,3H), 1.33(s,3H), 3.37(d, *J*=6.0Hz,2H), 3.63(dd, *J*=7.2Hz,8.4Hz,1H), 3.87(s,3H), 4.00(dd, *J*=6.0Hz,8.4Hz,1H), 4.30-4.36(m,1H), 7.10(dd, *J*=1.2Hz,8.0Hz,1H), 7.35(d, *J*=8.0Hz,8.0Hz,1H), 7.49(dd, *J*=1.2Hz,8.0Hz,1H), 10.35(s,1H).

### (2) Synthesis of 4-[2-(2,2-dimethyl-[1,3]dioxolan-4-ylmethyl) -3-methoxybenzyl]morpholine

330mg of 2-(2,2-dimethyl-[1,3]dioxolan-4-ylmethyl)-3-methoxybenzaldehyde, 174µl of morpholine were dissolved in 6ml of methylene chloride. 152µl of acetic acid was added to this solution, which was stirred for 10 minutes. Subsequently 420mg of sodium triacetoxyborohydride was added, which was stirred overnight at room temperature. The reaction liquid was diluted in chloroform, 10% sodium carbonate aqueous solution was added, which was stirred, the organic layer was separated. The organic layer was washed with brine and dried over magnesium sulfate. Magnesium sulfate was removed by filtration and the filtrate was concentrated under a reduced pressure. The residue was purified by NH silica gel column chromatography (hexane-ethyl acetate) to give 390mg of the title compound.
¹H-NMR(CDCl3)
δ : 1.34(s,3H), 1.44(s,3H), 2.42-2.47(m,4H), 3.04(dd, *J*=7.2Hz,13.2Hz,1H), 3.13(dd, *J*=5.6Hz,13.2Hz,1H), 3.47(d, *J*=12.8Hz,1H), 3.60(d, *J*=12.8Hz,1H), 3.67-3.68(m,4H), 3.73(dd, *J*=7.8Hz,8.2Hz,1H), 3.91(dd, *J*=5.8Hz,8.2Hz,1H), 3.80(s,3H), 3.91(dd, *J*=5.8Hz,8.2Hz,1H), 4.34-4.40(m,1H), 6.77-6.79(m,1H), 6.89-6.91(m,1H), 7.11-7.15(m,1H).

### (3) Synthesis of 1-{1-{2-[2-methoxy-6-(morpholinomethyl) phenyl]ethyl}piperidin-4-yl}-1H-indole-6-carboxamide

The title compound was obtained by synthesizing from 4-[2-(2,2-dimethyl-[1,3]dioxolan-4-ylmethyl)-3-methoxybenzyl] morpholine in accordance with the methods in example 53-(9) to (10).
¹H-NMR(CDCl3)
δ: 2.11-2.68(m,10H), 2.96-3.02(m,2H), 3.25-3.29(m,2H), 3.49(s,2H), 3.68-3.70(m,4H), 3.84(s,3H), 4.37-4.46(m,1H), 5.01-6.02(brs,2H), 6.56-6.57(m,1H), 6.80-6.82(m,1H), 6.86-6.88(m,1H), 7.11-7.15(m,1H), 7.39-7.41(m,2H), 7.64(d, *J*=8.4Hz, 1H), 8.11(s,1H).

### Example 69

### Synthesis of 1-{1-{[2-(3-hydroxydimethylmethane-1-yl)-6-methoxyphenyl]ethyl}piperidin-4-yl}-1H-indole-6-carboxamide

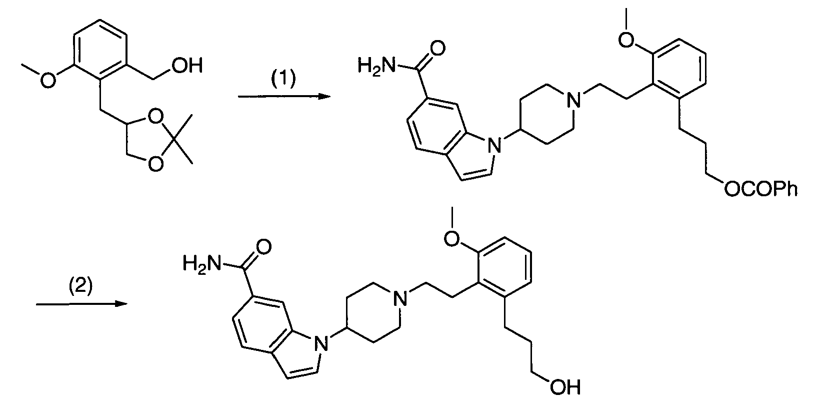

### (1) Synthesis of 3-{2-{2-[4-(6-carbamoylindol-1-yl)}-3-piperidin-1-yl]ethylmethoxyphenyl}propyl benzoate

The title compound was obtained by synthesizing from [2-(2,2-dimethyl-[1,3]dioxolan-4-ylmethyl)-3-methoxyphenyl] methanol prepared in example 65-(2) (syntesis of [2-(2,2-dimethyl-[1,3]dioxolan-4-ylmethyl)-3-methoxyphenyl] methanol) in accordance with the methods in example 53-(9) and (10).
¹H-NMR(CDCl3)
5: 2.01-2.14(m,6H), 2.13-2.27(m,2H), 2.52-2.56(m,2H), 2.80-2.84(m,2H), 2.90-2.94(m,2H), 3.16-3.19(m,2H), 3.83(s,3H), 4.31-4.37(m,1H), 4.39(t, J=6.2Hz,2H), 6.56(d, J=2.8Hz,1H), 6.74(d, *J*=8.4Hz,1H), 6.82(d, *J*=7.2Hz,1H), 7.12-7.16(m,1H), 7.40-7.45(m,4H), 7.52-7.56(m,1H), 7.64(d, *J*=8.4Hz, 1H), 8.03-8.08(m,3H).

### (2) Synthesis of 1-{1-{2-[2-(3-hydroxydimethylmethan-1-yl)-6-methoxyphenyl]ethyl}piperidin-4-yl}-1H-indole-6-carboxamide

470mg of 3-{2-{2-[4-(6-carbamoylindol-1-yl)}piperidin-1-yl] ethyl-3-methoxyphenyl}propyl benzoate was dissolved in 4ml methanol. 347µl of 5N aqueous sodium hydroxide was added thereto, which was stirred for 2 hours at room temperature. Water was added, which was extracted with ethyl acetate. The organic layer was washed with brine and dried over magnesium sulfate. Magnesium sulfate was removed by filtration, the filtrate was concentrated under a reduced pressure. The residue was purified by NH silica gel column chromatography (ethyl acetate-methanol) to give 200mg of the title compound.
¹H-NMR(CDCl3)
δ: 1.84-1.91(m,2H), 2.07-2.13(m,2H), 2.20-2.35(m,4H), 2.58-2.65(m,2H), 2.79(t, *J*=7.6Hz,2H), 2.93-2.98(m,2H), 3.24-3.30(m,2H), 3.69(t, *J*=6.2Hz,2H), 3.83(s,3H), 4.34-4.43(m, 1H), 6.11-3.40(brs, 2H), 6.55(dd, *J*=1.0Hz,3.2Hz,1H), 6.72-6.74(m,1H), 6.81-6.83(m,1H), 7.11-7.15(m,1H 7.38(d, *J*=3.2Hz,1H), 7.45(dd, *J*=1.0Hz, 8.2Hz, 1H), 7.62-7.65(m,1H), 8.13(s, 1H).

### Example 70

### Synthesis of 1-{1-[2-(2-dimethylcarbamoyl-6-methoxyphenyl) ethyl]piperidin-4-yl}-1H-indole-6-carboxamide

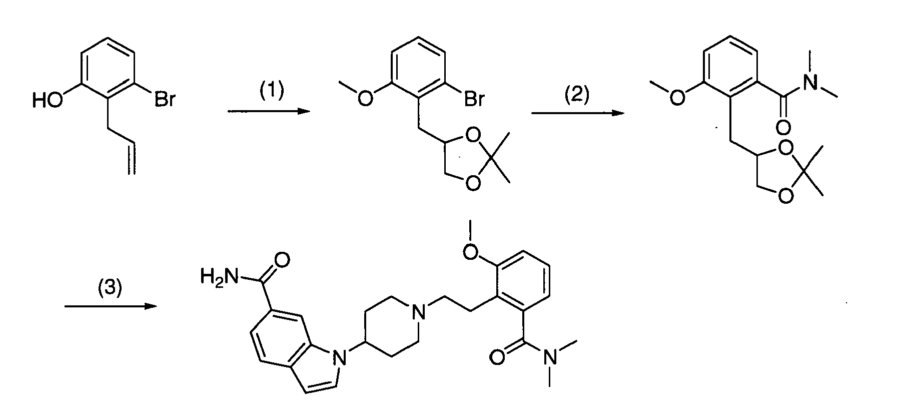

### (1) Synthesis of 4-(2-bromo-6-methoxybenzyl)-2,2-dimethyl-[1, 3] dioxolane

The title compound was obtained by synthesizing from 2-allyl-3-bromophenol prepared in example 53-(2), in accordance with the methods in example 53-(3) to 53-(5).
¹H-NMR(CDCl3)
δ: 1.35(s,3H), 1.48(s,3H), 3.10 (dd, *J*=8.4Hz,13.2Hz, 1H), 3.19(dd, *J*=5.2Hz,13.2Hz, 1H), 3.80-3.84(m,1H), 3.82(s,3H), 3.89(dd, *J*=5.8Hz,8.2Hz, 1H), 4.35-4.42(m,1H), 6.78-6.80(m,1H), 7.02-7.06(m,1H), 7.15(d, *J*=1.0Hz,8.2Hz, 1H).

### (2) Synthesis of 2-(2,2-dimethyl-[1,3]dioxolan-4-ylmethyl)-3-methoxy-N, N-dimethylbenzamide

440mg of 4-(2-bromo-6-methoxybenzyl)-2,2-dimethyl-[1,3]dioxolan was dissolved in 10ml of tetrahydrofuran, under nitrogen atmosphere, which was stirred in a dry ice-acetone bath. 1.09ml of *n-*butyllithium (1.6M hexane solution) was added dropwise, which was stirred for 10 minutes. 0.15ml of N,N-dimethylcarbamoyl chloride was added followed by stirring for 20 minutes. Saturated ammonium aqueous solution, water were added thereto, which was extracted with ethyl acetate. The organic layer was washed with brine and dried over magnesium sulfate. Magnesium sulfate was removed by filtration, the filtrate was concentrated under a reduced pressure. The residue was purified by NH silica gel column chromatography (*n-*hexane-ethyl acetate) to give 330mg of the title compound.
¹H-NMR(CDCl3)
δ: 1.39(s,3H), 1.58(s,3H), 2.76-2.86(m,1H), 2.84(s,3H), 2.96-3.05(m,1H), 3.11(s,3H), 3.68(dd, *J*=7.2Hz,8.4Hz,1H), 3.83(s,3H), 3.95-4.02(m,1H), 4.32-4.39(m,1H), 6.76-6.78(m,1H), 6.84-6.87-(m, 1H), 7.19-7.23(m,1H).

### (3) Synthesis of 2-(2,2-dimethyl-[1,3]dioxolan-4-ylmethyl)-3-methoxy-N, N-dimethylbenzamide

The title compound was obtained by synthesizing from 2-(2,2-dimethyl-[1,3]-dioxolan-4-ylmethyl)-3-methoxy-N,N-dimethylbenzamide in accordance with the methods in example 53 (9) to (10).
¹H-NMR(DMSO-d6)
δ: 1.95-2.07 (m, 4H), 2.21-2.84 (m, 6H), 2.76 (s, 3H), 3.00 (s, 3H), 2.97-3.06 (m, 2H), 3.82 (s, 3H), 4.34-4.45 (m,1H), 6.49 (d, J=2.8Hz,1H), 6.73(dd, *J*=1.2Hz,7.6Hz,1H), 7.00(dd, *J*=1.2Hz,8.2Hz,1H), 7.20(brs,1H), 7.24(dd, *J*=7.6Hz,8.2Hz,1H), 7.53-7.58 (m,2H), 7.65 (d, *J*=3.2Hz,1H), 7.89 (brs,1H), 8.11 (s,1H).

### Example 71

### Synthesis of 1-{1-{2-[2-methoxy-5-(2-oxo-2-piperidinoethyl)phenyl]ethyl}piperidin-4-yl}-1H-indole-6-carboxamide

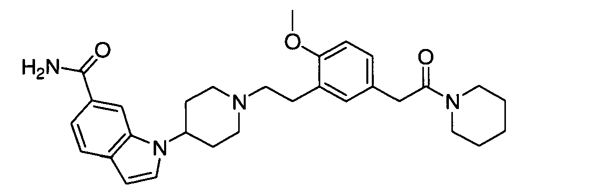

The title compound was obtained by synthesizing from 2-(4-allyloxyphenyl)-1-piperidino ethanone in accordance with the methods in example 1.
¹H-NMR(CDCl3)
δ: 1.43-1.63(m,6H), 2.10-2.19(m,4H), 2.26-2.33(m,2H), 2.51-2.55(m, 2H), 2.88-2.92(m,2H), 3.20-3.25(m,2H), 3.35-3.38(m,2H), 3.60-3.62(m,2H), 3.76(s,2H), 3.83(s,3H), 4.34-4.42(m,1H), 5.57(brs,1H), 6.20(brs,1H), 6.56(d, *J*=2.4Hz,1H), 6.75-6.79(m2H), 7.12-7.16(m,1H) 7.39-7.42(m,2H), 7.63(d, *J*=8.0Hz,1H), 8.10(s,1H)

### Example 72

### Synthesis of 1-{1-{2-[2-methoxy-5-(2-oxo-2-morpholinoethyl) phenyl]ethyl}piperidin-4-yl}-1H-indole-6-carboxamide

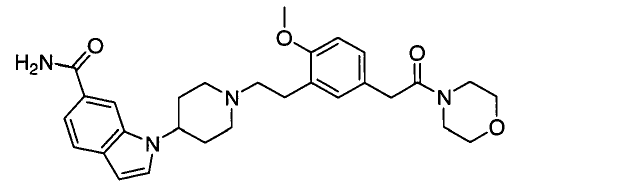

The title compound was obtained by synthesizing from 2-(4-allyloxy phenyl)-1-morpholino ethanone in accordance with the methods in example 1.
¹H-NMR(CDCl3)
δ: 2.07-2.19(m,4H), 2.26-2.33(m,2H), 2.50-2.54(m,2H), 2.87-2.91(m,2H), 3.19-3.24(m,2H), 3.41-3.44(m,2H), 3.56-3.66(m,6H), 3.76(s,2H), 3.84(s,3H), 4.35-4.42 (m; 1H), 5.60(brs,1H), 6.22(brs,1H), 6.56(d, *J*=3.2Hz,1H), 6.78-6.81(m,1H), 7.02-7.06(m,2H), 7.38-7.42(m,2H), 7.63(d, *J*=8.0Hz,1H), 8.10(s,1H).

### Example 73

### Synthesis of 1-{1-{2-[2-methoxy-6-(2-oxo-2-piperidinoethyl) phenyl]ethyl}piperidin-4-yl}-1H-indole-6-carboxamide

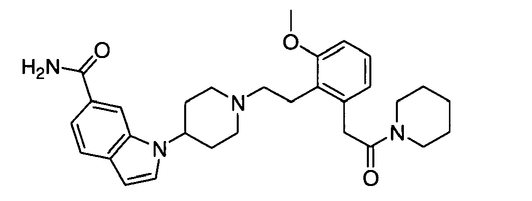

The title compound was obtained by synthesizing from 2-(3-allyloxyphenyl)-1-piperidino ethanone in accordance with the methods in example 1.
¹H-NMR(CDCl3)
δ : 1.35-1.41(m,2H), 1.48-1.63(m,4H), 2.06-2.19(m,4H), 2.24-2.32(m,2H), 2.61-2.65(m,2H), 2.82-2.86(m,2H), 3.17-3.22(m,2H), 3.38-3.40(m,2H), 3.55-3.58(m,2H), 3.65(s,2H), 3.82(s,3H), 4.34-4.42(s,1H), 5.59(brs,1H), 6.21(brs,1H), 6.56(d, J=3.2Hz,1H), 6.79(d, *J*=9.2Hz,1H), 7.05-7.06(m,2H),7.39-7.43(m,2H), 7.63(d, *J*=8.4Hz, 1H), 8.10(s,1H).

### Example 74

### Synthesis of 1-{2-[2-methoxy-6-(2-oxo-2-morpholinoethyl) phenyl]ethyl}piperidin-4-yl}-1H-indole-6-carboxamide

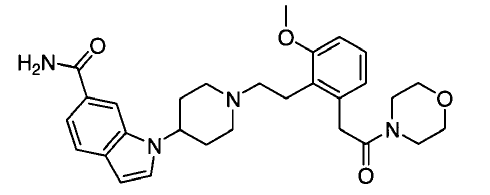

The title compound was obtained by synthesizing from 2-(3-allyloxyphenyl)-1-morpholino ethanone in accordance with example 1.
¹H-NMR(CDCl3)
δ : 2.09-2.19(m,4H), 2.25-2.31(m,2H), 2.61-2.65(m,2H), 2.82-2.86(m,2H), 3.17-3.22(m,2H), 3.44-3.52(m,4H), 3.63-3.65(m,4H), 3.65(s,2H), 3.82(s,3H), 4.34-4.42(m,1H), 5.60(brs,1H), 6.22(brs,1H), 6.56(d, *J*=3.2Hz,1H), 6.79-6.81(m,1H), 7.03-7.05(m,2H), 7.39-7.42(m,2H), 7.63(d, *J*=8.0Hz, 1H), 8.10(s,1H).

### Example 75

### Synthesis of 1-{1-{2-[2-methoxy-5-(2-piperidinoethyl)phenyl] ethyl}piperidin-4-yl}-1H-indole-6-carboxamide

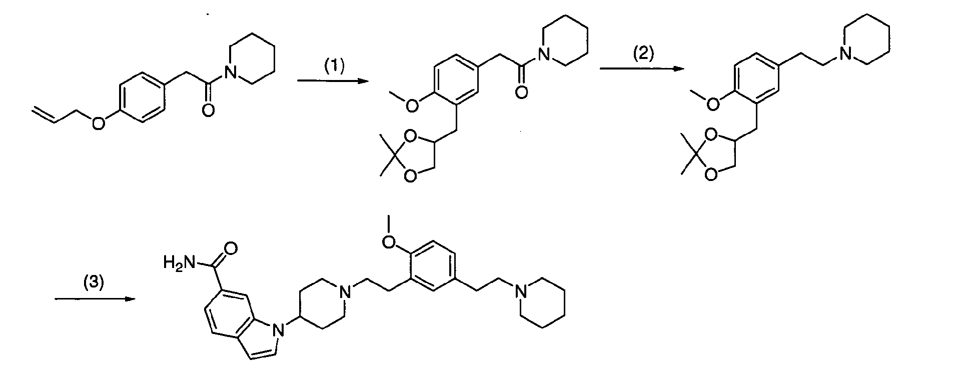

### (1) Synthesis of 2-[3-(2, 2-dimethyl-[1,3]dioxolan-4-ylmethyl)-4-methoxyphenyl]-1-piperidinoethanone

The title compound was obtained by synthesizing from 2-(4-allyloxyphenyl)-1-piperidino ethanone in accordance with the methods in example 1-(2) to (5).
¹H-NMR(CDCl₃)
δ: 1.32-1.38(m,2H), 1.34(s,3H), 1.42(s,3H), 1.48-1.61(m,4H), 2.77(dd, *J*=7.6Hz,13.2Hz,1H), 2.99(dd, J=1.6Hz,13.2Hz,1H), 3.35-3.38(m,2H), 3.54-3.57(m,2H), 3.62-3.65(m,1H), 3.63(s,2H), 3.79(s,3H), 3.89(dd, *J*=5.8Hz,8.2Hz,1H), 4.31-4.37(m,1H), 6.77(d, *J*=8.4Hz,1H), 7.03(d, *J*=2.0Hz,1H), 7.08(dd, *J*=2.0Hz,8.4Hz,1H).

### (2) Synthesis of 1-{2-[3-(2,2-dimethyl-[1,3]dioxolan-4-ylmethyl)-4-methoxyphenyl]ethyl}piperidine

0.59g of 2-[3-(2,2-dimethyl-[1,3]dioxolan-4-ylmethyl)-4-methoxyphenyl]-1-piperidinoethanone was dissolved in 5ml of tetrahydrofuran under nitrogen atmosphere, which was stirred at room temperature. 8mg of tris(triphenylphosphine) rhodium-(I) carbonyl hydride, 656µl of diphenylsilane were added thereto sequentially. A reaction mixture was stirred for 2 hours. The reaction mixture was purified by NH silica gel column chromatography (*n-*hexane-ethyl acetate) to give 305mg of the title compound.
¹H-NMR(CDCl3)
δ: 1.35(s,3H), 1.43(s,3H), 1.42-1.48(m,2H), 1.58-1.64(m,4H), 2.43-2.52(m,6H), 2.70-2.79(m,3H), 3.00(dd, J=5.4Hz,13.4Hz,1H), 3.65(dd, *J*=6.8Hz,8.0Hz,1H), 3.78(s,3H), 3.91(dd, *J*=5.8Hz,8.0Hz,1H), 4.31-4.38(m,1H), 6.74(d, *J*=8.0Hz,1H), 6.98-7.03(m,2H).

### (3) Synthesis of 1-{1-{2-[2-methoxy-5-(2-piperidinoethyl) phenyl]ethyl}piperidin-4-yl}-1H-indole-6-carboxamide

The title compound was obtained by synthesizing from 1-{2-[3-(2,2-dimethyl-[1,3]dioxolan-4-ylmethyl)-4-methoxyphenyl]ethyl}piperidine in accordance with the methods in example 53-(9) to (10).
¹H-NMR(CDCl3)
δ: 1.43-1.49(m,2H), 1.59-1.65(m,4H), 2.09-2.18(m,4H), 2.25-2.32(m,2H), 2.44-2.54(m,6H), 2.61-2.66(m,2H), 2.72-2.76(m,2H), 2.81-2.85(m,2H), 3.19-3.24(m,2H), 3.81(s,3H), 4.35-4.43(m,1H), 6.55-6.56(m,1H), 6.77(d, *J*=8.0Hz,1H), 6.98-7.03(m,2H), 7.39-7.41(m,2H), 7.62-7.64 (m,1H),8.09 (s,1H).

### Example 76

### Synthesis of 1-{1-{2-[6-dimethylcarbamoyl-3,6-dimethoxyphenyl]ethyl}piperidin-4-yl}-1H-indole-6-carboxamide

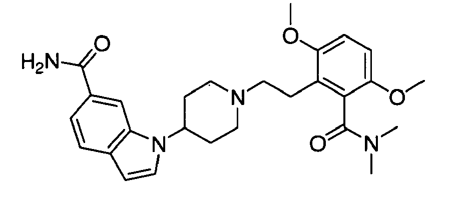

The title compound was obtained by synthesizing from 5-allyloxy-2-methoxy-N,N-dimethylbenzamide in accordance with the methods in example 1.
¹H-NMR (CDCl3)
δ: 2.08-3.37(m,12H), 2.83(s,3H), 3.14(s,3H), 3.77(s,3H), 3.81(s,3H), 4.34-4.45(m,1H), 5.55(brs,1H), 6.28(brs,1H), 6.55-6.56(m,1H), 6.72-6.74(m,1H), 6.79-6.81(m,1H), 7.37(d, J=3.2Hz, 1H), 7.43-7.47(m,1H), 7.63(d, J=8.0Hz, 1H), 8.10(s, 1H).

### Example 77

### Synthesis of 1-{1-{2-[2-methoxy-6-(3-piperidinopropyl)phenyl] ethyl}piperidin-4-yl}-1H-indole-6-carboxamide

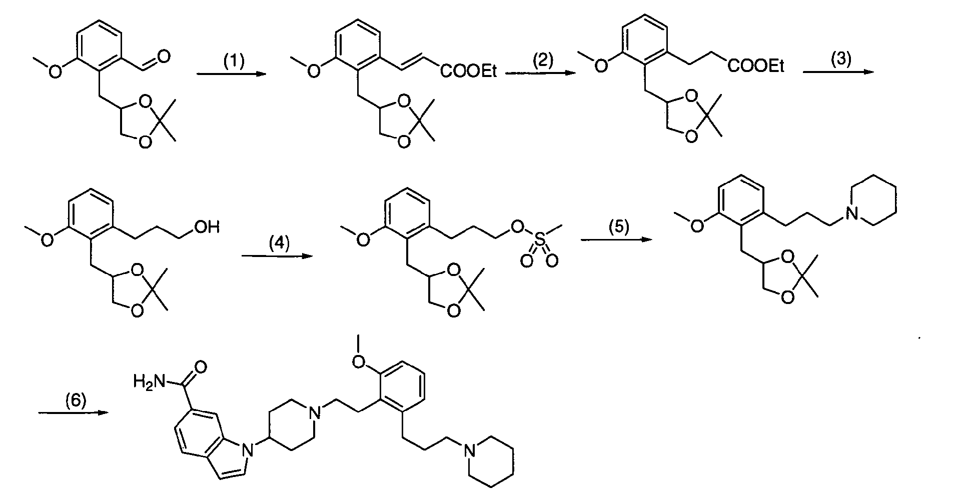

### (1) Synthesis of ethyl (E)-3-[2-(2,2-dimethyl-[1,3]dioxolan -4-ylmethyl)-3-methoxyphenyl]acrylate

Sodium hydride (60% dispersion) was washed with *n-*hexane, suspended in 1ml of tetrahydrofuran, which was stirred in an ice cooling under nitrogen atmosphere. 1.14g of triethyl phosphonoacetate were dissolved in 3ml of tetrahydrofuran and added thereto. The reaction liquid was stirred for 5 minutes at room temperature, ice cooling was carried out again, followed by stirring. Next, 1.16g of 2-(2,2-dimethyl-[1,3]dioxolan-4-ylmethyl)-3-methoxybenzaldehyde prepared in example 68-(1) was dissolved in 10ml of tetrahydrofuran, and then added. The reaction liquid was stirred overnight at room temperature. Water was added, which was extracted with ethyl acetate. The organic layer was washed with brine and dried over magnesium sulfate. Magnesium sulfate was removed by filtration, the filtrate was concentrated under a reduced pressure. The residue was purified by NH silica gel column chromatography (*n-*hexane-ethyl acetate) to give 1.47g of the title compound.
¹H-NMR(CDCl3)
δ: 1.31(s,3H), 1.33(t, *J*=7.0Hz,3H), 1.45 (s, 3H), 3.02-3.12(m,2H), 3.67(dd, *J*=7.0Hz,8.2Hz,1H), 3.83(s,3H), 3.96(dd, *J*=6.2Hz,8.2Hz,1H), 4.25-4.32(m,1H), 4.26(q, *J*=7.0Hz,2H), 6.33(d, *J*=15.8Hz,1H), 6.88(dd, *J*=1.4Hz,7.8Hz,1H), 7.16-7.23(m,2H), 8.14(d, *J*=15.8Hz,1H).

### (2) Synthesis of 3-[2-(2,2-dimethyl-[1,3]dioxolan-4-ylmethyl) ethyl-3-methoxyphenyl]propionate

0.51g of ethyl (*E*)-3-[2-(2,2-dimethyl-[1,3]dioxolan-4-yl methyl) -3-methoxyphenyl] acrylate was dissolved in 15ml of ethyl acetate. 0.03g of 10% Pd-C (50% wet) was added, under hydrogen atmosphere, which was stirred overnight at room temperature. Pd-C was removed by filtration and the filtrate was concentrated under a reduced pressure to give 0.48g of the title compound.
¹H-NMR(CDCl3)
δ: 1.25 (t, *J*=7.2Hz, 3H), 1.32(s,3H), 1.43(s,3H), 2.52-2.67(m,2H), 2.92(dd, *J*=6.6Hz,13.4Hz,1H), 2.98-3.09(m,3H), 3.67(dd, *J*=7.4Hz, 8.2Hz, 1H), 3.80(s,3H), 3.97(dd, *J*=5.8Hz,8.2Hz,1H), 4.13(q, *J*=7.2Hz,2H), 4.27-4.34(m,1H), 6.71-6.73(m,1H), 6.78-6.80(m,1H), 7.11-7.15(m,1H).

### (3) Synthesis of 3-[2-(2,2-dimethyl-[1,3]dioxolan-4-ylmethyl)-3-methoxyphenyl]propan-1-ol

0.06g of lithium aluminum hydride was suspended in 0.5ml of tetrahydrofuran and then stirred in an ice-cooling. 0.48g of 3-[2-(2,2-dimethyl-[1,3]dioxolan-4-ylmethyl)ethyl-3-methoxyphenyl]propionate was dissolved in 5ml of tetrahydrofuran and added thereto. The reaction liquid was stirred for 15 minutes. With stirring in an ice-cooling, 56µl of water, 56µl of 5N aqueous sodium hydroxide, 168µl of water were sequentially added and the precipitate was removed by filtration. The filtrate was concentrated under a reduced pressure to give 0.42g of the title compound.
¹H-NMR(CDCl3)
δ: 1.33 (s, 3H), 1.43 (s, 3H), 1.84-1.91 (m, 2H), 2.78-2.82 (m, 2H), 2.93-3.03(m,2H), 3.66-3.70(m,3H), 3.80(s,3H), 3.96(dd, *J*=5.8Hz,8.2Hz,1H), 4.27-4.33(m,1H), 6.69-6.72(m,1H), 6.80-6.82(m,1H), 7.11-7.15(m,1H).

### (4) Synthesis of 3-[2-(2,2-dimethyl-[1,3]dioxolan-4-ylmethyl)-3-methoxyphenyl]propyl methanesulfonate

1.18g of 3-[2-(2,2-dimethyl-[1,3]dioxolan-4-ylmethyl)-3-methoxyphenyl]propan-1-ol, 2.93ml of triethylamine were dissolved in 20ml of tetrahydrofuran, which was stirred in an ice-cooling. 489µl of methanesulfonyl chloride was added thereto. After stirring it in an ice-cooling for 5 minutes, stirred was carried out for 1 hour at room temperature. Water was added thereto, which was extracted with ethyl acetate. The organic layer was washed with brine and dried over magnesium sulfate. Magnesium sulfate was removed by filtration, and the filtrate was concentrated under a reduced pressure to give 690mg of the title compound. This was used in the next reaction without further purification.
¹H-NMR(CDCl3)
5: 1.32(s,3H), 1.42(s,3H), 2.01-2.08(m,2H), 2.77-2.92(m,3H), 2.96-3.02 (m,1H), 3.02(s,3H), 3.66(dd *J*=7.0Hz,8.2Hz,1H), 3.80(s, 3H), 3.96 (dd, *J*=5,8Hz, 8.2Hz, 1H), 4.25 (t, *J*=6.4Hz, 2H), 4.26-4.32(m, 1H), 6.72-6.74(m, 1H), 6.77-6.79(m, 1H), 7.12-7.16(m, 1H).

### (5) Synthesis of 1-{3-[2-(2,2-dimethyl-[1,3]dioxolan-4-ylmethyl)-3-methoxyphenyl]propyl}piperidine

To a mixture of 0.81g of 3- [2- (2,2-dimethyl- [1,3] dioxolan-4-ylmethyl)-3-methoxyphenyl]propyl methanesulfonate, 0.32g of NaI, 0.01g of tetrabutylammonium bromide in 20ml of acetonitrile was added 1.04ml of piperidine, which was stirred for 5 hours at 65 °C. Ethyl acetate, and 10% sodium carbonate aqueous solution were added thereto followed by stirred. The organic layer was separated, washed with brine and dried over magnesium sulfate. Magnesium sulfate was removed by filtration, the filtrate was concentrated under a reduced pressure. The residue was purified by NH silica gel column chromatography (*n-*hexane-ethyl acetate) to give 0.69g of the title compound.
¹H-NMR(CDCl3)
δ: 1.32(s,3H), 1.43(s,3H), 1.41-1.88(m,8H), 2.32-2.63(m,6H), 2.63-2.77(m,2H), 2.91-3.01(m,2H), 3.68(dd, *J*=7.0Hz,8.4Hz,1H), 3.79(s,3H), 3.92(dd, *J*=6.0Hz,8.4Hz,1H), 4.24-4.31(m,1H), 3.69-3.71(m,1H), 6.78-6.80(m,1H), 7.09-7.13(m,1H).

### (6) Synthesis of 1-{1-{2-[2-methoxy-6-(3-piperidinopropyl) phenyl]ethyl}piperidin-4-yl}-1H-indole-6-carboxamide

The title compound was obtained by synthesizing from 1-{3-[2-(2,2-dimethyl-[1,3]dioxolan-4-ylmethyl)-3-methoxyphenyl]propyl}piperidine in accordance with the methods in example 53-(9) to (10).
¹H-NMR(CDCl3)
5: 1.37-1.48(m,2H), 1.55-1.66(m,4H), 1.77-1.87(m,2H), 2.06-3.00(m, 18H), 3.20-3.29(m,2H), 3.82(s,3H), 4.33-4.43(m,1H), 5.83(brs,1H), 6.32(brs,1H), 6.54-6.58(m,1H), 6.69-6.84(m,2H), 7.09-7.13(m,1H 7.39-7.44(m,2H), 7.62-7.64(m,1H), 8.11(s,1H)

### Example 78

### Synthesis of 1-{1-{2-[2-methoxy-5-(3-piperidinopropyl)phenyl] ethyl}piperidin-4-yl}-1H-indole-6-carboxamide

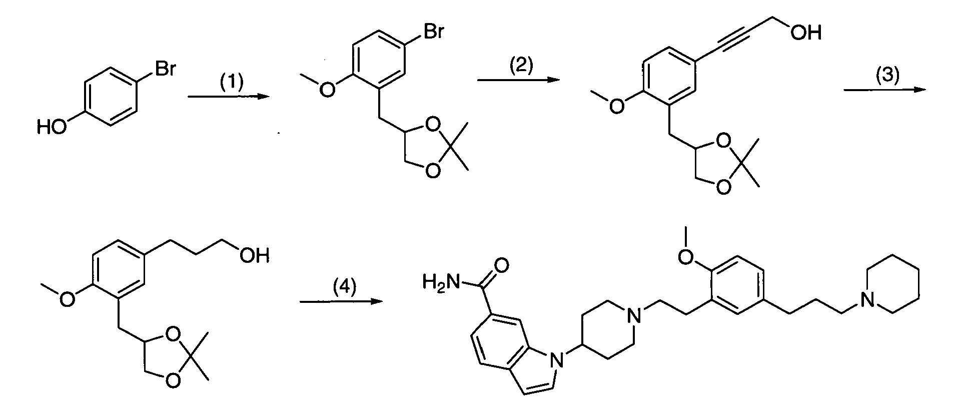

### (1) Synthesis of 4-(5-bromo-2-methoxybenzyl)-2,2-dimethyl-[1,3]dioxolane

The title compound was obtained by synthesizing from a 4-bromophenol in accordance with the methods in example 53-(1) to (5).
¹H-NMR(CDCl3)
δ: 1.35(s, 3H),1.43 (s, 3H) , 2.78(dd, *J*=7.0Hz, 13.4Hz, 1H), 2.93(dd, *J*=6.2Hz, 13.4Hz, 1H), 3.63(dd, *J*=6.6Hz, 8.2Hz, 1H), 3.79(s,3H), 3.95(dd, *J*=5.8Hz, 8.2Hz, 1H), 4.30-4.36(m,1H), 6.69-6.71(m,1H), 7.28-7.30(m2H).

### (2) Synthesis of 3-[3-(2,2-dimethyl-[1,3]dioxolan-4-ylmethyl)-4-methoxyphenyl]propyn-1-ol

A mixture of 0.74g of 4-(5-bromo-2-methoxybenzyl)-2,2-dimethyl-[1,3]dioxolane, 285µl of propargyl alcohol, 0.09g of dichlorobis(triphenylphosphine) palladium(II), 0.04g of copper, 0.32g of triphenylphosphine, 20ml of triethylamine and 10ml of pyridine was stirred overnight at 80 °C under nitrogen atmosphere. Ethyl acetate, water were added thereto, and the insoluble precipitate was removed by filtration. The organic layer of the filtrate was separated, washed with brine and dried over magnesium sulfate. Magnesium sulfate was removed by filtration, the filtrate was concentrated under a reduced pressure. The residue was purified by NH silica gel column chromatography (*n-*hexane-ethyl acetate) to give 0.31g of the title compound.
¹H-NMR(CDCl₃)
δ: 1.35(s,3H), 1.43(s,3H), 1.64(t, *J*=6.0Hz, 1H), 2.77(dd, *J*=7.0Hz,13.4Hz,1H), 2.95(dd, *J*=5.8Hz,13.4Hz,1H), 3.63(dd, *J*=7.0Hz,8.2Hz,1H), 3.82(s,3H), 3.93(dd, *J*=5.8Hz,8.2Hz,1H), 4.30-4.37(m,1H), 4.47(d, *J*=6.0Hz,2H), 6.77(d, *J*=8.4Hz,1H), 7.26(d, *J*=2.0Hz,1H), 7.30(dd, *J*=2.0Hz,8.4Hz,1H).

### (3) Synthesis of 3-[3-(2,2-dimethyl-[1,3]dioxolan-4-ylmethyl)-4-methoxyphenyl]propan-1-ol

0.51g of 3-[3-(2,2-dimethyl-[1,3]dioxolan-4-ylmethyl)-4-methoxyphenyl]propyn-1-ol was dissolved in 20ml of ethyl acetate. 0.07g of 10% Pd-C (50% wet) was added, under hydrogen atmosphere, which was stirred overnight at room temperature. Pd-C was removed by filtration, the filtrate was concentrated under a reduced pressure to give 0.48g of the title compound.
¹H-NMR(CDCl3)
δ: 1.35(s,3H), 1.43(s,3H), 1.82-1.89(m,2H), 2.63(t, *J*=7.6Hz,2H), 2.78(dd, *J*=7.4Hz,13.2Hz,1H), 2.99(dd, *J*=5.2Hz,13.2Hz,1H), 3.63-3.68(m,3H), 3.79(s,3H), 3.91(dd, J=5.8Hz,8.2Hz,1H), 4.32-4.38 (m,1H), 6.75(d, *J*=8.2Hz,1H), 6.99(d, *J*=2.4Hz,1H), 7.02(dd, *J*=2.4Hz,8.2Hz,1H).

### (4) Synthesis of 1-{1-{2-[2-methoxy-5-(3-piperidinopropyl) phenyl]ethyl}piperidin-4-yl}-1H-indole-6-carboxamide

The title compound was obtained by synthesizing from 3-[3-(2,2-dimethyl-[1,3]dioxolan-4-ylmethyl)-4-methoxyphenyl] propan-1-ol in accordance with the methods in example 77-(4) to (6).
¹H-NMR(CDCl3)
δ: 1.45-2.74(m,24H), 2.81-2.85(m,2H), 3.19-3.26(m,2H), 3.81(s,3H), 4.35-4.43(m,1H), 5.60(br,1H), 6.20(brs, 1H),6.56(d, *J*=3.2Hz,1H), 6.76(d, *J*=8.4Hz,1H), 6.95-7.42(m,2H), 7.39-7.42(m,2H), 7.63(d, *J*=8.4Hz,1H), 8.10(s,1H).

### Example 79

### Synthesis of 1-{1-{2-[2-methoxy-5-(3-piperidinopropyl)phenyl] ethyl}piperidin-4-yl}-1H-indole-6-carboxamide

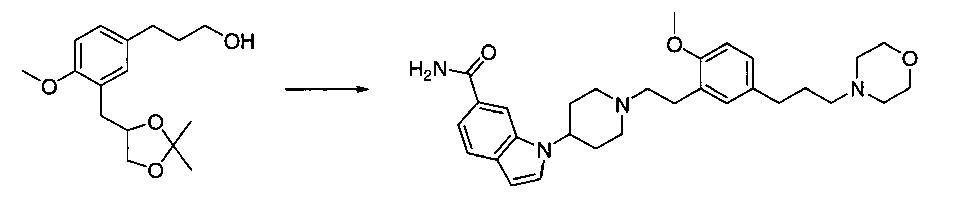

The title compound was obtained by synthesizing from 3-[3-(2,2-dimethyl-[1,3]dioxolan-4-ylmethyl)-4-methoxyphenyl] propan-1-ol prepared in example 78-(3) in accordance with the methods in example 78-(4).
¹H-NMR(CDCl3)
δ: 1.77-2.88(m,20H), 3.25-3.37(m,2H), 3.72-3.74(m,4H), 3.82(s,3H), 4.38-4.48(m,1H), 5.62(brs,1H), 6.19(brs,1H), 6.57(d, *J*=3.2Hz,1H), 6.77(d, *J*=8.2Hz,1H), 6.98-7.02(m,2H), 7.39-7.42(m,1H), 7.63(d, *J*=8.2Hz,1H),8.10(s,1H).

### Example 80

### Synthesis of 1-{1-{2-{2-methoxy-5-(piperidinomethyl)phenyl] ethyl}piperidin-4-yl-1H-indole-6-carboxamide

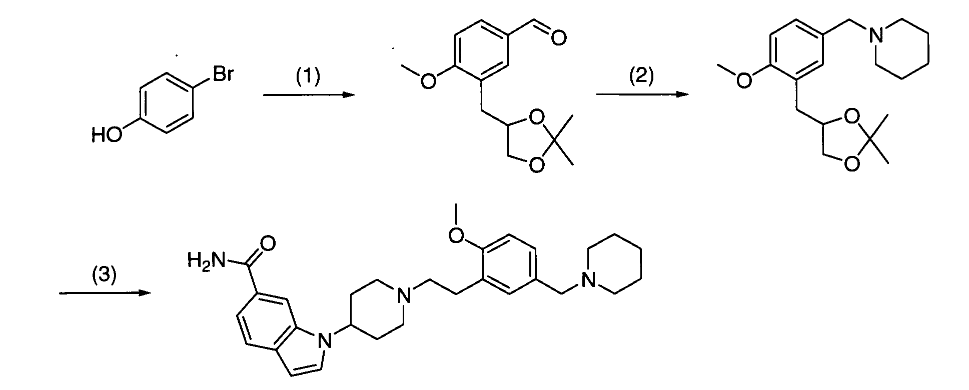

### (1) Synthesis of 3-(2,2-dimethyl-[1,3]dioxolan-4-ylmethyl)-4-methoxybenzaldehyde

The title compound was obtained by synthesizing from 4-bromophenol in accordance with the methods in example 53-(1) to (6).
¹H-NMR(CDCl3)
δ 1.35 (s, 3H), 1.43(s,3H), 2.88(dd, *J*=6.4Hz,13.6Hz,1H), 3.01(dd, *J*=5.6Hz,13.6Hz,1H), 3.65(dd, *J*=6.8Hz,8.0Hz,1H), 3.91(s,3H), 3.97(d, *J*=6.0Hz, 8.0Hz, 1H), 4.35-4.41(m,1H), 6.95(d, *J*=8.4Hz, 1H), 7.73(dd, *J*=2.0Hz,1H), 7.76(dd. *J*=2.0Hz,8.4Hz,1H), 9.86(s,1H).

### (2) Synthesis of 1-{1-{2-[2-methoxy-5-(piperidinomethyl) phenyl]ethyl}piperidin-4-yl}-1H-indole-6-carboxamide

The title compound was obtained by synthesizing from 3-(2,2-dimethyl-[1,3]dioxolan-4-ylmethyl)-4-methoxybenzaldehyde in accordance with the methods in example 68-(2) to (3).
¹H-NMR(CDCl3)
δ: 1.68-2.90(m,20H), 3.20-3.23(m,2H), 3.52-3.58(m,2H), 3.83(s,3H), 4.35-4.43(m, 1H), 5.60(brs,1H), 6.21(brs,1H), 6.56(d, *J*=3.0Hz,1H), 6.81-6.83(m,1H), 7.17-7.19(m,2H), 7.38(d, *J*=3.0Hz,1H), 7.41(dd, *J*=1.4Hz,8.2Hz,1H), 7.63(d, *J*=8.2Hz,1H), 8.10 (s, 1H).

### Example 81

### Synthesis of 1-{1-{2-[2-methoxy-5-(morpholinomethyl)phenyl] ethyl}piperidin-4-yl}-1H-indole-6-carboxamide

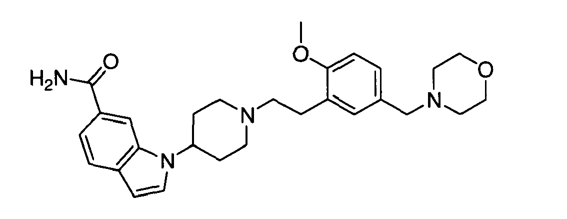

The title compound was obtained by synthesizing from 4-bromophenol in accordance with the methods in example 80.
¹H-NMR(CDCl3)
δ: 2.18-2.48(m,10H), 2.62-2.70(m,2H), 2.84-2.88(m,2H), 3.18-3.27(m,2H), 3.43(s,2H), 3.70-3.74(m,4H), 3.83(s,3H), 4.35-4.43 (m,1H), 5.59 (brs,1H), 6.20 (brs,1H), 6.56(d, *J*=2.8Hz,1H), 6.80(d, *J*=8.8Hz,1H), 7.11-7.14(m,2H), 7.39-7.41(m, 2H), 7.63(d, *J*=8.0Hz,1H), 8.10 (s,1H).

### Example 82

### Synthesis of 1-{1-{2-[2-methoxy-5-(pyridin-4-yl)phenyl]ethyl} piperidin-4-yl}-1H-indole-6-carboxamide

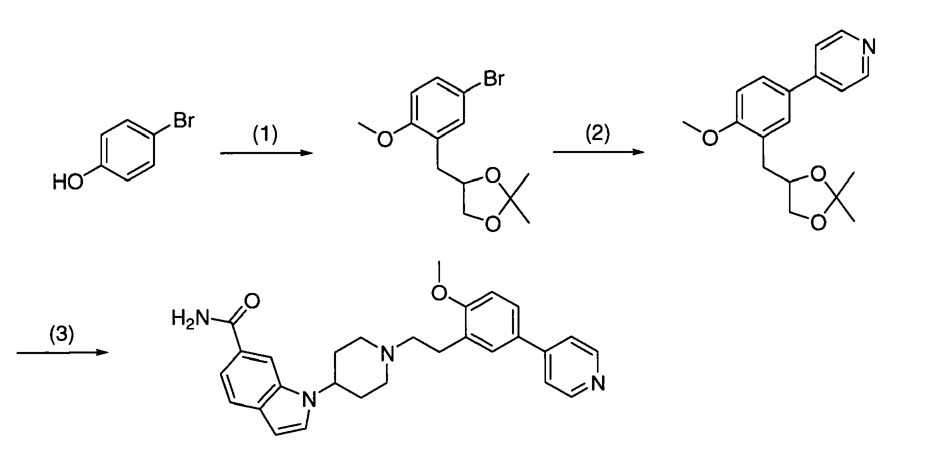

### (1) Synthesis of 4-(5-bromo-2-methoxybenzyl)-2,2-dimethyl -[1,3]dioxolane

The title compound was obtained by synthesizing from 4-bromophenol in accordance with the methods in example 53-(1) to (5).
¹H-NMR(CDCl3)
δ: 1.35(s,3H), 1.43(s,3H), 2.78(dd, *J*=7.0Hz, 13.4Hz, 1H), 2.93(dd, *J*=6.2Hz,13.4Hz,1H), 3.63(dd, *J*=6.6Hz,8.2Hz,1H), 3.79(s,3H), 3.95(dd, *J*=5.8Hz,8.2Hz,1H), 4.30-4.36(m,1H), 6.69-6.71(m,1H), 7.28-7.30 (m2H).

### (2) Synthesis of 4-[3-(2,2-dimethyl-[1,3]dioxolan-4-ylmethyl)-4-methoxyphenyl]pyridine

To a mixture of 0.72g of 4-(5-bromo-2-methoxybenzyl)-2,2-dimethyl-[1,3]dioxolane, 0.38g of sodium carbonate, 3.9ml of 1,2-dimethoxyethane and 1.3ml of water was added sequentially 0.35g of 4-pyridylboronic acid, 0.10g of [1,1'-bis-(diphenylphosphino)ferrocene] dichloropalladium(II). A reaction mixture was stirred for 3 hours at 100 °C under nitrogen atmosphere. Ethyl acetate and 10% sodium carbonate aqueous solution were added thereto, and the insoluble precipitate was removed by filtration. The organic layer of the filtrate was separated, washed with water (twice) and brine sequentially and dried over magnesium sulfate. Magnesium sulfate was removed by filtration, and the filtrate was concentrated under a reduced pressure. The residue was purified by NH silica gel column chromatography (*n-*hexane-ethyl acetate) to give 0.61g of the title compound.
¹H-NMR(CDCl3)
δ: 1.36(s,3H), 1.44 (s,3H), 2.90 (dd, *J*=6.8Hz,13.6Hz,1H), 3.05 (dd, *J*=6.2Hz,13.6Hz,1H), 3.68(dd, J=6.6Hz,8.2Hz,1H), 3.88(s,3H), 3.97(dd, *J*=5.8Hz,8.2Hz,1H), 4.38-4.44(m,1H), 6.94(d, *J*=8.4Hz,1H), 7.45 (d, J=6.2Hz,2H), 7.48 (d, *J*=2.4Hz,1H), 7.51 (dd, J=2.4Hz,8.4Hz,1H), 8.60(d, J=6.2Hz,2H).

### (3) Synthesis of 1-1-{2-[2-methoxy-5-(pyridin-4-yl)phenyl] ethyl}piperidin-4-yl}-1H-indole-6-carboxamide

The title compound was obtained by synthesizing from 4-[3-(2,2-dimethyl-[1,3]dioxolan-4-ylmethyl)-4-methoxy phenyl]pyridine in accordance with the methods in example 68- (3).
¹H-NMR(CDCl3)
δ: 2.08-2.18(m,4H), 2.28-2.35(m,2H), 2.67-2.71(m,2H), 2.92-2.99(m,2H), 3.22-3.27(m,2H), 3.90(s,3H), 4.36-4.44(m,1H), 5.60(brs,1H), 6.17(brs, 1H), 6.56-6.57(m, 1H), 6.96(d, *J*=8.4Hz, 1H), 7.25-7.41(m,2H), 7.47-7.52(m,4H), 7.62-7.64(m,1H), 8.11(s, 1H), 8.61(d, *J*=6.0Hz, 1H).

### Example 83

### Synthesis of 1-{1-{2-[2-methoxy-5-(pyridin-3-yl)phenyl]ethyl} piperidin-4-yl}-1H-indole-6-carboxamide

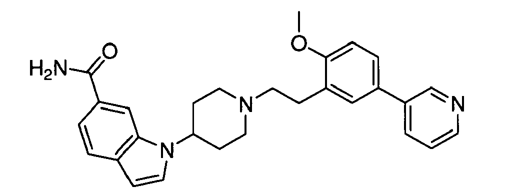

The title compound was obtained by synthesizing from 4-bromophenol in accordance with the methods in example 82. ¹H-NMR(CDCl3)
δ : 2.11-2.20(m,4H), 2.28-2.34(m,2H), 2.68-2.72(m,2H), 2.92-2.96(m,2H), 3.21-3.26(m,2H), 3.90(s,3H), 4.36-4.44(m,1H), 5.69(brs,1H), 6.18(brs,1H), 6.56(d, J=2.8Hz,1H), 6.96(d, *J*=8.4Hz,1H), 7.31-7.44(m,5H), 7.63(d, *J*=8.4Hz,1H), 7.82-7.85-(m,1H), 8.10(s,1H), 8.53(dd, *J*=1.4Hz,5.0Hz,41H), 8.81(d, *J*=2.4Hz,1H).

### Example 84

### Synthesis of 1-{1-{2-[2-methoxy-5-(2-hydroxyethyl)phenyl] ethyl}piperidin-4-yl}-1H-indole-6-carboxamide

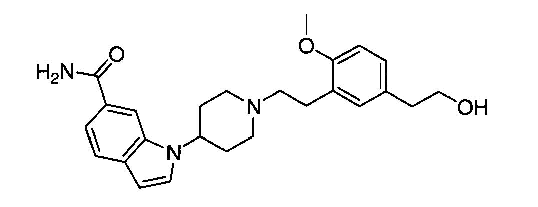

The title compound was obtained by synthesizing from 2-(4-hydroxyphenyl)ethanol in accordance with the methods in example 63.
¹H-NMR(CDCl3)
δ: 2.06-2.29(m,6H), 2.62-2.66(m,2H), 2.80(t, J=6.6Hz,2H), 2.82-2.86(m,2H), 3.16-3.21(m, 2H), 3.82(s,3H), 3.83(t, *J*=6.6Hz,2H), 4.32-4.40(m,1H), 5.76(brs,1H), 6.31(brs,1H), 6.55(d, *J*=2.8Hz,1H), 6.80(d, *J*=8.0Hz,1H), 7.03-7.06(m,2H), 7.37(d, *J*=3.2Hz,1H), 7.41(dd, *J*=1.6Hz,8.0Hz,1H), 7.62(d, *J*=8.0Hz,1H), 8.10(s,1H).

### Example 85

### Synthesis of 1-{1-{2-[2-methoxy-6-(2-hydroxyethyl)phenyl] ethyl}piperidin-4-yl}-1H-indole-6-carboxamide

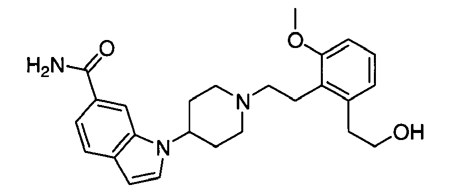

The title compound was obtained by synthesizing from 3-(4-hydroxyphenyl)ethanol in accordance with the methods in example 63.
¹H-NMR(CDCl3)
δ: 2.06-2.09(m,2H), 2.21-2.32(m,4H), 2.62(t, J=7.2Hz,2H), 2.91-2.97(m,4H), 3.17-3.20(m,2H), 3.84(s,3H), 3.92(t, *J*=6.4Hz,2H), 4.30-4.38(m,1H), 5.53 (brs, 1H) 6.35 (brs, 1H), 6.54(d, *J*=3.0Hz, 1H), 6.76(d, *J*=8.4Hz, 1H), 6.84-6.86(m,1H), 7.15-7.19 (m,1H), 7.36(d, *J*=3.0Hz,1H), 7.46-7.48 (m,1H), 7.63(d, *J*=8.4Hz,1H), 8.15 (s,1H).

### Example 86

### Synthesis of 1-{1-{2-[2-methoxy-6-(2-oxo-2-morpholinoethyl) phenyl]ethyl}piperidin-4-yl}-1H-indole-6-carboxamide

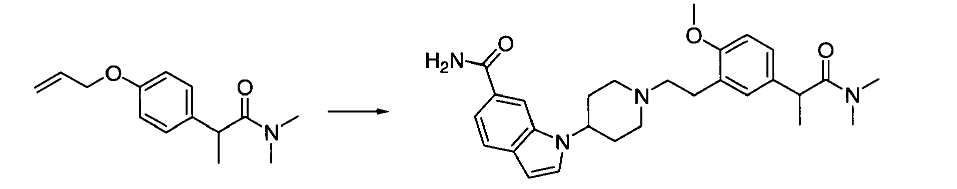

The title compound was obtained by synthesizing from 2-(4-allyloxyphenyl)-N,N-dimethyl propionamide in accordance with the methods in example 1.
¹H-NMR(CDCl3)
δ: 1.42(d, J=7.2Hz,3H), 2.07-2.18(m,4H), 2.25-2.31(m,2H), 2.62-2.65(m,2H), 2.82-2.86(m,2H), 2.93(s,3H), 2.95(s,3H), 3.17-3.22(m,2H), 3.81-3.86 (m,1H), 3.82(s,3H), 4.34-4.42(m,1H), 5.77 (brs,1H), 6.31 (brs,1H), 6.57(d, *J*=3.2Hz,1H), 6.80(d, *J*=8.4Hz,1H), 7.08-7.10 (m,1H), 7.40-7.45 (m, 2H), 7.64(d, *J*=8.4Hz,1H), 8.12 (s,1H).

### Example 87

### Synthesis of 1-{1-{2-[2-methoxy-5-(1-methylpiperidin-4-yl) phenyl]ethyl}piperidin-4-yl}-1H-indole-6-carboxamide

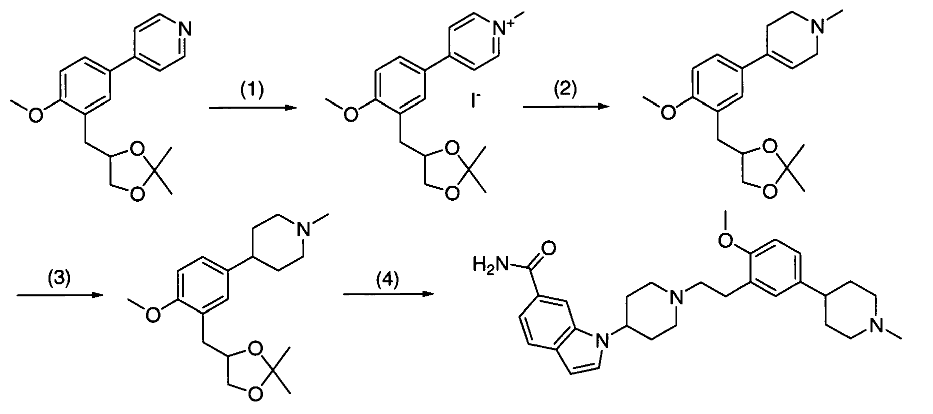

### (1) Synthesis of 4-[3-(2,2-dimethyl-[1,3]dioxolan-4-ylmethyl) -4-methoxyphenyl]-1-pyridinium iodide

1.17g of 4-[3-(2,2-dimethyl-[1,3]dioxolan-4-ylmethyl)-4-methoxyphenyl]-1-pyridine obtained in example 82-(2) was dissolved in 15ml of acetonitrile. 4ml of iodomethane was added thereto , which was stirred in a sealed flask overnight at room temperature. Concentration was carried out under a reduced pressure. Diethyl ether was added to the residue, and collected by filtration to give 1.60g of the title compound.
¹H-NMR(DMSO-d6)
δ: 1.25(s,3H), 1.34(s,3H), 2.84(dd, *J*=7.2Hz,13.6Hz,1H), 2.97(dd, *J*=6.6Hz,13.6Hz,1H), 3.65(dd, J=6.2Hz,8.2Hz,1H), 3.89-3.93(m,1H), 3.92(s,3H), 4.28(s,3H), 4.33-4.40(m,1H), 7.22(d, J=8.8Hz,1H), 8.00(d, *J*=2.8Hz,1H), 8.04(dd, *J*=2.8Hz,8.8Hz,1H), 8.44(d, *J*=7.0Hz,2H), 8.90(d, *J*=7.0Hz,2H).

### (2) Synthesis of 4- [3-(2,2-dimethyl-[1,3] dioxolan-4-ylmethyl)-4-methoxyphenyl]-1-methyl-1,2,3,6-tetrahydropyridine

1. 60g of 4- [3- (2,2-dimethyl- [1, 3] dioxolan-4-ylmethyl) -4-methoxyphenyl]-1-pyridinium iodide was dissolved in 30ml of methanol, which was stirred in an ice cooling. Sodium borohydride was added thereto in small portions until the starting material disappeared. Stirring was carried out for 15 minutes at room temperature, and stirring in an ice-cooling was again carried out. Ethyl acetate, 10% sodium carbonate aqueous solution was added thereto, the organic layer was separated, washed with brine, and dried over magnesium sulfate. Magnesium sulfate was removed by filtration, the filtrate was concentrated under a reduced pressure. The residue was purified by NH silica gel column chromatography (*n-*hexane-ethyl acetate) to give 1.07g of the title compound.
¹H-NMR(CDCl3)
δ: 1.35(s,3H), 1.43(s,3H), 2.40(s,3H), 2.53-2.57(m,2H), 2.64-2.67(m,2H), 2.79(dd, *J*=7.6Hz,13.2Hz,1H), 3.02(dd, *J*=5.0Hz,13.2Hz,1H), 3.08-3.11(m,2H), 3.66(dd, J=6.4Hz, 8.0Hz,1H), 3.81(s,3H), 3.92(dd, *J*=6.0Hz,8.0Hz,1H), 4.33-4.39(m,1H), 5.94-5.98(m,1H), 6.78-6.80(m,1H), 7.13-7.26(m,2H).

### (3) Synthesis of 4-[3-(2,2-dimethyl-[1,3]dioxolan-4-ylmethyl)-4-methoxyphenyl]-1-methyl-piperidine

1.07g of 4-[3-(2,2-dimethyl-[1,3]dioxolan-4-ylmethyl)-4-methoxyphenyl]-1-methyl-1,2,3,6-tetrahydropyridine was dissolved in 30ml of ethyl acetate. 0.12g of 10% Pd-C (50% wet) was added, which was stirred overnight at room temperature under hydrogen atmosphere. Pd-C was removed by filtration, the filtrate was concentrated under a reduced pressure. The residue was dissolved in 30ml of ethyl acetate. 0.12g of 10% Pd-C (50% wet) was added, which was stirred overnight at room temperature under hydrogen atmosphere. Pd-C was removed by filtration, the filtrate was concentrated under a reduced pressure to give 0.96g of the title compound.
¹H-NMR(CDCl3)
δ: 1.35(s,3H), 1.43(s,3H), 1.70-1.83(m,4H), 1.99-2.06(m,2H), 2.31(s,3H), 2.36-2.44(m,1H), 2.78(dd, *J*=7.6Hz,13.2Hz,1H), 2.93-3.03(m,3H), 3.65(dd, *J*=6.8Hz,8.0Hz,1H), 3.79(s,3H), 3.92(dd, *J*=6.0Hz, 8.0Hz,1H), 4.32-4.38(m,1H), 6.78(d, *J*=8.4Hz,1H), 7.03(d, *J*=2.4Hz,1H), 7.06(dd, *J*=2.4Hz, 8.4Hz, 1H).

### (4) Synthesis of 4-{1-{2-[2-methoxy-5-(1-methylpiperidin-4-yl) phenyl]ethyl}piperidin-4-yl}-1H-indole-6-carboxamide

The title compound was obtained by synthesizing from 4-[3-(2,2-dimethyl-[1,3]dioxolan-4-ylmethyl)-4-methoxyphenyl] -1-methyl- piperidine in accordance with the methods in example 82.
¹H-NMR(CDCl3)
δ: 1.72-1.84 (m, 4H), 2.05-2.17 (m, 6H), 2.26-2.32 (m,1H), 2.32(s,3H), 2.38-2.45(m,1H), 2.62-2.66(m,2H), 2.83-2.87-(m,2H), 2.95-2.99(m,2H), 3.19-3.33(m,2H), 3.82(s,3H), 4.36-4.44(m,1H), 5.61(brs,1H), 6.16(brs,1H), 6.57(d, *J*=3.2Hz,1H), 6.81(d, *J*=8.0Hz,1H), 7.04-7.07(m,2H), 7.40-7.42(m,2H), 7.64(d, *J*=8.0Hz,1H), 8.10-(s, 1H).

### Example 88

### Synthesis of 1-{1-{2-[2-methoxy-5-(1-methylpiperidin-3-yl) phenyl]ethyl}piperidin-4-yl}-1H-indole-6-carboxamide

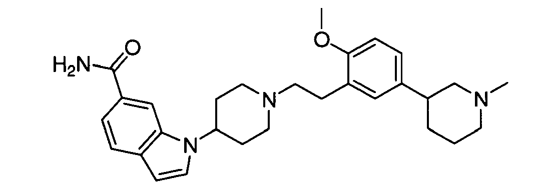

The title compound was obtained by synthesizing from 4-bromophenol in accordance with the methods in example 87.
¹H-NMR (CDCl3)
5: 1.34-1.44 (m,1H), 1.69-1.82(m,2H), 1.89-1.95(m,4H), 2.10-2.15(m,4H), 2.30(s,3H), 2.62-2.96(m,8H), 3.20-3.24(m, 2H), 3.82(s,3H), 4.36-4.44(m,1H), 5.60(brs,1H), 6.15(brs,1H), 6.57(d, *J*=3.2Hz, 1H), 6.80(d, *J*=8.0Hz,1H), 7.03-7.07(n,2H), 7.39-7.42(m,2H), 7.64(d, *J*=8.0Hz,1H), 8.11(s,1H).

### Example 89

### Synthesis of 1-{1-{2-[2-methoxy-6-(1-methylpiperidin-3-yl) phenyl]ethyl}piperidin-4-yl}-1H-indole-6-carboxamide

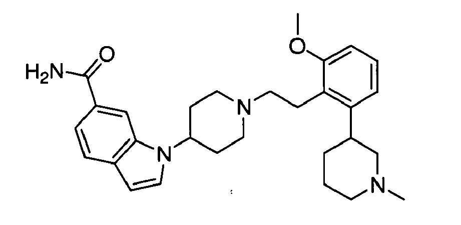

The title compound was obtained by synthesizing from 3-bromophenol in accordance with the methods in example 87.
¹H-NMR(CDCl3)
δ: 1.24-2.33(m,15H), 2.45-2.61(m,2H), 2.86-3.12(m,5H), 3.21-3.27(m,2H), 3.83(s,3H), 4.33-4.41(m,1H), 5.65(brs,1H), 6.45(brs,1H), 6.56(d, *J*=3.2Hz,1H),6.75(d, *J*=8.4Hz,1H), 6.88(d, *J*=7.6Hz,1H), 7.16-7.20(m,1H), 7.40(d, *J*=3.2Hz,1H), 7.47-7.49(m,1H), 7.65(d, *J*=8.0Hz,1H), 8.14(s,1H)

### Example 90

### Synthesis of 1-{1-{2-[4-hydroxymethyl-2-methoxyphenyl]ethyl} piperidin-4-yl}-1H-indole-6-carboxamide

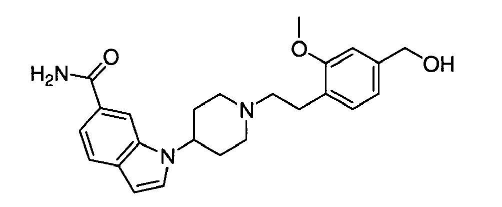

The title compound was obtained by synthesizing from 3-bromophenol in accordance with the methods in example 69.
¹H-NMR(CDCl3)
δ: 2.09-2.18(m,4H), 2.26-2.32(m,2H), 2.62-2.66(m,2H), 2.84-2.88(m,2H), 3.19-3.23(m,2H), 3.86(s,3H), 3.35-3.43(m,1H), 4.67(s,2H), 5.57(brs,1H), 6.17(brs,1H), 6.56-6.57(m,1H), 6.87-6.90(m,2H), 7.14(d, *J*=7.2Hz,1H), 7.38-7.41(m, 2H), 7.62-7.64(m,1H), 8.09(s,1H).

### Example 91

### Synthesis of 1-{1-{2-[2-methoxy-4-(piperidinomethyl)phenyl] ethyl}piperidin-4-yl}-1H-indole-6-carboxamide

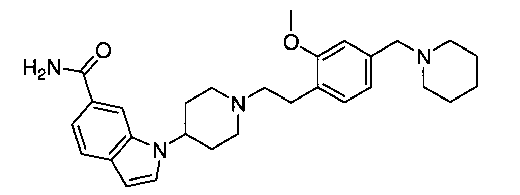

The title compound was obtained by synthesizing from 3-bromophenol in accordance with the methods in example 68.
¹H-NMR(CDCl3)
δ: 1.41-1.60(m,6H), 2.09-2.41(m,10H), 2.62-2.66(m,2H), 2.82-2.86(m,2H), 3.19-3.24(m,2H), 3.44(s,2H), 3.84(s,3H), 4.35-4.43(m,1H), 5.62(brs,1H), 6.13(brs, 1H) 6.56-(d, *J*=3.2Hz,1H), 6.81-6.85(m,2H), 7.08(d, *J*=7.6Hz, 1H), 7.39-7.41(m,2H), 7.63(d, *J*=8.4Hz, 1H), 8.09(s,1H).

### Example 92

### Synthesis of 1-{1-{2-[4-(3-dimethylaminopropyl)-2-methoxyphenyl]ethyl}piperidin-4-yl}-1H-indole-6-carboxamide

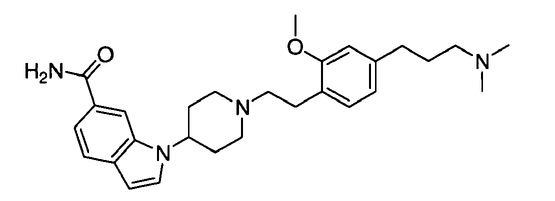

The title compound was obtained by synthesizing from 3-bromophenol in accordance with the methods in example 68.
¹H-NMR(CDCl3)
δ: 1.76-1.83(m,2H), 2.09-2.18(m,4H), 2.23(s,6H), 2.23-2.33(m,4H), 2.60-2.65(m,4H), 2.81-2.85(m,2H), 3.19-3.24(m,2H), 3.83(s,3H), 4.34-4.42(m,1H), 5.61(brs,1H), 6.17(brs,1H), 6.56(d, *J*=7.6Hz,1H), 6.70-6.74(m,2H), 7.06(d, *J*=7.6Hz,1H), 7.39-7.41(m,2H), 7.63(d, *J*=8.4Hz,1H), 8.09(s,1H).

### Example 93

### Synthesis of 1-{1-{2-[4-dimethylcarbamoyl-2,5-dimethoxyphenyl]ethyl}piperidin-4-yl}-1H-indole-6-carboxamide

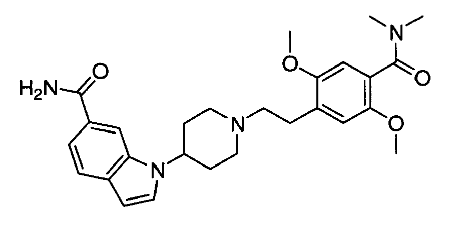

The title compound was obtained by synthesizing from 5-allyloxy-2-methoxy-N,N-dimethylbenzamide in accordance with the methods in example 1. ¹H-NMR(CDCl3)
δ: 2.11-2.90(m,10H), 2.88(s,3H), 3.12(s,3H), 3.16-3.25(m,2H), 3.80(s,3H), 3.80(s,3H), 4.36-4.43(m,1H), 5.58(brs,1H), 6.14(brs,1H), 6.56-6.57(m,1H), 6.76-6.77(m,2H), 7.38-7.41(m,2H), 7.63(d, *J*=8.4Hz,1H), 8.10(s,1H).

### Example 94

### Synthesis of 1-{1-{2-[5-dimethylcarbamoyl-2,4-dimethoxy phenyl]ethyl}piperidin-4-yl}-1H-indole-6-carboxamide

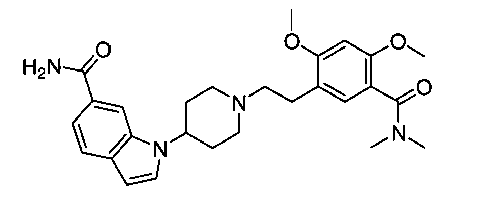

The title compound was obtained by synthesizing from 4-allyloxy-2-methoxy-N,N-dimethylbenzamide in accordance with the methods in example 1.
¹H-NMR(CDCl3)
δ: 2.04-2.34(m,6H), 2.58-2.65(m,2H), 2.75-2.83(m,2H), 2.88 (s,3H), 3.10 (s, 3H), 3.14-3.23 (m, 2H), 3.84(s,3H), 3.87 (s, 3H), 4.34-4.42(m,1H), 5.58(brs,1H), 6.22(brs,1H), 6.42(s,1H), 6.55-6.56(m,1H), 7.05(s,1H), 7.38-7.43(m,2H), 7.62-7.64(m,1H), 8.10(s,1H).

### Example 95

### Synthesis of 1-{1-{2-[5-dimethylcarbamoyl-2,6-dimethoxyphenyl]ethyl}piperidin-4-yl}-1H-indole-6-carboxamide

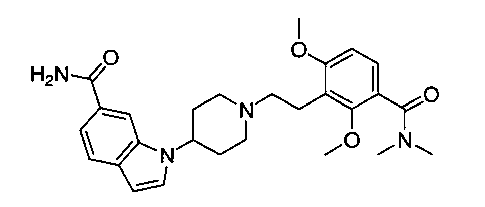

The title compound was obtained by synthesizing from 4-allyloxy-2-methoxy-N,N-dimethyl benzamide in accordance with the methods in example 1.
¹H-NMR(CDCl3)
δ : 2.08-2.35(m,6H), 2.53-2.62(m,2H), 2.86-2.94(m,2H), 2.89(s,3H), 3.13(s,3H), 3.22-3.27(m,2H), 3.79(s,3H), 3.85(s,3H), 4.35-4.43(m,1H), 5.55(brs,1H), 6.16(s,1H), 6.56-(dd, *J*=0.8Hz, 3.2Hz, 1H), 6.67(d, *J*=8.4Hz, 1H), 7.14(d, *J*=8.4Hz, 1H), 7.38-7.42(m,2H), 7.62-7.64(m,1H), 8.10(s,1H).

### Example 96

### Synthesis of 1-{1-{2-[2-dimethylcarbamoyl-4,6-dimethoxyphenyl]ethyl}piperidin-4-yl}-1H-indole-6-carboxamide

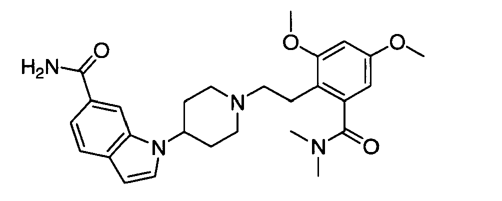

The title compound was obtained by synthesizing from 3-allyloxy-5-methoxy-N,N-dimethylbenzamide in accordance with the methods in example 1.
¹H-NMR(CDCl3)
δ: 2.18-3.32(m,12H), 2.87(s,3H), 3.13(s,3H), 3.79(s,3H), 3.83(s,3H), 4.33-4.44(m, 1H), 5.56 (brs, 1H), 6.23 (brs, 1H), 6.31 (d, J=2.4Hz,1H), 6.44(d, *J*=2.4Hz, 1H), 6.55-6.56(m, 1H), 7.37(d, *J*=3.2Hz, 1H), 7.42-7.46(m,1H), 7.62-7.64(m,1H), 8.10(s,1H).

### Example 97

### Synthesis of 1-{1-{2-[4-dimethylcarbamoyl-2,6-dimethoxyphenyl]ethyl}piperidin-4-yl}-1H-indole-6-carboxamide

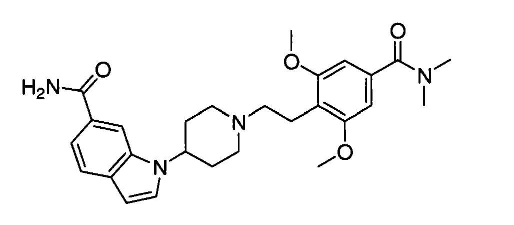

The title compound was obtained by synthesizing from 3-allyloxy-5-methoxy-N,N-dimethylbenzamide in accordance with the methods in example 1.
¹H-NMR(CDCl3)
δ: 2.08-2.34-(m, 6H), 2.48-2.56-(m ,2H), 2.88-2.96(m, 2H), 3.01 (brs, 3H), 3.11 (brs,3H), 3.20-3.27 (m,2H), 3.83 (s,6H), 4.34-4.43 (m,1H), 5.58 (brs,1H), 6.16 (brs,1H) 6.55-6.56 (m,1H) 6.57(s,2H), 7.39-7.43(m,2H), 7.62-7.64 (m,1H), 8.10 (s,1H).

### Example 98

### Synthesis of 1-[1-{2-(2-methoxy-6-piperidinophenyl)ethyl} piperidin-4-yl]-1H-indole-6-carboxamide

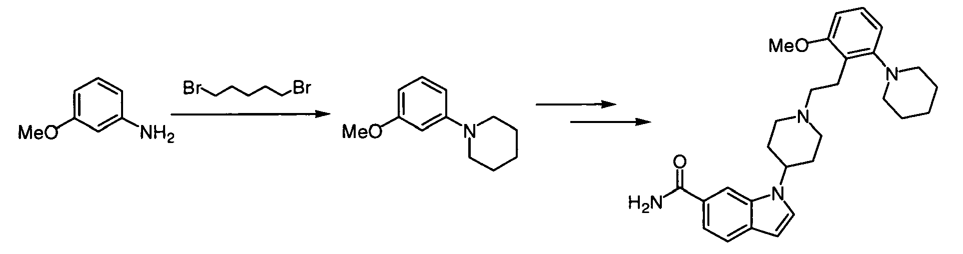

The title compound was obtained by synthesizing from 3-methoxyaniline in accordance with the methods in example 56.
¹H-NMR(CDCl3)
δ: 1.50-1.64(m,2H), 1.66-1.78(m,4H), 2.06-2.22(m,4H), 2.28-2.38(m,2H), 2.60-2.68(m,2H), 2.78-2.84(m,4H), 2.94-3.00(m,2H), 3 .25-3.32 (m, 2H), 3.83 (s, 3H), 4.34-4.44 (m,1H), 6.56(d,1H, *J*=3.2Hz), 6.65(dd,1H, *J*=8.4Hz,0.8Hz), 6.78(dd,1H, *J*=8.0Hz,0.8Hz), 7.15(dd,1H, *J*=8.4Hz,8.0Hz), 7.38-7.43(m,2H), 7.63(d,1H *J*=8.0Hz), 8.11(s,1H).
Mass Spectrum: 461 (M+1), 921(2M+1).

### Example 99

### Synthesis of 1-[1-{2-(2-methoxy-6-pyrrolidinophenyl)ethyl} piperidin-4-yl]-1H-indole-6-carboxamide

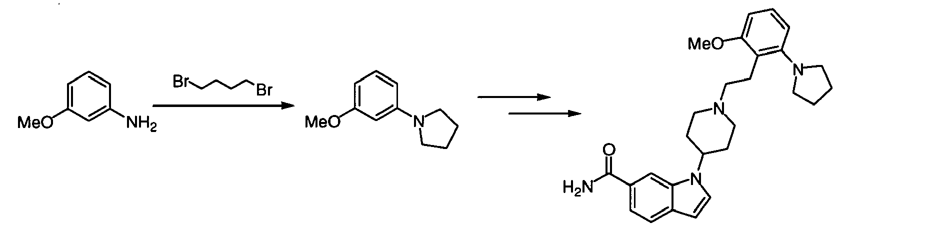

The title compound was obtained by synthesizing from 3-methoxyaniline in accordance with the methods in example 56.
¹H-NMR(CDCl₃)
5: 1.89-1.96(m,4H), 2.08-2.22(m,4H), 2.28-2.35(m,2H), 2.64-2.70(m,2H), 2.94-3.00(m,2H), 3.12-3.17(m,4H), 3.22-3.30(m,2H), 3.82(s,3H), 4.32-4.42(m,1H), 6.54-6.58(m,2H), 6.70(dd,1H, *J*=8.4Hz), 7.11(dd,1H *J*=8.4Hz,8.0Hz), 7.39-7.42(m,2H), 7.63 (d,1H *J*=8.8Hz), 8.10(s,1H).
Mass Spectrum: 447(M+1).

### Example 100

### Synthesis of 1-[1-[2-(2-methoxy-6-piperazinylphenyl)-ethylpiperidin-4-yl]-1H-indole-6-carboxamide

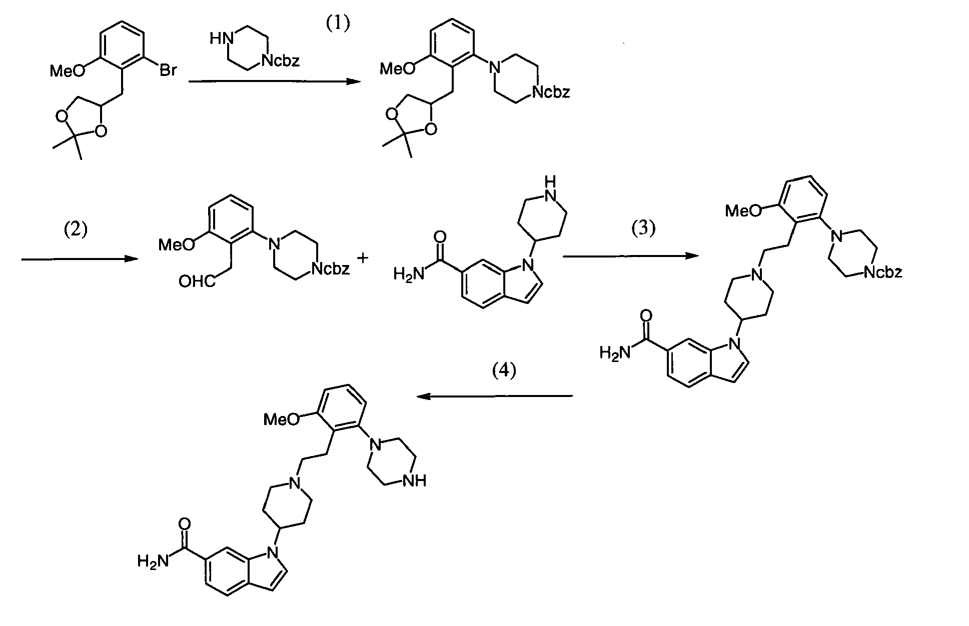

### (1) Synthesis of 1-benzyloxycarbonyl-4-[2-(2,2-dimethyl-[1,3]dioxolan-4-ylmethyl)-3-methoxyphenyl]piperazine

418mg of 4-(2-bromo-6-methoxybenzyl)-2,2-dimethyl-[1,3] dioxolane, 321µl of 1-carbobenzoxypiperazine, 51mg of tris(dibenzylideneacetone)dipalladium and 101mg of (±)-2,2'-bis(biphenylphosphino)-1,1'-binaphthyl were suspended in 5ml toluene and then 204mg of sodium *tert*-butoxide was added at room temperature. This reaction liquid was heated with stirring at 90°C for 2.5 hours under nitrogen atmosphere. After standing to cool, the reaction liquid was filtered through Celite, and the solvent was concentrated under a reduced pressure. The residue was purified by NH silica gel column chromatography (hexane-ethyl acetate) to give 263mg of the title compound.
¹H-NMR(CDCl3)
δ (ppm): 1.33(s,3H), 1.45(s,3H), 2.70-2.80(m,2H), 2.88-3.03(m,3H), 3.14(dd,1H, *J*=12.4Hz,5.2Hz), 3.55-3.75(m,4H), 3.73(dd,1H, *J*=8.0Hz,6.8Hz), 3.80(s,3H), 3.84(dd,1H, *J*=8.0Hz,6.0Hz), 4.34-4.42(m,1H), 5.16(s,2H), 6.67(dd,1H, *J*=8.4Hz,0.8Hz), 6.73(dd,1H, *J*=8.0Hz,0.8Hz), 7.16(dd,1H *J*=8.4Hz,8.0Hz), 7.30-7.40(m,5H).

### (2) Synthesis of [2-methoxy-6-(4-benzyloxycarbonylpiperazin-1-yl)phenyl]acetaldehyde

263mg of 1-benzyloxycarbonyl-4-[2-(2,2-dimethyl-[1,3]dioxolan-4-ylmethyl)-3-methoxyphenyl]piperazine was dissolved in 4ml methanol, 1ml of 4N HCl-ethyl acetate solution was added at room temperature. After stirring for 25 minutes at room temperature, solvent was evaporated, methanol was added, and solvent was evaporated again. 2ml of water was added to the residue, neutralized with saturated sodium bicarbonate aqueous solution, and 5ml of tetrahydrofuran was then added. 269mg of sodium periodate was added thereto and stirred for 3 hours at room temperature. Saturated sodium bicarbonate aqueous solution and dichloromethane were added to this reaction liquid, the organic layer was separated. The resulting organic layer was washed with brine and then dried over anhydrous sodium sulfate. After removing the drying agent by filtration, the organic layer was concentrated under a reduced pressure to give 206mg of the title compound.
¹H-NMR(CDCl3)
δ (ppm) : 2.78-2.88(m, 4H), 3.50-3.70 (m, 4H), 3.70(d,2H, *J*=1.8Hz), 3.81(s,3H), 5.14(s,2H), 6.73(d,1H, *J*=8.4Hz), 6.77(dd,1H, *J*=8.0Hz,1.2Hz), 7.24-7.38(m,6H), 9.55(t,1H, J=1.8Hz).

### (3) Synthesis of 1-[1-[2-{2-methoxy-6-(4-benzyloxycarbonyl piperazin-1-yl)phenyl}ethyl]piperidin-4-yl]-1H-indole-6-carboxamide

140mg of 1-(piperidin-4-yl)-1H-indole-6-carboxamide, which was synthesized in Production example 1, and 206mg of [2-methoxy-6-(4-benzyloxycarbonylpiperazin-1-yl)phenyl] acetaldehyde were dissolved in 5ml of dichloromethane, 66µl of acetic acid and 186mg of sodium triacetoxyborohydride were added to the reaction liquid, the reaction liquid was stirred overnight at room temperature. Aqueous sodium hydroxide, dichloromethane were added to the reaction liquid, and the organic layer was separated. The resulting organic layer was washed with brine and then dried over anhydrous sodium sulfate. After removal of drying agent by filtration, the organic layer was concentrated under a reduced pressure. The residue was purified by NH silica gel column chromatography (ethyl acetate) to give 279mg of the title compound.
¹H-NMR(CDCl3)
δ (ppm): 2.10-2.21(m, 4H), 2.29-2.37(m, 2H), 2.57-2.63(m, 2H), 2.78-2.90(m,4H), 2.96-3.02(m,2H), 3.22-3.30(m,2H), 3.60-3.75(m,4H), 3.83(s,3H), 4.34-4.44(m,1H), 5.16(s,2H), 6.56(dd,1H, *J*=3.2Hz,0.8Hz), 6.70(d,1H, *J*=8.0Hz), 6.75(dd,1H, *J*=8.4Hz,1.2Hz), 7.17(dd,1H, *J*=8.4Hz,8.0Hz), 7.30-7.44(m,7H), 7.63(dd,1H, *J*=8.4Hz,0.4Hz), 8.09(s,1H).
mass spectrum: 596-(M+1).

### (4) Synthesis of 1-[1-[2-(2-methoxy-6-piperazinylphenyl) ethylpiperidin-4-yl]-1H-indole-6-carboxamide

279mg of 1-[1-[2-{2-methoxy-6-(4-benzyloxycarbonyl piperazin-1-yl)phenyl}ethyl]piperidin-4-yl]-1H-indole-6-carboxamide was dissolved in 15ml of methanol, catalytic amount of palladium carbon was added, which was stirred for 3 hours under hydrogen atmosphere at room temperature. Hydrogen in the reaction vessel was replaced with nitrogen, catalyst was removed by filtration through a filter paper. The solvent was concentrated under a reduced pressure to give 204mg of the title compound.
¹H-NMR(CDCl3)
5 (ppm): 2.10-2.22(m, 4H), 2.28-2.38(m,2H), 2.60-2.65(m,2H), 2.83-2.89(m,4H), 2.96-3.06-(m,6H), 3.25-3.33(m,2H), 3.83(s,3H), 4.34-4.44(m,1H), 6.56(dd,1H, *J*=3.2Hz, 0.8Hz), 6.68(d,1H, J=8.0Hz), 6.81(dd,1H, J=8.4Hz,1.2Hz), 7.17(dd,1H, *J*=8.4Hz, 8.0Hz), 7.38-7.42(m,2H), 7.63(dd, 1H, *J*=8.4Hz, 0.4Hz), 8.11(s, 1H).
mass spectrum: 462-(M+1).

### Example 101

### Synthesis of 1-[1-[2-{2-methoxy-6-(4-acetylpiperazin-1-yl) phenyl}ethyl]piperidin-4-yl]-1H-indole-6-carboxamide

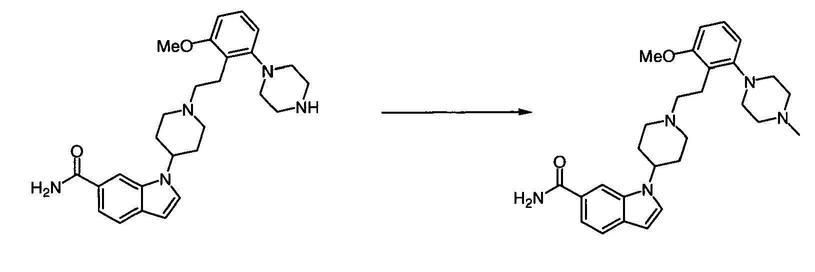

47mg of carbonyldiimidazole was dissolved in 1ml of N, N-dimethylformamide, 17µl of acetic acid was added at room temperature. After this reaction liquid was stirred for 15 minutes at room temperature, 47mg of 1-[1-[2-(2-methoxy-6-piperazinylphenyl)-ethylpiperidin-4-yl]-1*H*-indole-6-carboxamide synthesized in example 100 was dissolved in 1ml of N, N-dimethylformamide and added at room temperature. This reaction liquid was stirred overnight at room temperature. Water, 10% methanol-chloroform mixed solvent were added to the reaction liquid, and the organic layer was separated. The resulting organic layer was washed with brine and then dried over anhydrous sodium sulfate. After removal of drying agent by filtration, the organic layer was concentrated under a reduced pressure. The residue was purified by NH silica gel column chromatography (ethyl acetate-methanol) to give 57mg of the title compound.
¹H-NMR(CDCl3)
δ (ppm): 2.10-2.25 (m, 4H), 2.15 (s,3H), 2.29-2.39 (m,2H), 2.60-2.68(m,2H), 2.84-2.94(m,6H), 2.96-3.06(m,2H), 3.24-3.32(m,2H), 3.10-3.17 (m,2H), 3.84 (s,3H), 4.34-4.44(m,1H), 6.57(dd,1H, *J*=3.2.Hz,0.8Hz), 6.71(d,1H, *J*=8.4Hz), 6.75(dd,1H, *J*=8.0Hz,0.8Hz), 7.18(dd,1H, *J*=8.4Hz,8.0Hz), 7.38-7.44(m,2H), 7.64(dd,1H, *J*=8.4Hz,0.8Hz), 8.11(s,1H)
mass spectrum: 504-(M+1), 526-(M+23).

### Example 102

### Synthesis of 1-[1-[2-{2-methoxy-6-(4-methyl piperazin-1-yl) phenyl}ethyl]piperidin-4-yl]-1H-indole-6-carboxamide

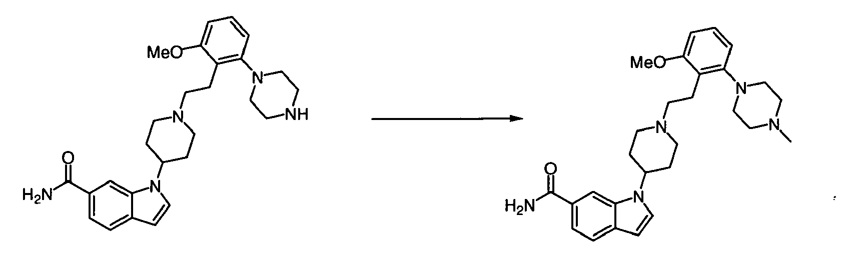

57mg of 1-[1-[2-(2-methoxy-6-piperazinylphenyl) ethylpiperidin-4-yl]-1*H*-indole-6-carboxamide synthesized in example 100, 1ml of 37% formaldehyde aqueous solution, 100µl of acetic acid was dissolved in 5ml of acetonitrile, 41mg of sodium cyanoborohydride were added at room temperature. This reaction liquid was stirred overnight at room temperature. Saturated sodium bicarbonate aqueous solution, 10% methanol-chloroform mixed solvent were added to reaction liquid, and the organic layer was separated. The resulting organic layer was washed with brine and then dried over anhydrous sodium sulfate. After removal of drying agent by filtration, the organic layer was concentrated under a reduced pressure. The residue was purified by NH silica gel column chromatography (ethyl acetate-methanol) to give 35mg of the title compound.
¹H-NMR(CDCl3)
δ (ppm): 2.09-2.23(m,4H), 2.28-2.40(m,2H), 2.37(s,3H), 2.52-2.68(m,6H), 2.90-3.02(m,6H), 3.24-3.32(m,2H), 3.83(s,3H), 4.35-4.45(m,1H), 6.57(dd,1H, *J*=3.2Hz, 0.8Hz), 6.68-(dd,1H, *J*=8.4Hz,0.8Hz), 6.82(dd,1H, *J*=8.0Hz, 0.8Hz), 7.17(dd,1H *J*=8.4Hz, 8.0Hz), 7.38-7.44(m,2H), 7.63(dd,1H, *J*=8.0Hz, 0.8Hz), 8.12(s,1H).
mass spectrum: 476-(M+1).

### Example 103

### Synthesis of 1-[1-[2-{2-methoxy-6-(N-acetyl-N-methylamino) phenyl}ethylpiperidin-4-yl]-1H-indole-6-carboxamide [126]

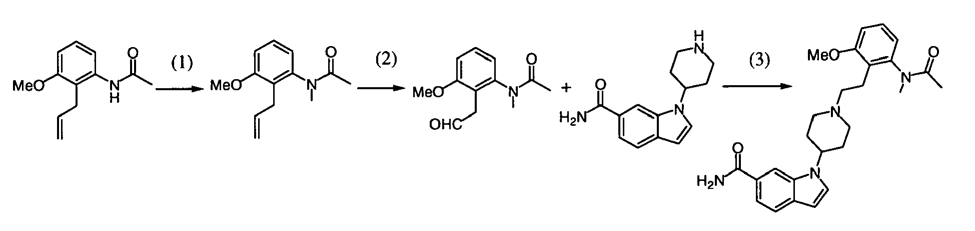

### (1) Synthesis of the N-methyl-N-(2-allyl-3-methoxyphenyl) acetamide

193mg of N-(2-allyl-3-methoxyphenylacetamide synthesized in Production example 5 and 88µl of methyl iodide were dissolved in 3ml of N, N-dimethylformamide, 60mg of sodium hydride was added at room temperature. This reaction liquid was stirred overnight at room temperature. Water and ethyl acetate were added to reaction liquid, and the organic layer was separated. The resulting organic layer was washed with brine and then dried over anhydrous sodium sulfate. After removing the drying agent by filtration, the organic layer was concentrated under a reduced pressure, and the residue was purified by silica gel column chromatography (hexane-ethyl acetate) to give 194mg of the title compound.
¹H-NMR(CDCl3)
δ (ppm) : 1.76(s,3H), 3.16(s,3H), 3.32(td,2H, *J*=6.4Hz, *J*=1.6Hz), 3.86(s,3H), 4.92-5.02(m, 2H), 5.85-5.95(m,1H), 6.74(dd,1H, *J*=8.0Hz,0.8Hz), 6.88(dd,1H, *J*=8.0Hz,0.8Hz), 7.23(t,1H, *J*=8.0Hz).

### (2) Synthesis of [2-methoxy-6-(N-acetyl-N-methylamino)phenyl] acetaldehyde

194mg of N-methyl-N-(2- allyl-3-methoxyphenyl)acetamide was dissolved in a mixed solvent of tetrahydrofuran 4ml and water 1ml, 3330µl of 3% osmium tetroxide aqueous solution was added at room temperature. 557mg of sodium periodate was then added at room temperature followed by stirring overnight at room temperature. Sodium sulfite aqueous solution and dichloromethane were added to reaction liquid, and the organic layer was separated. The resulting organic layer was washed with brine and then dried over anhydrous sodium sulfate. After removing the drying agent by filtration, the organic layer was concentrated under a reduced pressure to give 152mg of the title compound. The compound was used in the next reaction without further purification.

### (3) Synthesis of 1-[1-[2-{2-methoxy-6-(N-acetyl-N-methylamino) phenyl}ethylpiperidin-4-yl]-1H-indole-6-carboxamide

156mg of 1-(piperidin-4-yl)-1*H*-indole-6-carboxamide synthesized in Production example 1 and 152mg of [2-methoxy-6-(N-acetyl N-methylamino)phenyl]acataldehyde were dissolved in 5ml of dichloromethane, 73µl of acetic acid and 200mg of sodium triacetoxyborohydride were added to reaction liquid, reaction liquid was stirred overnight at room temperature. Aqueous sodium hydroxide and dichloromethane were added to reaction liquid, and the organic layer was separated. The resulting organic layer was washed with brine and then dried over anhydrous sodium sulfate. After removal of drying agent by filtration, the organic layer was concentrated under a reduced pressure. The residue was purified by silica gel column chromatography (chloroform / methanol / ammonia aqueous solution) to give 200mg of the title compound.
¹H-NMR(CDCl3)
δ (ppm): 1.82(s,3H), 2.06-2.20(m,4H), 2.22-2.36(m,2H), 2.50-2.70(m,2H), 2.76-2.82(m,2H), 3.12-3.22(m,2H), 3.23(s,3H), 3.88(s,3H), 4.32-4.42(m,1H), 6.56(d,1H, J=3.2Hz), 6.75(d,1H, J=8.0Hz), 6.88(d,1H, J=7.6Hz), 7.21-7.26(m,1H), 7.38(d,1H, J=3.2Hz), 7.42(d,1H, J=8.0Hz,1.6Hz), 7.63(d,1H, J=8.0Hz), 8.08(s,1H).
mass spectrum: 449-(M+1), 471-(M+23).

### Example 104

### Synthesis of 1-[1-[2-{2-methoxy-6-(N-acetylamino)phenyl} ethylpiperidin-4-yl]-1H-indole-6-carboxamide

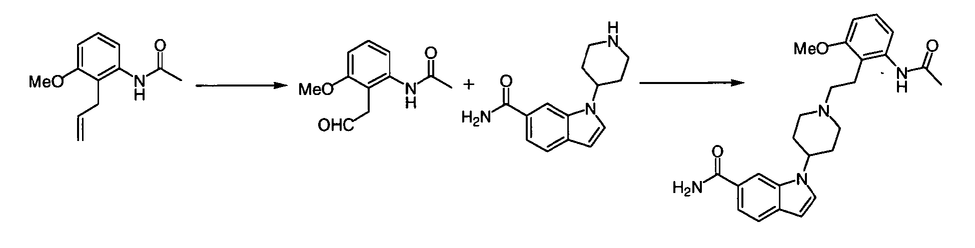

The title compound was obtained by synthesizing from N-methyl-N-(2-allyl-3-methoxyphenyl) acetamide (synthesized in 103-(1)) in accordance with the methods in example 103.
¹H-NMR(DMSO-d6)
δ (ppm): 1.92-2.08(m,4H), 2.06(s,3H), 2.24-2.34(m,2H), 2.38-2.46(m,2H), 2.75-2.82(m,2H), 3.05-3.14(m,2H), 3.79(s,3H), 4.38-4.46(m,1H), 6.82(d,1H, *J*=8.0Hz), 6.95(d,1H, *J*=8.0Hz), 7.13(t,1H, *J*=8.0Hz), 7.11(br,1H) 7.52-7.60(m,2H), 7.65(d,1H, J=3.2Hz), 7.89(br,1H), 8.12(s,1H) 9.46(br,1H).
mass spectrum: 435-(M+1).

### Example 105

### Synthesis of 1-[2-{2-methoxy-6-(2-tolyl)phenyl} ethylpiperidin-4-yl]-1H-indole-6-carboxamide

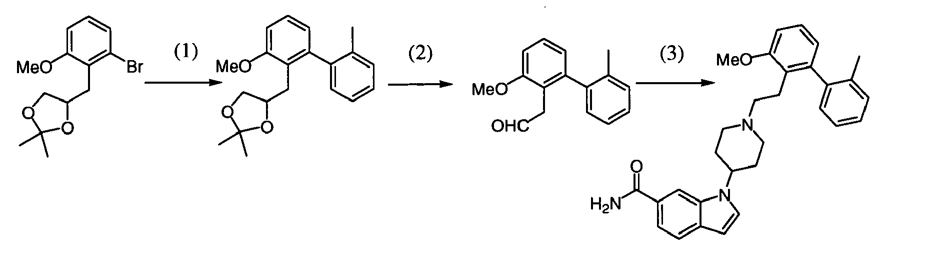

### (1) Synthesis of 2-[2-(2,2-dimethyl-[1,3]dioxolan-4-ylmethyl) -3-methoxyphenyl]methylbenzene

334mg of 4-(2-bromo-6-methoxybenzyl)-2,2-dimethyl-[1,3]dioxolane synthesized in Production example 4, 620mg of o-tolyl boronic acid and 925mg of potassium phosphate were dissolved in 5ml of dioxane, 201mg of 1,1'-bis(biphenylphosphino)ferrocene dichloropalladium was added at room temperature. This reaction liquid was heated with stirring at 90 °C overnight under nitrogen atmosphere. After standing to cool, water and ethyl acetate was added, the reaction liquid was filtered through Celite, and the organic layer was separated. After the resulting organic layer was washed with water twice and brine once, and dried over anhydrous sodium sulfate. After removing the drying agent by filtration, the organic layer was concentrated under a reduced pressure, the residue was purified by NH silica gel column chromatography (hexane-ethyl acetate) to give 330mg of the title compound.
¹H-NMR(CDCl3)
δ (ppm) : 1.16 (s,1.5H), 1.23 (s,1.5H), 1.25 (s,1.5H), 1.26 (s,1.5H), 2.03 (s,1.5H), 2.05(s,1.5H), 2.53(dd,0.5H, *J*=14Hz,8.0Hz), 2.62(dd,0.5H, J=14Hz,6.4Hz), 2.85(dd,0.5H, J=14Hz,7.2Hz), 3.01(dd,0.5H, *J*=14Hz,5.2Hz), 3.30(dd,0.5H, *J*=8.0Hz,7.4Hz), 3.44(dd,0.5H, *J*=7.6Hz,7.6Hz), 3.74(dd,0.5H, *J*=8.0Hz,5.6Hz), 3.82(dd,0.5H, *J*=8.0Hz, 2.0Hz), 3.86(s,1.5H), 3.87(s,1.5H), 4.11-4.19(m,0.5H), 4.20-4.28(m,0.5H), 6.72(d,1H, *J*=8.0Hz), 6.85(d,1H, *J*=8.0.Hz), 7.08(d,0.5H, *J*=7.2Hz), 7.06-7.26(m,4H).

### (2) Synthesis of [2-methoxy-6-(2-tolyl)phenyl]acetaldehyde

263mg of 2-[2-(2,2-dimethyl-[1,3]dioxolan-4-ylmethyl)-3-methoxyphenyl]methylbenzene was dissolved in 5ml of methanol, 1.5ml of 4N HCl-ethyl acetate solution was added at room temperature. Solvent was evaporated under a reduced pressure after stirring for 3 hours at room temperature, methanol was added, solvent was evaporated again. Water was added to the residue, neutralized with saturated sodium bicarbonate aqueous solution, and 5ml of tetrahydrofuran was then added. 503mg of sodium periodate was added thereto, which was stirred overnight at room temperature. Saturated sodium bicarbonate aqueous solution and dichloromethane were added to this reaction liquid, the organic layer was separated. The resulting organic layer was washed with brine and then dried over anhydrous sodium sulfate. After removing the drying agent by filtration, the organic layer was concentrated under a reduced pressure to give 334mg of the title compound. The compound was used in the next reaction without further purification.

### (3) Synthesis of 1-[2-{2-methoxy-6-(2-tolyl)phenyl} ethylpiperidin-4-yl]-1H-indole-6-carboxamide

278mg of 1-(piperidin-4-yl)-1H-indole-6-carboxamide synthesized in Production example 1 and 334mg of [2-methoxy-6-(2-tolyl)phenyl]acetaldehyde were dissolved in 10ml of dichloromethane, 131µl of acetic acid and 412mg of sodium triacetoxyborohydride were added to reaction liquid, the reaction liquid was stirred for 6.5 hours at room temperature. Aqueous sodium hydroxide and dichloromethane were added to reaction liquid, and the organic layer was separated. The resulting organic layer was washed with brine and then dried over anhydrous sodium sulfate. After removal of drying agent by filtration, the organic layer was concentrated under a reduced pressure. The residue was purified by NH silica gel column chromatography (ethyl acetate / hexane) to give 182mg of the title compound.
¹H-NMR(CDCl3)
δ (ppm): 1.94-2.18(m,6H), 2.35-2.55(m,3H), 2.78-2.98(m,3H), 3.89(s,3H), 4.23-4.33(m,1H), 6.54(dd,1H, *J*=7.2Hz,0.8Hz), 6.75(dd,1H, *J*=7.6Hz,0.8Hz), 6.88(dd,1H, *J*=8.0Hz,0.8Hz), 7.14(d,1H, *J*=6.8Hz), 7.20-7.28(m,4H), 7.33(d,1H, *J*=3.2Hz), 7.40(d,1H, *J*=8.4Hz), 7.62(dd,1H, *J*=8.4Hz,0.8Hz), 8.07(s,1H).
mass spectrum: 468-(M+1), 935-(2M+1).

### Example 106

### Synthesis of 1-[1-[2-{2-methoxy-6-(2-methoxyphenyl)phenyl} ethylpiperidin-4-yl]-1H-indole-6-carboxamide

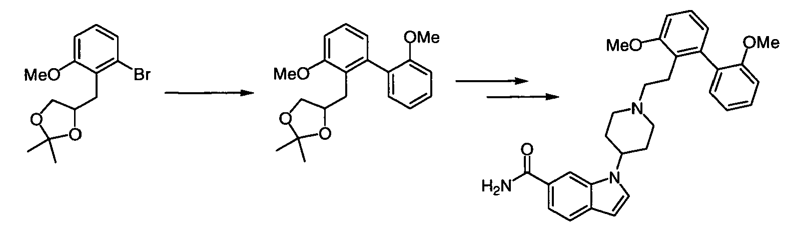

The title compound was obtained by synthesizing from 4-(2-bromo-6-methoxybenzyl)-2,2-dimethyl-[1,3]dioxolane synthesized in Production example 4 in accordance with the methods in example 105.
¹H-NMR(CDCl3) δ (ppm): 1.93-2.08(m,6H), 2.38-2.64(m,3H), 2.75-2.83(m,1H), 2.85-2.93(m,2H), 3.78(s,3H), 3.88(s,3H), 4.23-5.35(m,1H), 6.54(dd,1H, *J*=3.6Hz,0.8Hz), 6.81(dd,1H, *J*=7.6Hz,1.2Hz), 6.89(dd,1H, *J*=8.0Hz, 0.8Hz), 6.97-7.03(m,2H), 7.15(dd,1H *J*=7.6Hz, 2.0Hz), 7.23(d, 1H, *J*=8.0Hz), 7.32-7.42(m,3H), 7.63(d,1H, J=8.0Hz), 8.07(s, 1H).
mass spectrum: 484-(M+1).

### Example 107

### Synthesis of 1-[2-{4-methoxybiphenyl-3-yl}ethylpiperidin-4-yl]-1H-indole-6-carboxamide

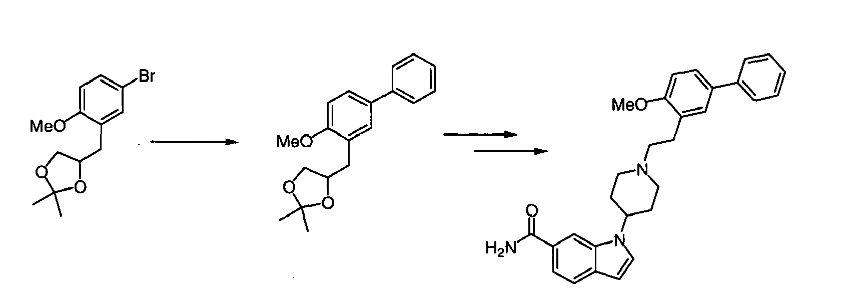

The title compound was obtained by synthesizing from 4-(3-bromo-6-methoxybenzyl)-2,2-dimethyl-[1,3]dioxolane synthesized in production example 6 in accordance with the methods in example 105.
¹H-NMR(CDCl3)
δ (ppm): 2.05-2.18(m, 4H), 2.23-2.35(m, 2H), 2.66-2.74(m, 2H), 2.90-2.98(m, 2H), 3.20-3.28(m, 2H), 3.89(s, 3H), 4.36-4.44(m,1H), 6.95(d,1H, *J*=2.8Hz), 6.94(t,2H, *J*=8.4Hz), 7.30-7.46(m,5H), 7.52-7.56(m,3H), 7.64-(d,1H, *J*=8.0Hz), 8.10(s,1H).
mass spectrum: 454-(M+1).

### Example 108

### Synthesis of 1-{2-(2-methoxy-S-piperidinylphenyl) ethylpiperidin-4-yl}-1H-indole-6-carboxamide

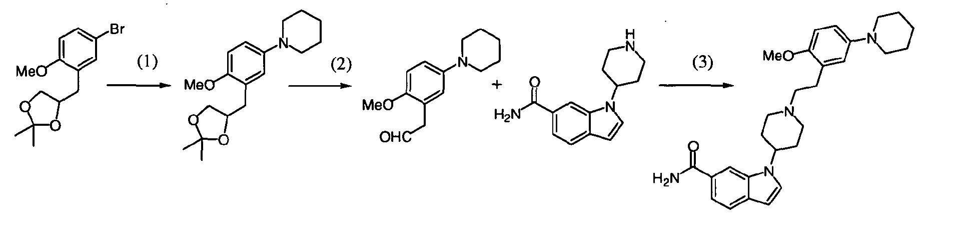

### (1) Synthesis of 4-(3-piperidinyl-6-methoxybenzyl)-2,2-dimethyl-[1,3]dioxolane

611mg of 4-(3-bromo-6-methoxybenzyl)-2,2-dimethyl-[1,3]dioxolane synthesized in Production example 6, 302µl of piperidine, 88mg of tris(dibenzylideneacetone) dipalladium and 140mg of (±)2,2'-bis(biphenylphosphino)-1,1'-binaphthyl was suspended in 5ml of toluene, 293mg of sodium *tert*-butoxide was added at room temperature. This reaction liquid was heated with stirring at 90 °C for 5 hours under nitrogen atmosphere. After standing to cool, water and ethyl acetate was added thereto, the reaction liquid was filtered through Celite, and the organic layer was separated. After having washed the resulting organic layer with water and brine, which was dried over anhydrous sodium sulfate. The residue was purified by NH silica gel column chromatography (hexane-ethyl acetate) to give 165mg of the title compound.
¹H-NMR(CDCl3)
δ (ppm): 1.35(s,3H), 1.44(s,3H), 1.51-1.57(m,2H), 1.66-1.76(m,4H), 2.76(dd,1H, *J*=13.2Hz,8.4Hz), 2.98-3.06(m,5H), 3.67(dd,1H, *J*=8.0Hz,6.4Hz), 3.76(s,3H), 3.90(dd,1H, *J*=8.4Hz,2.0Hz), 4.32-4.40(m,1H), 6.45-6.84(m,3H).

### (2) Synthesis of (2-methoxy-5-piperidinylphenyl)acetaldehyde

1.358g of 4-(3-piperidinyl-6-methoxybenzyl)-2,2-dimethyl-[1,3]dioxolane was dissolved in 10ml of methanol, 6ml of 4N HCl ethyl acetate solution was added at room temperature. After stirring for 5 hours at room temperature, solvent was removed, methanol was added, and solvent was removed again. 3ml of water was added to the residue, neutralized with saturated sodium bicarbonate aqueous solution and then 15ml tetrahydrofuran was added thereto. 2.115g of sodium periodate was added thereto, which was stirred for 50 minutes at room temperature. Saturated sodium bicarbonate aqueous solution and dichloromethane was added to this reaction liquid, the organic layer was separated. The resulting organic layer was washed with brine and then dried over anhydrous sodium sulfate. After removing the drying agent by filtration, the organic layer was concentrated under a reduced pressure to give 439mg of the title compound. The compound was used in the next reaction without further purification.
¹H-NMR(CDCl3)
δ (ppm): 1.51-1.60(m,2H), 1.67-1.74(m,4H), 3.00-3.06(m,4H), 3.59(s,2H), 3.77(s,3H), 6.76-6.90(m,3H), 9.65(s,1H).

### (3) Synthesis of 1-{2-(2-methoxy-5-piperidinylphenyl)ethyl piperidin-4-yl}-1H-indole-6-carboxamide

94mg of 1-(piperidin-4-yl)-1H-indole-6-carboxamide synthesized in Production example 1 and 439mg of (2-methoxy-5-piperidinylphenyl)acetaldehyde was dissolved in 10ml of dichloromethane, 44µl of acetic acid and 123mg of sodium triacetoxyborohydride were added to reaction liquid, the reaction liquid was stirred overnight at room temperature. Aqueous sodium hydroxide, dichloromethane were added to reaction liquid, and the organic layer was separated. The resulting organic layer was washed with brine and then dried over anhydrous sodium sulfate. After removal of drying agent by filtration, the organic layer was concentrated under a reduced pressure. The residue was purified by NH silica gel column chromatography (ethyl acetate / hexane) to give 164mg of the title compound.
¹H-NMR(CDCl3)
δ (ppm): 1.52-1.62(m,2H), 1.68-1.78(m,4H), 2.07-2.15(m,4H), 2.25-2.35(m,2H), 2.60-2.68(m,2H), 2.80-2.88(m,2H), 3.01-3.05(m,4H), 3.18-3.24(m,2H), 3.80(s,3H), 4.35-4.45-(m,1H), 6.57(d,1H, *J*=3.2Hz), 6.76-6.80(m,2H), 6.86(s,1H), 7.40-7.44(m,2H), 7.65(d,1H, *J*=8.4Hz), 8.11(s,1H) mass spectrum: 461-(M+1).

### Example 109

### Synthesis of 1-[1-[2-{2-methoxy-5-(4-benzyloxycarbonyl piperazin-1-yl)phenyl}ethyl]piperidin-4-yl]-1H-indole-6-carboxamide

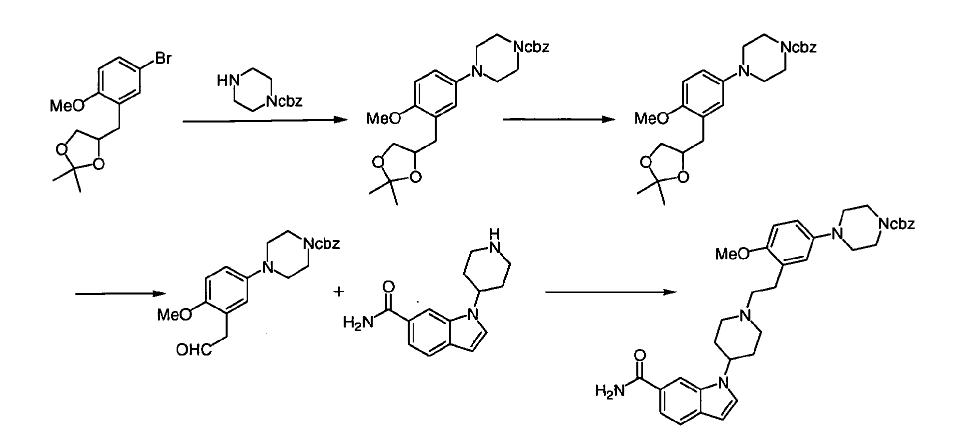

The title compound was obtained by synthesizing in accordance with the methods in example 100.
¹H-NMR(CDCl3)
δ (ppm): 2.08-2.16 (m,4H), 2.25-2.35(m,2H), 2.60-2.68(m,2H), 2.80-2.88(m,2H), 2.98-3.08-(m,4H), 3.18-3.24(m,2H), 3.62-3.70(m,4H), 3.80(s,3H), 4.35-4.45(m,1H), 5.17(s,2H), 6.57 (d,1H, *J*=3.2Hz), 6.76-6.85(m,3H), 7.28-7.43(m,7H), 7.65 (d,1H, *J*=8.0Hz), 8.11 (s,1H).
mass spectrum: 596-(M+1).

### Example 110

### Synthesis of 1-[1-[2-(2-methoxy-6-piperazinylphenyl)ethyl piperidin-4-yl]-1H-indole-6-carboxamide

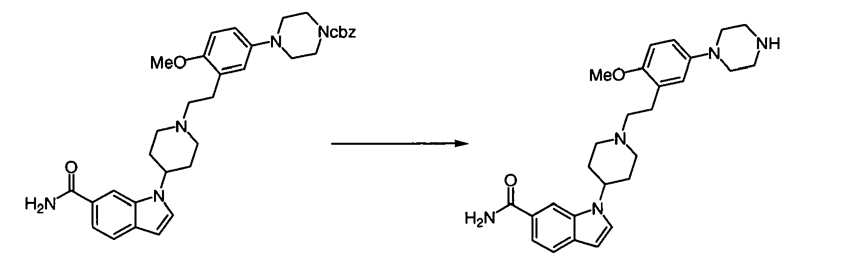

The title compound was obtained by synthesizing in accordance with the methods in example 100.
¹H-NMR(CDCl₃)
δ (ppm): 2.08-2.20(m,4H), 2.25-2.38(m,2H), 2.62-2.70(m,2H), 2.80-2.90(m,2H), 3.00-3.16(m,8H), 3.18-3.28(m,2H), 3.80(s,3H), 4.35-4.45(m,1H), 6.59(d,1H, *J*=2.8Hz), 6.75-6.88(m,3H), 7.38-3.46(m,2H), 7.65(d,1H, *J*=8.4Hz), 8.10(s,1H).

### Example 111

### Synthesis of 1-[1-[2-{2-methoxy-6-(4-acetylpiperazin-1-yl) phenyl}ethyl]piperidin-4-yl]-1H-indole-6-carboxamide

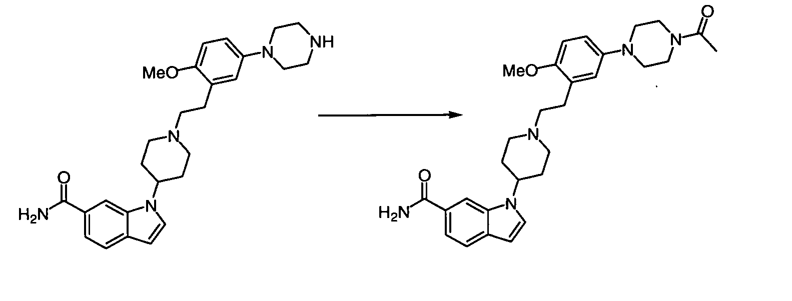

The title compound was obtained by synthesizing in accordance with the methods in example 101.
¹H-NMR(CDCl3)
5 (ppm): 2.09-2.15(m,4H), 2.15(s,3H), 2.26-2.36(m,2H), 2.62-2.68(m,2H), 2.81-2.86(m,2H), 3.01-3.10(m,4H), 3.18-3.25(m,2H), 3.60-3.65(m,2H), 3.76-3.80(m,2H), 3.81(s,3H), 4.35-4.45(m,1H), 6.58(d,1H, *J*=3.2Hz), 6.78-6.80(m,2H), 6.85(d,1H, *J*=2.8Hz), 7.39-7.43(m, 2H), 7.65(d,1H, *J*=8.4Hz), 8.12 (s,1H).
mass spectrum: 504-(M+1).

### Example 112

### Synthesis of 1-[1-[2-{2-methoxy-6-(4-methyl piperazin-1-yl) phenyl}ethyl]piperidin-4-yl]-1H-indole-6-carboxamide

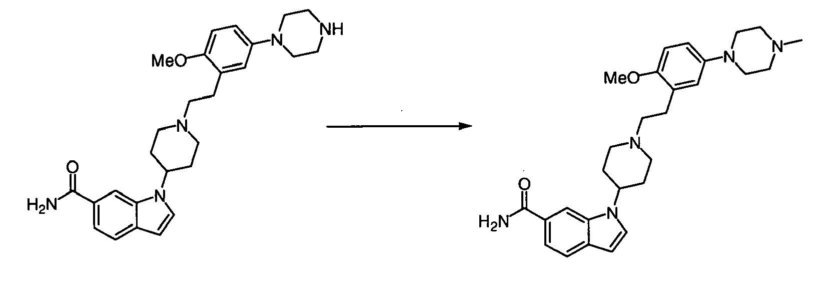

The title compound was obtained by synthesizing in accordance with the methods in example 102.
¹H-NMR(CDCl3)
δ (ppm): 2.06-2.20(m,4H), 2.25-2.35(m,2H), 2.36(s,3H), 2.58-2.63(m,4H), 2.63-2.68(m,2H), 2.80-2.88(m,2H), 3.10-3.15(m,4H), 3.21-3.25(m,2H), 3.80(s,3H), 4.35-4.45(m,1H), 6.58-(d,1H, *J*=3.2Hz), 6.75-6.86(m,3H), 7.38-7.45(m,2H), 7.65(d,1H, *J*=8.4Hz), 8.11(s,1H).
mass spectrum: 476-(M+1).

### Example 113

### Synthesis of 1- [1- (2- [2-{(1-acetylpiperidin-4-yl)methylamino}-6-methoxyphenyl)ethyl)piperidin-4-yl]-1H-indole-6-carboxamide

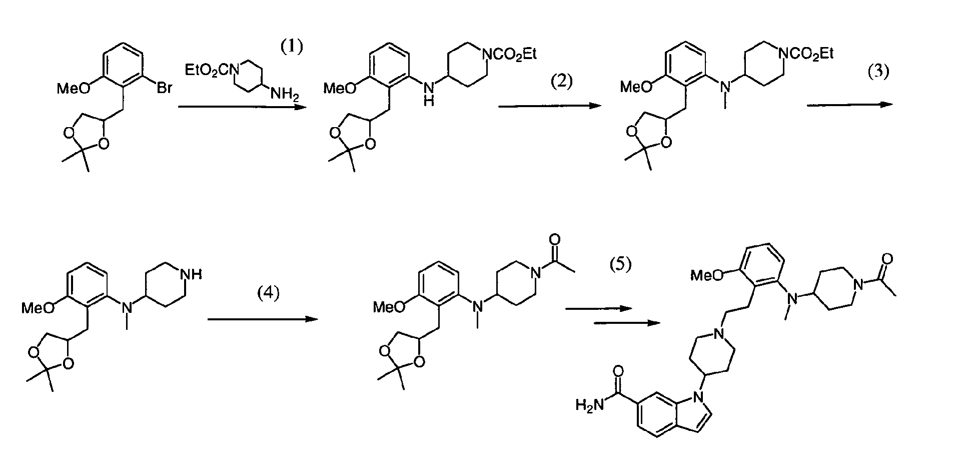

### (1) Synthesis of 4-[2-(2,2-dimethyl-[1,3]dioxolan-4-ylmethyl) ethyl-3-methoxyphenylamino]piperidine-1-carboxylate

489mg of 4-(2-bromo-6-methoxybenzyl)-2,2-dimethyl-[1,3]dioxolane synthesized in Production example 4, 418µl of 4-amino-1-carboethoxypiperazine, 87mg of tris (dibenzylideneacetone)dipalladium and 117mg of (±) 2,2'-bis(biphenylphosphino)-1,1'-binaphthyl suspended in 5ml of toluene, 242mg of sodium *tert*-butoxide was added at room temperature. This reaction liquid was heated with stirring at 90 °C for 4 hours under nitrogen atmosphere. After standing to cool, water and ethyl acetate was added thereto, Celite was filtered. The resulting organic layer was washed with water and brine, and then dried over anhydrous sodium sulfate. After removal of drying agent by filtration, the organic layer was concentrated under a reduced pressure. The residue was purified by silica gel column chromatography (hexane-ethyl acetate) to give 438mg of the title compound.
¹H-NMR(CDCl3)
δ (ppm): 1.27(t,3H, *J*=6.8Hz), 1.31(s,3H), 1.37(s,3H), 1.33-1.50(m,2H), 1.98-2.08(m,2H), 2.60-2.68(m, 1H), 3.04-3.12 (m, 3H), 3.42-3.52 (m, 1H), 3.57-3.62 (m, 1H), 3.77 (s, 3H), 3.96-4.18(m,5H), 4.22-4.32(m,1H), 4.70-4.76(br,1H), 6.32(d,1H, J=8.4Hz), 6.35(d,1H, *J*=8.0Hz), 7.09(dd,1H, J=8.4Hz,8.OHz).

### (2) Synthesis of 4-{[2-(2,2-dimethyl-[1,3]dioxolan-4-ylmethyl) ethyl-3-methoxyphenyl]methylamino}piperidine-1-carboxylate

438mg of 4-[2-(2,2-dimethyl-[1,3]dioxolan-4-ylmethyl)ethyl-3-methoxyphenylamino]piperidine-1-carboxylate, 238ul of methyl iodide were dissolved in 5ml of N, N-dimethylformamide, 94mg of 60% sodium hydride was added at room temperature. This reaction liquid was heated with stirring at 70 °C for 2 days. After standing to cool, water and ethyl acetate was added thereto, the organic layer was separated. The resulting organic layer was washed with brine and then dried over anhydrous sodium sulfate. After removal of drying agent by filtration, the organic layer was concentrated under a reduced pressure. The residue was purified by NH silica gel column chromatography (hexane-ethyl acetate) to give 271mg of the title compound.

### (3) Synthesis of 4-{[2-(2,2-dimethyl-[1,3]dioxolan-4-ylmethyl) -3-methoxyphenyl]methylamino}piperidine

271mg of 4-{[2-(2,2-dimethyl-[1,3]dioxolan-4-ylmethyl) ethyl-3-methoxyphenyl]methylamino}piperidine-1-carboxylate was dissolved in 5ml of methanol, 2ml of 5N aqueous sodium hydroxide was added at room temperature. This reaction liquid was heated with stirring at 90 °C for 1 day. After standing to cool, water and ethyl acetate was added thereto, the organic layer was separated. The resulting organic layer was washed with brine and then dried over anhydrous sodium sulfate. After removing the drying agent by filtration, the organic layer was concentrated under a reduced pressure to give 221mg of the title compound. The compound was used in the next reaction without further purification.

### (4) Synthesis of 1-acetyl-4-{[2-(2,2-dimethyl-[1,3]dioxolan -4-ylmethyl)-3-methoxyphenyl]methylamino}piperidine

221mg of 4-{[2-(2,2-dimethyl-[1,3]dioxolan-4-ylmethyl)-3-methoxyphenyl]methylamino}piperidine, 60µl of acetic acid and 240µl of diisopropylethylamine were dissolved in 5ml of dichloromethane, 473mg of benzotriazol-1-yloxy tris(dimethylamino)phosphonium hexafluorophosphate was added thereto at room temperature. This reaction liquid was stirred for 1.5 hours at room temperature, and dichloromethane was evaporated under a reduced pressure. Water and ethyl acetate was added to the residue, the organic layer was separated. The resulting organic layer was washed with brine and then dried over anhydrous sodium sulfate. After removal of drying agent by filtration, the organic layer was concentrated under a reduced pressure. The residue was purified by NH silica gel column chromatography (hexane-ethyl acetate) to give 189mg of the title compound.

### (5) Synthesis of 1-[1-(2-[2-{(1-acetylpiperidin-4-yl) methylamino}-6-methoxyphenyl)ethyl)piperidin-4-yl]-1H-indole-6-carboxamide

The title compound was obtained from 1-acetyl-4-{[2-(2,2-dimethyl-[1,3]dioxolan-4-ylmethyl)-3-methoxyphenyl]methylamino}piperidine in accordance with the methods in example 108.
¹H-NMR(CDCl3)
δ (ppm): 1.42-1.55(m,2H), 1.78-1.88(m,2H), 2.09(s,3H), 2.07-2.20(m,4H), 2.26-2.34(m,2H), 2.54-2.64(m,2H), 2.63(s,3H), 2.82-3.08(m,4H), 3.20-3.28(m,2H), 3.72-3.84(m,2H), 3.84(s,3H), 4.33-4.43 (m,1H), 4.50-4.58 (m,1H), 6.57 (d,1H, *J*=3.6Hz), 6.70 (d,1H, *J*=7.6Hz), 6.83 (d,1H, *J*=8.0Hz), 7.17(dd,1H, *J*=8.0Hz, 7.6Hz), 7.39-7.45 (m,2H), 7.65 (d,1H, *J*=8.4Hz), 8.11(s,1H).
mass spectrum: 532-(M+1).

### Example 114

### Synthesis of 1-(1-{2-{2-methoxy-5-(3-oxopiperazin-1-ylmethyl) phenyl}ethyl}piperidin-4-yl)-1H-indole-6-carboxamide

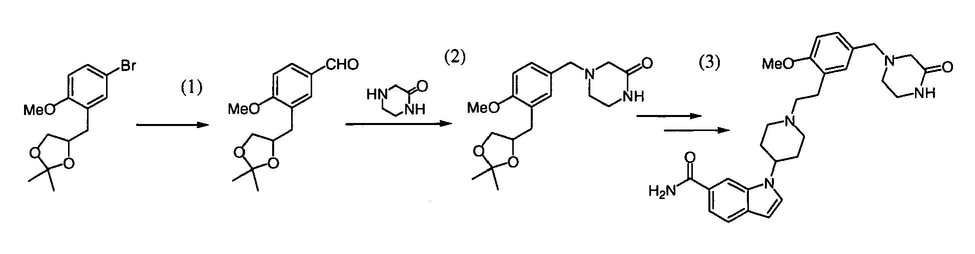

### (1) Synthesis of 3-(2,2-dimethyl-[1,3]dioxolan-4-ylmethyl)-4-methoxybenzaldehyde

1.1g of 4-(3-bromo-6-methoxybenzyl)-2,2-dimethyl-[1,3] dioxolane synthesized in Production example 6 was dissolved in 15ml of anhydrous tetrahydrofuran, under nitrogen atmosphere, 3.3ml of *n-*butyllithium (1.6M hexane solution) was added dropwise at -70 °C. After dropping, stirring was carried out for 40 minutes at -70 °C, N, N-dimethylformamide 540ul was then added dropwise at -70 °C. After completion of dropping, dry ice-acetone bath was removed, which was stirred for 1 hour at room temperature. Water and ethyl acetate were added to reaction liquid, and the organic layer was separated. The resulting organic layer was washed with brine and then dried over anhydrous sodium sulfate. After removal of drying agent by filtration, the organic layer was concentrated under a reduced pressure. The residue was purified by silica gel column chromatography (hexane-ethyl acetate) to give 621mg of the title compound.
¹H-NMR(CDCl3)
δ (ppm): 1.35(s,3H), 1.43(s,3H), 2.89 (dd,1H, *J*=13.6Hz,6.8Hz), 3.01(dd,1H, *J*=13.6Hz,6.4Hz), 3.66 (dd,1H, J=12.0Hz,6.4Hz), 3.92(s,3H), 3.98(dd,1H, *J*=12.0Hz, 6.0Hz), 4.39(dddd,1H, *J*=6.8Hz, 6.4Hz, 6.4Hz, 6.0Hz), 6.97(d,1H, *J*=8.4Hz), 7.74(d,1H, J=2.4Hz), 7.77 (dd, 1H, *J*=8.4Hz, 2.4Hz), 9.88(s, 1H).

### (2) Synthesis of 4-[3-(2,2-dimethyl-[1,3]dioxolan-4-ylmethyl)-4-methoxy benzyl]piperazin-2-one

621mg of 3-(2,2-dimethyl-[1,3]dioxolan-4-ylmethyl)-4-methoxybenzaldehyde, 376mg of piperazin-2-one and 284µl of acetic acid were dissolved in 10ml of dichloromethane, 794mg of sodium triacetoxyborohydride was added thereto, and the reaction liquid was stirred overnight at room temperature. Aqueous sodium hydroxide and dichloromethane were added to the reaction liquid, and the organic layer was separated. The resulting organic layer was washed with brine and then dried over anhydrous sodium sulfate. After removal of drying agent by filtration, the organic layer was concentrated under a reduced pressure. The residue was purified by NH silica gel column chromatography (hexane-ethyl acetate / methanol) to give 776mg of the title compound.
¹H-NMR(CDCl3)
δ (ppm): 1.35(s,3H), 1.43(s,3H), 2.60-2.65(m,2H), 2.80(dd,1H, *J*=13.6Hz, 7.2Hz), 3.01(dd,1h, J=13.6Hz,5.6hz), 3.14(s,2H), 3.32-3.37(m,2H), 3.50(s,2H), 3.65(dd,1H, *J*=8.4Hz,6.8Hz), 3.81(s,3H), 3.92(dd,1H, *J*=8.4Hz, 6.0Hz), 4.36(dddd,1H, *J*=7.2Hz, 6.8Hz, 6.0Hz,5.6Hz), 6.00(br,1H), 6.79(d,1H, *J*=8.4Hz), 7.10-7.15(m,2H).

### (3) Synthesis of 1-(1-{2-{2-methoxy-5-(3-oxopiperazin-1-ylmethyl)phenyl)ethyl}piperidin-4-yl)-1H-indole-6-carboxamide

The title compound was obtained by synthesizing from 4-[3-(2,2-dimethyl-[1,3]dioxolan-4-ylmethyl)-4-methoxybenzyl] piperazin-2-one in accordance with the methods in example 108.
¹H-NMR(CDCl3)
δ (ppm): 2.08-2.20(m,4H), 2.24-2.34(m,2H), 2.60-2.70(m,4H), 2.83-2.90(m,2H), 3.15(s,2H), 3.18-3.25(m,2H), 3.33-3.40(m,2H), 3.52 (s,2H), 3.84 (s,3H), 4.34-4.44 (m,1H), 5.85 (br, 1H), 6.57 (d, 1H, *J*=3.2Hz), 6.81 (d, 1H, *J*=8.8Hz), 7.10-7.14 (m, 2H), 7.40-7.44 (m,2H), 7.65 (d,1H, *J*=8.0Hz), 8.11 (s,1H).
mass spectrum: 490-(M+1).

### Example 115

### Synthesis of 1-(1-{2-{2-methoxy-6-(3-oxopiperazin-1-ylmethyl) phenyl}ethyl}piperidin-4-yl)-1H-indole-6-carboxamide

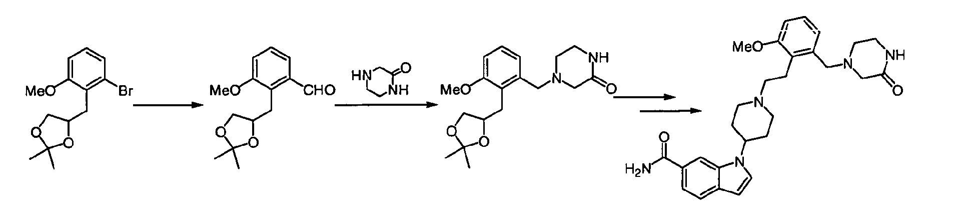

The title compound was obtained by synthesizing from 4-(2-bromo-6-methoxybenzyl)-2,2-dimethyl-[1,3]dioxolane synthesized in Production example 4 in accordance with the methods in example 114.
¹H-NMR(CDCl3)
5 (ppm): 2.08-2.22(m,4H), 2.26-2.36(m,2H), 2.56-2.70(m,4H), 2.94-3.03(m,2H), 3.18(s,2H), 3.20-3.27(m,2H), 3.33-3.39(m,2H), 3.58(s,2H), 3.85 (s,3H), 4.32-4.43(m,1H), 5.90(br,1H), 6*.*57(d,1H, *J*=3.2Hz), 6.84(d,1H, *J*=7.6Hz), 6.87(d,1H, *J*=8.0Hz), 7.15(dd,1H, *J*=8.0Hz,7.6Hz), 7.40-7.45(m,2H), 7.65(d,1H, *J*=8.4Hz), 8.10(s,1H).
mass spectrum: 490-(M+1).

### Example 116

### Synthesis of 1-[1-(2-{2-[4-acetylmethylaminopiperidin-1-yl]-6-methoxyphenyl}ethyl)piperidin-4-yl]-1H-indole-6-carboxamide

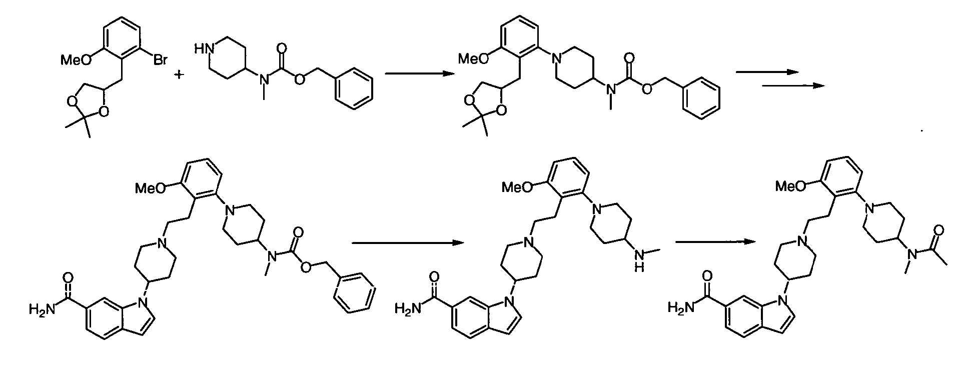

The title compound was obtained by synthesizing from 4-benzyloxycarbonylmethylaminopiperidine synthesized in Production example 7 in accordance with the methods in example 101.
¹H-NMR(CDCl3)
δ (ppm): 1.65-1.95(m,4H), 2.03-2.20(m,7H), 2.22-2.30(m,2H), 2.58-2.66(m,2H), 2.78-3.15-(m,9H), 3.25-3.35(m,2H), 3.83(s,3H), 4.36-4.44(m,1H), 4.59-4.66(m,1H), 6.57(d,1H, *J*=3.6Hz), 6.68(dd,1H, *J*=8.0Hz,7.6Hz), 6.79(d,1H, *J*=7.6Hz), 7.14-7.20(m,1H), 7.36-7.44(m,2H), 7.64(d,1H, *J*=8.4Hz), 8.12(s,1H).
mass spectrum: 532-(M+1).

### Example 117

### Synthesis of 1-{1-[2-(4-methoxy-6-oxo-1-phenyl-1,6-dihydropyridin-3-yl)ethyl]piperidin-4-yl}-1H-indole-6-carboxamide

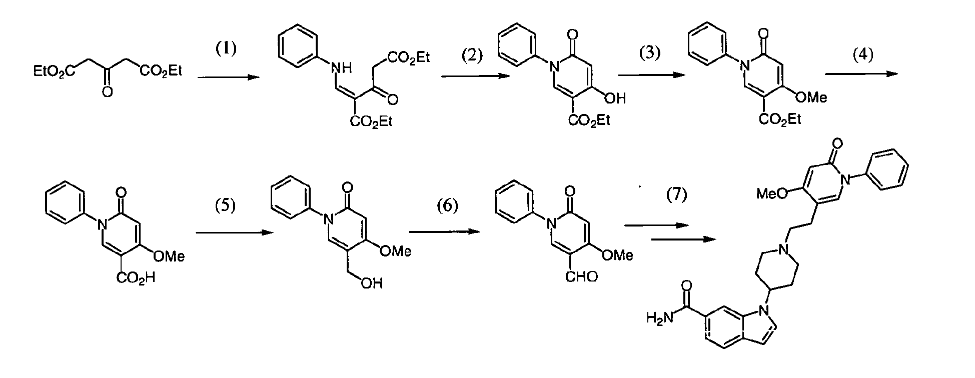

### (1) Synthesis of 1,3-diethyl 1-(phenylaminomethylidene)acetone dicarboxylate

A mixture of 3.42g of 1,3-diethyl acetonedicarboxylate, 1. 84g of aniline, 4. 65g of triethyl orthoformate and 4ml of acetic acid was heated to reflux for 4 hours. This reaction liquid was concentrated under a reduced pressure, the residue was purified by silica gel column chromatography (hexane-ethyl acetate) to give 4.09g of the title compound. The compound was used in the next reaction without further purification.

### (2) Synthesis of ethyl 4-hydroxy-6-oxo-1-phenyl-1,6-dihydro pyridine-3-carboxylate

20ml of polyphosphoric acid was added to 3.49g of 1,3-diethyl 1-(phenylaminomethylidene)acetone dicarboxylate, which was heated with stirring for 6 hours. Iced water and ethyl acetate was added to reaction liquid, sodium bicarbonate powder was then added thereto. The organic layer was separated and the resulting organic layer was washed with brine and then dried over anhydrous sodium sulfate. After removing the drying agent by filtration, the organic layer was concentrated under a reduced pressure, and the residue was purified by silica gel column chromatography (hexane-ethyl acetate) to give 1.74g of the title compound.
¹H-NMR (CDCl3)
δ (ppm): 1.36(t,3H, J=7.2Hz), 4.37(q,2H, J=7.2Hz), 6.02 (s,1H), 7.34-7.37(m,2H), 7.45-7.53(m,3H), 8.18(s,1H).

### (3) Synthesis of ethyl 4-methoxy-6-oxo-1-phenyl-1,6-dihydro pyridine-3-carboxylate

1.74g of ethyl 4-hydroxy-6-oxo-1-phenyl-1,6-dihydro pyridine-3-carboxylate was dissolved in 20ml of N, N-dimethylformamide, 1.4g of potassium carbonate and 630µl of methyl iodide were added at room temperature. This reaction liquid was stirred overnight at room temperature. Water and ethyl acetate were added to reaction liquid, and the organic layer was separated. The resulting organic layer was washed with brine and then dried over anhydrous sodium sulfate. After removing the drying agent by filtration, the organic layer was concentrated under a reduced pressure, and the residue was purified by NH silica gel column chromatography (hexane-ethyl acetate) to give 833mg of the title compound.
¹H-NMR(CDCl3)
δ (ppm): 1.33(t,3H, J=7.2Hz), 3.91(s,3H), 4.29(q,2H, *J*=7.2Hz), 5.99(s,1H), 7.35-7.38(m,2H), 7.45-7.51(m,3H), 8.13(s,1H).

### (4) Synthesis of 4-methoxy-6-oxo-1-phenyl-1,6-dihydropyridine-3-carbonxylic acid

544mg of ethyl 4-methoxy-6-oxo-1-phenyl-1,6-dihydropyridine-3-carboxylate was dissolved in 20ml of methanol, 3ml of 5N aqueous sodium hydroxide was added at room temperature. This reaction liquid was heated at 80 °C with stirring for 2 hours. After standing to cool, 5N HCl was added thereto, the precipitate was collected by filtration to give 426mg of the title compound. The compound was used in the next reaction without further purification.

### (5) Synthesis of 5-hydroxymethyl-4-methoxy-1-phenyl-1H-pyridin-2-one

426mg of 4-methoxy-6-oxo-1-phenyl-1,6-dihydropyridine-3-carbonxylic acid was dissolved in 20ml of anhydrous tetrahydrofuran, 5.2ml of diborane (1.0M tetrahydrofuran solution) was added dropwise in an ice-cooling. An ice bath was removed, which was stirred overnight at room temperature. Methanol was added to this reaction liquid, concentrated under a reduced pressure, and the residue was purified by NH silica gel column chromatography (ethyl acetate / methanol) to give 215mg of the title compound.
¹H-NMR(CDCl3)
δ (ppm): 2.04(br,1H), 3.87(s,3H), 4.47(s,2H), 6.01(s,1H), 7.26(s,1H), 7.32-7.46(m,5H).

### (6) Synthesis of 4-methoxy-6-oxo-1-phenyl-1,6-dihydropyridine-3-carbaldehyde

215mg of 5-hydroxymethyl-4-methoxy-1-phenyl-1*H*-pyridin-2-one was dissolved in 5ml of dimethyl sulfoxide, 650µl of triethylamine and 776mg of sulfur trioxide / pyridine complex were added followed by stirring for 2.5 hours at room temperature. Water and ethyl acetate were added, and the organic layer was separated. The resulting organic layer was washed with brine and then dried over anhydrous sodium sulfate. After removing the drying agent by filtration, the organic layer was concentrated under a reduced pressure, and the residue was purified by silica gel column chromatography (hexane-ethyl acetate) to give 54mg of the title compound.
¹H-NMR(CDCl3)
δ (ppm): 3.94(s,3H), 5.98(s,1H), 7.25-7.35(m,2H), 7.45-7.50(m,3H), 8.08(s,1H), 10.03(s,1H).

### (7) Synthesis of 1-{1-[2-(4-methoxy-6-oxo-1-phenyl-1,6-dihydropyridin-3-yl)-ethyl]-piperidin-4-yl}-1H-indole-6-carboxamide

The title compound was obtained by synthesizing from 4-methoxy-6-oxo-1-phenyl-1,6-dihydropyridine-3-carbaldehyde in accordance with the methods in example 56.
¹H-NMR(CDCl3)
δ (ppm): 2.02-2.12(m,4H), 2.23-2.30(m,2H), 2.58-2.65(m,4H), 3.11-3.18(m,2H), 3.86(s,3H), 4.35-4.45(m, 1H), 6.00(s, 1H), 6.57(d,1H, J=3.2Hz), 7.13(s,1H 7.36-7.41(m,5H), 7.46-7.48(m,2H), 7.63(d, 1H, *J*=7.6Hz), 8.10(s, 1H).
mass spectrum: 471-(M+1).

### Example 118

### Synthesis of 1-{1-[2-(4,6-dimethoxypyridin-3-yl)ethyl] piperidin-4-yl}-1H-indole-6-carboxamide

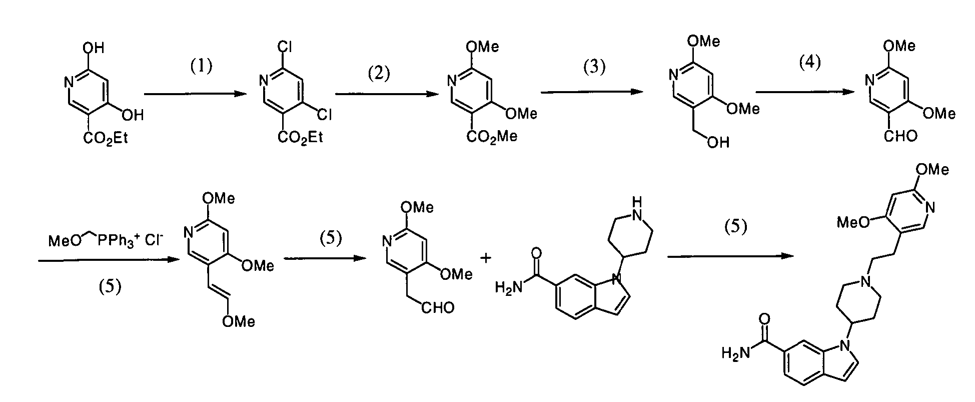

### (1) Synthesis of ethyl 4,6-dichloropyridine-3-carboxylate

To 3.43g of ethyl 4,6-dihydroxypyridine-3-carboxylate synthesized according to the known literature *(J. Heterocyclic.* Chem., 20, 1363- (1983)) was added 15ml of phosphorus oxychloride at room temperature, this reaction liquid was heated to reflux for 3 hours. After standing to cool, removal under a reduced pressure was done of phosphorus oxychloride, the residue was poured to iced water, which was stirred at room temperature. Diethyl ether was added to reaction liquid, and the organic layer was separated. The resulting organic layer was washed with brine and then dried over anhydrous sodium sulfate. After removing the drying agent by filtration, the organic layer was concentrated under a reduced pressure, and the residue was purified by silica gel column chromatography (hexane-ethyl acetate) to give 3.49g of the title compound.
¹H-NMR(CDCl3)
δ (ppm): 1.41(t,3H, *J=*8.0Hz), 4.42(q,2H, *J*=8.0Hz), 7.44(s,1H), 8.83(s,1H).

### (2) Synthesis of methyl 4,6-dimethoxy pyridine-3-carboxylate

3.49g of ethyl 4,6-dichloropyridine-3-carboxylate was dissolved in 20ml of methanol, 15ml of sodium methoxide (28% methanol solution) was added at room temperature, which was heated to reflux for 1.5 hours. After standing to cool, removal under reduced pressure was done of methanol, water and ethyl acetate were added, and the organic layer was separated. The resulting organic layer was washed with brine and then dried over anhydrous sodium sulfate. After removing the drying agent by filtration, the organic layer was concentrated under a reduced pressure to give 733mg of the title compound.
In addition, the aqueous layer after having extracted with ethyl acetate was acidified by adding 5N HCl, mixed solvent of the 10% methanol-chloroform was added, and the organic layer was separated. The resulting organic layer was washed with brine and then dried over anhydrous sodium sulfate. After removing the drying agent by filtration, the organic layer was concentrated under a reduced pressure to yield 1. 85g of 4, 6-dimethoxynicotinic acid. The compound was dissolved in 30 ml of N, N-dimethylformamide, 1.85g of potassium carbonate and 1.24ml of dimethyl sulfate were added, which was stirred for 3.5 hours at room temperature. This reaction liquid was poured into iced water, ethyl acetate was added, and the organic layer was separated. The resulting organic layer was washed with brine and then dried over anhydrous sodium sulfate. After removing the drying agent by filtration, the organic layer was concentrated under a reduced pressure to give 1.96g of the title compound.
¹H-NMR (CDCl3)
δ (ppm): 3.84(s,3H), 3.88(s,3H), 3.96(s,3H), 6.21(s,1H), 8.60(s,1H).

### (3) Synthesis of (4,6-dimethoxypyridin-3-yl)methanol

To a suspension of 865mg of lithium aluminum hydride in 30ml of anhydrous tetrahydrofuran was added a solution of 2.69g of methyl 4,6-dimethoxypyridine-3-carboxylate in anhydrous 15ml of tetrahydrofuran in an ice-cooling. This reaction liquid was stirred for 1 hour in an ice-cooling. To the reaction liquid were sequentially added 865µl of water, 865µl of 5N aqueous sodium hydroxide, 2.6ml of water in an ice-cooling, which was stirred. After an insoluble material was filtered, and then removed, solvent was concentrated under a reduced pressure to give 2.29g of the title compound.
¹H-NNR (CDCl3)
δ (ppm): 3.87(s,3H), 3.92(s,3H), 4.59(s,2H), 6.20 (s,1H), 7.91(s,1H).

### (4) Synthesis of 4,6-dimethoxypyridine-3-carbaldehyde

2.29g of (4,6-dimethoxypyridin-3-yl)methanol was dissolved in 30ml of toluene, 2g of manganese dioxide was added. This reaction liquid was stirred overnight at room temperature. After an insoluble material was removed by filtration, solvent was concentrated under a reduced pressure, and the residue was purified by silica gel column chromatography (hexane-ethyl acetate) to give 300mg of the title compound (and starting material recovery 1.59g).
¹H-NMR (CDCl3)
δ (ppm): 3.93(s,3H), 4.00(s,3H), 6.23 (s,1H), 8.55 (s,1H), 10.23 (s,1H).

### (5) Synthesis of 1-{1-[2-(4,6-dimethoxypyridin-3-yl)ethyl] piperidin-4-yl}-1H-indole-6-carboxamide

The title compound was obtained by synthesizing from (4,6-dimethoxypyridin-3-yl)aldehyde in accordance with the methods in example 56.
¹H-NMR(CDCl3)
δ (ppm): 2.08-2.12(m,4H), 2.24-2.31(m,2H), 2.57-2.61(m,2H), 2.72-2.76(m,2H), 3.16-3.20(m,2H), 3.84(s,3H), 3.91(s,3H), 4.32-4.42 (m,1H), 6.18 (s,1H), 6.56 (d,1H, *J*=2.8Hz), 7.38 (d, 1H, *J*=2.8Hz), 7.39 (d,1H, *J*=8.0Hz), 7.63 (d,1H, *J*=8.0Hz), 7.82(s, 1H), 8.09 (s,1H).
mass spectrum: 409-(M+1), 431-(M+23).

### Example 119

### Synthesis of 1-{1-[2-(3,6-dimethoxypyridin-2-yl)ethyl] piperidin-4-yl}-1H-indole-6-carboxamide

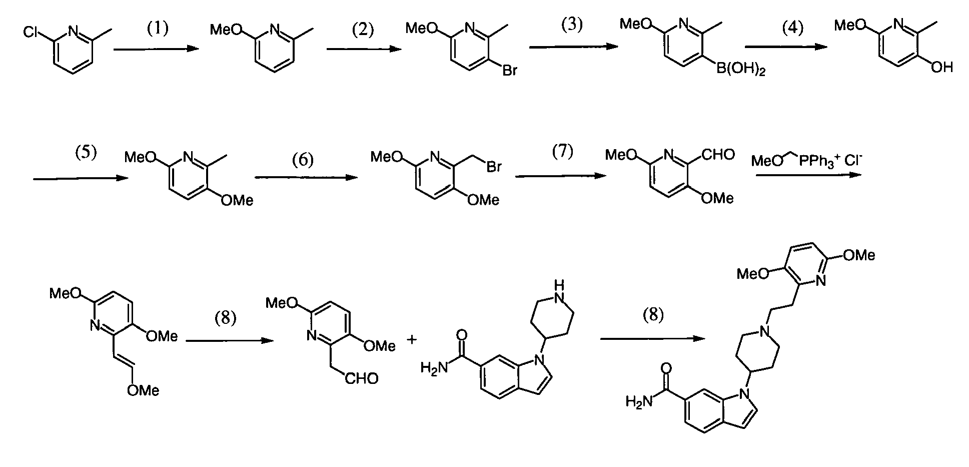

### (1) 2-Methoxy-6-methylpyridine

100ml of sodium methoxide (28% methanol solution) was added to 20.lg of 2-chloro-6-methylpyridine at room temperature, this reaction liquid was heated to reflux for 2 days. After standing to cool, water and ethyl acetate were added thereto, and the organic layer was separated. The resulting organic layer was washed with brine and then dried over anhydrous sodium sulfate. After removing the drying agent by filtration, the organic layer was concentrated under a reduced pressure to give 16.8g of the title compound.
¹H-NMR (CDCl₃)
δ (ppm) : 2.44(s,3H), 3.90(s,3H), 6.51(d, 1H, *J*=8.4Hz), 6.68(d, 1H, *J*=8.0Hz), 7.43(dd, 1H, *J*=8.4Hz,8.0Hz).

### (2) Synthesis of 3-bromo-6-methoxy-2-methylpyridine

15.3g of 2-methoxy-6-methylpyridine was dissolved in 100ml of potassium bromide aqueous solution, prepared that 50.1g of potassium bromide was dissolved in 200ml of water, 11.9g of potassium hydroxide and 40g of tetraethylammonium chloride were added. The solution of 7.6ml of bromine in 100ml of potassium bromide aqueous solution was added in on ice-cooling to the reaction liquid using a dropping funnel for 40 minutes. After completion of dropping, an ice bath was removed, which was stirred for 17 hours at room temperature. Sodium sulfite aqueous solution and ethyl acetate were added to reaction liquid, and the organic layer was separated. The resulting organic layer was washed with brine and then dried over anhydrous magnesium sulfate. After removing the drying agent by filtration, the organic layer was concentrated under a reduced pressure to give 19.6g of the title compound.
¹H-NMR(CDCl3)
δ (ppm) : 2.56 (s,3H), 3.89 (s, 3H), 6.45 (d, 1H, J=8.0Hz), 7.60(d, 1H, *J*=8.0Hz).

### (3) The synthesis of 6-methoxy-2-methylpyridine-3-boronic acid

21.2g of 3-bromo-6-methoxy-2-methylpyridine was dissolved in 100ml of anhydrous tetrahydrofuran, 79ml of *n-*butyllithium (1.6M hexane solution) was added dropwise for 30 minutes at -70 °C under nitrogen atmosphere. After dropping, stirring was carried out for 30 minutes at -70 °C, a solution of 37ml of triisopropyl borate in 50ml of anhydrous tetrahydrofuran was added dropwise for 40 minutes at -70 °C. After completion of dropping, dry ice-acetone bath was removed, which was stirred for 16 hours at room temperature. Afterwards, 12ml of acetic acid was added at room temperature followed by stirring for another hour. Water and ethyl acetate were added to reaction liquid, and the organic layer was separated. The resulting organic layer was washed with brine and then dried over anhydrous magnesium sulfate. After removing the drying agent by filtration, the organic layer was concentrated under a reduced pressure, and recrystallized from diethyl ether to give 6.31g of the title compound.

### (4) Synthesis of 6-methoxy-2-methylpyridin-3-ol

2.33g of 6-methoxy-2-methylpyridin-3-boronic acid was dissolved in 50ml of water, 1.6ml of hydrogen peroxide (30% aqueous solution) was added at room temperature, which was stirred for 5 hours at room temperature. Methanol-chloroform mixed solvent was added to reaction liquid, the organic layer was separated. The resulting organic layer was washed with brine and then dried over anhydrous sodium sulfate. After removing the drying agent by filtration, the organic layer was concentrated under a reduced pressure to give 1.66g of the title compound.

### (5) Synthesis of 3,6-dimethoxy-2-methylpyridine

6-methoxy-2-methylpyridin-3-ol was dissolved in 30ml of N, N-dimethylformamide, 2.5g of potassium carbonate and 1.1ml of methyl iodide were added at room temperature. This reaction liquid was stirred for 4 hours at room temperature. Water and ethyl acetate were added to reaction liquid, and the organic layer was separated. The resulting organic layer was washed with brine and then dried over anhydrous sodium sulfate. After removing the drying agent by filtration, the organic layer was concentrated under a reduced pressure, the residue was purified by silica gel column chromatography (hexane-ethyl acetate) to give 1.6g of the title compound.
¹H-NMR(CDCl3)
δ (ppm) : 2.39(s, 3H), 3.78(s, 3H), 3.88(s, 3H), 6.53(d, 1H, *J*=8.8Hz), 7.13(d, 1H, *J*=8.8Hz).

### (6) Synthesis of 2-bromomethyl-3,6-dimethoxypyridine

1.6g of 3,6-dimethoxy-2-methylpyridine was dissolved in 30ml of carbon tetrachloride, 2g of N-bromosuccinimide and 1.2ml of acetic acid were added. This reaction liquid was heated to reflux by infrared lamp irradiation for 30 minutes. After standing to cool, an insoluble material was removed by filtration, and concentrated under a reduced pressure. Saturated sodium bicarbonate aqueous solution and ethyl acetate were added to the residue, and the organic layer was separated. The resulting organic layer was washed with brine and then dried over anhydrous sodium sulfate. After removing the drying agent by filtration, the organic layer was concentrated under a reduced pressure to give 2.5g of the title compound. The compound was used in the next reaction without further purification.

### (7) Synthesis of 3,6-dimethoxypyridine-2-carbaldehyde

521mg of 2-bromomethyl-3,6-dimethoxypyridine was dissolved in 5ml of dimethyl sulfoxide, 252mg of sodium bicarbonate and 511mg of sodium iodide were added, which was heated at 100 °C with stirring for 1.5 hours. Water and ethyl acetate were added to reaction liquid, and the organic layer was separated. The resulting organic layer was washed with brine and then dried over anhydrous sodium sulfate. After removing the drying agent by filtration, the organic layer was concentrated under a reduced pressure, the residue was purified by silica gel column chromatography (hexane-ethyl acetate) to give 154mg of the title compound.
¹H-NMR(CDCl3)
δ (ppm) : 3.94(s,3H), 3.98(s,3H), 6.99 (d,1H,*J*=8. 8Hz), 7.43(d,1H, J=8.8Hz), 10.26(s,1H).

### (8) Synthesis of 1-{1-[2-(3,6-dimethoxypyridin-2-yl)ethyl] piperidin-4-yl}-1H-indole-6-carboxamide

The title compound was obtained by synthesizing from 3,6-dimethoxypyridine-2-carbaldehyde in accordance with the methods in example 56.
¹H-NMR(CDCl3)
δ (ppm): 2.05-2.18(m,4H), 2.28-2.38(m,2H), 2.83-2.87(m,2H), 2.98-3.02(m,2H), 3.20-3.28(m,2H), 3.80(s,3H), 3.89(s,3H), 4.32-4.42(m,1H), 6.55-6.57(m,2H), 7.17(d,1H, *J*=8.8Hz), 7.37-7.43(m,2H), 7.64(d,1H, *J*=8.8Hz), 8.11(br,1H) mass spectrum: 409-(M+1).

### Example 120

### Synthesis of 1-{1-[2-(2,4-dimethoxypyridin-3-yl)ethyl] piperidin-4-yl}-1H-indole-6-carboxamide

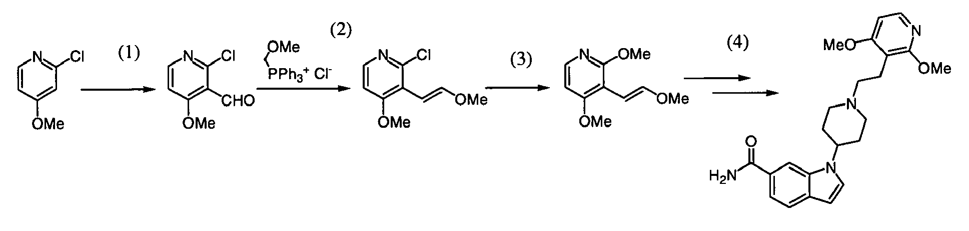

### (1) Synthesis of 2-chloro-4-methoxypyridine-3-carbaldehyde

5.61g of 2-chloro-4-methoxypyridine was dissolved in 100ml of anhydrous tetrahydrofuran, 40ml of *n-*butyllithium (1. 6M hexane solution) was added dropwise at -70 °C for 20 minutes under nitrogen atmosphere. Stirring was carried out for 20 minutes at -70 °C after dropping, N, N-dimethylformamide 6.2ml was dissolved in anhydrous tetrahydrofuran 50ml afterwards and was added thereto at -70 °C. This reaction liquid was stirred for another 2 hours at room temperature. Water and diethyl ether were added to reaction liquid, and the organic layer was separated. The resulting organic layer was washed with brine and then dried over anhydrous sodium sulfate. After removal of drying agent by filtration, the organic layer was concentrated under a reduced pressure. The residue was purified by silica gel column chromatography (hexane-ethyl acetate) to give 2.69g of the title compound.
¹H-NMR(CDCl₃)
δ (ppm): 4.00(s,3H), 6.91(d,1H, J=5.6Hz), 8.38(d,1H, *J*=5.6Hz), 10.45 (s, 1H).

### (2) Synthesis of 2-chloro-4-methoxy-3-(2-methoxyvinyl)pyridine

Under nitrogen atmosphere, 4.07g of (methoxymethyl) triphenylphosphonium chloride was suspended in 50ml of tetrahydrofuran, 1.37g of potassium *tert*-butoxide was added in an ice-cooling, which was stirred for 10 minutes. 1.02g of 2-chloro-4-methoxypyridine-3-carbaldehyde was dissolved in 20ml of tetrahydrofuran, which was added to reaction liquid, and was stirred for another 40 minutes. Water and diethyl ether were added to reaction liquid, and the organic layer was separated. The resulting organic layer was washed with brine and then dried over anhydrous sodium sulfate. The reaction liquid was concentrated under a reduced pressure after removal of drying agent by filtration. The residue was purified by NH silica gel column chromatography (hexane-ethyl acetate) to give 1.18g of the title compound as a mixture of the geometric isomers.

### (3) Synthesis of 2,4-dimethoxy-3-(2-methoxyvinyl)pyridine

10ml of sodium methoxide (28% methanol solution) was added to 1.18g of a mixture of the geometric isomers of 2-chloro-4-methoxy-3-(2-methoxyvinyl)pyridine at room temperature, this reaction liquid was heated to reflux for 1 day. After standing to cool, water and ethyl acetate were added, and the organic layer was separated. The resulting organic layer was washed with brine and then dried over anhydrous sodium sulfate. After removing the drying agent by filtration, the organic layer was concentrated under a reduced pressure, the residue was purified by NH silica gel column chromatography (hexane-ethyl acetate) to give 1.02g of the title compound as a mixture of the geometric isomers.

### (4) Synthesis of 1-{1-[2-(2,4-dimethoxypyridin-3-yl)ethyl] piperidin-4-yl}-1H-indole-6-carboxamide

The title compound was obtained by synthesizing from 2,4-dimethoxy-3-(2-methoxyvinyl)pyridine in accordance with the methods in example 56.
¹H-NMR(CDCl3)
δ (ppm): 2.08-2.18(m,4H), 2.25-2.35(m,2H), 2.50-2.60(m,2H), 2.80-2.90(m,2H), 3.18-3.28(m,2H), 3.87(s,3H), 3.95(s,3H), 4.35-4.45(m,1H), 6.52(d,1H, *J*=6.0Hz), 6.57(d,1H, *J*=3.6Hz), 7.40-7.46(m,1H), 7.65(d,1H, *J*=8.0Hz), 7.98(d,1H, *J*=6.0Hz), 8.12(br, 1H).
mass spectrum: 409-(M+1).

### Example 121

### Synthesis of 1-{1-[2-(4-methoxy-1-methyl-2-oxo-1,2-dihydropyridin-3-yl)ethyl]piperidin-4-yl}-1H-indole-6-carboxamide

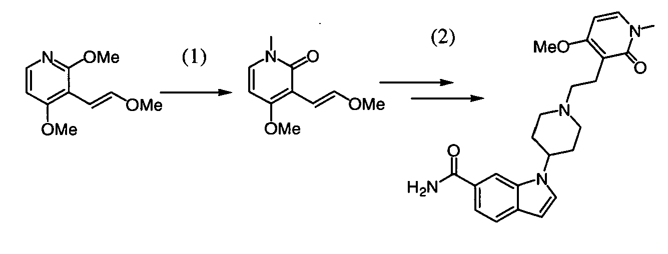

### (1) Synthesis of 4-methoxy-3-(2-methoxyvinyl)-1-methy-1H-pyridin-2-one

3ml of methyl iodide was added to 311mg of 2,4-dimethoxy-3-(2-methoxyvinyl)pyridine, which was heated at 130 °C with stirring overnight in the sealed tube. Methanol was added to this reaction liquid, concentrated under a reduced pressure to give 276mg of the title compound as a mixture of the geometric isomers.

### (2) Synthesis of 1-{1- [2-(-4-methoxy-1-methyl-2-oxo-1,2-dihydropyridin-3-yl)ethyl]piperidin-4-yl}-1H-indole-6-carboxamide

The title compound was obtained by synthesizing from 4-methoxy-3-(2-methoxyvinyl)-1-methyl-1H-pyridin-2-one in accordance with the methods in example 56.
¹H-NMR(CDCl3)
δ (ppm): 2.05-2.11(m,4H), 2.22-2.38(m,2H), 2.55-2.63(m,2H), 2.78-2.88(m,2H), 3.20-3.30(m,2H), 3.54(s,3H), 3.85(s,3H), 4.32-4.42(m,1H), 6.07(d,1H, *J*=7.6Hz), 6.56(d,1H, *J*=3.2Hz), 7.22(d,1H, J=7.6Hz), 7.40(d,1H, *J*=3.2Hz), 7.44(d,1H, *J*=8.0Hz), 7.64(d,1H, *J*=8.0Hz), 8.10(s, 1H).
mass spectrum: 409-(M+1).

### Example 122

### Synthesis of 1-{1-[2-(2-chloro-4-methoxypyridin-3-yl)ethyl] piperidin-4-yl}-1H-indole-6-carboxamide

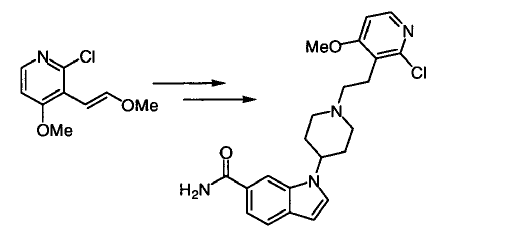

The title compound was obtained by synthesizing from 2-chloro-4-methoxy-3-(2-methoxyvinyl) pyridine in accordance with the methods in example 56.
¹H-NMR(d6-DMSO)
δ (ppm): 1.98-2.10(m,4H), 2.22-2.38(m,2H), 2.42-2.58(m,2H), 2.84-2.92(m,2H), 3.07-3.18(m,2H), 3.92(s,3H), 4.39-4.48(m,1H), 6.50(d,1H, *J*=3.2Hz), 7.11(d,1H, *J*=6.0Hz), 7.21(br,1H), 7.54-7.58(m,2H), 7.67(d,1H, J=3.2Hz), 7.90(br,lH), 8.18(d,1H, *J*=6.0Hz).
mass spectrum: 413-(M+1), 847-(2M+23).

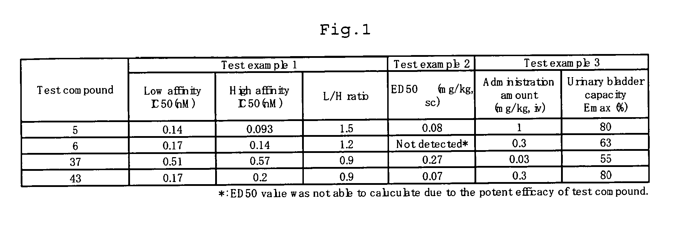

A test showing the usability of the compound with the present invention next is explained.
Test 1 Affinity test at serotonin 1A-(5-HT_{1A}) receptors
(1) Affinity test for the 5-HT_{1A} receptor of the subject material is prepared from inhibition experiment against binding of [³H]-4H-4-(2'-methoxy)phenyl-1-[2'-(N-2"-pyridinyl)-*p-*fluorobenzamido]ethyl-piperazine-(MPPF) which bind selectively to 5-HT_{1A} receptors to membrane fraction of porcine hippocampal. The 5-HT_{1A} receptor which is a G protein coupled receptor becomes G protein couples state by MgCl₂ addition, while becomes G protein non-couples state by guanylylimidophosphate-(Gpp(NH)P) addition. In general terms, because it is known the G protein receptor agonist which shows strong affinity for a receptor of the G protein coupled state depends on the intrinsic active strength, each affinity for the receptor G protein non-coupled state and G protein coupled stated is prepared, intrinsic strength of the subject material was presumed by comparing with them. If a value (L/H) that divided affinity (IC50 value) for the receptor of the low affinity state by affinity (IC50 value) for the high affinity state is equal to or less than 1as the inner activity is a zero, and this value grows big, intrinsic activity becomes strong. As a practical matter, as for the subject material of less than or equal to 1 did not show intrinsic activity, it was determined that the subject material of more than of equal to 2 had intrinsic activity.
   Porcine hippocampus was homogenized in ice-cooled 50mM Tris hydrochloric acid buffer solution (pH 7.4; hereinafter referred to as buffer solution A). Suspension liquid was centrifuged at 40,000xg for 15 minutes. Provided precipitate is suspended in buffer solution A, it was centrifuged at 40,000xg for 15 minutes. Further, similar operation was repeated two or three times. The precipitate which was finally provided was suspended it in buffer solution A of about 10 -fold quantity of the porcine hippocampus wet weight, and it was done with membrane fraction, it stored at -80 °C to time of use.
   The admixture (0.5mL) for incubation was contained, appropriate amount of membrane fraction, the subject material of desired concentration, MgCl₂ (final concentration: 10mM) or Gpp(NH)p (final concentration: 1mM) [³H] MPPF (final concentration: 0.5nM), dimethyl sulfoxide (final concentration:) 1% (v/v)) and 50mM Tris hydrochloric acid buffer solution (pH 7.4). Reaction was initiated by addition of the membrane fraction, it was incubated at 37 °C for 30 minutes. After incubation, the admixture was filtered by suction through a glass filter using Cell Harvester. After having washed in ice cooled buffer solution A, binding to receptor radioactivity was measured using a liquid scintillation counter. Non-specific binding was defined as a binding detected in the presence of 10 micro M WAY-100, 635. As for FIG. 1, the data of the affinity show an IC50 value prepared from the inhibition curve.
(2) From results shown in FIG. 1, the compounds according to the present invention showed superior receptor binding property.

Test 2 [Antagonistic action to the 5-HT_{1A} receptor agonist induced hypothermic effect in mice]
(1) Probed was inserted about 2cm in the intestinum rectum of CD-1 (ICR) male mice (25-45g), body temperature was measured. Temperature falls by subcutaneous administration of 5-HT_{1A} receptor agonist (8-hydroxy-dipropylamino tetrahydronaphthalene) at 0.5mg/kg. Because the 5-HT_{1A} antagonist inhibits this effect, antagonistic action for the 5-HT_{1A} receptor of the subject material was evaluated for an index in this, results are shown in table 3 described below. The subject material administered subcutaneously 15 minutes before the administration of 5-HT_{1A} agonist. Note that the 5-HT_{1A} receptor agonist decreased body temperature depends on a grade of the operation gender after a single administration. Also, the antagonistic action to 8-hydroxy dipropylamino tetrahydronaphthalene elicitation hypothermia was weak.
(2) Results: From results shown in FIG. 1, the compounds according to the present invention showed superior pharmacological action.

Test 3 [Inhibitory effect for the brain colliculus superior destruction micturition reflex enhancement effect rats]
(1) Sprague-Dawley female Rats (200-350g) were used for this examination. After midline incised rat abdomen under anaesthesia, opened the hole of the narrow path in the bladder cupular part, and catheter for the cystometry use was placed. Catheter for subject material administration is detained in a unilateral femoral vein, it was fixed in a rat occipital region through subcutis with urocystic catheter. One day after, micturition reflex of rat was measured by cystometgram. Rats was fixed to brain stereotaxis apparatus under anaesthesia, afterwards, after midline incised scalp, in accordance with coordinates of the brain map, a hole was able to be opened with dental drill in crania of the colliculus superior upper part. After the insert of microelectrode (a diameter: 0.7mm; length; 1. 5mm) of legion generator to the colliculus superior part through the hole, brain tissue was damaged by energization of current (65 °C, four minutes). After the operation was finished, at a point in time when rats awoke from anesthesia, cystometgram was performed again, enhancement state of the micturition reflex was confirmed. The subject material was administrated from the catheter of the femoral vein, effect to the micturition reflex of the subject material was evaluated. Also, the comparison of the effect of each subject material used the miximal response (Emax). The results are shown in FIG. 1.
(2) Rsults: From results shown in FIG. 1 the compounds according to the present invention showed superior pharmacological action.

The compounds according to the present invention has affinity for a 5-HT_{1A} receptor selectively and has the receptor antagonistic action in the central nervous system, and can show superior effect for the treatment and prevention to the lower urinary tract symptoms, especially to storage symptoms containing frequent urination, a feeling of urgency of urination, urinary incontinence.

Pharmaceutical formulation example containing the compounds according to the present invention will be explained below. A tablet containing a composition shown in the following can be produced by a conventional method.
Pharmaceutical formualtion example
Talet

| | |
|---|---|
| A compound of example 5 | 10mg |
| Lactose | 68.5mg |
| Crystalline cellulose | 20mg |
| Carboxymethylcellulose | 5mg |
| Silicic anhydride | 0.5mg |
| Magnesium stearate | 1mg |
| | 105mg |

### Industrial Applicability

1-(Piperidin-4-yl)-1*H*-indole derivatives represented by the formula (1) according to the present invention pharmacologically acceptable salts thereof, or hydrates thereof have superior antagonistic effect against 5-HT_{1A} receptor and are useful as an agent for treating or preventing the lower urinary tract symptoms.

### BRIEF DESCRIPTION OF DRAWINGS

[FIG. 1] Figure 1 shows the results of various tests using 1-(piperidin-4-yl)-1*H*-indole derivatives according to the present invention as representative compounds.

## Claims

1. A compound represented by the following formula (1) or a pharmacologically acceptable salt thereof, or a hydrate thereof, 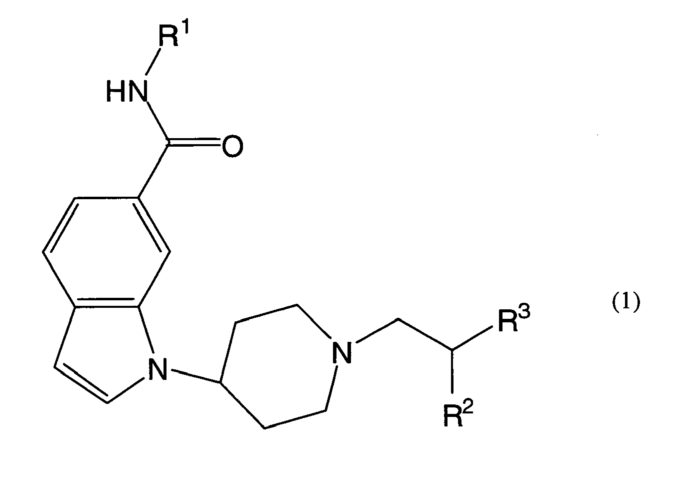 wherein R1 represents a hydrogen atom or a methyl group;
R2 represents a hydrogen atom, a halogen atom, a C₁₋₆ alkyl group, or a hydroxyl group; and
R3 represents a group selected from the group consisting of the formulas: 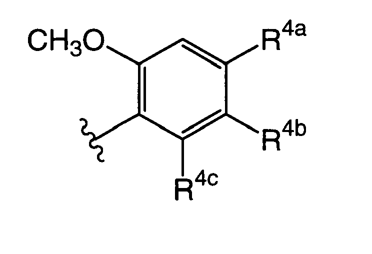 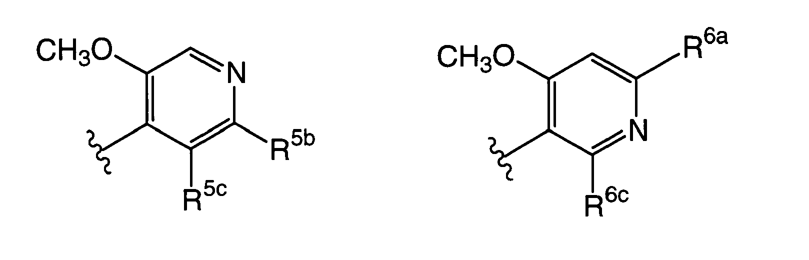 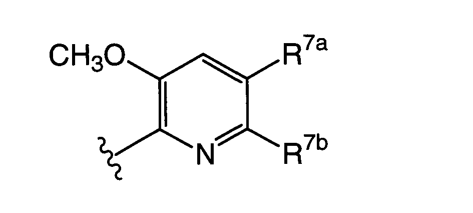 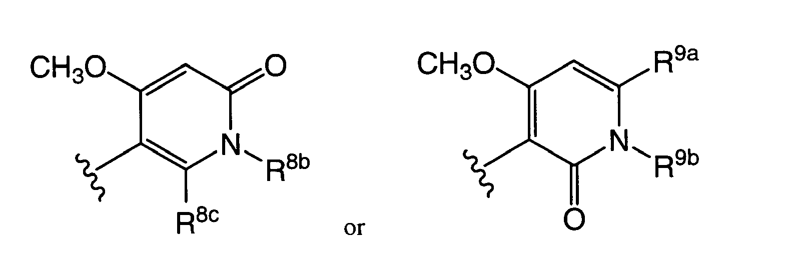
wherein R4a, R4b, R4c, R5b, R5c, R6a, R6c, R7a, R7b, R8c, and R9a are the same as or different from each other and each represents the group selected from following A1 group; R8b and R9b represent a phenyl group which may be substituted by one to three groups selected from a C₁₋₆ alkyl group or following B1 group; (provided that a compound in which all of R4a, R4b, and R4c are hydrogen atoms and a compound in which all of R6a, and R6c are hydrogen atoms are excluded.)
<A1 group>
(1) a hydrogen atom;
(2) a halogen atom;
(3) a hydroxyl group;
(4) a nitro group;
(5) a cyano group;
(6) a C₁₋₆ alkyl group (wherein said C₁₋₆ alkyl group may be substituted by the substituent selected from the group consisting of a hydroxyl group, a cyano group, a C₁₋₆ alkoxy group, a di (C₁₋₆ alkyl)amino group, and a di(C₁₋₆ alkyl)aminocarbonyl group),
(7) a trifluoromethyl group;
(8) a C₁₋₆ alkoxy group (wherein said C₁₋₆ alkoxy group may be substituted by the substituent selected from the group consisting of a hydroxyl group, a C₁₋₆ alkoxy group, a di (C₁₋₆ alkyl) amino group, and a piperidyl group);
(9) a phenyl group which may be substituted with one to three group selected from following B1 group;
(10) a benzyloxy group which may be substituted with one to three group selected from following B1 group;
(11) a group represented by the formula -NR²¹-R²² (wherein R²¹ represents a hydrogen atom or a C₁₋₆ alkyl group, R²² represents a hydrogen atom, a C₁₋₆ alkyl group, a C₂₋₇ alkylcarbonyl group, or a C₁₋₆ alkylsulfonyl group) ;
(12) formula -CO-R²³ (wherein R²³ represents a C₁₋₆ alkyl group or a di (C₁₋₆ alkyl) amino group.) ; and
(13) formula -X-R²⁴ (wherein R²⁴ represents a 5- to 6-membered heterocyclic group or a heteroaryl group which may be substituted with one to three group selected from C1 group, X represents a single bond, - (CH₂)ₘ- group (m represents 1 to 3), an oxygen atom, a carbonyl group, a -(CH₂)ₙCO- group (n represents 1 to 3), or a -N(CH₃)- group.).
<B1 group>
a C₁₋₆ alkyl group;
a C₁₋₆ alkoxy group; and
a halogen atom.
<C1 group>
a C₁₋₆ alkyl group;
a C₁₋₆ alkoxy group;
a C₂₋₇ alkyl carbonyl group;
a halogen atom,
a benzyloxycarbonyl group; and
a N-methylacetamide group.

2. The compound according to Claim 1 or a pharmacologically acceptable salt thereof, or a hydrate thereof, wherein R1 represents a hydrogen atom.

3. The compound according to Claim 1 or 2 or a pharmacologically acceptable salt thereof, or a hydrate thereof, wherein R2 represents a hydrogen atom.

4. The compound according to any one of Claims 1 to 3 or a pharmacologically acceptable salt thereof, or a hydrate thereof, wherein R3 is represented by the formula: 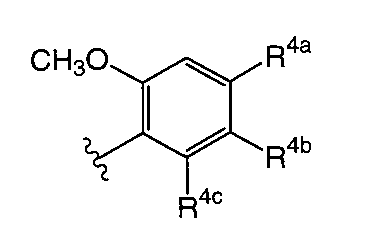 (wherein R4a, R4b, and R4c are the same meaning as defined in claim 1).

5. The compound according to any one of Claims 1 to 3 or a pharmacologically acceptable salt thereof or a hydrate thereof, wherein R3 is represented by the following formula: 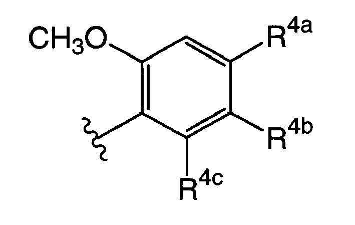 (wherein R4a, R4b, and R4c are the same as or different from each other, and each represents the group selected from the following A2 group.)
<A2 group>
(1) a hydrogen atom;
(2) a halogen atom;
(3) a hydroxyl group;
(4) a cyano group;
(5) a C₁₋₆ alkyl group (wherein said C₁₋₆ alkyl group may be substituted by the substituent selected from the group consisting of a hydroxyl group, a cyano group, a C₁₋₆ alkoxy group, a di (C₁₋₆ alkyl)amino group, and a di(C₁₋₆ alkyl)aminocarbonyl group);
(6) a trifluoromethyl group;
(7) a C₁₋₆ alkoxy group (wherein said C₁₋₆ alkoxy group may be substituted by the substituent selected from the group consisting of a hydroxyl group, a C₁₋₆ alkoxy group, a di (C₁₋₆ alkyl) amino group, and a piperidyl group);
(8) a phenyl group which may be substituted by one to three group selected from the following B1 group;
(9) a group represented by the formula -NR²¹- R²² (wherein R²¹ represents a hydrogen atom or a C₁₋₆ alkyl group; R²² represents a hydrogen atom, a C₁₋₆ alkyl group, C₂₋₇ alkylcarbonyl group, or C₁₋₆ alkylsulfonyl group);
(10) formula -CO-R²³ (wherein R²³ represents a C₁₋₆ alkyl group or a di (C₁₋₆ alkyl) amino group.); and
(11) formula -X-R²⁴ (wherein R²⁴ represents a 5- to 6-membered heterocyclic group or a heteroaryl group which may be substituted by one to three group selected from a C1 group; X represents a single bond, a - (CH₂)ₘ-group (m represents 1 to 3), an oxygen atom, a carbonyl group, a -(CH₂)ₙCO- group (n represents 1 to 3) or a -N (CH₃) -group.)
<B1 group>
a C₁₋₆ alkyl group;
a C₁₋₆ alkoxy group; and
a halogen atom.
<C1 group>
a C₁₋₆ alkyl group;
a C₁₋₆ alkoxy group;
a C₂₋₇ alkylcarbonyl group;
a halogen atom;
a benzyloxycarbonyl group; and
a N-methylacetamide group.

6. The compound according to any one of Claims 1 to 3 or a pharmacologically acceptable salt thereof or a hydrate thereof, wherein R3 is represented by the formula: 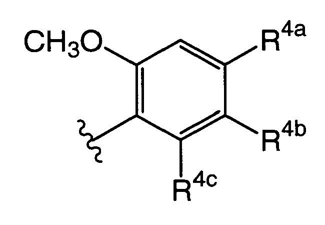 (wherein R4a, R4b, and R4c are the same as or different from each other and each represent the group selected from the following A3 group.)
<A3 group>
(1) a hydrogen atom;
(2) a halogen atom;
(3) a cyano group;
(4) a C₁₋₆ alkyl group (wherein said C₁₋₆ alkyl group may be substituted by the substituent selected from the group consisting of a hydroxyl group, a cyano group, and C₁₋₆ alkoxy group.);
(5) a trifluoromethyl group;
(6) a C₁₋₆ alkoxy group (wherein said C₁₋₆ alkoxy group may be substituted by the substituent selected from the group consisting of a hydroxyl group and a C₁₋₆ alkoxy group.) ;
(7) a group represented by the formula -NR²¹-R²² (wherein R²¹ represents a hydrogen atom or a C₁₋₆ alkyl group; R²² represents a hydrogen atom, a C₁₋₆ alkyl group, a C₂₋₇ alkylcarbonyl group, or a C₁₋₆ alkylsulfonyl group.);
(8) formula -CO-R²³ (wherein R²³ represents a C₁₋₆ alkyl group.); and
(9) formula -X-R²⁴ (Wherein R²⁴ represents a 5- to 6-membered heterocyclic group or a heteroaryl group which may be substituted by one to three group selected from the following C1 group; X represents a single bond and a -(CH₂)ₘ-group (m represents 1 to 3), an oxygen atom, a carbonyl group, a -(CH₂)ₙCO- group (n represents 1 to 3), or a -N(CH₃)-group.)
<C1 group>
a C₁₋₆ alkyl group;
a C₁₋₆ alkoxy group;
a C₂₋₇ alkylcarbonyl group;
a halogen atom;
a benzyloxycarbonyl group; and
a N-methylacetamide group.

7. The compound according to any one of Claims 1 to 3 or a pharmacologically acceptable salt thereof, or a hydrate thereof, wherein R3 is represented by the formula: 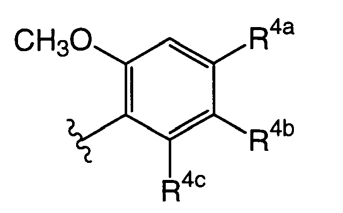 (wherein R4a, R4b, and R4c are the same as or different from each other and each represents the group selected by following A4 group.)
<A4 group>
(1) a hydrogen atom;
(2) an unsubstituted C₁₋₆ alkoxy group;
(3) a C₁₋₆ alkoxy-C₁₋₆ alkyl group; and
(4) a morpholino group.

8. The compound according to any one of Claims 1 to 3 or a pharmacologically acceptable salt thereof or a hydrate thereof, wherein R3 is represented by the formula: 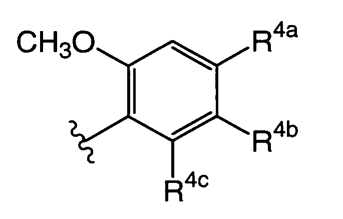 (wherein R4a, R4b, and R4c are the same as or different from each other and each represents the group selected by following A5 group.)
<A5 group>
(1) a hydrogen atom;
(2) a methoxy group;
(3) a methoxymethyl group; and
(4) a morpholino group.

9. The compound according to any one of Claims 1 to 6 or a pharmacologically acceptable salt thereof, or a hydrate thereof, wherein R24 represents a group selected from the following D1 group (wherein said group may be substituted by the one to three group selected from C1 group.)
<C1 group>
a C₁₋₆ alkyl group;
a C₁₋₆ alkoxy group;
a C₂₋₇ alkyl carbonyl group;
a halogen atom;
a benzyloxycarbonyl group; and
N-methylacetamide group.
<D1 group>
a pyrrolidinyl group;
a piperidyl group;
a pyridyl group;
a piperazinyl group;
an oxazolyl group;
an isoxazolyl group;
an oxo pyrrolidinyl group;
an oxo piperazinyl group;
a pyridonyl group; and
a morpholinyl group.

10. The compound according to any one of Claims 1 to 6 or a pharmacologically acceptable salt thereof, or a hydrate thereof, wherein R24 represents a group selected from the following D2 group (wherein said group may be substituted by the one to three group selected from C2 group.)
<C2 group>
a methyl group;
a acetyl group; and
a N-methylacetamide group.
<D2 group>
a pyrrolidinyl group;
a piperidyl group;
an oxo pyrrolidinyl group; and
a morpholinyl group.

11. A compound selected from the group consisting of the following compound groups or a pharmacologically acceptable salt thereof, or a hydrate thereof:
1-[1-[2-(2-methoxy-4-methoxymethylphenyl)ethyl]piperidin-4-yl ]-1H-indole-6-carboxamide;
1-[1-[2-(2,4,6-trimethoxyphenyl)ethyl]piperidin-4-yl]-1H-indo le-6-carboxamide;
1-[1-[2-(2,3,6-trimethoxyphenyl)ethyl]piperidine-4-yl]-1*H*-ind ole-6-carboxamide;
1-[1-[2-(4,6-dimethoxypyridin-3-yl)ethyl]piperidine-4-yl]-1*H-*indole-6-carboxamide;
1-[1-[2-(2,5-dimethoxyphenyl)ethyl]piperidin-4-yl]-1*H*-indole-6-carboxamide;
1-[1-[2-(2,6-dimethoxyphenyl)ethyl]piperidin-4-yl]-1*H*-indole-6-carboxamide; and
1-[1-[2-(2-methoxy-6-morpholinophenyl)ethyl]piperidin-4-yl]-1 H-indole-6-carboxamide.

12. A pharmaceutical composition comprising the compound according to any one of Claims 1 to 11 or a pharmacologically acceptable salt thereof, or a hydrate thereof, as an active ingredient.

13. A treating or preventing agent for a lower urinary tract symptom comprising the compound according to any one of Claims 1 to 11 or a pharmacologically acceptable salt thereof, or a hydrate thereof, as an active ingredient.

14. A treating or preventing agent for a micturition symptom comprising the compound according to any one of Claims 1 to 11 or a pharmacologically acceptable salt thereof, or a hydrate thereof, as an active ingredient.

15. A treating or preventing agent for a frequent urination or urinary incontinence comprising the compound according to any one of Claims 1 to 11 or a pharmacologically acceptable salt thereof, or a hydrate thereof, as an active ingredient.
